# EUROPEAN PATENT SPECIFICATION

(11) **EP 3 452 021 B1**
(45) Date of publication and mention of the grant of the patent: **29.12.2021**
(21) Application number: 17793073.2
(22) Date of filing: 01.05.2017
(51) Int. Cl.: A61K 9/22, A61K 38/00, A61K 47/42

(54) **ELP FUSION PROTEINS FOR CONTROLLED AND SUSTAINED RELEASE**
ELP-FUSIONSPROTEINE ZUR GESTEUERTEN UND VERZÖGERTEN FREISETZUNG
PROTÉINES DE FUSION ELP POUR LIBÉRATION CONTRÔLÉE ET PROLONGÉE

(30) Priority: 06.05.2016 US 201662332803 P; 04.01.2017 US 201762442057 P
(43) Date of publication of application: 13.03.2019
(73) Proprietor: Phasebio Pharmaceuticals, Inc., Malvern, Pennsylvania 19355 (US)
(72) Inventor: JOWETT, James, Malvern PA 19355 (US); BALLANCE, David, James, Malvern PA 19355 (US)
(74) Representative: Cooley (UK) LLP
(86) International application number: PCT/US2017/030403
(87) International publication number: WO 2017/192449

(56) References cited:
- WO-A1-2005/023863
- WO-A1-2009/099763
- WO-A2-00/53755
- WO-A2-00/66142
- WO-A2-98/24813
- WO-A2-2004/081198
- WO-A2-2009/067639
- WO-A2-2012/006598
- CA-A1- 2 953 975
- CN-A- 102 703 459
- US-A- 5 428 014
- US-A1- 2004 220 111
- US-A1- 2006 019 339
- US-A1- 2010 316 623
- US-A1- 2011 020 342
- US-A1- 2011 039 776
- US-A1- 2011 245 173
- US-A1- 2011 245 592
- US-A1- 2012 016 033
- US-A1- 2014 187 746
- US-A1- 2015 005 233
- US-A1- 2015 290 328
- WALKER ET AL.: 'Cell Penetrating Peptides Fused to a Thermally Targeted Biopolymer Drug Carrier Improve the Delivery and Antitumor Efficacy of an Acid-sensitive Doxorubicin Derivative' INT J PHARM vol. 436, no. 1-2, 15 October 2012, pages 825 - 832, XP055438207

## Description

### FIELD OF INVENTION

The present disclosure relates to pharmaceutical formulations for sustained release, and their use in methods for delivering a treatment regimen.

### BACKGROUND

The effectiveness of peptide and small molecule drugs is often limited by the half-life of such drugs in the circulation, as well as difficulties in obtaining substantially constant plasma levels. For example, the incretin GLP-1 must be administered at relatively high doses to counter its short half-life in the circulation, and these high doses are associated with nausea, among other things (Murphy and Bloom, Nonpeptidic glucagon-like peptide 1 receptor agonists: A magic bullet for diabetes? PNAS 104 (3):689-690 (2007)). Further, the peptide agent vasoactive intestinal peptide (VIP) exhibits a half-life, in some estimates, of less than one minute, making this agent impractical for pharmaceutical use (Domschke et al., Vasoactive intestinal peptide in man: pharmacokinetics, metabolic and circulatory effects, Gut 19:1049-1053 (1978); Henning and Sawmiller, Vasoactive intestinal peptide: cardiovascular effects, Cardiovascular Research 49:27-37 (2001)). A short plasma half-life for peptide drugs is often due to fast renal clearance as well as to enzymatic degradation during systemic circulation.

### SUMMARY OF THE INVENTION

The invention is defined by the appended claim set.

The present disclosure, which comprises the invention, provides pharmaceutical formulations for sustained release, and their use for delivering a treatment regimen. The disclosure thereby provides improved pharmacokinetics for peptide and small molecule drugs.

In some aspects, the disclosure provides a sustained release pharmaceutical formulation. The formulation includes a therapeutic agent for systemic administration, where the therapeutic agent includes an active agent and an amino acid sequence capable of forming a reversible matrix at the body temperature of a subject. The reversible matrix is formed from hydrogen bonds (*e.g.,* intra- and/or intermolecular hydrogen bonds) as well as from hydrophobic contributions. The formulation further includes one or more pharmaceutically acceptable excipients and/or diluents. The matrix provides for a slow absorption to the circulation from an injection site. The sustained release, or slow absorption from the injection site, is due to a slow reversal of the matrix as the concentration dissipates at the injection site. Once product moves into the circulation, the formulation confers long half-life and improved stability. Thus, a unique combination of slow absorption and long half-life is achieved leading to a desirable PK profile with a low peak to trough (Cmax to Cmin) and delayed or late Tmax.

The amino acid sequence capable of forming a reversible matrix at the body temperature of a subject is an Elastin-Like-Peptide (ELP) sequence. The ELP sequence includes or consists of structural peptide units or sequences that are related to, or mimics of, repeat sequence present in the elastin protein. In accordance with the invention, the ELP sequence comprises or consists of at least 60 ELP structural units of SEQ ID NO.3. The ELP amino acid sequence may exhibit a visible and reversible inverse phase transition in the selected formulation. That is, the amino acid sequence may be structurally disordered and highly soluble in the formulation below a transition temperature (Tt), but exhibit a sharp (2-3°C range) disorder-to-order phase transition when the temperature of the formulation is raised above the Tt. In some instances, the present disclosure provides therapeutic agents having transition temperatures between about 26°C and about 37°C. In addition to temperature, length of the ELP polymer, amino acid composition of the ELP, ionic strength, pH, pressure, selected solvents, presence of organic solutes, and protein concentration may also affect the transition properties, and these may be tailored for the desired absorption profile. The protein concentration and salt concentration affect the transition properties (e.g. transition temperature).

The active agent for systemic administration is a protein or peptide, which may have a short circulatory half-life, such as from about 30 seconds to about 1 hour, to about 2 hours, or to about 5 hours, or from 30 seconds to about 10 hours. The therapeutic agent may be a recombinant fusion protein between the protein active agent and the amino acid sequence capable of forming the matrix. In accordance with the invention, the peptide active agent is a GLP-2 receptor agonist comprising the amino acid sequence of SEQ ID NO: 70. By providing a slow absorption from the injection site, renal clearance and degradation can be controlled, thereby achieving the desired PK profile.

In other aspects, the disclosure provides the formulation of the invention for use as a medicament. The formulation may be administered from about 1 to about 8 times per month. The formulation may be administered about weekly, and may be administered subcutaneously or intramuscularly (for example). Optionally, the site of administration is not a pathological site, that is, the therapeutic agent is not administered directly to the intended site of action.

### DESCRIPTION OF THE DRAWINGS

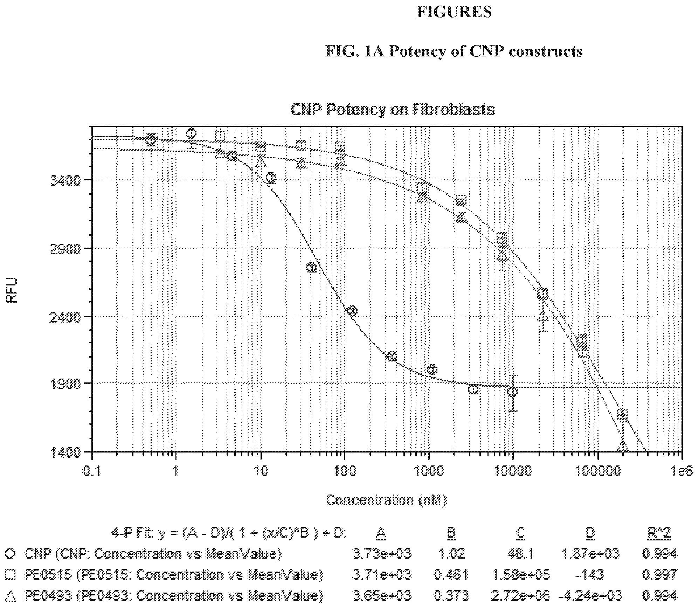

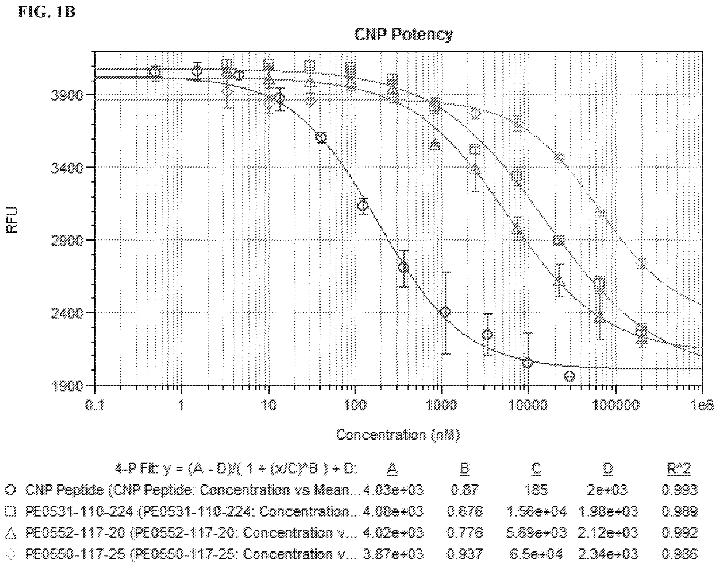

**Figure 1 A-B** shows the potency of CNP constructs.

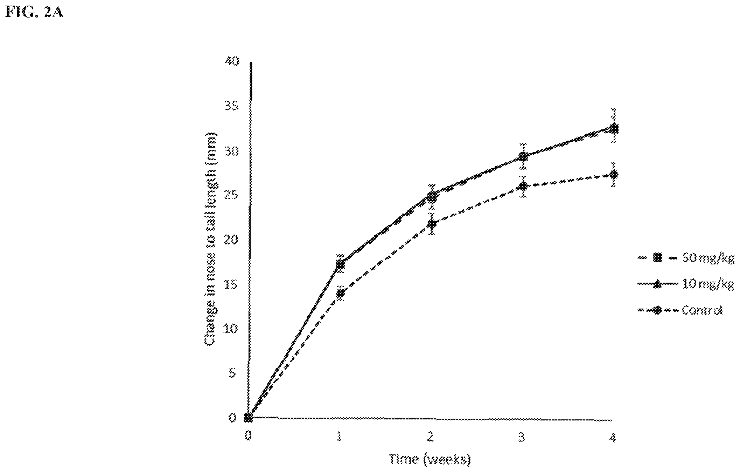

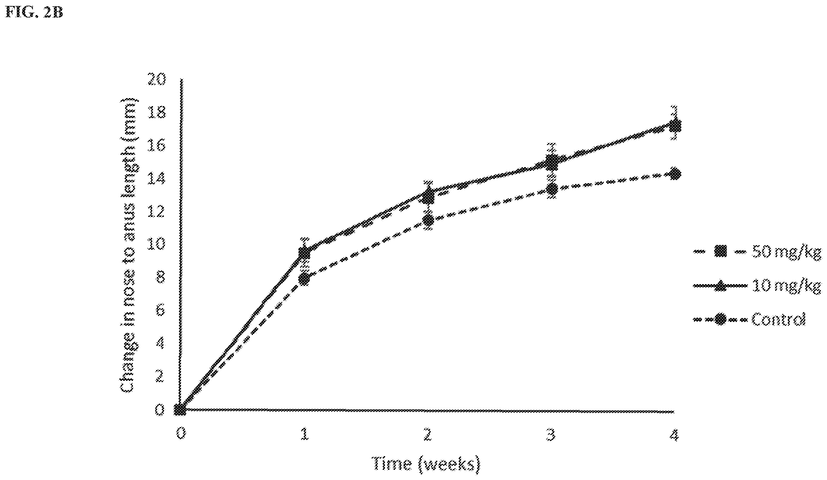

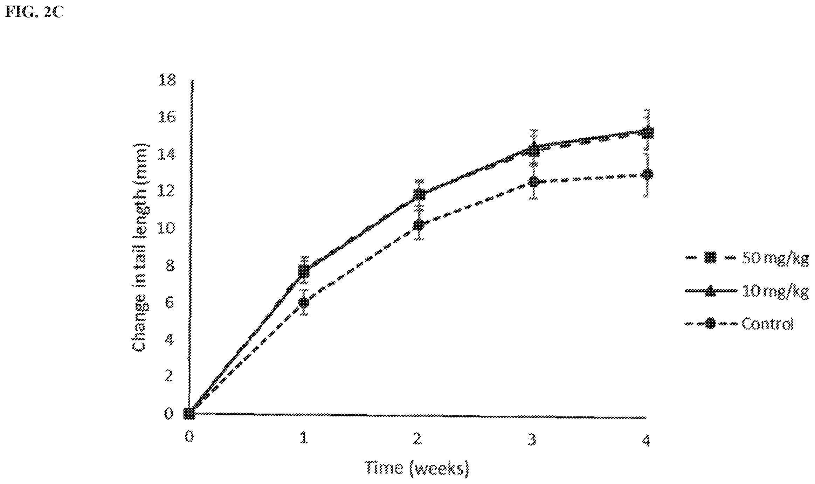

**Figure 2A-C** shows the effect on growth of subcutaneous injection of five week old male FVB/nJ mice (n = 12/group) with PE0552 or saline (control) three times per week. Effect on growth was determined by measuring nose to tail length (A), nose to anus (B) and length of tail (C). The graphs show mean measurements and standard error for each group.

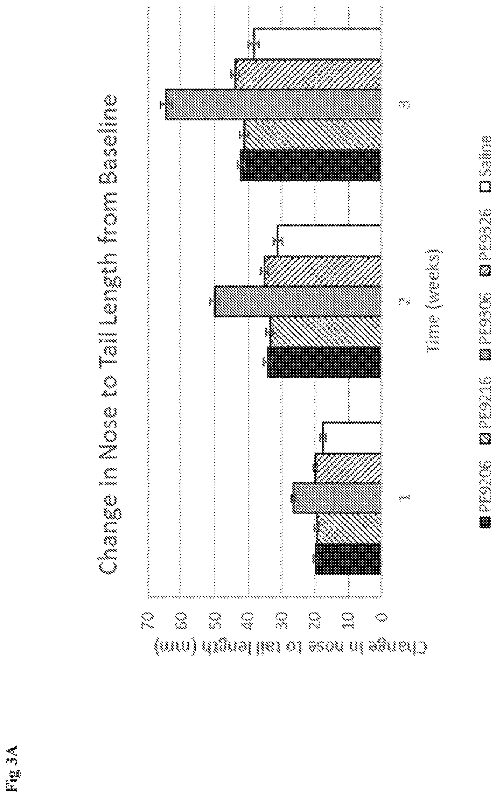

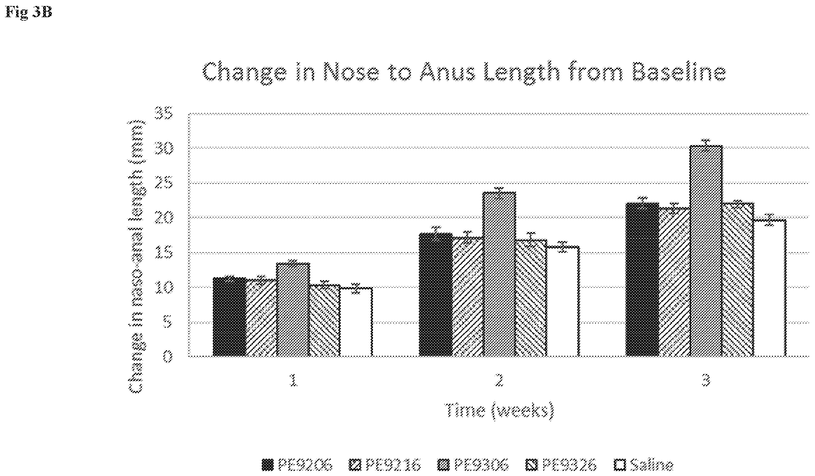

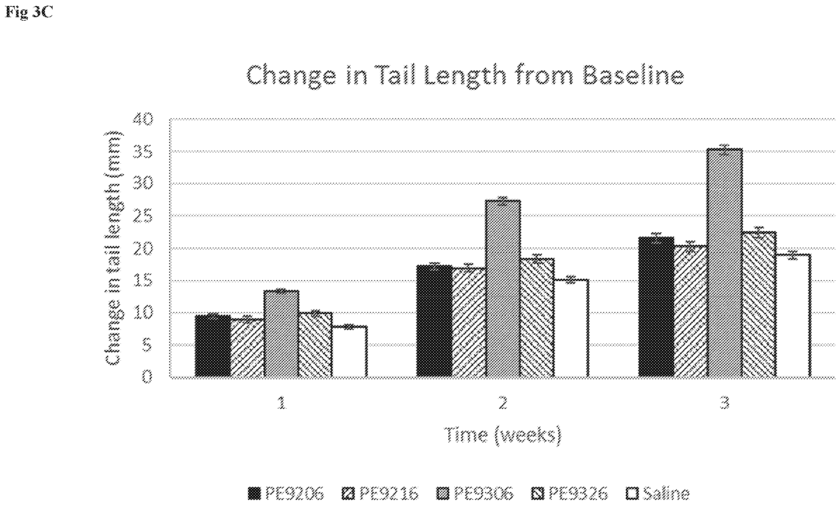

**Figure 3A-C** shows the effect on growth of subcutaneous injection of three week old male FVB/nJ mice (n = 11/group) with PE9206, PE9216, PE9306, PE9326 or saline (control) daily for 3 weeks. Effect on growth was determined by measuring nose to tail length (A), nose to anus length (B), and tail length (C). The graphs show the mean change in growth versus baseline over the 3 week period.

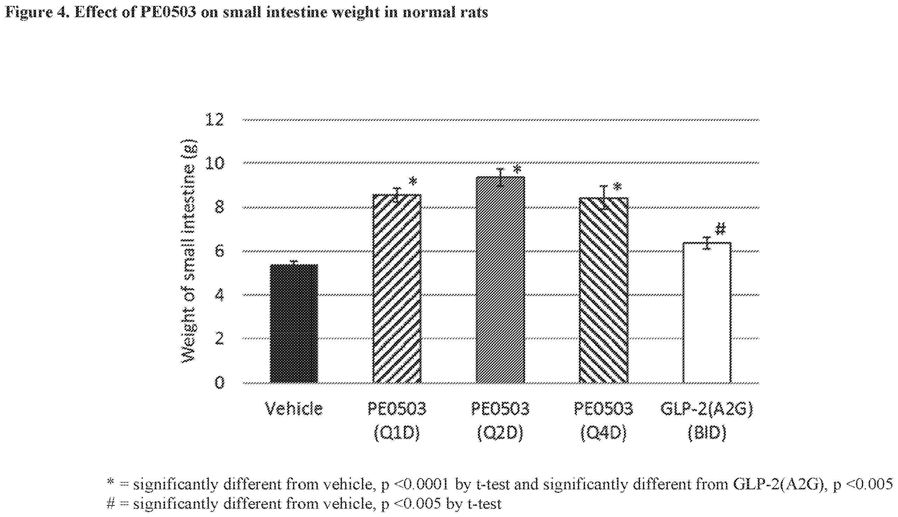

**Figure 4** shows the effect on small intestine weight of subcutaneous injection of PE0503 or GLP-2 (A2G) over an eleven-day dosing period in male Sprague Dawley rats (200 - 220 g, n = 12/group). Dosing was as follows: Vehicle group dosed with saline daily; PE0503 Q1D dosed with PE0503 at 1.3 mg/kg once per day; PE0503 Q2D dosed with PE0503 at 5.1 mg/kg every other day; PE0503 Q4D dosed with PE0503 at 20.5 mg/kg once every four days; GLP-2(A2G) TID dosed twice per day with 0.1 mg/kg GLP2(A2G).

### DETAILED DESCRIPTION

The invention is defined by the appended claim set. The present disclosure comprising the invention provides pharmaceutical formulations for sustained release, and methods for delivering a treatment regimen with the sustained release formulations. The pharmaceutical compositions disclosed herein have enhanced efficacy, bioavailability, circulatory half-life, persistence, degradation resistance, *etc.* The disclosure thereby provides improved pharmacokinetics for active agents, such as peptides and small molecule drugs, including a relatively flat PK profile with a low ratio of peak to trough, and/or a long Tmax. The PK profile can be maintained with a relatively infrequent administration schedule, such as from one to eight injections per month in some instances.

In some aspects, the disclosure provides sustained release pharmaceutical formulations. The formulation includes therapeutic agents for systemic administration, where the therapeutic agent includes an active agent and an amino acid sequence capable of forming a matrix or coacervate at the body temperature of a subject. The reversible matrix is formed from hydrogen bonds (*e.g.,* intra- and/or intermolecular hydrogen bonds) as well as from hydrophobic contributions. The formulation further includes one or more pharmaceutically acceptable excipients and/or diluents. The matrix provides for a slow absorption to the circulation from an injection site. Without being bound by theory, this slow absorption is due to the slow reversal of the matrix or coacervate at the periphery of the injection site depot. The slow absorption profile provides for a flat PK profile, as well as convenient and comfortable administration regimen. For example, the plasma concentration of the active agent over the course of days (*e.g.,* from 2 to about 60 days, or from about 4 to about 30 days) does not change by more than a factor of 20, or by more than a factor of about 10, or by more than a factor of about 5, or by more than a factor of about 3. Generally, this flat PK profile is seen over a plurality of (substantially evenly spaced) administrations, such as at least about 2, at least about 5, or at least about 10 administrations of the formulation. In some instances, the slow absorption is manifest by a Tmax (time to maximum plasma concentration) of greater than about 5 hours, greater than about 10 hours, greater than about 20 hours, greater than about 30 hours, or greater than about 50 hours.

### AMINO ACID SEQUENCES FORMING A REVERSIBLE MATRIX

The sustained release, or slow absorption from the injection site, is controlled by the amino acid sequence capable of forming a hydrogen-bonded matrix or coacervate at the body temperature of the subject.

The amino acid sequence contains structural units that form hydrogen-bonds through protein backbone groups and/or side chain groups, and which may contribute hydrophobic interactions to matrix formation. In some instances, the amino acid side chains do not contain hydrogen bond donor groups, with hydrogen bonds being formed substantially through the protein backbone. Exemplary amino acids include proline, alanine, valine, glycine, and isoleucine, and similar amino acids. In some instances, the structural units are substantially repeating structural units, so as to create a substantially repeating structural motif, and substantially repeating hydrogen-bonding capability. In these and other instances, the amino acid sequence contains at least about 10%, at least about 20%, at least about 40%, or at least about 50% proline, which may be positioned in a substantially repeating pattern. In this context, a substantially repeating pattern means that at least about 50% or at least about 75% of the proline residues of the amino acid sequence are part of a definable structural unit. In still other instances, the amino acid sequence contains amino acids with hydrogen-bond donor side chains, such as serine, threonine, and/or tyrosine. In some instances, the repeating sequence may contain from one to about four proline residues, with remaining residues independently selected from non-polar residues, such as glycine, alanine, leucine, isoleucine, and valine. Non-polar or hydrophobic residues may contribute hydrophobic interactions to the formation of the matrix.

In other instances, the amino acid sequence capable of forming the matrix at body temperature may include a random coil or non-globular extended structure. For example, the amino acid sequence capable of forming the matrix at body temperature may comprise an amino acid sequence disclosed in U.S. Patent Publication No. 2008/0286808, WIPO Patent Publication No. 2008/155134, and U.S. Patent Publication No. 2011/0123487.

In some instances the amino acid sequence includes an unstructured recombinant polymer of at least 40 amino acids. The unstructured polymer may include more than about 100, about 150, about 200 or more contiguous amino acids. In some instances, the amino acid sequence forms a random coil domain. In particular, a polypeptide or amino acid polymer having or forming "random coil conformation" substantially lacks a defined secondary and tertiary structure. In some instances, the unstructured polymer is defined as a polymer having at least 40 amino acids where the total number of glycine (G), aspartate (D), alanine (A), serine (S), threonine (T), glutamate (E) and proline (P) residues constitutes more than about 80% of the total amino acids in the polymer. In some instances, at least 50% of the amino acids are devoid of secondary structure as determined by the Chou-Fasman algorithm.

The amino acid sequences may form a "gel-like" state upon injection at a temperature higher than the storage temperature. Exemplary sequences have repeating peptide units, and/or may be relatively unstructured at the lower temperature, and achieve a hydrogen-bonded, structured, state at the higher temperature.

### Elastin-Like Peptides (ELPs)

In some instances, the amino acid sequence capable of forming a matrix at body temperature is a peptide having repeating units of from four to ten amino acids. The repeating unit may form one, two, or three hydrogen bonds in the formation of the matrix. In certain instances, the amino acid sequence capable of forming a matrix at body temperature is an amino acid sequence of silk, elastin, collagen, keratin, or mimic thereof, or an amino acid sequence disclosed in U.S. Patent 6,355,776.

In certain instances, the amino acid sequence is an Elastin-Like-Peptide (ELP) sequence. The ELP sequence includes or consists of structural peptide units or sequences that are related to, or mimics of, the elastin protein. The ELP sequence is constructed from structural units of from three to about twenty amino acids, or in some instances, from about four to about ten amino acids, such as about four, about five or about six amino acids. The length of the individual structural units may vary or may be uniform. This disclosure provides exemplary structural units defined by SEQ ID NOS: 1-13 (below), which may be employed as repeating structural units, including tandem-repeating units, or may be employed in some combination. In accordance with the invention, the ELP comprises structural units of SEQ ID NO:3. Thus, the ELP of the invention may comprise or consist essentially of structural units selected from SEQ ID NO: 3, as defined below.

In some instances, including instances in which the structural units are ELP units, the amino acid sequence includes or consists essentially of from about 1 to about 500 structural units, or about 9 to about 200 structural units, or about 10 to 200 structural units, or about 50 to about 200 structural units, or from about 80 to about 200 structural units, or from about 80 to about 150 structural units. In accordance with the invention, the amino acid sequence includes or consists essentially of 60 structural units, wherein the structural units are ELP units defined by SEQ ID NO: 3. The disclosure also provides instances wherein the ELP includes a combination of units defined by SEQ ID NOS: 1-13. In exemplary instances, the amino acid sequence of the ELP structural unit includes or consists essentially of about 80 structural units, of about 90 structural units, of about 100 structural units, of about 120 structural units, of about 140 structural units, about 144 structural units, of about 160 structural units, of about 180 structural units, of about 200 structural units, or of about 500 structural units. In exemplary instances, the structural units collectively have a length of about 300 amino acid residues, of about 400 amino acid residues, of about 500 amino acid residues, of about 600 amino acid residues, of about 700 amino acid residues, of about 720 amino acid residues, of about 800 amino acid residues, or of about 1000 amino acid residues.

The amino acid sequence may exhibit a visible and reversible inverse phase transition with the selected formulation. That is, the amino acid sequence may be structurally disordered and highly soluble in the formulation below a transition temperature (Tt), but exhibit a sharp (2-3°C range) disorder-to-order phase transition, or coacervation, when the temperature of the formulation is raised above the Tt. In addition to temperature, length of the amino acid polymer, amino acid composition, ionic strength, pH, pressure, selected solvents, presence of organic solutes, and protein concentration may also affect the transition properties, and these may be tailored in the formulation for the desired absorption profile. Absorption profile can be easily tested by determining plasma concentration or activity of the active agent over time.

In accordance with the invention the ELP component may be formed of the structural unit:
the pentapeptide Val-Pro-Gly-X-Gly (SEQ ID NO: 3), or VPGXG, where X is any natural or non-natural amino acid residue except proline, and where X optionally varies among polymeric or oligomeric repeats.

The disclosure also provides further ELP structural units including:
(a) the tetrapeptide Val-Pro-Gly-Gly, or VPGG (SEQ ID NO: 1);
(b) the tetrapeptide Ile-Pro-Gly-Gly, or IPGG (SEQ ID NO: 2);
(c) the pentapeptide Ala-Val-Gly-Val-Pro, or AVGVP (SEQ ID NO: 4);
(d) the pentapeptide Ile-Pro-Gly-X-Gly, or IPGXG (SEQ ID NO: 5), where X is any natural or non-natural amino acid residue, and where X optionally varies among polymeric or oligomeric repeats;
(e) the pentapeptide Ile-Pro-Gly-Val-Gly, or IPGVG (SEQ ID NO: 6);
(f) the pentapeptide Leu-Pro-Gly-X-Gly, or LPGXG (SEQ ID NO: 7), where X is
   any natural or non-natural amino acid residue, and where X optionally varies among polymeric or oligomeric repeats;
(g) the pentapeptide Leu-Pro-Gly-Val-Gly, or LPGVG (SEQ ID NO: 8);
(h) the hexapeptide Val-Ala-Pro-Gly-Val-Gly, or VAPGVG (SEQ ID NO: 9);
(i) the octapeptide Gly-Val-Gly-Val-Pro-Gly-Val-Gly, or GVGVPGVG (SEQ ID NO: 10);
(j) the nonapeptide Val-Pro-Gly-Phe-Gly-Val-Gly-Ala-Gly, or VPGFGVGAG (SEQ ID NO: 11);
(k) the nonapeptides Val-Pro-Gly-Val-Gly-Val-Pro-Gly-Gly, or VPGVGVPGG (SEQ ID NO: 12); and
(l) the pentapeptide Xaa-Pro-Gly-Val-Gly, or XPGVG (SEQ ID NO:13) where X is any natural or non-natural amino acid residue, and where X optionally varies among polymeric or oligomeric repeats.

In instances of the disclosure, such structural units defined by SEQ ID NOS: 1-13 may form structural repeating units, or may be used in combination to form an ELP. In some instances, the ELP component is formed entirely (or almost entirely) of one or a combination of (*e.g.,* 2, 3, 4, 5, 6, 7, 8, 9, or 10) structural units selected from SEQ ID NOS: 1-13. In other instances, at least about 75%, or at least about 80%, or at least about 90% of the ELP component is formed from one or a combination of structural units selected from SEQ ID NOS: 1-13, and which may be present as repeating units.

In certain instances, the ELP contains repeat units, including tandem repeating units, of Val-Pro-Gly-X-Gly (SEQ ID NO: 3), where X is as defined above, and where the percentage of Val-Pro-Gly-X-Gly (SEQ ID NO: 3) units taken with respect to the entire ELP component (which may comprise structural units other than VPGXG (SEQ ID NO: 3)) is greater than about 50%, or greater than about 75%, or greater than about 85%, or greater than about 95% of the ELP. The ELP may contain motifs of 5 to 15 structural units (*e.g.* about 9 or about 10 structural units) of SEQ ID NO: 3, with the guest residue X varying among at least 2 or at least 3 of the units in the motif. The guest residues may be independently selected, such as from non-polar or hydrophobic residues, such as the amino acids V, I, L, A, G, and W (and may be selected so as to retain a desired inverse phase transition property). In certain instances, the guest residues are selected from V, G, and A.

In certain instances, the ELP contains repeating units, including tandem repeating units of any of SEQ ID NOs: 1-13 either alone or in combination. In some instances, the ELP contains repeats of two or more of any of SEQ ID NOs: 1-13 in combination. In certain instances, the ELP contains repeats of SEQ ID NO: 3 and SEQ ID NO: 13. In some instances, the ELP contains repeats of SEQ ID NO: 3 and SEQ ID NO: 13, wherein the guest residues are independently selected, such as from non-polar or hydrophobic residues, such as the amino acids V, I, L, A, G, and W (and may be selected so as to retain a desired inverse phase transition property). In certain instances, the guest residues are selected from V, G, and A.

In some instances, the ELP includes 9-mers including nine copies of one or more ELP structural units disclosed herein. In some instances, the ELP includes 9-mers including nine copies of a pentapeptide disclosed herein. In some instances, the ELP includes 9-mers including SEQ ID NOs: 3 and 13 in any combination. In some instances, the ELP includes a sequence alternating between SEQ ID NOs: 3 and 13. ELPs of varying numbers of 9-mers can be combined to produce ELPs with, for instance, 18, 27, 36, 45, 54, 63, 72, 81, 90, 99, 108, 117, 126, 135, 144, 153, 162, 171, or 180 copies of the 9-mer.

In certain instances, the ELP includes 9-mers including SEQ ID NO: 3, wherein the guest residue is selected from V, G, and A. In certain instances, the ELP includes 9-mers including SEQ ID NO: 3, wherein V, G, and A are in the ratio of 7:2:0 (alpha). In certain instances, the ELP includes 9-mers including SEQ ID NO:3, wherein V, G, and A are in the ratio of 7:0:2 (beta v1). In certain instances, the ELP includes 9-mers including SEQ ID NO:3, wherein V, G, and A are in the ratio of 6:0:3 (beta v2). In certain instances, the ELP includes 9-mers including SEQ ID NO:3, wherein V, G, and A are in the ratio of 5:2:2 (gamma). In certain instances, the ELP includes 9-mers including SEQ ID NO: 13, wherein the guest residue is selected from V, G, and A. In certain instances, the ELP includes 9-mers including SEQ ID NO:13, wherein V, G, and A are in the ratio of 5:0:4 (delta). Exemplary 9-mers are disclosed in **Table 1. Table 2** demonstrates the transition temperatures of several exemplary 9-mers.

**Table 1 Guest residue ratios in exemplary 9-mers.** The ELP polymers have hydrophobicities between the 10-mer ELP 1 series (least hydrophobic) and the 10-mer ELP 4 series (most hydrophobic).

| **ELP series** | **Pentamer motif** | **Guest residue ratio** |
|---|---|---|
| 1 series | VPG*X*G | 5 Val: 3 Gly : 2 Ala |
| alpha | VPG*X*G | 7 Val: 2 Gly : 0 Ala |
| beta v1 | VPG*X*G | 7 Val: 0 Gly : 2 Ala |
| beta v2 | VPG*X*G | 6 Val: 0 Gly : 3 Ala |
| gamma | VPG*X*G | 5 Val: 2 Gly : 2 Ala |
| delta | *X*PGVG | 5 Val: 0 Gly : 4 Ala |
| | VPG*X*G | 6 Val: 3 Gly : 0 Ala |
| | VPG*X*G | 6 Val: 2 Gly : 1 Ala |
| | VPG*X*G | 6 Val: 1 Gly : 2 Ala |
| | VPG*X*G | 6 Val: 0 Gly : 3 Ala |
| | VPG*X*G | 7 Val: 1 Gly : 1 Ala |
| | VPG*X*G | 8 Val: 0 Gly : 1 Ala |
| | VPG*X*G | 8 Val: 1 Gly : 0 Ala |
| 4 series | VPG*X*G | 10 Val: 0 Gly : 0 Ala |

**Table 2** Comparison of measured transition temperatures of exemplary 9-mers to ELP1 series. The inflection of turbidity measured using a Cary spectrophotometer is the result of the ELP biopolymer phase transitioning.

| **ELP series (10mg/ml)** | **Transition temp** |
|---|---|
| 1 series (pPB1023) | 37°C |
| alpha (pPE0253) | 29°C |
| beta v1 (pPE0254) | 28°C |
| beta v2 (pPE0311) | 31°C |
| gamma (pPE0255) | 29°C |
| delta (pPE0256) | 35°C |
| 4 series (pPE0002) | 26°C |

In some instances, the ELP includes combinations of the 9-mers listed in **Table 1.** In some instances, the ELP includes combinations of the alpha, beta v1, beta v2, and/or delta 9-mers. For example, the gamma ELP is constructed by alternating between an alpha 9-mer and a beta v1 9-mer for 16 copies until a 144mer is constructed. In certain instances, the ELP includes combinations of alpha and beta v1 9-mers. In certain instances, the ELP includes combinations of alpha and beta v2 9-mers. In certain instances, the ELP includes combinations of alpha and delta 9-mers. In certain instances, the ELP includes combinations of beta v1 and beta v2 9-mers. In certain instances, the ELP includes combinations of beta v1 and delta 9-mers. In certain instances, the ELP includes combinations of beta v2 and delta 9-mers. In certain instances, the ELP includes combinations of alpha, beta v1, and beta v2 9-mers. In certain instances, the ELP includes combinations of alpha, beta v1, and delta 9-mers. In certain instances, the ELP includes combinations of alpha, beta v2, and delta 9-mers. For example, in particular arrangements, the ELPbeta v2 may include the following guest residues in structural units iterated in the following sequence: A-V-A-V-V-A-V-A-V. The iterated sequence may be repeated sequentially in the ELP about 10 times, about 12 times, about 15 times, about 16 times, about 20 times, about 25 times, about 30 times, or about 35 times or more. In some aspects, the ELP contains about 10 to about 20 iterated sequences. In other aspects, the ELP contains about 15 to 20 iterated sequences. In some aspects, the ELP contains about 16 iterated sequences.

In some instances, the ELP includes 10-mers including ten copies of one or more ELP structural units disclosed herein. In some instances, the ELP includes 10-mers including ten copies of a pentapeptide disclosed herein. In some instances, the ELP includes 10-mers including SEQ ID NOs: 3 and 13 in any combination. In some instances, the ELP includes a sequence alternating between SEQ ID NOs: 3 and 13. ELPs of varying numbers of 10-mers can be combined to produce ELPs with, for instance, 20, 30, 40, 60, 90, 100, 120, 150, 160, or 200 copies of the 10-mer. Exemplary 10-mers are disclosed in **Table 3.**

**Table 3 Guest residue ratios in exemplary 10-mers.** The ELP polymers have hydrophobicities between the ELP 1 series (least hydrophobic) and the ELP 4 series (most hydrophobic).

| **ELP series** | **Pentamer motif** | **Guest residue ratio** |
|---|---|---|
| 1 series | VPG*X*G | 5 Val: 3 Gly : 2 Ala |
| | VPG*X*G | 5 Val: 4 Gly : 1 Ala |
| | VPGXG | 5 Val: 5 Gly : 0 Ala |
| | VPG*X*G | 5 Val: 2 Gly : 3 Ala |
| | VPGXG | 5 Val: 1 Gly : 4 Ala |
| | VPG*X*G | 5 Val: 0 Gly : 5 Ala |
| | VPG*X*G | 6 Val: 4 Gly : 0 Ala |
| | VPG*X*G | 6 Val: 3 Gly : 1 Ala |
| | VPG*X*G | 6 Val: 2 Gly : 2 Ala |
| | VPG*X*G | 6 Val: 1 Gly : 3 Ala |
| | VPG*X*G | 6 Val: 0 Gly : 4 Ala |
| | VPG*X*G | 7 Val: 3 Gly : 0 Ala |
| | VPG*X*G | 7 Val: 2 Gly : 1 Ala |
| | VPG*X*G | 7 Val: 1 Gly : 2 Ala |
| | VPG*X*G | 7 Val: 0 Gly : 3 Ala |
| | VPG*X*G | 8 Val: 2 Gly : 0 Ala |
| | VPG*X*G | 8 Val: 0 Gly : 2 Ala |
| | VPG*X*G | 8 Val: 1 Gly : 1 Ala |
| | VPG*X*G | 9 Val: 1 Gly : 1 Ala |
| | VPG*X*G | 9 Val: 0 Gly : 1 Ala |
| 4 series | VPG*X*G | 10 Val: 0 Gly : 0 Ala |

In some instances, the ELP may form a β-turn structure. Exemplary peptide sequences suitable for creating a β-turn structure are described in International Patent Application PCT/US96/05186. For example, the fourth residue (X) in the sequence VPGXG (SEQ ID NO: 3), can be varied without eliminating the formation of a β-turn.

The structure of exemplary ELPs may be described using the notation ELPₖ [XᵢYⱼ-n], where k designates a particular ELP repeat unit, the bracketed capital letters are single letter amino acid codes, and their corresponding subscripts designate the relative ratio of each guest residue X in the structural units (where applicable), and n describes the total length of the ELP in number of the structural repeats. For example, ELP1 [V₅A₂G₃-10] designates an ELP component containing 10 repeating units of the pentapeptide VPGXG (SEQ ID NO: 3), where X is valine, alanine, and glycine at a relative ratio of about 5:2:3; ELP1 [K₁V₂F₁-4] designates an ELP component containing 4 repeating units of the pentapeptide VPGXG (SEQ ID NO: 3), where X is lysine, valine, and phenylalanine at a relative ratio of about 1:2:1; ELP1 [K₁V₇F₁-9] designates a polypeptide containing 9 repeating units of the pentapeptide VPGXG (SEQ ID NO: 3), where X is lysine, valine, and phenylalanine at a relative ratio of about 1:7:1; ELP1 [V-5] designates a polypeptide containing 5 repeating units of the pentapeptide VPGXG (SEQ ID NO:3), where X is valine; ELP1 [V-20] designates a polypeptide containing 20 repeating units of the pentapeptide VPGXG (SEQ ID NO: 3), where X is valine; ELP2 [5] designates a polypeptide containing 5 repeating units of the pentapeptide AVGVP (SEQ ID NO: 4); ELP3 [V-5] designates a polypeptide containing 5 repeating units of the pentapeptide IPGXG (SEQ ID NO: 5), where X is valine; ELP4 [V-5] designates a polypeptide containing 5 repeating units of the pentapeptide LPGXG (SEQ ID NO: 7), where X is valine.

With respect to ELP, the Tt is a function of the hydrophobicity of the guest residue. Thus, by varying the identity of the guest residue(s) and their mole fraction(s), ELPs can be synthesized that exhibit an inverse phase transition over a broad range of temperatures. Thus, the Tt at a given ELP length may be decreased by incorporating a larger fraction of hydrophobic guest residues in the ELP sequence. Examples of suitable hydrophobic guest residues include valine, leucine, isoleucine, phenylalanine, tryptophan and methionine. Tyrosine, which is moderately hydrophobic, may also be used. Conversely, the Tt may be increased by incorporating residues, such as those selected from: glutamic acid, cysteine, lysine, aspartate, alanine, asparagine, serine, threonine, glycine, arginine, and glutamine.

For polypeptides having a molecular weight >100,000, the hydrophobicity scale disclosed in PCT/US96/05186 provides one means for predicting the approximate Tt of a specific ELP sequence. For polypeptides having a molecular weight <100,000, the Tt may be predicted or determined by the following quadratic function: Tt = M0 + MIX + M2X2 where X is the MW of the fusion protein, and M0 = 116.21; M1 = -1.7499; M2 = 0.010349.

The ELP in some instances is selected or designed to provide a Tt ranging from about 10 to about 37°C, such as from about 20 to about 37°C, or from about 25°C to about 37°C. In some instances, the transition temperature at physiological conditions (*e.g.,* 0.9% saline) is from about 34°C to 36°C, to take into account a slightly lower peripheral body temperature.

In certain instances, the ELP includes [VPGXG]ₘ, where m is any number from 1 to 200. In certain instances, the ELP includes [VPGXG]ₘ, where m is any number from 1 to 200, and each X is selected from V, G, and A. In certain instances, the ELP includes [VPGXG]ₘ, where m is any number from 1 to 200, each X is selected from V, G, and A, and wherein the ratio of V:G:A may be about 5:3:2. In certain instances, the ELP includes [VPGXG]₆₀, where each X is selected from V, G, and A, and wherein the ratio of V:G:A may be about 5:3:2. In certain instances, the ELP includes [VPGXG]₉₀, where each X is selected from V, G, and A, and wherein the ratio of V:G:A may be about 5:3:2. For example, the amino acid sequence capable of forming the hydrogen-bonded matrix at body temperature includes [VPGXG]₁₂₀, where each X is selected from V, G, and A, and wherein the ratio of V:G:A may be about 5:3:2. As shown herein, 120 structural units of this ELP can provide a transition temperature at about 37°C with about 5 to 15 mg/ml (e.g., about 10 mg/ml) of protein. At concentrations of about 50 to about 100 mg/mL the phase transition temperature is about 35.5 degrees centigrade (just below body temperature), which allows for peripheral body temperature to be just less than 37°C. In some instances, the ELP may include [VPGXG]₁₄₄, where each X is selected from V, G, and A, and wherein the ratio of V:G:A may be about 5:3:2. In some instances, the ELP includes [VPGXG]₁₈₀, where each X is selected from V, G, and A, and wherein the ratio of V:G:A may be about 5:3:2.

In certain instances, the ELP includes [VPGXG]ₘ, where m is any number from 1 to 200, where each X is selected from V, G, and A, and wherein the ratio of V:G:A is about 7:2:0. In certain instances, the ELP includes [VPGXG]₆₀, where each X is selected from V, G, and A, and wherein the ratio of V:G:A is about 7:2:0. In certain instances, the ELP includes [VPGXG]₉₀, where each X is selected from V, G, and A, and wherein the ratio of V:G:A is about 7:2:0. In certain instances, the ELP includes [VPGXG]₁₀₈, where each X is selected from V, G, and A, and wherein the ratio of V:G:A is about 7:2:0. In certain instances, the ELP includes [VPGXG]₁₂₀, where each X is selected from V, G, and A, and wherein the ratio of V:G:A is about 7:2:0. In certain instances, the ELP includes [VPGXG]₁₄₄, where each X is selected from V, G, and A, and wherein the ratio of V:G:A is about 7:2:0. In certain instances, the ELP includes [VPGXG]₈₀, where each X is selected from V, G, and A, and wherein the ratio of V:G:A is about 7:2:0.

In certain instances, the ELP includes [VPGXG]ₘ, where m is any number from 1 to 200, where each X is selected from V, G, and A, and wherein the ratio of V:G:A is about 7:0:2. In certain instances, the ELP includes [VPGXG]₆₀, where each X is selected from V, G, and A, and wherein the ratio of V:G:A is about 7:0:2. In certain instances, the ELP includes [VPGXG]₉₀, where each X is selected from V, G, and A, and wherein the ratio of V:G:A is about 7:0:2. In certain instances, the ELP includes [VPGXG]₁₀₈, where each X is selected from V, G, and A, and wherein the ratio of V:G:A is about 7:0:2. In certain instances, the ELP includes [VPGXG]₁₂₀, where each X is selected from V, G, and A, and wherein the ratio of V:G:A is about 7:0:2. In certain instances, the ELP includes [VPGXG]₁₄₄, where each X is selected from V, G, and A, and wherein the ratio of V:G:A is about 7:0:2. In certain instances, the ELP includes [VPGXG]₁₈₀, where each X is selected from V, G, and A, and wherein the ratio of V:G:A is about 7:0:2.

In certain instances, the ELP includes [VPGXG]ₘ, where m is any number from 1 to 200, where each X is selected from V, G, and A, and wherein the ratio of V:G:A is about 6:0:3. In certain instances, the ELP includes [VPGXG]₆₀, where each X is selected from V, G, and A, and wherein the ratio of V:G:A is about 6:0:3. In certain instances, the ELP includes [VPGXG]₉₀, where each X is selected from V, G, and A, and wherein the ratio of V:G:A is about 6:0:3. In certain instances, the ELP includes [VPGXG]₁₀₈, where each X is selected from V, G, and A, and wherein the ratio of V:G:A is about 6:0:3. In certain instances, the ELP includes [VPGXG]₁₂₀, where each X is selected from V, G, and A, and wherein the ratio of V:G:A is about 6:0:3. In certain instances, the ELP includes [VPGXG]₁₄₄, where each X is selected from V, G, and A, and wherein the ratio of V:G:A is about 6:0:3. In certain instances, the ELP includes [VPGXG]₁₈₀, where each X is selected from V, G, and A, and wherein the ratio of V:G:A is about 6:0:3.

In certain instances, the ELP includes [VPGXG]ₘ, where m is any number from 1 to 200, where each X is selected from V, G, and A, and wherein the ratio of V:G:A is about 5:2:2. In certain instances, the ELP includes [VPGXG]₆₀, where each X is selected from V, G, and A, and wherein the ratio of V:G:A is about 5:2:2. In certain instances, the ELP includes [VPGXG]₉₀, where each X is selected from V, G, and A, and wherein the ratio of V:G:A is about 5:2:2. In certain instances, the ELP includes [VPGXG]₁₀₈, where each X is selected from V, G, and A, and wherein the ratio of V:G:A is about 5:2:2. In certain instances, the ELP includes [VPGXG]₁₂₀, where each X is selected from V, G, and A, and wherein the ratio of V:G:A is about 5:2:2. In certain instances, the ELP includes [VPGXG]₁₄₄, where each X is selected from V, G, and A, and wherein the ratio of V:G:A is about 5:2:2. In certain instances, the ELP includes [VPGXG]₁₈₀, where each X is selected from V, G, and A, and wherein the ratio of V:G:A is about 5:2:2.

In certain instances, the ELP includes [VPGXG]ₘ, where m is any number from 1 to 200, where each X is selected from V, G, and A, and wherein the ratio of V:G:A is about 10:0:0. In certain instances, the ELP includes [VPGXG]₆₀, where each X is selected from V, G, and A, and wherein the ratio of V:G:A is about 10:0:0. In certain instances, the ELP includes [VPGXG]₉₀, where each X is selected from V, G, and A, and wherein the ratio of V:G:A is about 10:0:0. In certain instances, the ELP includes [VPGXG]₁₀₈, where each X is selected from V, G, and A, and wherein the ratio of V:G:A is about 10:0:0. In certain instances, the ELP includes [VPGXG]₁₂₀, where each X is selected from V, G, and A, and wherein the ratio of V:G:A is about 10:0:0. In certain instances, the ELP includes [VPGXG]₁₄₄, where each X is selected from V, G, and A, and wherein the ratio of V:G:A is about 10:0:0. In certain instances, the ELP includes [VPGXG]₁₈₀, where each X is selected from V, G, and A, and wherein the ratio of V:G:A is about 10:0:0.

In certain instances, the ELP includes [VPGXG]ₘ, where m is any number from 1 to 100, where each X is selected from V and L, and wherein the ratio of V:L is about 3:7 or about 4:6 or about 1:1 or about 6:4 or about 3:7. In certain instances, the ELP includes [VPGXG]₆₀, where each X is selected from V and L, and wherein the ratio of V:L is about 3:7 or about 4:6 or about 1:1 or about 6:4 or about 3:7. In certain instances, the ELP includes [VPGXG]₅₀, where each X is selected from V and L, and wherein the ratio of V:L is about 3:7 or about 4:6 or about 1:1 or about 6:4 or about 3:7. In certain instances, the ELP includes [VPGXG]₄₀, where each X is selected from V and L, and wherein the ratio of V:L is about 3:7 or about 4:6 or about 1:1 or about 6:4 or about 3:7. In certain instances, the ELP includes [VPGXG]₃₀, where each X is selected from V and L, and wherein the ratio of V:L is about 3:7 or about 4:6 or about 1:1 or about 6:4 or about 3:7 In certain instances, the ELP includes [VPGXG]₂₀, where each X is selected from V and L, and wherein the ratio of V:L is about 3:7 or about 4:6 or about 1:1 or about 6:4 or about 3:7.

The disclosure also provides certain instances wherein the ELP includes [XPGVG]ₘ, where m is any number from 1 to 200. In certain instances, the ELP includes [XPGVG]ₘ, where m is any number from 1 to 200, and each X is selected from V, G, and A. In certain instances, the ELP includes [XPGVG]ₘ, where m is any number from 1 to 200, each X is selected from V, G, and A and wherein the ratio of V:G:A is about 5:0:4. In certain instances, the ELP includes [XPGVG]₆₀, where each X is selected from V, G, and A, and wherein the ratio of V:G:A is about 5:0:4. In certain instances, the ELP includes [XPGVG]₉₀, where each X is selected from V, G, and A, and wherein the ratio of V:G:A is about 5:0:4. In certain instances, the ELP includes [XPGVG]₁₂₀, where each X is selected from V, G, and A, and wherein the ratio of V:G:A is about 5:0:4. In certain instances, the ELP includes [XPGVG]₁₄₄, where each X is selected from V, G, and A, and wherein the ratio of V:G:A is about 5:0:4. In certain instances, the ELP includes [XPGVG]₁₈₀, where each X is selected from V, G, and A, and wherein the ratio of V:G:A is about 5:0:4.

In certain instances, the ELP includes [VPGVG]ₘ where m is any number from 1 to 200. In some instances, the ELP includes [VPGVG]₆₀, [VPGVG]₉₀, or [VPGVG]₁₂₀. As shown herein, 120 structural units of this ELP can provide a transition temperature at about 37°C with about 0.005 to about 0.05 mg/ml (e.g., about 0.01 mg/ml) of protein. Alternatively, the ELP includes [VPGXG]₁₄₄ or [XPGVG]₁₄₄. As shown herein **(Table 2)**, 144 structural units of either of these ELPs can provide a transition temperature at 28°C to 35°C inclusive.

In various instances, the intended subject is human, and the body temperature is about 37°C, and thus the therapeutic agent is designed to provide a sustained release at or near this temperature (e.g. between about 28°C to about 37°C). A slow release into the circulation with reversal of hydrogen bonding and/or hydrophobic interactions is driven by a drop in concentration as the product diffuses at the injection site, even though body temperature remains constant. In other instances, the subject is a non-human mammal, and the therapeutic agent is designed to exhibit a sustained release at the body temperature of the mammal, which may be from about 30 to about 40°C in some instances, such as for certain domesticated pets (*e.g.,* dog or cat) or livestock (*e.g.,* cow, horse, sheep, or pig). Generally, the Tt is higher than the storage conditions of the formulation (which may be from 2 to about 25°C, or from 15 to 22°C), such that the therapeutic agent remains in solution for injection.

In some instances, the ELP can provide a transition temperature at a range of 27°C to 36°C inclusive. In some instances, the ELP can provide a transition temperature at a range of 28 °C to 35°C inclusive. In some instances, the ELP can provide a transition temperature at a range of 29 °C to 34°C inclusive. In some instances, the ELP can provide a transition temperature at a range of 27°C to 33°C inclusive. In some instances, the ELP can provide a transition temperature at a range of 30°C to 33°C inclusive. In some instances, the ELP can provide a transition temperature at a range of 31°C to 31°C inclusive. In some instances, the ELP can provide a transition temperature of 27°C, 28°C, 29°C, 30°C, 31°C, 32°C, 33°C, 34°C, 35°C, or 36°C. In some instances, the ELP can provide a transition temperature at a range of 28 °C to 35 °C inclusive at a protein concentration of 10 mg/mL in 110 mM NaCl.

Elastin-like-peptide (ELP) protein polymers and recombinant fusion proteins can be prepared as described in U.S. Patent Publication No. 2010/0022455. In some instances, the ELP protein polymers are constructed through recursive ligation to rapidly clone DNA encoding highly repetitive polypeptides of any sequence and specified length over a large range of molecular weights. In a single cycle, two halves of a parent plasmid, each containing a copy of an oligomer, are ligated together, thereby dimerizing the oligomer and reconstituting a functional plasmid. This process is carried out recursively to assemble an oligomeric gene with the desired number of repeats. For example, one ELP structural subunit (e.g. a pentapeptide or a 9-mer of pentapeptides) is inserted into a vector. The vector is digested, and another ELP structural unit (*e.g.* a pentapeptide or a 9-mer of pentapeptides) is inserted. Each subsequent round of digestion and ligation doubles the number of ELP structural units contained in the resulting vector until the ELP polymer is the desired length. By varying the number of pentapeptides in the initial structural unit, ELPs of varying length can easily be constructed. Alternative means of construction (i.e. other than recursive ligation) can be used to produce alternative lengths of ELP.

In some instances, the vector contains one or more additional amino acids or ELP structural unit repeats. For example, pPE0248 adds an additional pentamer repeat to the N terminus of the 144mer with valine in the guest position and an additional pentamer to the C terminus with a tryptophan in the guest residue position. The tryptophan may be used as a means to increase the extinction coefficient of the molecule, allowing for better measurement of absorbance, for instance at 280nm, which can be useful for determination of protein concentration, or for monitoring protein content during purification. The pentamers added to either end can also be designed so as the encoding DNA contains restriction enzyme recognition sites for cloning of fusion partners on to either end of the ELP coding sequence.

In some instances, the therapeutic agent includes an active agent and one or more ELPs. In some instances, the therapeutic agent includes an active agent with one or more ELPs at either the N- or C-terminus. In some instances, the therapeutic agent includes an active agent with one or more ELPs at both the N- or C-termini. In some instances, the ELPs are approximately the same size. In some instances, the ELPs differ in size. In some instances, an ELP at one terminus is larger than an ELP at the other terminus. In some instances, an ELP at the N-terminus is larger than an ELP at the C-terminus. In some instances, an ELP at the C-terminus is larger than an ELP at the N-terminus.

### ACTIVE AGENTS

### Protein Active Agents

In accordance with the invention, the active agent comprised in the sustained release formulation is a Glucagon-like peptide (GLP)-2 receptor agonist comprising the amino acid sequence of SEQ ID NO: 70.

The disclosure provides that the active agent may be a protein or peptide, which by itself may have a short circulatory half-life, such as from about 30 seconds to about 1 hour. The therapeutic agent may be a recombinant fusion protein between the protein active agent and the amino acid sequence capable of forming the hydrogen-bonded matrix at the body temperature of the subject (*e.g.* an ELP). Any appropriate protein active agent may be used in the therapeutic agents of the present disclosure. The disclosure exemplifies various alternative protein active agents, such as Activin receptor 2A extracellular domain, Adrenocorticotrophic hormone (ACTH), alpha-2 macroglobulin, alpha-MSH/Afamelanotide, Amylin (pramlintide), Angiotensin (1-7), Annexin A1, Apelin, Arginase, Asparaginase, Bradykinin B2 receptor antagonist, Compstatin, Coversin, CTLA-4, C-type natriuretic peptide, cenderitide (CD-NP), Elafin, Exendin-4, Fibroblast growth factor (FGF)-18/sprifermin, FGF-19, FGF-21, Galanin, Granulocyte colony stimulating factor (G-CSF), Ghrelin, GLP-1/GIP dual agonist, GLP-1/glucagon dual agonist, GLP-1/GLP-2 dual agonist, Glucagon, Glucagon-like peptide (GLP)-1 receptor antagonist, Granulocyte macrophage colony stimulating factor (GM-CSF), Hepcidin, Human growth hormone (hGH), hGH antagonist, Icatibant, Insulin-like growth factor (IGF)-1, Interleukin 1 receptor antagonist (IL-IRa), Infestin-4, Kisspeptin, L4F peptide, Lacritin, Parathyroid hormone (PTH), Parathyroid hormone-related protein (PTHrP), PYY, Relamorelin, Relaxin, Somatostatin, Thioredoxin, Thymosin β4, TNF-related apoptosis-inducing ligand (TRAIL), Urate oxidase, Urodilatin, Uroguanylin, Parathyroid hormone fragments (*e.g.* residues 1-34), full length parathyroid hormone, Adrenocorticotrophic hormone, Coversin, Kisspeptin, kisspeptin fragments (*e.g.* amino acid residues 1-10 or amino acid residues 1-54), Annexin A1-derived peptides (*e.g.* amino acid residues 2-26), analogs, derivatives, mimetics, fragments, combinations, or functional variants thereof. The present disclosure provides therapeutic agents including a protein active agent and an amino acid sequence providing sustained release. In some instances, the amino acid sequence providing sustained release is an Elastin-like peptide (ELP).

The half-life of protein active agents can be extended by a variety of means, including increasing the size and thus the hydrodynamic volume of the protein active agent, adding modified or unnatural amino acids, conjugation of moieties (*e.g.* pegylation), the addition of synthetic sequences (*e.g*. XTEN^{®} sequences, PASylation^{®}), carboxy-terminal extension from hCG (CTP), addition of albumin-binding sequences (*e.g.* AlbudAb^{®}), conjugation of albumin-binding compounds, *e.g.* fatty acids, post-translational modifications such as N-glycosylation and fusion to other peptides, or fusion with a mammalian heterologous protein, such as albumin, transferrin, or antibody Fc sequences. Such sequences are described in US Patent No. 7,238,667 (particularly with respect to albumin conjugates), US Patent No. 7,176,278 (particularly with respect to transferrin conjugates), and US Patent No. 5,766,883.

In some instances, the disclosure provides mimetics, analogs, derivatives, variants, or mutants of one or more active protein agents disclosed herein. In some instances, the mimetic, analog, derivative, variant, or mutant contains one or more amino acid substitutions compared to the amino acid sequence of the native therapeutic peptide agent. In some instances, one to 20 amino acids are substituted. In some instances, the mimetic, analog, derivative, variant, or mutant contains about 1, about 2, about 3, about 4, about 5, about 6, about 7, about 8, about 9, or about 10 amino acid substitutions compared to the amino acid sequence of the native therapeutic peptide agent. In some instances, the mimetic, analog, derivative, variant, or mutant contains one or more amino acid deletions compared to the amino acid sequence of the native therapeutic peptide agent. In some instances, one to 20 amino acids are deleted compared to the amino acid sequence of the native protein agent. In some instances, the mimetic, analog, derivative, variant, or mutant has about 1, about 2, about 3, about 4, about 5, about 6, about 7, about 8, about 9, or about 10 amino acid deletions compared to the amino acid sequence of the native protein agent. In some instances, one to ten amino acids are deleted at either terminus compared to the amino acid sequence of the native protein agent. In some instances, one to ten amino acids are deleted from both termini compared to the amino acid sequence of the native protein agent. In some instances, the amino acid sequence of the mimetic, analog, derivative, variant, or mutant is at least about 70% identical to the amino acid sequence of the native protein agent. In some instances, the amino acid sequence of the mimetic, analog, derivative, variant, or mutant is about 70%, about 80%, about 85%, about 90%, about 95%, about 96%, about 97%, about 98%, or about 99% identical to the amino acid sequence of the native protein agent. Percentage identity can be calculated using the alignment program EMBOSS Needle, available at http://www.ebi.ac.uk/Tools/psa/emboss_needle/. The following default parameters may be used for Pairwise alignment: Protein Weight Matrix = BLOSUM62; Gap Open = 10; Gap Extension = 0.1.

In some instances, the disclosure provides for co-formulation of any two or more active agents disclosed herein. In some instances, the co-formulation includes two or more peptide active agents and small molecule active agents. In some instances, the co-formulation includes two or more small molecule active agents. In some instances, the co-formulation includes two or more peptide active agents. In some instances, one or more of the active agents in the co-formulation is not conjugated to an ELP. In some instances, all of the active agents in the co-formulation are conjugated to an ELP.

### Active Agent of the Invention : Glucagon-Like Peptide (GLP)-2 Receptor Agonists

In accordance with the invention, the therapeutic agent includes an ELP fused or conjugated to a GLP-2 receptor agonist comprising the amino acid sequence of SEQ ID NO:70. The disclosure also describes derivatives, analogs, mimetics, fragments, or functional variants of a GLP-2 receptor agonist. In some instances, the GLP-2 receptor agonist is GLP-2.

GLP-2 is a 33-amino-acid peptide released from the posttranslational processing of proglucagon in the enteroendocrine L cells of the intestine and in specific regions of the brainstem. It is co-secreted together with GLP-1, oxyntomodulin and glicentin, in response to nutrient ingestion. GLP-2 shows remarkable homology in terms of amino acid sequence to glucagon and GLP-1. Different mammalian forms of GLP-2 are also highly conserved. For example, the human GLP-2 (hGLP-2) and degu (a south American rodent) GLP-2 differ from rat GLP-2 (rGLP-2) by one and three amino acids respectively. GLP-2 binds to a single G protein-coupled receptor belonging to the class II glucagon secretin family. The GLP-2 receptor is localized in the small intestine, colon and stomach, which are sites that are known to be responsive to GLP-2 (Yusta et al., 2000, Gastroenterology, 119: 744-55).

In certain instances of the disclosure, the active agent includes an amino acid sequence providing sustained release fused or conjugated to a GLP-2 receptor agonist, derivatives, analogs, mimetics, fragments, or functional variants thereof. In certain instances of the disclosure, the active agent includes an amino acid sequence providing sustained release fused or conjugated to a GLP-2 receptor agonist and hGH. In some instances, the GLP-2 receptor agonist and the hGH are attached at different termini of the amino acid sequence providing sustained release. In some instances, the GLP-2 receptor agonist is attached to the amino terminus of the amino acid sequence providing sustained release and the hGH is attached at the carboxy terminus.. In some instances, the hGH is attached to the amino terminus of the amino acid sequence providing sustained release and the GLP-2 receptor agonist is attached at the carboxy terminus. In some instances, the amino acid sequence providing sustained release is ELP.

In certain instances, the GLP-2 receptor agonist is a mammalian GLP-2 receptor agonist, such as a human GLP-2 receptor agonist. Human GLP-2 peptide is encoded by SEQ ID NO: 68. The GLP-2 receptor agonist used in the invention is a GLP-2 peptide analog wherein the Alanine (A) in position 2 of the N-terminus of GLP-2 peptide has been replaced by Glycine (G) (SEQ ID NO: 70). The disclose also provides the GLP-2 receptor agonist wherein it includes a sequence HXDGSFSDEMNTILDNLAARDFINWLIQTKITD (SEQ ID NO: 74), wherein X is Alanine (A), Glycine (G), Leucine (L), Isoleucine (I), or Valine (V). In some instances, X is Alanine (A) or Glycine (G).

The disclosure also describes instances wherein the GLP-2 receptor agonist is a functional variant of a mammalian GLP-2 peptide, including functional fragments truncated at the C-terminus of human GLP-2 peptide by from about 1 to about 25 amino acids, including, for example, by up to about 3 amino acids, up to about 5 amino acids, up to about 10 amino acids, up to about 15 amino acids, up to about 20 amino acids, or up to about 25 amino acids. In other instances, functional variants may contain from about 1 to about 25 amino acid insertions, deletions, and/or substitutions with respect to a native or consensus sequence (*e.g.,* SEQ ID NOs: 68 or 74). For example, functional variants may have up to about 3 amino acid, up to about 5 amino acid, up to about 10 amino acid, up to about 15 amino acid, up to about 20 amino acid, or up to about 25 amino acid insertions, deletions, and/or substitutions with respect to a or consensus sequence (e.g., SEQ ID NOs: 68 or 74). Protein activity may be confirmed or assayed using any available assay. Also described here are other instances wherein the GLP-2 receptor agonist has at least about 75% identity, about 80% identity, about 90% identity, about 95% identity, about 96% identity, about 97% identity, about 98% identity, or about 99% identity with a native or consensus sequence (e.g., SEQ ID NOs: 68 or 74). Percentage identity can be calculated using the alignment program EMBOSS needle, available at http://www.ebi.ac.uk/Tools/psa/emboss_needle/. The following default parameters may be used for Pairwise alignment: Protein Weight Matrix = BLOSUM62; Gap Open = 10; Gap Extension = 0.1. In some instances, the GLP-2 receptor agonist may contain additional chemical modifications known in the art.

In certain instances, the therapeutic agent includes an ELP fused to the N-terminus or the C-terminus of a GLP-2 receptor agonist, fragments, or functional variants thereof. Described herein as instances wherein the therapeutic agent includes an ELP fused to the C-terminus of a GLP-2 peptide (SEQ ID NOs: 69, 71, and 73).

In some instances, the GLP-2 receptor agonist is in a fusion protein with more than one ELP sequence. In some instances, the GLP-2 receptor agonist has one or more ELPs at both the N- and C-termini. In some instances, the ELP at the C-terminus and/or the N-terminus of the GLP-2 receptor agonist includes about 90 to about 180 repeating structural units. In other instances, the ELP at the C-terminus and/or the N-terminus of the GLP-2 receptor agonist includes fewer than about 90 repeating structural units. In other aspects, the ELP at the C-terminus and/or the N-terminus of the GLP-2 receptor agonist includes greater than about 180 repeating structural units. In some instances, the two or more ELPs at the N- and C- termini are approximately the same size. In some instances, the two or more ELPs at the N- and C- termini differ in size. In some instances, the ELP at the N-terminus of the GLP-2 receptor agonist is larger than the ELP at the C-terminus of the GLP-2 receptor agonist. In other instances, the ELP at the C-terminus of the GLP-2 receptor agonist is larger than the ELP at the N-terminus of the GLP-2 receptor agonist. Described herein are instances wherein the ELP is attached to the C-terminus and/or the N-terminus of the GLP-2 receptor agonist via a linker (SEQ ID NOs: 71 and 73). In some instances, the linker comprises a tripeptide GGS sequence (SEQ ID NO: 87). In some instances, the linker comprises a single copy of the tripeptide GGS or alternatively the linker comprises multiple repeats of the tripeptide GGS. In some instances, the linker comprises two repeats of the GGS sequence (SEQ ID NO: 102). In some instances, the linker comprises three repeats of the GGS sequence (SEQ ID NO: 103). In some instances, the linker comprises a pentapeptide GGGGS sequence (SEQ ID NO: 93). In some aspects, the linker comprises a single copy of the pentapeptide GGGGS (SEQ ID NO: 93). In another aspect, the linker comprises multiple repeats of the pentapeptide GGGGS. In some instances, the linker comprises two repeats of the GGGGS sequence (SEQ ID NO: 104). In some instances, the linker comprises three repeats of the GGGGS sequence (SEQ ID NO: 105). In some instances, the linker comprises a pentapeptide PAPAP sequence (SEQ ID NO: 94). In some aspects, the linker comprises a single copy of the pentapeptide PAPAP. In another aspect, the linker comprises multiple repeats of the pentapeptide PAPAP. In some instances, the linker comprises two repeats of the PAPAP sequence (SEQ ID NO: 106). In some instances, the linker comprises three repeats of the PAPAP sequence (SEQ ID NO: 107). In some instances, the linker comprises a pentapeptide EAAAK sequence (SEQ ID NO: 95). In some aspects, the linker comprises a single copy of the pentapeptide EAAAK. In another aspect, the linker comprises multiple repeats of the pentapeptide EAAAK In some instances, the linker comprises two repeats of the EAAAK sequence (SEQ ID NO: 108). In some instances, the linker comprises three repeats of the EAAAK sequence (SEQ ID NO: 109).

In other aspects, the present disclosure provides a therapeutic agent including an ELP and a GLP-2 receptor agonist (as described above) for use in methods for treating or preventing intestinal diseases including, but not limited to, intestinal diseases such as Inflammatory Bowel Disease (IBD), such as Ulcerative Colitis (UC) and Crohn's Disease (CD), or Short Bowel Syndrome (SBS); chemotherapy-induced mucositis; chemotherapy-induced diarrhea; or ischemia-reperfusion injury. The methods include administering the therapeutic agent including an ELP and a GLP-2 receptor agonist (as described above) to a patient in need of such treatment. Generally, the patient may be a human or non-human animal patient (*e.g.,* dog, cat, cow, or horse). Preferably, the patient is human. In some instances, treatment with a GLP-2 receptor agonist according to the present disclosure may also be combined with one or more pharmacologically active substances, *e.g.* selected from agents for the treatment and/or prevention of various complications and disorders, resulting from or associated with various diseases including without limitation intestinal diseases including, but not limited to, intestinal diseases such as IBD (such as UC or CD) or SBS; chemotherapy-induced mucositis; chemotherapy-induced diarrhea; or ischemia-reperfusion injury.

In addition to the active agent comprised within the sustained release formulation of the invention (GLP-2 receptor agonist comprising the amino acid sequence of SEQ ID NO:7), the disclosure also provides a description of the following active agents.

### Active agent of the disclosure: Apelin

An example of a protein active agent is apelin, analogs, mimetics, derivatives, fragments, or functional variants thereof. Human apelin is an endogenous ligand for the Apelin Receptor (APJ Class A G-protein coupled receptor). Apelin is expressed in several cell types including human cardiomyocytes, endothelial cells, and vascular smooth muscle cells. The *apelin* gene has been identified in various species, including human, dog, bovine, rat, mouse, rhesus monkey, and zebra fish and codes for an apelin preproprotein of 77 amino acids. Spanning 1726 base pairs of genomic DNA with 3 exons, the apelin locus is highly conserved between species. In apelin preproprotein processing, a signal peptide corresponding to residues 1-22 of the apelin preproprotein is cleaved off, resulting in a 55 amino acid apelin proprotein, which is processed into several active molecular forms including apelin-12, apelin-13, apelin-16, apelin-17 and apelin-36. (Kawamata et al., 2001, Biochim Biophys Acta 1538:162-71). Such active molecular forms are collectively referred to as apelin and are named according to their length and/or modification state. The full length mature peptide, Apelin-36, is a 36 amino acid long peptide derived from the 55 amino acid long apelin proprotein (Tatemoto et al., 1998, Biochem. Biophys. Res. Comm. 251:471-476, 1998) and corresponds to residues 42-77 of the preproprotein. Apelin-17 and apelin-13 are derived from the carboxy (C)-terminal end of apelin. Apelin-17 corresponds to residues 61-77 of the apelin proprotein. Apelin-13 corresponds to residues 65-77 of the apelin proprotein. The apelin proteins may include amino acid derivatives or modifications. For example, the most active isoform is the pyroglutamated form of apelin-13 (pyr-apelin 13).

Apelin is an endogenous vasodilator and inotrope which may lower blood pressure by antagonizing the renin-angiotensin system, possibly via formation of receptor heterotrimers.

Described herein are instances wherein the active agent includes an amino acid sequence providing sustained release fused or conjugated to apelin, mimetics, analogs, derivatives, fragments, or functional variants thereof. In some instances, the amino acid sequence providing sustained release is ELP. In certain instances, the apelin is a mammalian apelin. In some instances, the apelin is human apelin *(e.g.,* SEQ ID NO: 39), which is the full length mature human apelin peptide, apelin-36 (SEQ ID NO: 39). In other instances, the apelin is a truncation of apelin-36 including, but not limited to, apelin-16, apelin-17 (SEQ ID NO: 41), apelin-13 (SEQ ID NO: 43), and apelin-12 (SEQ ID NO: 44). In some instances, the apelin includes one or more modified amino acids. In some instances, the apelin includes one or more amino acid derivatives. In some instances, the apelin is pyr-apelin 13 (SEQ ID NO: 42).

In some instances, the apelin is a functional analog of mammalian apelin, including functional fragments truncated at the N-terminus and/or the C-terminus of apelin by from about 1 to about 30 amino acids, including, for example, by up to about 3 amino acids, up to about 5 amino acids, up to about 10 amino acids, up to about 15 amino acids, up to about 20 amino acids, up to about 25 amino acids, or up to about 30 amino acids. In other instances, functional variants contain from about 1 to about 30 amino acid insertions, deletions, and/or substitutions with respect to a native or truncated sequence (*e.g.,* SEQ ID NOs: 39, 41, 42, 43, or 44). For example, functional variants may have up to about 3 amino acid, up to about 5 amino acid, up to about 10 amino acid, up to about 15 amino acid, up to about 20 amino acid, up to about 25 amino acid, or up to about 30 amino acid insertions, deletions, and/or substitutions with respect to a native or truncated sequence (*e.g.,* SEQ ID NOs: 39, 41, 42, 43, or 44). Protein activity may be confirmed or assayed using any available assay. In other instances, the apelin has at least about 75% identity, about 80% identity, about 90% identity, about 95% identity, about 96% identity, about 97% identity, about 98% identity, or about 99% identity with a native or truncated sequence (*e.g.,* SEQ ID NOs: 39, 41, 42, 43, or 44). Percentage identity can be calculated using the alignment program EMBOSS needle, available at http://www.ebi.ac.uk/Tools/psa/emboss_needle/. The following default parameters may be used for Pairwise alignment: Protein Weight Matrix = BLOSUM62; Gap Open = 10; Gap Extension = 0.1. In some instances, the apelin may contain additional chemical modifications known in the art. In some instances, the functional analog of apelin is Elabella/Toddler or an analog or derivative thereof (Yang et al., 2015, Trends in Pharmacol. Sci. 36:560-567)

Described herein are instances wherein the therapeutic agent includes an ELP fused to the N-terminus or the C-terminus of apelin, mimetics, analogs, derivatives, fragments, or functional variants thereof. In some instances, the therapeutic agent includes an ELP fused to the N-terminus of human apelin-36 (SEQ ID NO: 40). In some instances, the apelin is in a fusion protein with more than one ELP sequence. In some instances, the apelin has one or more ELPs at both the N- and C-termini. In some instances, the ELP at the C-terminus and/or the N-terminus of the apelin includes about 90 to about 180 repeating structural units. In other instances, the ELP at the C-terminus and/or the N-terminus of the apelin includes fewer than about 90 repeating structural units. In other aspects, the ELP at the C-terminus and/or the N-terminus of the apelin includes greater than about 180 repeating structural units. In some instances, the two or more ELPs at the N- and C- termini are approximately the same size. In other instances, the two or more ELPs at the N- and C- termini differ in size. In some instances, the ELP at the N-terminus of the apelin is larger than the ELP at the C-terminus of the apelin. In other instances, the ELP at the C-terminus of the apelin is larger than the ELP at the N-terminus of the apelin.

In other aspects, the present disclosure provides methods for treating or preventing diseases including, but not limited to, heart failure, pulmonary arterial hypertension (PAH), ischemic heart disease, diabetes, cancer, obesity, cardiovascular defects, or pathological conditions involving angiogenesis (*e.g.,* cancer) by administering an ELP and an apelin. In some instances, the therapeutic agents disclosed herein may be administered to provide effects including, but not limited to, vasodilatory effects (*e.g.,* mediated by endothelial Nitric Oxide Synthase (eNOS)), positive ionotropic effects (*e.g.,* due to increased intracellular calcium and increased myofilament calcium sensitivity), protection against ischemia-reperfusion injury (*e.g.* via Nitric Oxide (NO)-dependent and NO-independent pathways), and APJ receptor-mediated cardiac hypertrophy (*e.g.,* β-arrestin mediated, rather than G protein mediated). The methods include administering a therapeutic agent including an ELP and an apelin (as described above) to a patient in need of such treatment. Generally, the patient may be a human or non-human animal patient *(e.g.,* dog, cat, cow, or horse). Preferably, the patient is human.

In some instances, treatment with an apelin therapeutic agent according to the present disclosure may also be combined with one or more pharmacologically active substances, *e.g.* selected from agents for the treatment and/or prevention of complications and disorders resulting from or associated with various diseases, including without limitation heart failure, PAH, ischemic heart disease, diabetes, cancer, obesity, cardiovascular defects, or pathological conditions involving angiogenesis (*e.g.,* cancer). In additional instances, treatment with an apelin therapeutic agent according to the present disclosure may be combined with one or more pharmacologically active substances, *e.g.* selected from agents providing effects including, but not limited to, vasodilatory effects (*e.g.,* mediated by endothelial Nitric Oxide Synthase (eNOS)), positive ionotropic effects (e.g. due to increased intracellular calcium and increased myofilament calcium sensitivity), protection against ischemia-reperfusion injury (*e.g.,* via Nitric Oxide (NO)-dependent and NO-independent pathways), and APJ receptor-mediated cardiac hypertrophy (*e.g.,* β-arrestin mediated, rather than G protein mediated).

### Active agent of the disclosure: Arginase

An example of a protein active agent is Arginase (EC 3.5.3.1; L-arginine amidinohydrolase), isozymes, fragments, or functional variants thereof. Arginase is a 35 kDa key enzyme of the urea cycle that catalyzes the conversion of L-arginine to ornithine and urea, which is the final cytosolic reaction of urea formation in the mammalian liver. Arginase serves three important functions: production of urea, production of ornithine, and regulation of substrate arginine levels for nitric oxide synthase (Jenkinson et al., 1996, Comp. Biochem. Physiol. 114B: 107-132; Kanyo et al., 1996, Nature 383:554-557; Christianson, 1997, Prog. Biophys. Molec. Biol. 67:217-252). Urea production provides a mechanism to excrete nitrogen in the form of a highly soluble, non-toxic compound, thus avoiding the potentially dangerous consequences of high ammonia levels. L-ornithine is a precursor for the biosynthesis of polyamines, spermine, and spermidine, which have important roles in cell proliferation and differentiation. Finally, arginase modulates production of nitric oxide by regulating the levels of arginine present within tissues.

In general, arginase is expressed in liver, kidney and testis of urea-producing animals (*e.g.* mammals, elasmobranchs, amphibians, and turtles). In most mammals, including humans, the family of arginase proteins includes the isozymes arginase I, which is mainly expressed in the liver cells, and arginase II, which is mainly expressed in the kidney and erythrocytes.

A deficiency in human liver arginase may result in argininemia, an inherited autosomal recessive disorder accompanied by hyperammonemia, a condition where excess arginine leads to seizures, impaired cognitive abilities, and neuronal impairment. Clinically significant hyperargininemia results from mutations in the *arginase I* gene (Cederbaum S. D. et al., 1979, Pediat. Res. 13:827-833; Cederbaum S. et al., 1977, J. Pediatr. 90:569-573; Michel V. V. et al., 1978, Clin. Genet. 13:61-67). Due to an arginase deficiency, arginine cannot be degraded into urea and participate in the ornithine metabolism cycle, resulting in blood arginine levels about 7 to about 10 times higher than normal, increased arginine levels in the cerebrospinal fluid, increased urine production, and increased urea excretion of creatinine. Arginase I deficient patients may present with spasticity, growth retardation, progressive mental impairment and episodic hyperammonemia (Cederbaum S.D. et al., 1979, Pediat Res 13:827-833; Cederbaum S.D., et al., 1977, J. Pediatr 90:569-573; Thomas K.R. and Capecchi M.R., 1987, Cell 51:503-512).

Described herein are instances wherein the arginase therapeutic agent includes an amino acid sequence providing sustained release fused or conjugated to an arginase, mimetics, analogs, derivatives, fragments, or functional variants thereof. In some instances, the amino acid agent providing sustained release is an ELP.

In certain instances, the arginase is a mammalian arginase. In some instances, the arginase is arginase I. In other instances, the arginase is arginase II. In some instances, the arginase is human arginase (SEQ ID NO: 45).

In some instances, the arginase is a functional analog of mammalian arginase, including functional fragments truncated at the N-terminus and/or the C-terminus of arginase by from about 1 to about 50 amino acids, including, for example, by up to about 3 amino acids, up to about 5 amino acids, up to about 10 amino acids, up to about 15 amino acids, up to about 20 amino acids, up to about 25 amino acids, up to about 30 amino acids, up to about 35 amino acids, up to about 40 amino acids, up to about 45 amino acids, or up to about 50 amino acids. In other instances, functional variants contain from about 1 to about 50 amino acid insertions, deletions, and/or substitutions with respect to a native sequence (*e.g.,* SEQ ID NO: 45). For example, functional variants may have up to about 3 amino acid, about 5 amino acid, up to about 10 amino acid, up to about 15 amino acid, up to about 20 amino acid, up to about 25 amino acid, up to about 30 amino acid, up to about 35 amino acid, up to about 40 amino acid, up to about 45 amino acid, or up to about 50 amino acid insertions, deletions, and/or substitutions with respect to a native sequence (*e.g.,* SEQ ID NO: 45). Protein activity may be confirmed or assayed using any available assay. In other instances, the arginase has at least about 75% identity, about 80% identity, about 90% identity, about 95% identity, about 96% identity, about 97% identity, about 98% identity, or about 99% identity with a native sequence (*e.g.,* SEQ ID NO: 45). Percentage identity can be calculated using the alignment program EMBOSS needle, available at http://www.ebi.ac.uk/Tools/psa/emboss_needle/. The following default parameters may be used for Pairwise alignment: Protein Weight Matrix = BLOSUM62; Gap Open = 10; Gap Extension = 0.1. In some instances, the arginase may contain additional chemical modifications known in the art. In some instances, the arginase may utilize manganese as a cofactor, instead of cobalt.

In certain instances, the therapeutic agent includes an ELP fused to the N-terminus or the C-terminus of an arginase, mimetics, analogs, derivatives, fragments, or functional variants thereof. In some instances, the therapeutic agent includes an ELP fused to the C-terminus of human arginase (SEQ ID NO: 46). In some instances, the arginase is in a fusion protein with more than one ELP sequence. In some instances, the arginase has one or more ELPs at both the N- and C-termini. In some instances, the ELP at the C-terminus and/or the N-terminus of the arginase includes about 90 to about 180 repeating structural units. In other instances, the ELP at the C-terminus and/or the N-terminus of the arginase includes fewer than about 90 repeating structural units. In other aspects, the ELP at the C-terminus and/or the N-terminus of the arginase includes greater than about 180 repeating structural units. In some instances, the two or more ELPs at the N- and C- termini are approximately the same size. In other instances, the two or more ELPs at the N- and C- termini differ in size. In some instances, the ELP at the N-terminus of the arginase is larger than the ELP at the C-terminus of the arginase. In other instances, the ELP at the C-terminus of the arginase is larger than the ELP at the N-terminus of the arginase.

In other aspects, the present disclosure provides methods for treating or preventing diseases associated with, or caused by, a defect in an arginase gene or arginase gene expression, such as, for example, urea cycle diseases; hypertension; hypotension; hyperammonemia; episodic hyperammonemia; defects in biosynthesis of proline, glutamate, nitric oxide and ornithine; hyperargininemia and its related spasticity; growth retardation; progressive mental impairment; prostate disease, prostate cancer, prostatitis or benign prostatic hyperplasia or hypertrophy, prostate damage; kidney disease, and kidney damage; cancers including, without limitation, non-Hodgkin's lymphoma, hepatocarcinomas, melanomas or renal cell carcinomas; autoimmune disease; fibrotic diseases; erectile dysfunction; pulmonary hypertension; atherosclerosis; renal disease; asthma; T-cell dysfunction; ischemia reperfusion injury; neurodegenerative diseases; wound healing; arginine-dependent hyperplasia; tumors; hepatocarcinomas; melanomas; renal cell carcinomas; HCC; or melanoma. The methods include administering a therapeutic agent including an ELP and an arginase (as described above) to a patient in need of such treatment. Generally, the patient may be a human or non-human animal patient (*e.g.,* dog, cat, cow, or horse). Preferably, the patient is human.

In some instances, treatment with an arginase therapeutic agent according to the present disclosure may also be combined with one or more pharmacologically active substances, *e.g.* selected from agents for the treatment and/or prevention of complications and disorders resulting from or associated with various diseases, including without limitation hypertension; hypotension; hyperammonemia; episodic hyperammonemia; defects in biosynthesis of proline, glutamate, nitric oxide and ornithine; hyperargininemia and its related spasticity; growth retardation; progressive mental impairment; prostate disease, prostate cancer; prostatitis or benign prostatic hyperplasia or hypertrophy; prostate damage; kidney disease; kidney damage; cancers including, without limitation, non-Hodgkin's lymphoma, hepatocarcinomas, melanomas or renal cell carcinomas; autoimmune disease; fibrotic diseases; erectile dysfunction; pulmonary hypertension; atherosclerosis; renal disease; asthma; T-cell dysfunction; ischemia reperfusion injury; neurodegenerative diseases; wound healing; arginine-dependent hyperplasia; tumors; hepatocarcinomas; melanomas; renal cell carcinomas; HCC; or melanoma.

### Active agent of the disclosure: C-type Natriuretic Peptide (CNP)

An example of a protein active agent is C-type natriuretic peptide (CNP), mimetics, analogs, derivatives, fragments, or functional variants thereof. Natriuretic peptides play a role in cardiovascular homeostasis, diuresis, natriuresis, and vasodilation. The natriuretic peptide family consists of three structurally related peptides: atrial natriuretic peptide (ANP), brain natriuretic peptide (BNP), and C-type natriuretic peptide (CNP). These small, single chain peptides (ANP, BNP, and CNP) have a 17-amino acid loop structure (Levin et al., N. Engl. J. Med., 339: 863-870 (1998)) and play important roles in multiple biological processes. ANP and BNP are produced primarily within the muscle cells of the heart, and have important roles in cardiovascular homeostasis (Science, 252: 120-123 (1991)). CNP is expressed more widely, including in the central nervous system, reproductive tract, bone and endothelium of blood vessels (Hypertension, 49: 419-426 (2007)), and acts, for example, as a regulator of bone growth, a vasodilator and inotrope, and have cardiovascular effects.

In humans, CNP is initially produced from the natriuretic peptide precursor C (NPPC) gene as a single chain 126-amino acid pre-pro polypeptide (Sudoh, T. et al., Biochem. Biophys. Res. Commun., 1990, 168: 863-870). Removal of the signal peptide yields pro-CNP, and further cleavage by the endoprotease furin generates an active 53-amino acid peptide (CNP-53), which is secreted and cleaved again to produce the mature 22-amino acid peptide (CNP-22) (Wu, J. Biol. Chem., 2003, 278: 25847-852). CNP-53 and CNP-22 differ in their distribution, with CNP-53 predominating in tissues, while CNP-22 is mainly found in plasma and cerebrospinal fluid (J. Alfonzo, Recept. Signal. Transduct. Res., 2006, 26: 269-297). The predominant bioactive form of CNP is CNP-22.

Natriuretic peptides act mainly through two receptors: natriuretic peptide receptor-A (NPR-A) and natriuretic peptide receptor B (NPR-B). These receptors have cytoplasmic guanylyl cyclase domains, which are activated upon ANP, BNP, or CNP binding and lead to accumulation of intracellular cGMP. Both CNP-53 and CNP-22 bind similarly to NPR-B and they can both induce cGMP production in a dose-dependent and similar fashion (Yeung, V.T., Peptides, 1996, 17: 101-106). A third receptor, NPR-C, binds each of the natriuretic peptides with high affinity and functions primarily to capture the peptides from the extracellular compartment and deposit the peptides into lysosomes, where they are degraded (Maak, T. et al., Science, 1987, 238: 675-678).

CNP binds to and activates NPR-B resulting in increased intracellular cyclic guanosine monophosphate (cGMP) levels. Downstream signaling mediated by cGMP generation influences a diverse array of biological processes including endochondral ossification (the process that governs longitudinal long-bone growth).

The disclosure provides instances wherein the therapeutic agent includes an amino acid sequence providing sustained release fused or conjugated to CNP, fragments, or functional variants thereof. In some instances, the amino acid sequence providing sustained release is ELP.

In certain instances, the CNP is a mammalian CNP. In some instances, the CNP is human CNP-53 (SEQ ID NO: 51). In other instances, the CNP is CNP-22 (SEQ ID NO: 47). In other instances, the CNP is CNP-37 (SEQ ID NO: 49). In yet other instances, the CNP is CNP-39 (SEQ ID NO: 52).

In some instances, the CNP is a fragment or functional variant of mammalian CNP, including functional fragments truncated at the N-terminus and/or the C-terminus of CNP by from about 1 to about 30 amino acids, including, for example, by up to about 3 amino acids, up to about 5 amino acids, up to about 10 amino acids, up to about 15 amino acids, up to about 20 amino acids, up to about 25 amino acids, or up to about 30 amino acids. In other instances, functional variants contain from about 1 to about 30 amino acid insertions, deletions, and/or substitutions with respect to a native or truncated sequence (*e.g.,* SEQ ID NOs: 47, 49, 51, or 52). For example, functional variants may have up to about 3 amino acid, up to about 5 amino acid, up to about 10 amino acid, up to about 15 amino acid, up to about 20 amino acid, up to about 25 amino acid, or up to about 30 amino insertions, deletions, and/or substitutions with respect to a native or truncated sequence *(e.g.,* SEQ ID NOs: 47, 49, 51, or 52). Protein activity may be confirmed or assayed using any available assay. In other instances, the CNP has at least about 75% identity, about 80% identity, about 90% identity, about 95% identity, about 96% identity, about 97% identity, about 98% identity, or about 99% identity with a native or truncated sequence (*e.g.,* SEQ ID NOs: 47, 49, 51, or 52). Percentage identity can be calculated using the alignment program EMBOSS needle, available at http://www.ebi.ac.uk/Tools/psa/emboss_needle/. The following default parameters may be used for Pairwise alignment: Protein Weight Matrix = BLOSUM62; Gap Open = 10; Gap Extension = 0.1. In some instances, the CNP may contain additional chemical modifications known in the art.

In some aspects, the therapeutic agent includes an ELP fused to the N-terminus or the C-terminus of CNP, fragments, or functional variants thereof. In some instances, the therapeutic agent includes an ELP fused to the N-terminus of CNP-22 (SEQ ID NO: 48). In another aspect, the therapeutic agent includes an ELP fused to the N-terminus of CNP-37 (SEQ ID NO: 50). In some instances, the therapeutic agent includes an ELP fused to the C-terminus of CNP (*e.g.* SEQ ID NO: 57 or 98). In some instances, the therapeutic agent includes an ELP fused to a pro-CNP (SEQ ID NO: 56). In some instances, the CNP is in a fusion protein with more than one ELP sequence. In some instances, the CNP has one or more ELPs at both the N- and C-termini. In some instances, the ELP at the C-terminus and/or the N-terminus of the CNP includes about 90 to about 180 repeating structural units. In other instances, the ELP at the C-terminus and/or the N-terminus of the CNP includes fewer than about 90 repeating structural units. In other instances, the ELP at the C-terminus and/or the N-terminus of the CNP includes fewer than about 40 repeating structural units. In other aspects, the ELP at the C-terminus and/or the N-terminus of the CNP includes greater than about 180 repeating structural units. In some instances, the two or more ELPs at the N- and C- termini are approximately the same size. In other instances, the two or more ELPs at the N- and C- termini differ in size. In some instances, the ELP at the N-terminus of the CNP is larger than the ELP at the C-terminus of the CNP. In other instances, the ELP at the C-terminus of the CNP is larger than the ELP at the N-terminus of the CNP.

In some instances, the ELP is attached to the C-terminus and/or the N-terminus of the CNP via a linker (SEQ ID NOs: 83 and 55). In some instances, the linker comprises a tripeptide GGS sequence (SEQ ID NO: 87). In some aspects, the linker comprises a single copy of the tripeptide GGS (SEQ ID NO: 87). In another aspect, the linker comprises multiple repeats of the tripeptide GGS. In some instances, the linker comprises two repeats of the GGS sequence (SEQ ID NO: 102). In some instances, the linker comprises three repeats of the GGS sequence (SEQ ID NO: 103). In some instances, the linker comprises a pentapeptide GGGGS sequence (SEQ ID NO: 93). In some aspects, the linker comprises a single copy of the pentapeptide GGGGS (SEQ ID NO: 93). In another aspect, the linker comprises multiple repeats of the pentapeptide GGGGS. In some instances, the linker comprises two repeats of the GGGGS sequence (SEQ ID NO: 104). In some instances, the linker comprises three repeats of the GGGGS sequence (SEQ ID NO: 105). In some instances, the linker comprises a pentapeptide PAPAP sequence (SEQ ID NO: 94). In some aspects, the linker comprises a single copy of the pentapeptide PAPAP. In another aspect, the linker comprises multiple repeats of the pentapeptide PAPAP. In some instances, the linker comprises two repeats of the PAPAP sequence (SEQ ID NO: 106). In some instances, the linker comprises three repeats of the PAPAP sequence (SEQ ID NO: 107). In some instances, the linker comprises a pentapeptide EAAAK sequence (SEQ ID NO: 95). In some aspects, the linker comprises a single copy of the pentapeptide EAAAK. In another aspect, the linker comprises multiple repeats of the pentapeptide EAAAK. In some instances, the linker comprises two repeats of the EAAAK sequence (SEQ ID NO: 108). In some instances, the linker comprises three repeats of the EAAAK sequence (SEQ ID NO: 109).

In some instances, the ELP is attached to the C-terminus and/or the N-terminus of the CNP via a peptide sequence comprising a protease recognition site. In some instances, the protease recognition site is cleaved *in vivo,* thereby releasing the CNP. In some instances, the protease recognition site is cleaved at the injection site, thereby releasing the CNP. In some instances, the protease recognition site is cleaved in the circulation, thereby releasing the CNP. In some instances, the protease recognition site is cleaved in the joint in proximity to the growth plate, thereby releasing the CNP where it is required and minimizing side effects. Protease recognition sites include those for Factor Xa (*e.g.* IEGR↓, IDGR↓, GR↓), thrombin (*e.g.* LVPR↓GS, LVPR↓GF), cathepsin (*e.g.* cathepsin K, RKPR↓G, RKLR↓G), matrix metalloprotease (MMP) (*e.g.* PLGL↓WAG "consensus" recognition sequence for MMP1, MMP2, MMP3, MMP7, MMP8, and MMP9 (Eckhard et al. (2016) Matrix Biology 49, 37-60). Analysis of the preferred cleavage site for human cathepsin K by Chou et al. (2006. J. Biol. Chem. 281, 12824-12832) suggests alternative cleavage sites that can be used, *e.g.* P3 could be K, G, H or M, P2 could be P, I or L, P1 could be Q, R or K.

In other aspects, the present disclosure provides methods for treating or preventing diseases including, but not limited to, diseases associated with regulation of bone growth (*e.g.,* skeletal dysplasias including dwarfism and Achondroplasia); juvenile idiopathic arthritis; osteoarthritis; cardiovascular diseases; diseases affecting cardiovascular homeostasis, diuresis, natriuresis, or vasodilation; diseases of the central nervous system, diseases of the reproductive tract; or diseases affecting the endothelium of blood vessels. The methods include administering a therapeutic agent including an ELP and a CNP (as described above) to a patient in need of such treatment. Generally, the patient may be a human or non-human animal patient (*e.g.,* dog, cat, cow, or horse). Preferably, the patient is human.

In some instances, treatment with a CNP therapeutic agent according to the present disclosure may also be combined with one or more pharmacologically active substances, **e.g.** selected from agents for the treatment and/or prevention of complications and disorders resulting from or associated with various diseases including, without limitation, diseases affecting regulation of bone growth (*e.g*., skeletal dysplasias including dwarfism and Achondroplasia); juvenile idiopathic arthritis; osteoarthritis; cardiovascular diseases; diseases affecting cardiovascular homeostasis, diuresis, natriuresis, or vasodilation; diseases of the central nervous system, diseases of the reproductive tract; or diseases affecting the endothelium of blood vessels.

### Active agent of the disclosure: Glucagon-Like Peptide (GLP)-1 Receptor Antagonists

Also described herein are instances wherein the therapeutic agent includes an ELP component fused or conjugated to a GLP-1 receptor antagonist, such as exendin-4 lacking amino acids 1-8, derivatives, analogs, mimetics, fragments, or functional variants thereof.

Human GLP-1 is a 37 amino acid residue peptide originating from preproglucagon which is synthesized in the L-cells in the distal ileum, in the pancreas, and in the brain. Processing of preproglucagon to give GLP-1 (7-36) amide, GLP-1 (7-37) and GLP-2 occurs mainly in the L-cells.

After processing in the intestinal L-cells, GLP-1 is released into the circulation, most notably in response to a meal. The plasma concentration of GLP-1 rises from a fasting level of approximately 15 pmol/L to a peak postprandial level of 40 pmol/L.

In some aspects, the protein active agents of the present invention are GLP-1 receptor antagonists. In some instances, the GLP-1 receptor antagonists are N-terminal and/or C-terminal fragments of GLP-1. In certain instances, the GLP-1 receptor antagonists include the amino acid sequence: DVSSYLEGQAAKEFIAWLVKGR (SEQ ID NOs: 54, 64, and 65), or fragments thereof. In some aspects, the GLP-1 receptor antagonist is GLP-1 (9-31) (SEQ ID NO: 54). In other aspects, the GLP-1 receptor antagonist is GLP-1 (9-29) (SEQ ID NO: 64). In yet other aspects, the GLP-1 receptor antagonist is GLP-1-exendin-4 (SEQ ID NO: 65).

In other instances, the GLP-1 receptor antagonists are N-terminal and/or C-terminal fragments of exendin-4. In certain instances, the GLP-1 receptor antagonists comprise the following amino acid sequence: DLSKQMEEEAVRLFIEWLKNGGP (SEQ ID NOs: 27, 28, 60, 61, 62, and 63), or a fragment thereof. In certain instances, the exendin-4 fragments include, but are not limited to, exendin-4 (9-39) (SEQ ID NO: 27), exendin-4 (9-31) (SEQ ID NO: 28), exendin-4 (9-30) (SEQ ID NO: 60), M-exendin-4 (9-39) (SEQ ID NO: 62), M-exendin-4 (9-31) (SEQ ID NO: 61), or M-exendin-4 (9-30) (SEQ ID NO: 63).

In other instances, the GLP-1 receptor antagonists include the amino acid sequence: DVSSYLEGQAAKEFIAWLVKGR (SEQ ID NOs: 66 and 67), or a fragment thereof. In some aspects, the GLP-1 receptor antagonist is Jant-4 (9-30) (SEQ ID NO: 66). In another aspect, the GLP-1 receptor antagonist is Jant-4 (9-39) (SEQ ID NO: 67).

In certain instances, the GLP-1 receptor antagonist is a mammalian GLP-1 receptor antagonist. In some instances, the GLP-1 receptor antagonist is a human GLP-1 receptor antagonist (*e.g*., SEQ ID NOs: 27, 28, 54, 56, 60, 61, 62, 63, 64, 65, 66, or 67). In some instances, the GLP-1 receptor antagonist is a functional analog of mammalian GLP-1 receptor antagonist, including functional fragments truncated at the N-terminus and/or C-terminus of a GLP-1 receptor antagonist by from about 1 to about 30 amino acids, including, for example, by up to about 3 amino acids, up to about 5 amino acids, up to about 10 amino acids, up to about 15 amino acids, up to about 20 amino acids, up to about 25 amino acids, or up to about 30 amino acids. In other instances, functional variants contain from about 1 to about 30 amino acid insertions, deletions, and/or substitutions with respect to a native or truncated sequence (*e.g.,* SEQ ID NOs: 27, 28, 54, 56, 60, 61, 62, 63, 64, 65, 66, or 67). For example, functional variants may have up to about 3 amino acid, up to about 5 amino acid, up to about 10 amino acid, up to about 15 amino acid, up to about 20 amino acid, up to about 25 amino acid, or up to about 30 amino acid insertions, deletions, and/or substitutions with respect to a native or truncated sequence (*e.g.,* SEQ ID NOs: 27, 28, 54, 56, 60, 61, 62, 63, 64, 65, 66, or 67). Protein activity may be confirmed or assayed using any available assay. In other instances, the GLP-1 receptor antagonist has an amino acid sequence with at least about 75% identity, about 80% identity, about 90% identity, about 95% identity, about 96% identity, about 97% identity, about 98% identity, or about 99% identity with a native or truncated sequence (*e.g.,* SEQ ID NOs: 27, 28, 54, 56, 60, 61, 62, 63, 64, 65, 66, or 67). Percentage identity can be calculated using the alignment program EMBOSS needle, available at http://www.ebi.ac.uk/Tools/psa/emboss_needle/. The following default parameters may be used for Pairwise alignment: Protein Weight Matrix = BLOSUM62; Gap Open = 10; Gap Extension = 0.1. In some instances, the GLP-1 receptor antagonist may contain additional chemical modifications known in the art.

In certain instances, the therapeutic agent includes an ELP fused to C-terminus of GLP-1 receptor antagonist, analogs, mimetics, derivatives, fragments, or functional variants thereof. In some instances, the therapeutic agent includes an ELP fused to the N-terminus of a human GLP-1 receptor antagonist (*e.g.,* SEQ ID NOs: 58 and 59). In some instances, the GLP-1 receptor antagonist is in a fusion protein with more than one ELP sequence. In some instances, the GLP-1 receptor antagonist has one or more ELPs at both the N- and C-termini. In some instances, the ELP at the C-terminus and/or the N-terminus of the GLP-1 receptor antagonist includes about 90 to about 180 repeating structural units. In other instances, the ELP at the C-terminus and/or the N-terminus of the GLP-1 receptor antagonist includes fewer than about 90 repeating structural units. In other aspects, the ELP at the C-terminus and/or the N-terminus of the GLP-1 receptor antagonist includes greater than about 180 repeating structural units. In some instances, the two or more ELPs at the N- and C- termini are approximately the same size. In other instances, the two or more ELPs at the N- and C- termini differ in size. In some instances, the ELP at the N-terminus of the GLP-1 receptor antagonist is larger than the ELP at the C-terminus of the GLP-1 receptor antagonist. In other instances, the ELP at the C-terminus of the GLP-1 receptor antagonist is larger than the ELP at the N-terminus of the GLP-1 receptor antagonist.

In other instances the GLP-1 receptor antagonist therapeutic agent is used to treat diseases including, but not limited to, diabetes (type 1 or 2); metabolic disease; obesity; diseases resulting from excessive insulin secretion including, but not limited to, hypoglycemia associated with hyperinsulinemia; postgastric surgery acquired hyperinsulinemic hypoglycemia; or hyperinsulinism, such as congenital hyperinsulinism or acquired hyperinsulinism following gastric surgery, for instance gastric surgery to treat obesity. The methods include administering a therapeutic agent comprising an ELP and a GLP-1 receptor antagonist (as described above) to a patient in need of such treatment. Generally, the patient may be a human or non-human animal patient (*e.g.,* dog, cat, cow, or horse). Preferably, the patient is human.

In some instances, the treatment with a GLP-1 receptor antagonist therapeutic agent according to the present disclosure may also be combined with one or more pharmacologically active substances, e.g. selected from agents for the treatment and/or prevention of various complications and disorders, resulting from or associated with diseases including, without limitation, diabetes (type 1 or 2); metabolic disease; obesity; diseases resulting from excessive insulin secretion including, but not limited to, hypoglycemia associated with hyperinsulinemia; postgastric surgery acquired hyperinsulinemic hypoglycemia; or hyperinsulinism, such as congenital hyperinsulinism or acquired hyperinsulinism following gastric surgery, for instance gastric surgery to treat obesity.

### Active agent of the disclosure: Hepcidin

An example of an active agent is Hepcidin (aka LEAP-1 (liver-expressed antimicrobial peptide)), derivatives, analogs, mimetics, fragments, or functional variants thereof. Hepcidin is a 8 kDa peptide hormone produced by hepatocytes in response to inflammation or to rising levels of iron in the blood.

A hepcidin cDNA encoding an 83 amino acid pre-propeptide in mice and an 84 amino acid pre-propeptide in rat and human was identified in a search for liver specific genes that were regulated by iron (Pigeon et al., 2001, J. Biol. Chem. 276:7811 (2001)). The 24 residue N-terminal signal peptide is first cleaved to produce pro-hepcidin, which is then further processed to produce mature hepcidin which is found in both blood and urine. In human urine, the predominant form of hepcidin contains 25 amino acids, although shorter 22 and 20 amino acid peptides are also present.

Human hepcidin is a 25-amino acid peptide (Hep25). (*See* Krause et al., 2000, FEBS Lett 480:147-150, and Park et al., 2001, J. Biol. Chem. 276:7806-7810). The structure of the bioactive 25-amino acid form of hepcidin is a simple hairpin with 8 cysteines that form 4 disulfide bonds (Jordan et al., 2009, J. Biol. Chem. 284:24155-67).

Hepcidin binds to its receptor, the iron export channel ferroportin, and causes its internalization and degradation, thereby reducing ferroportin mediated release of iron into the blood. Internalization further reduces iron transport across gut mucosa and uptake by liver and macrophages. Hepcidin expression is increased in acute and chronic inflammation which decreases iron availability for erythropoiesis. In certain instances, the hepcidin therapeutic agent includes an amino acid sequence providing sustained release fused or conjugated to hepcidin, fragments, or functional variants thereof. In some instances, the amino acid sequence providing sustained release is ELP.

In certain instances, the hepcidin is a mammalian hepcidin. In some instances, the mammalian hepcidin is a human hepcidin (SEQ ID NO: 75). In some instances, the mammalian hepcidin includes the N-terminal fragment of Hepcidin, DTHFPICIF (SEQ ID NO: 88) which is critical to activity. In certain instances, the present invention provides peptide fragments that mimic the hepcidin activity of Hep25, the bioactive human 25-amino acid form. Such peptides are referred to as "mini-hepcidins."

In some instances, the hepcidin is a functional variant of human hepcidin, including functional fragments truncated at the C-terminus of hepcidin by from about 1 to about 20 amino acids, including, for example, by up to about 3 amino acids, up to about 5 amino acids, up to about 10 amino acids, up to about 15 amino acids, or up to about 20 amino acids. In other instances, functional variants contain from about 1 to about 20 amino acid insertions, deletions, and/or substitutions at the C-terminus with respect to the native sequence (*e.g.,* SEQ ID NO: 75). For example, functional variants may have up to about 3 amino acid, up to about 5 amino acid, up to about 10 amino acid, up to about 15 amino acid, or up to about 20 amino acid insertions, deletions, and/or substitutions with respect to a native sequence (*e.g.,* SEQ ID NO: 75).

Protein activity may be confirmed or assayed using any available assay. As used herein, in some instances, a compound having "hepcidin activity" indicates the compound has the ability to lower plasma iron concentrations in subjects (*e.g.* mice or humans), when administered thereto (*e.g.* parenterally injected or orally administered), in a dose-dependent and time-dependent manner. (*See, e.g.,* as demonstrated in Rivera et al., 2005, Blood, 106: 2196-2199). In some instances, the peptides of the present invention have *in vitro* activity as assayed by the ability to cause the internalization and degradation of ferroportin in a ferroportin-expressing cell line as taught in Nemeth et al., 2006, Blood, 107: 328-333. *In vitro* activity may be measured for example by the dose-dependent loss of fluorescence of cells engineered to display ferroportin fused to green fluorescent protein as taught in Nemeth et al., 2006, Blood, 107: 328-33.

In some instances, the hepcidin has at least about 75% identity, about 80% identity, about 90% identity, about 95% identity, about 96% identity, about 97% identity, about 98% identity, or about 99% identity with a native sequence (*e.g.,* SEQ ID NO: 75). Percentage identity can be calculated using the alignment program EMBOSS needle, available at http://www.ebi.ac.uk/Tools/psa/emboss_needle/. The following default parameters may be used for Pairwise alignment: Protein Weight Matrix = BLOSUM62; Gap Open = 10; Gap Extension = 0.1. In some instances, the hepcidin may contain additional chemical modifications known in the art.

In certain instances, the therapeutic agent includes an ELP fused to the N-terminus or the C-terminus of hepcidin, fragments, or functional variants thereof. In some instances, the therapeutic agent includes an ELP fused to the N-terminus of human hepcidin (SEQ ID NO: 76). In other instances, the therapeutic agent includes an ELP fused to the C-terminus of human hepcidin (SEQ ID NO: 77).

In some instances, the hepcidin is in a fusion protein with more than one ELP sequence. In some instances, the hepcidin has one or more ELPs at both the N- and C-termini. In some instances, the ELP at the C-terminus and/or the N-terminus of the hepcidin includes about 90 to about 180 repeating structural units. In other instances, the ELP at the C-terminus and/or the N-terminus of the hepcidin includes fewer than about 90 repeating structural units. In other aspects, the ELP at the C-terminus and/or the N-terminus of the hepcidin includes greater than about 180 repeating structural units. In some instances, the two or more ELPs at the N- and C-termini are approximately the same size. In other instances, the two or more ELPs at the N- and C- termini differ in size. In some instances, the ELP at the N-terminus of the hepcidin is larger than the ELP at the C-terminus of the hepcidin peptide. In some instances, the ELP at the C-terminus of the hepcidin is larger than the ELP at the N-terminus of the hepcidin.

In other aspects, the present disclosure provides methods for treating or preventing intestinal diseases including, but not limited to, iron overload diseases such as hereditary hemochromatosis (HH) or iron-loading anemias; type 2 hemochromatosis; cardiac diseases such as myocardial siderosis or heart failure; diseases causing endocrine damage; liver diseases such as hepatic cirrhosis or hepatocellular carcinoma; diabetes; beta-thalassemia; acute severe infections; or polycythemia vera. The methods include administering a therapeutic agent including an ELP and a hepcidin (as described above) to a patient in need of such treatment. Generally, the patient may be a human or non-human animal patient (*e.g.,* dog, cat, cow, or horse). Preferably, the patient is human.

In some instances, treatment with a hepcidin therapeutic agent according to the present disclosure may also be combined with one or more pharmacologically active substances, *e.g.* selected from agents for the treatment and/or prevention of various complications and disorders, resulting from or associated with various diseases, including without limitation iron overload diseases such as hereditary hemochromatosis (HH) or iron-loading anemias; type 2 hemochromatosis; cardiac diseases such as myocardial siderosis or heart failure; diseases causing endocrine damage; liver diseases such as hepatic cirrhosis or hepatocellular carcinoma; diabetes; beta-thalassemia; acute severe infections; or polycythemia vera.

### Active agent of the disclosure: Insulin-like Growth Factor-1 (IGF-1)

An example of a protein active agent is IGF-1, derivatives, analogs, mimetics, fragments, or functional variants thereof. IGF-I is a single chain peptide present in plasma and other body fluids as well as many cells/tissues which comprises 70 amino acids, including 3 disulphide bonds, and stimulates proliferation of a wide range of cell types, and mediates some of the effects of growth hormone.

IGF-1 has both systemic and local effects and is mostly associated with different specific binding proteins, four of which are termed IGFBP1, IGFBP2, IGFBP3 and IGFBP4. These binding proteins modulate the biological functions and availability of IGF-1 in both a positive and negative manner.

IGF-1 acts mainly by interactions with the IGF-type 1 receptor exposed on the outer surface of plasma membranes in many different cell types. IGF-1R is expressed on many different cellular types and therefore several organism's tissues depend on the action of IGF-1: liver, kidneys, lungs, muscles, bone tissue and cartilage, as well as nerve tissue.

In certain instances, the IGF-1 therapeutic agent of the present disclosure includes an amino acid sequence providing sustained release fused or conjugated to IGF-1, fragments, or functional variants thereof. In some instances, the amino acid sequence providing sustained release is ELP.

In certain instances, the IGF-1 is a mammalian IGF-1. In some instances, the mammalian IGF-1 is a human IGF-1 (SEQ ID NO: 78). In some instances, the IGF-1 is a fragment or functional variant of human IGF-1, including functional fragments truncated at the N-terminus and/or C-terminus of IGF-1 by from about 1 to about 50 amino acids, including, for example, by up to about 3 amino acids, up to about 5 amino acids, up to about 10 amino acids, up to about 15 amino acids, up to about 20 amino acids, up to about 25 amino acids, up to about 30 amino acids, up to about 35 amino acids, up to about 40 amino acids, up to about 45 amino acids, or up to about 50 amino acids. In other instances, fragments or functional variants contain from about 1 to about 50 amino acid insertions, deletions, and/or substitutions with respect to a native sequence (e.g., SEQ ID NO: 78). For example, fragments or functional variants may have up to about 3 amino acid, up to about 5 amino acid, up to about 10 amino acid, up to about 15 amino acid, up to about 20 amino acid, up to about 25 amino acid, up to about 30 amino acid, up to about 35 amino acid, up to about 40 amino acid, up to about 45 amino acid, or up to about 50 amino acid insertions, deletions, and/or substitutions with respect to a native sequence (*e.g.,* SEQ ID NO: 78). Protein activity may be confirmed or assayed using any available assay. In other instances, the IGF-1 derivative, analog, mimetic, fragment, or functional variant thereof has at least about 75% identity, about 80% identity, about 90% identity, about 95% identity, about 96% identity, about 97% identity, about 98% identity, or about 99% identity with a native sequence (*e.g.,* SEQ ID NO: 78). Percentage identity can be calculated using the alignment program EMBOSS needle, available at http://www.ebi.ac.uk/Tools/psa/emboss_needle/. The following default parameters may be used for Pairwise alignment: Protein Weight Matrix = BLOSUM62; Gap Open = 10; Gap Extension = 0.1. In some instances, the IGF-1 derivative, analog, mimetic, fragment, or functional variant thereof may contain additional chemical modifications known in the art.

In certain instances, the therapeutic agent includes an ELP fused to the N-terminus or the C-terminus of IGF-1, derivatives, analogs, mimetics, fragments, or functional variants thereof. In certain instances, the therapeutic agent includes an ELP fused to the C-terminus of IGF-1 (SEQ ID NOs: 79 and 80).

In some instances, the IGF-1 derivative, analog, mimetic, fragment, or functional variant thereof is in a fusion protein with more than one ELP sequence. In some instances, the IGF-1 has one or more ELPs at both the N- and C-termini. In some instances, the ELP at the C-terminus and/or the N-terminus of the IGF-1 includes about 90 to about 180 repeating structural units. In other instances, the ELP at the C-terminus and/or the N-terminus of the IGF-1 includes fewer than about 90 repeating structural units. In other aspects, the ELP at the C-terminus and/or the N-terminus of the IGF-1 includes greater than about 180 repeating structural units. In some instances, the two or more ELPs at the N- and C- termini are approximately the same size. In other instances, the two or more ELPs at the N- and C- termini differ in size. In some instances, the ELP at the N-terminus of the IGF-1 is larger than the ELP at the C-terminus of the IGF-1. In other instances, the ELP at the C-terminus of the IGF-1 is larger than the ELP at the N-terminus of the IGF-1.

In other aspects, the present disclosure provides methods for treating or preventing disease including, but not limited to, primary IGF-1 deficiency; neuropathy; neurological diseases; cancer; kidney disease; liver disease; diseases of the lungs; diabetes; growth defects; treatment of children with GH gene deletion who have developed neutralizing antibodies to recombinant GH; Laron's syndrome, or Laron-type dwarfism; heart disease; or stroke. The methods include administering a therapeutic agent comprising an ELP and an IGF-1 (as described above) to a patient in need of such treatment. Generally, the patient may be a human or non-human animal patient (*e.g.,* dog, cat, cow, or horse). Preferably, the patient is human.

In some instances, treatment with an IGF-1 compound according to the present disclosure may also be combined chemotherapy and irradiation, as well as with one or more pharmacologically active substances, *e.g.* selected from agents for the treatment and/or prevention of complications and disorders resulting from or associated with various diseases, including, without limitation, primary IGF-1 deficiency; neuropathy; neuronal diseases; cancer; kidney disease; liver disease; diseases of the lungs; diabetes; growth defects; treatment of children with GH gene deletion who have developed neutralizing antibodies to recombinant GH; Laron's syndrome, or Laron-type dwarfism; heart disease; or stroke.

### Active agent of the disclosure: Urodilatin

An example of a protein active agent is urodilatin, derivatives, analogs, mimetics, fragments, or functional variants thereof. Urodilatin is a 32 amino acid peptide hormone which is a natriuretic peptide receptor A (NPR-A) agonist. Urodilatin is the renal isoform of atrial natriuretic peptide (ANP). It is formed by a differential processing of the ANP prohormone in the kidney, as opposed to all other tissues, where instead of 126 amino acid prohormone being cleaved between amino acid 98 and 99 to form ANP and kaliuretic peptide, the prohormone is cleaved between amino acid 95 and 96. The cleavage of the ANP prohormone in the kidney results in 4 amino acids from the C-terminal end of kaliuretic peptide (*i.e.,* threonine-alanine-proline-arginine) being attached to the N-terminus of ANP. Therefore, urodilatin is a 32-amino acid-containing peptide with the same structure as the 28-amino acid-containing ANP, except for the addition of four amino acids (TAPR) at the N-terminal extension.

Urodilatin is produced in physiological quantities in the kidney, differentially processed and secreted into the urine and forms the basis for a paracrine system regulating water and sodium reabsorption at the level of the collecting duct (Forssmann, W.-G. et al., 1998, Histochemistry and Cell Biology 110(4): 335-357). Urodilatin causes diuresis through increasing renal blood flow. It is secreted in response to increased mean arterial pressure and increased blood volume from the cells of the distal tubule and collecting duct. It is important in oliguric patients (such as those with acute renal failure and chronic renal failure) as it lowers serum creatinine and increases urine output.

Urodilatin is more potent than ANP and has a half-life twice as long as ANP, which may be due to the N-terminus of urodilatin being resistant to deactivation by neutral endopeptidase. In certain instances, the urodilatin therapeutic agent of the present disclosure includes an amino acid sequence providing sustained release fused or conjugated to urodilatin, fragments, or functional variants thereof. In some instances, the amino acid sequence providing sustained release is ELP.

In certain instances, the urodilatin is a mammalian urodilatin. In some instances, the mammalian urodilatin is human urodilatin (SEQ ID NO: 84). In some instances, the urodilatin is a derivative, analog, mimetic, fragment, or functional variant of mammalian urodilatin, including functional fragments truncated at the C-terminus of urodilatin by from about 1 to about 25 amino acids, including, for example, by including, for example, by up to about 3 amino acids, up to about 5 amino acids, up to about 10 amino acids, up to about 15 amino acids, up to about 20 amino acids, or up to about 25 amino acids. In other instances, the urodilatin, derivatives, analogs, mimetics, fragments, or functional variants contain from about 1 to about 25 amino acid insertions, deletions, and/or substitutions with respect to a native sequence (*e.g.,* SEQ ID NO: 84). For example, the urodilatin, derivatives, analogs, mimetics, fragments, or functional variants may have up to about 3 amino acid, up to about 5 amino acid, up to about 10 amino acid, up to about 15 amino acid, up to about 20 amino acid, or up to about 25 amino acid insertions, deletions, and/or substitutions with respect to a native sequence (*e.g.,* SEQ ID NO: 84). Protein activity may be confirmed or assayed using any available assay. In other instances, the urodilatin, derivatives, analogs, mimetics, fragments, or functional variants have at least about 75% identity, about 80% identity, about 90% identity, about 95% identity, about 96% identity, about 97% identity, about 98% identity, or about 99% identity with a native sequence (*e.g.,* SEQ ID NO: 84). Percentage identity can be calculated using the alignment program EMBOSS needle, available at http://www.ebi.ac.uk/Tools/psa/emboss_needle/. The following default parameters may be used for Pairwise alignment: Protein Weight Matrix = BLOSUM62; Gap Open = 10; Gap Extension = 0.1. In some instances, urodilatin, derivatives, analogs, mimetics, fragments, or functional variants may contain additional chemical modifications known in the art.

In certain instances, the therapeutic agent includes an ELP fused to the N-terminus or the C-terminus of a urodilatin, derivatives, analogs, mimetics, fragments, or functional variants thereof. In some instances, the therapeutic agent includes an ELP fused to the C-terminus of human urodilatin (SEQ ID NO: 85). In other instances, the therapeutic agent includes an ELP fused to the N-terminus of human urodilatin (SEQ ID NO: 86).

In some instances, the urodilatin, derivative, analog, mimetic, fragment, or functional variant is in a fusion protein with more than one ELP sequence. In some instances, the urodilatin, derivative, analog, mimetic, fragment, or functional variant has one or more ELPs at both the N- and C-termini. In some instances, the ELP at the C-terminus and/or the N-terminus of the urodilatin, derivative, analog, mimetic, fragment, or functional variant includes about 90 to about 180 repeating structural units. In other instances, the ELP at the C-terminus and/or the N-terminus of the urodilatin, derivative, analog, mimetic, fragment, or functional variant includes fewer than about 90 repeating structural units. In other aspects, the ELP at the C-terminus and/or the N-terminus of the urodilatin, derivative, analog, mimetic, fragment, or functional variant includes greater than about 180 repeating structural units. In some instances, the two or more ELPs at the N- and C- termini are approximately the same size. In other instances, the two or more ELPs at the N- and C- termini differ in size. In some instances, the ELP at the N-terminus of the urodilatin is larger than the ELP at the C-terminus of the urodilatin, derivative, analog, mimetic, fragment, or functional variant. In other instances, the ELP at the C-terminus of the urodilatin is larger than the ELP at the N-terminus of the urodilatin, derivative, analog, mimetic, fragment, or functional variant.

In other aspects, the present disclosure provides methods for treating or preventing diseases including, but not limited to, heart diseases such as acute heart failure (AHF); or kidney disease such as acute renal failure or chronic renal failure. The methods include administering a therapeutic agent including an ELP and a urodilatin (as described above) to a patient in need of such treatment. Generally, the patient may be a human or non-human animal patient (*e.g.,* dog, cat, cow, or horse). Preferably, the patient is human.

In some instances, treatment with a urodilatin according to the present disclosure may also be combined with one or more pharmacologically active substances, including, but not limited to, diuretics and agents for the treatment and/or prevention of complications and disorders resulting from or associated with various diseases including without limitation heart diseases such as AHF; or kidney disease such as acute renal failure or chronic renal failure.

### Active agent of the disclosure: Thymosin β4

An example of a protein active agent is Thymosin β4, derivatives, analogs, mimetics, fragments, or functional variants thereof. Thymosins refer to a family of biochemically and functionally distinct proteins that were originally identified from the thymus, and are now known to be present in many other tissues, and which have a variety of different physiological functions. More than 20 isoforms of β-tbymosin have been identified in different species, and among humans three β-thymosins have been identified including thymosin β4, thymosin β10, and thymosin β15, all of which share significant amino acid sequence homology. Despite this homology, each of these thymosin beta proteins is a distinct gene product with different functions.

Thymosin β4, the most abundant of the β-thymosins, is a highly conserved, watersoluble acidic polypeptide. Thymosin β4 was initially identified as a protein that is up-regulated during endothelial cell migration and differentiation *in vitro.* The mammalian gene encoding thymosin β-4 localizes to the X-chromosome. Human thymosin β-4, X-linked, has 44 amino acids, and escapes X-inactivation by being processed into a 43 amino acid peptide, with a molecular weight of 4.9 kDa, by removal of the first methionyl residue (Girardi, M., et al., 2003, Immunology 109: 1-7). Thymosin β-4 is localized to both the cytoplasm and the nucleus of cells. Thymosin β-4 is present in many tissues, and has multiple biological functions. It potently regulates actin polymerization, stimulates tissue remodeling, cell differentiation, and cell and tissue healing after injury, and is also involved in the expression of a number of inflammatory chemokines and cytokines.

In certain instances, the Thymosin β4 therapeutic agent of the present disclosure includes an amino acid sequence providing sustained release fused or conjugated to Thymosin β4, fragments, or functional variants thereof. In some instances, the amino acid sequence providing sustained release is ELP.

In certain instances, the Thymosin β4, derivative, analog, mimetic, fragment, or functional variant is a mammalian Thymosin β4. In some instances, the mammalian Thymosin β4 is human Thymosin β4 (SEQ ID NO: 89). In some instances, the Thymosin β4 is a derivative, analog, mimetic, fragment, or functional variant of mammalian Thymosin β4, including functional fragments truncated at the N-terminus and/or C-terminus of Thymosin β4 by from about 1 to about 35 amino acids, including, for example, by up to about 3 amino acids, up to about 5 amino acids, up to about 10 amino acids, up to about 15 amino acids, up to about 20 amino acids, up to about 25 amino acids, up to about 30 amino acids, or up to about 35 amino acids. In other instances, Thymosin β4, derivatives, analogs, mimetics, fragments, or functional variants contain from about 1 to about 35 amino acid insertions, deletions, and/or substitutions with respect to a native sequence (*e.g.*, SEQ ID NO: 89). For example, Thymosin β4, derivatives, analogs, mimetics, fragments, or functional variants may have up to about 3 amino acid, up to about 5 amino acid, up to about 10 amino acid, up to about 15 amino acid, up to about 20 amino acid, up to about 25 amino acid, up to about 30 amino acid, or up to about 35 amino acid insertions, deletions, and/or substitutions with respect to a native sequence (*e.g.,* SEQ ID NO: 89). Protein activity may be confirmed or assayed using any available assay. In other instances, the Thymosin β4, derivatives, analogs, mimetics, fragments, or functional variants have at least about 75% identity, about 80% identity, about 90% identity, about 95% identity, about 96% identity, about 97% identity, about 98% identity, or about 99% identity with a native sequence (*e.g.,* SEQ ID NO: 89). Percentage identity can be calculated using the alignment program EMBOSS needle, available at http://www.ebi.ac.uk/Tools/psa/emboss_needle/. The following default parameters may be used for Pairwise alignment: Protein Weight Matrix = BLOSUM62; Gap Open = 10; Gap Extension = 0.1. In some instances, the Thymosin β4, derivatives, analogs, mimetics, fragments, or functional variants may contain additional chemical modifications known in the art.

In certain instances, the therapeutic agent includes an ELP fused to the N-terminus or the C-terminus of Thymosin β4, derivatives, analogs, mimetics, fragments, or functional variants thereof. In some instances, the therapeutic agent includes an ELP fused to the C-terminus of Thymosin β4 (SEQ ID NO: 90).

In some instances, the Thymosin β4, derivative, analog, mimetic, fragment, or functional variant is in a fusion protein with more than one ELP sequence. In some instances, the Thymosin β4, derivative, analog, mimetic, fragment, or functional variant has one or more ELPs at both the N- and C-termini. In some instances, the ELP at the C-terminus and/or the N-terminus of the Thymosin β4, derivative, analog, mimetic, fragment, or functional variant includes about 90 to about 180 repeating structural units. In other instances, the ELP at the C-terminus and/or the N-terminus of the Thymosin β4, derivative, analog, mimetic, fragment, or functional variant includes fewer than about 90 repeating structural units. In other aspects, the ELP at the C-terminus and/or the N-terminus of the Thymosin β4, derivative, analog, mimetic, fragment, or functional variant includes greater than about 180 repeating structural units. In some instances, the two or more ELPs at the N- and C- termini are approximately the same size. In other instances, the two or more ELPs at the N- and C- termini differ in size. In some instances, the ELP at the N-terminus of the Thymosin β4, derivative, analog, mimetic, fragment, or functional variant is larger than the ELP at the C-terminus of Thymosin β4. In other instances, the ELP at the C-terminus of the Thymosin β4, derivative, analog, mimetic, fragment, or functional variant is larger than the ELP at the N-terminus.

In other aspects, the present disclosure provides methods for treating or preventing disease including, but not limited to, heart failure; pulmonary hypertension; ischemic heart disease; dry eye; or liver fibrosis. The methods include administering a therapeutic agent including an ELP and a Thymosin β4 (as described above) to a patient in need of such treatment. Generally, the patient may be a human or non-human animal patient (*e.g.,* dog, cat, cow, or horse). Preferably, the patient is human.

In some instances, treatment with a Thymosin β4 according to the present disclosure may also be combined with one or more pharmacologically active substances, *e.g.* selected from agents for the treatment and/or prevention of complications and disorders resulting from or associated with various diseases including, without limitation, heart failure; pulmonary hypertension; ischemic heart disease; dry eye; or liver fibrosis.

### Active agent of the disclosure: TRAIL

An example of a protein active agent is TRAIL (TNF-related apoptosis-inducing ligand), derivatives, analogs, mimetics, fragments, or functional variants thereof.

The extrinsic cell death pathway is triggered by ligand-receptor interactions that lead to intracellular signaling events, which ultimately result in the death of the target cell (apoptosis). One such ligand is a member of the tumor necrosis factor (TNF) superfamily, TRAIL or Apo2L (Wiley, S. R., et al., 1995, Immunity 3:673-682; Pitti, R. M., et al., 1996, J. Biol. Chem. 271: 12687-12690). TRAIL is a type II membrane protein of 281 amino acids. Its extracellular region comprises amino acid residues 114-281 and, upon cleavage by proteases, forms soluble sTRAIL molecule of 20 kDa size, which is also biologically active. Endogenous TRAIL exists as a homotrimer which is a critical requirement for its biological function. Activated T lymphocytes and NK cells express high levels of TRAIL.

TRAIL protein acts, for example, by binding to and activating pro-apoptotic TRAIL surface receptors 1 and 2 (TRAIL-R1/R2 or DR4 and DR5). Both TRAIL and sTRAIL are capable of triggering apoptosis via interaction with TRAIL receptors present on target cells.

In certain instances, the TRAIL therapeutic agent of the present disclosure includes an amino acid sequence providing sustained release fused or conjugated to TRAIL, fragments, or functional variants thereof. In some instances, the amino acid sequence providing sustained release is ELP.

In certain instances, the TRAIL is a mammalian TRAIL. In some instances, the mammalian TRAIL is human TRAIL (SEQ ID NO: 91). In some instances, the TRAIL may be a derivative, analog, mimetic, fragment, or functional variant thereof of mammalian TRAIL, including functional fragments truncated at the N-terminus and/or C-terminus of TRAIL by from about 1 to about 160 amino acids, including, for example, by up to about 3 amino acids, up to about 5 amino acids, up to about 10 amino acids, up to about 15 amino acids, up to about 20 amino acids, up to about 25 amino acids, up to about 30 amino acids, up to about 35 amino acids, up to about 40 amino acids, up to about 45 amino acids, up to about 50 amino acids, up to about 55 amino acids, up to about 60 amino acids, up to about 65 amino acids, up to about 70 amino acids, up to about 75 amino acids, up to about 80 amino acids, up to about 85 amino acids, up to about 90 amino acids, up to about 95 amino acids, up to about 100 amino acids, up to about 105 amino acids, up to about 110 amino acids, up to about 115 amino acids, up to about 120 amino acids, up to about 125 amino acids, up to about 130 amino acids, up to about 135 amino acids, up to about 140 amino acids, up to about 145 amino acids, up to about 150 amino acids, up to about 155 amino acids, or up to about 160 amino acids. TRAIL, derivatives, analogs, mimetics, fragments, or functional variants may contain from about 1 to about 160 amino acid insertions, deletions, and/or substitutions with respect to a native sequence (*e.g.,* SEQ ID NO: 91). For example, TRAIL, derivatives, analogs, mimetics, fragments, or functional variants may have up to about 3 amino acid, up to about 5 amino acid, up to about 10 amino acid, up to about 15 amino acid, up to about 20 amino acid, up to about 25 amino acid, up to about 30 amino acid, up to about 35 amino acid, up to about 40 amino acid, up to about 45 amino acid, up to about 50 amino acid, up to about 55 amino acid, up to about 60 amino acid, up to about 65 amino acid, up to about 70 amino acid, up to about 75 amino acid, up to about 80 amino acid, up to about 85 amino acid, up to about 90 amino acid, up to about 95 amino acid, up to about 100 amino acid, up to about 105 amino acid, up to about 110 amino acid, up to about 115 amino acid, up to about 120 amino acid, up to about 125 amino acid, up to about 130 amino acid, up to about 135 amino acid, up to about 140 amino acid, up to 1 about 45 amino acid, up to about 150 amino acid, up to about 155 amino acid, or up to about 160 amino acid insertions, deletions, and/or substitutions with respect to a native sequence (*e.g.,* SEQ ID NO: 91). Protein activity may be confirmed or assayed using any available assay. In other instances, the TRAIL, derivative, analog, mimetic, fragment, or functional variant has at least about 75% identity, about 80% identity, about 90% identity, about 95% identity, about 96% identity, about 97% identity, about 98% identity, or about 99% identity with a native sequence (*e.g.,* SEQ ID NO: 91). Percentage identity can be calculated using the alignment program EMBOSS needle, available at http://www.ebi.ac.uk/Tools/psa/emboss_needle/. The following default parameters may be used for Pairwise alignment: Protein Weight Matrix = BLOSUM62; Gap Open = 10; Gap Extension = 0.1. In some instances, the TRAIL, derivative, analog, mimetic, fragment, or functional variant may contain additional chemical modifications known in the art.

In certain instances, the therapeutic agent includes an ELP fused to the N-terminus or the C-terminus of the TRAIL, derivative, analog, mimetic, fragment, or functional variant thereof. In some instances, the therapeutic agent includes an ELP fused to the N-terminus of TRAIL (SEQ ID NO: 92).

In some instances, the TRAIL, derivative, analog, mimetic, fragment, or functional variant is in a fusion protein with more than one ELP sequence. In some instances, the TRAIL, derivative, analog, mimetic, fragment, or functional variant has one or more ELPs at both the N- and C-termini. In some instances, the ELP at the C-terminus and/or the N-terminus of the TRAIL, derivative, analog, mimetic, fragment, or functional variant includes about 90 to about 180 repeating structural units. In other instances, the ELP at the C-terminus and/or the N-terminus of the TRAIL, derivative, analog, mimetic, fragment, or functional variant includes fewer than about 90 repeating structural units. In other aspects, the ELP at the C-terminus and/or the N-terminus of the TRAIL, derivative, analog, mimetic, fragment, or functional variant includes greater than about 180 repeating structural units. In some instances, the two or more ELPs at the N- and C- termini are approximately the same size. In other instances, the two or more ELPs at the N- and C- termini differ in size. In some instances, the ELP at the N-terminus of the TRAIL, derivative, analog, mimetic, fragment, or functional variant is larger than the ELP at the C-terminus. In other instances, the ELP at the C-terminus of the TRAIL is larger than the ELP at the N-terminus.

In other aspects, the present disclosure provides methods for treating or preventing diseases including, but not limited to, tumorigenesis and various types of cancer; non-alcoholic fatty liver disease (NAFLD); nonalcoholic steatohepatitis (NASH); cirrhosis; post-transplant liver fibrosis or cirrhosis in post-orthotopic liver transplant (POLT) recipients as a result of recurrent hepatitis C virus (HCV) infection; pancreatic fibrosis. The methods include administering a therapeutic agent including an ELP and a TRAIL (as described above) to a patient in need of such treatment. Generally, the patient may be a human or non-human animal patient (*e.g.,* dog, cat, cow, or horse). Preferably, the patient is human.

In some instances, treatment with a TRAIL according to the present disclosure may also be combined with chemotherapy or radiation therapy. In other instances, the treatment with a TRAIL according to the present disclosure may also be combined with one or more pharmacologically active substances, *e.g.* selected from agents for the treatment and/or prevention of complications and disorders associated with various diseases, including without limitation tumorigenesis and various types of cancer; non-alcoholic fatty liver disease (NAFLD); nonalcoholic steatohepatitis (NASH); cirrhosis; post-transplant liver fibrosis or cirrhosis in post-orthotopic liver transplant (POLT) recipients as a result of recurrent hepatitis C virus (HCV) infection; pancreatic fibrosis.

### Active agent of the disclosure: Fibroblast growth factor 21 (FGF21)

Fibroblast growth factor 21 (FGF21) is a member of the FGF family which produces beneficial effects on lipid levels, body weight and glucose metabolism in animals. Overexpression of FGF21 in transgenic mice has been shown to result in reduced glucose and triglyceride levels, and resistance to diet-induced obesity. (Kharitonenkov et al. (2005), J. Clin. Invest. 115; 1627-1635). The administration of exogenous FGF21 to rodents and primates results in normalization of blood glucose levels, reduced triglyceride and cholesterol levels, improved glucose tolerance and improved insulin sensitivity (Kharitonenkov et al. (2007), Endocrinol. 48:774-781). FGF21 administration in experimental animal models has been shown to reduce body weight and body fat by increasing energy expenditure, physical activity, and metabolic rate. (Long and Kharitonenkov (2011) Biochim. Biophys. Acta 1812:791-795).

FGF21 signaling is mediated through its interaction with a receptor complex that includes β(Klotho (KLB) and one of three different FGF receptors (FGFR1c, FGFR2c or FGFR3c) (Ogawa et al. (2007), Proc. Natl. Acad. Sci. USA 104:7432-7437; Suzuki et al. (2008), Mol. Endocrinol. 22:1006-1014). It is believed that the main functional receptor for FGF21 signaling *in vivo* is the KLB/FGFRlc complex (this complex is referred to herein as "FGF21R").

Pharmacological activation of FGF21 signaling has been proposed for the treatment of various diseases and disorders in humans including type 2 diabetes, obesity, dyslipidemia, non-alcoholic fatty liver disease (nonalcoholic steatohepatitis, NASH), and other metabolic conditions (Gimeno and Moller (2014) Trends Endocrinol. Metab. 25, 303-311). An FGF analog, LY2405319 (Kharitonenkov et al. (2013) PLOS One, 8, e58575) was evaluated in a clinical trial in patients with type 2 diabetes and obesity (Gaich et al. (2013) Cell Metabolism 18, 333-340). Hecht et al. (2012, PLOS One, 7(11): e49345) developed a long-acting FGF21 analog fused to the Fc domain of human IgG1.

In certain instances, the FGF21 therapeutic agent of the present disclosure includes an amino acid sequence providing sustained release fused or conjugated to FGF21, fragments, or functional variants thereof. In some instances, the amino acid sequence providing sustained release is ELP.

In certain instances, the FGF21 is a mammalian FGF21. In some instances, the mammalian FGF21 is human FGF21 (SEQ ID NO: 110). In some instances, the FGF21 may be a derivative, analog, mimetic, fragment, or functional variant thereof of mammalian FGF21, including functional fragments truncated at the N-terminus and/or C-terminus of FGF21 by from about 1 to about 160 amino acids, including, for example, by up to about 3 amino acids, up to about 5 amino acids, up to about 10 amino acids, up to about 15 amino acids, up to about 20 amino acids, up to about 25 amino acids, up to about 30 amino acids, up to about 35 amino acids, up to about 40 amino acids, up to about 45 amino acids, up to about 50 amino acids, up to about 55 amino acids, up to about 60 amino acids, up to about 65 amino acids, up to about 70 amino acids, up to about 75 amino acids, up to about 80 amino acids, up to about 85 amino acids, up to about 90 amino acids, up to about 95 amino acids, up to about 100 amino acids, up to about 105 amino acids, up to about 110 amino acids, up to about 115 amino acids, up to about 120 amino acids, up to about 125 amino acids, up to about 130 amino acids, up to about 135 amino acids, up to about 140 amino acids, up to about 145 amino acids, up to about 150 amino acids, up to about 155 amino acids, or up to about 160 amino acids. FGF21, derivatives, analogs, mimetics, fragments, or functional variants may contain from about 1 to about 160 amino acid insertions, deletions, and/or substitutions with respect to a native sequence (*e.g*., SEQ ID NO: 110). For example, FGF21, derivatives, analogs, mimetics, fragments, or functional variants may have up to about 3 amino acid, up to about 5 amino acid, up to about 10 amino acid, up to about 15 amino acid, up to about 20 amino acid, up to about 25 amino acid, up to about 30 amino acid, up to about 35 amino acid, up to about 40 amino acid, up to about 45 amino acid, up to about 50 amino acid, up to about 55 amino acid, up to about 60 amino acid, up to about 65 amino acid, up to about 70 amino acid, up to about 75 amino acid, up to about 80 amino acid, up to about 85 amino acid, up to about 90 amino acid, up to about 95 amino acid, up to about 100 amino acid, up to about 105 amino acid, up to about 110 amino acid, up to about 115 amino acid, up to about 120 amino acid, up to about 125 amino acid, up to about 130 amino acid, up to about 135 amino acid, up to about 140 amino acid, up to 1 about 45 amino acid, up to about 150 amino acid, up to about 155 amino acid, or up to about 160 amino acid insertions, deletions, and/or substitutions with respect to a native sequence (*e.g.,* SEQ ID NO: 110). Protein activity may be confirmed or assayed using any available assay. In other instances, the FGF21, derivative, analog, mimetic, fragment, or functional variant has at least about 75% identity, about 80% identity, about 90% identity, about 95% identity, about 96% identity, about 97% identity, about 98% identity, or about 99% identity with a native sequence (*e.g.,* SEQ ID NO: 110). Percentage identity can be calculated using the alignment program EMBOSS needle, available at http://www.ebi.ac.uk/Tools/psa/emboss_needle/. The following default parameters may be used for Pairwise alignment: Protein Weight Matrix = BLOSUM62; Gap Open = 10; Gap Extension = 0.1. In some instances, the TRAIL, derivative, analog, mimetic, fragment, or functional variant may contain additional chemical modifications known in the art.

In certain instances, the therapeutic agent includes an ELP fused to the N-terminus or the C-terminus of the FGF21, derivative, analog, mimetic, fragment, or functional variant thereof. In some instances, the therapeutic agent includes an ELP fused to the N-terminus of FGF21 (SEQ ID NO: 110).

In some instances, the FGF21, derivative, analog, mimetic, fragment, or functional variant is in a fusion protein with more than one ELP sequence. In some instances, the FGF21, derivative, analog, mimetic, fragment, or functional variant has one or more ELPs at both the N-and C-termini. In some instances, the ELP at the C-terminus and/or the N-terminus of the FGF21, derivative, analog, mimetic, fragment, or functional variant includes about 90 to about 180 repeating structural units. In other instances, the ELP at the C-terminus and/or the N-terminus of the FGF21, derivative, analog, mimetic, fragment, or functional variant includes fewer than about 90 repeating structural units. In other aspects, the ELP at the C-terminus and/or the N-terminus of the FGF21, derivative, analog, mimetic, fragment, or functional variant includes greater than about 180 repeating structural units. In some instances, the two or more ELPs at the N- and C- termini are approximately the same size. In other instances, the two or more ELPs at the N- and C- termini differ in size. In some instances, the ELP at the N-terminus of the FGF21, derivative, analog, mimetic, fragment, or functional variant is larger than the ELP at the C-terminus. In other instances, the ELP at the C-terminus of the FGF21 is larger than the ELP at the N-terminus.

In some instances, the therapeutic agent includes an ELP fused to the N-terminus of the FGF21, derivative, analog, mimetic, fragment, or functional variant thereof and a GLP-1 agonist is fused to the N-terminus of the ELP (SEQ ID NOs: 133 and 134). Such a therapeutic agent combines the benefits of both FGF21 and GLP-1 activity for the treatment of diabetes, obesity and related disorders.

In other aspects, the present disclosure provides methods for treating or preventing diseases including, but not limited to, tumorigenesis and various types of cancer; non-alcoholic fatty liver disease (NAFLD); nonalcoholic steatohepatitis (NASH); cirrhosis; post-transplant liver fibrosis or cirrhosis in post-orthotopic liver transplant (POLT) recipients as a result of recurrent hepatitis C virus (HCV) infection; pancreatic fibrosis. The methods include administering a therapeutic agent including an ELP and an FGF21 (as described above) to a patient in need of such treatment. Generally, the patient may be a human or non-human animal patient (*e.g.,* dog, cat, cow, or horse). Preferably, the patient is human.

In some instances, treatment with a FGF21 according to the present disclosure may also be combined with one or more pharmacologically active substances, e.g. selected from agents for the treatment and/or prevention of complications and disorders associated with various diseases, including without limitation tumorigenesis and various types of cancer; non-alcoholic fatty liver disease (NAFLD); nonalcoholic steatohepatitis (NASH); cirrhosis; post-transplant liver fibrosis or cirrhosis in post-orthotopic liver transplant (POLT) recipients as a result of recurrent hepatitis C virus (HCV) infection; pancreatic fibrosis.

### FORMULATIONS

The present disclosure provides sustained release formulations including a therapeutic agent disclosed herein and one or more pharmaceutically acceptable excipients and/or diluents. For example, such excipients include salts, and other excipients that may act to stabilize hydrogen bonding. Any appropriate excipient known in the art may be used. Exemplary excipients include, but are not limited to, amino acids such as histidine, glycine, or arginine; glycerol; sugars, such as sucrose; surface active agents such as polysorbate 20 and polysorbate 80; citric acid; sodium citrate; antioxidants; salts including alkaline earth metal salts such as sodium, potassium, and calcium; counter ions such as chloride and phosphate; sugar alcohols (e.g. mannitol); preservatives; sugar alcohols (*e.g.* mannitol, sorbitol); and buffering agents. Exemplary salts include sodium chloride, potassium chloride, magnesium chloride, calcium chloride, sodium phosphate dibasic, sodium phosphate monobasic, sodium phosphate, and potassium phosphate.

The therapeutic agent is formulated at a pH, ionic strength, and generally with excipients sufficient to enable the formation of the matrix at body temperature (*e.g*., 37°C, or at from 34 to 36°C). The therapeutic agent is generally prepared such that it does not form the matrix at storage conditions. The formulation can be stored frozen, refrigerated or at room temperature. Storage conditions are generally less than the transition temperature of the formulation, such as less than about 32°C, or less than about 30°C, or less than about 27°C, or less than about 25°C, or less than about 20°C, or less than about 15°C. For example, the formulation may be isotonic with blood or have an ionic strength that mimics physiological conditions. For example, the formulation may have an ionic strength of at least that of 25 mM Sodium Chloride, or at least that of 30 mM Sodium chloride, or at least that of 40 mM Sodium Chloride, or at least that of 50 mM Sodium Chloride, or at least that of 75 mM Sodium Chloride, or at least that of 100 mM Sodium Chloride, or at least that of 150 mM Sodium Chloride. The formulation may have an ionic strength equivalent to that of 0.9% saline (154 mM sodium chloride).

In some instances, the formulation is stable at storage conditions. Storage conditions may be any conditions used to stably store a formulation. In some instances, the formulation is refrigerated. In some instances, the formulation is frozen. In some instances, the storage conditions include temperatures of less than about 30°C. In some instances, the storage conditions include temperatures of about 2°C to about 8°C. In some instances, the storage conditions include temperatures below 0°C. In some instances, the storage conditions include temperatures of about -15°C to about -80°C.

Stability can be measured using any appropriate means in the art. Generally, a stable formulation is one that shows less than a 5% increase in degradation products or impurities. In some instances, the formulation is stable for at least about 1 month, at least about 2 months, at least about 3 months, at least about 4 months, at least about 5 months, at least about 6 months, or at least about one year or more at the storage conditions. In some instances, the formulation is stable for at least about 1 month, at least about 2 months, at least about 3 months, at least about 4 months, at least about 5 months, at least about 6 months, at least about one year, or at least about two years or more at 2-8 °C. In some instances, the formulation is stable for at least about 1 month, at least about 2 months, at least about 3 months, at least about 4 months, at least about 5 months, at least about 6 months, at least about one year, or at least about two years or more at 25 °C. In some instances, the formulation is stable for at least about 1 month, at least about 2 months, at least about 3 months, at least about 4 months, at least about 5 months, at least about 6 months, at least about one year, or at least about two years or more at -15°C to about -80°C.

In some instances, the formulation includes two or more of calcium chloride, magnesium chloride, potassium chloride, potassium phosphate monobasic, sodium chloride, sodium phosphate dibasic, sodium phosphate monobasic, histidine, arginine, glycine, glycerol, antimicrobial preservative (*e.g.* metacresol), tonicity-adjusting agent (*e.g.* mannitol), glacial acetic acid, sodium acetate trihydrate; sucrose, sodium phosphate monobasic monohydrate, sodium phosphate dibasic heptahydrate, zinc, m-cresol, phenol, sorbitol, polysorbate 80, and polysorbate 20.

In some instances, the formulation includes histidine or another amino acid at a range of about 10 mM to about 100 mM histidine. In some instances, the formulation includes histidine or another amino acid at a range of about 10 mM to about 30 mM histidine. In some instances, the formulation includes histidine or another amino acid at a range of about 15 mM to about 25 mM histidine. In some instances, the formulation includes NaCl at a range of about 10 mM to about 165 mM NaCl. In some instances, the formulation includes between about 50 mM and about 165 mM NaCl. In some instances, the formulation includes between about 54 mM and about 162 mM NaCl. In some instances, the formulation includes between about 110 mM and about 162 mM NaCl. In some instances, the formulation includes sodium phosphate at a range of about 1 mM to about 20 mM. In some instances, the formulation includes sodium phosphate at a range of about 5 mM to about 15 mM. In some instances, the formulation includes sodium phosphate monobasic at a range of about 2mM to about 10mM. In some instances, the formulation includes sodium phosphate monobasic at a range of about 4 mM to about 8 mM. In some instances, the formulation includes sodium phosphate dibasic at a range of about 1mM to about 10 mM. In some instances, the formulation includes sodium phosphate dibasic at a range of about 2 mM to about 7 mM. In some instances, the formulation includes sodium phosphate dibasic at a range of about 2 mM to about 5 mM. In some instances, the formulation includes polysorbate 20 at a range of about 0.01% to about 0.2%. In some instances, the formulation includes polysorbate 80 at a range of about 0.01% to about 0.2%. In some instances, the formulation includes sodium phosphate, sodium chloride, sodium phosphate monobasic, sodium phosphate dibasic, and polysorbate 20. In some instances, the formulation includes about 10mM sodium phosphate (about 7mM sodium phosphate monobasic and about 3mM sodium phosphate dibasic), about 110mM sodium chloride, and about 0.1% polysorbate 20.

In some instances, the formulation is formulated at physiological pH. In some instances, the formulation is formulated at a pH in the range of about 5.5 to about 7.5. In some instances, the formulation is formulated at a pH in the range of about 6.0 to about 7.0. In some instances, the formulation is formulated at a pH in the range of about 6.5 to about 7.0. In some instances, formulations with a lower pH demonstrate improved formulation stability compared to formulations at a higher pH. In some instances, formulations with a pH of about 6.5 demonstrate improved stability compared to formulations with a pH of about 7.0. In some instances, formulations with a pH of about 6.0 demonstrate improved stability compared to formulations with a pH of about 6.5. In some instances, formulations with a lower pH maintain a higher percentage of monomers compared to formulations at a higher pH. In some instances, formulations with a pH of about 6.5 maintain a higher percentage of monomers compared to formulations with a pH of about 7.0. In some instances, formulations with a pH of about 6.0 maintain a higher percentage of monomers compared to formulations with a pH of about 6.5.

The protein concentration of the therapeutic agent in the formulation is tailored to drive the formation of the matrix at the temperature of administration. For example, higher protein concentrations help drive the formation of the matrix, and the protein concentration needed for this purpose varies depending on the ELP series used. For example, in instances using an ELP1-120, or amino acid sequences with comparable transition temperatures, the protein is present in the range of about 1 mg/mL to about 200 mg/mL, or is present in the range of about 30 mg/mL to about 150 mg/mL. In instances using an ELP4-120, or amino acid sequences with comparable transition temperatures, the protein is present in the range of about 0.005 mg/mL to about 10 mg/mL, or is present in the range of about 0.01 mg/mL to about 5 mg/mL.

In some instances, the therapeutic agent may be present in the range of about 0.5 mg/mL to about 200 mg/mL, or is present in the range of about 30 mg/mL to about 150 mg/mL. In some instances, the therapeutic agent is present in the range of about 50 mg/mL to about 125 mg/mL, or the range of about 75 mg/mL to about 110 mg/mL. In some instances, the therapeutic agent is present at a concentration of about 100 mg/mL.

In some aspects, the disclosure provides a method for delivering a sustained release regimen of an active agent disclosed herein. The method includes administering the pharmaceutical composition described herein to a subject in need, wherein the pharmaceutical composition is administered from about 1 to about 8 times per month. In some instances, the pharmaceutical composition is administered about 1 time, about 2 times, about 3 times, and/or about 4 times per month. In some instances, the pharmaceutical composition is administered weekly. In some instances, the pharmaceutical composition is administered daily. In some instances, the pharmaceutical composition is administered from one to three times weekly. In some instances, the pharmaceutical composition is administered once every two weeks. In some instances, the pharmaceutical composition is administered from one to two times a month. In particular instances, the pharmaceutical composition is administered about 1 time per month. In some instances, the pharmaceutical composition is administered about once every 2 months, about once every 3 months, about once every 4 months, about once every 5 months, and/or about once every 6 months. The pharmaceutical composition can be packaged in the form of pre-filled pens or syringes for administration once per week, twice per week, or from one to eight times per month, or alternatively filled in conventional vials and the like.

In some instances, the formulation is administered about monthly, and may be administered subcutaneously or intramuscularly. In some instances, the formulation is administered about weekly, and may be administered subcutaneously or intramuscularly. In some instances, the site of administration is not a pathological site, for example, is not the intended site of action.

In some instances, the pharmaceutical compositions disclosed herein are administered chronically. In some instances, the pharmaceutical compositions disclosed herein are administered for about 6 months, for about 7 months, for about 8 months, for about 9 months, for about 10 months, for about 11 months, for about 1 year, for about 2 years, for about 3 years, for about 4 years, for about 5 years, for about 10 years or more. The pharmaceutical compositions may be administered at any required dose and/or frequency disclosed herein.

In some instances, the pharmaceutical compositions disclosed herein are administered until disease or disorder symptoms improve. In some instances, the pharmaceutical compositions disclosed herein are administered until disease or disorder symptoms are ameliorated, delayed, and/or cured.

In some instances, the pharmaceutical compositions disclosed herein are administered before the patient begins to exhibit one or more disease or disorder symptoms. In some instances, the pharmaceutical compositions disclosed herein are administered at the onset of disease or disorder symptoms.

The therapeutic agent is formulated generally for "systemic delivery," meaning that the agent is not delivered locally to a pathological site or a site of action. Instead, the agent is absorbed into the bloodstream from the injection site, where the agent acts systemically or is transported to a site of action via the circulation. The therapeutic agent may be administered by any known route, such as for example, orally, intravenously, intramuscularly, nasally, subcutaneously, intra-vaginally, and intra-rectally. In some instances, the formulation is generally for subcutaneous administration. In some instances, the pharmacokinetic (PK) parameters are prolonged when the agent is administered subcutaneously. In some instances, the half-life of the fusion protein is prolonged. In some instances, the PK parameters when the agent is administered subcutaneously are prolonged compared with the agent administered by other means (e.g. intravenously). In some instances, the depot of the agent is prolonged when the agent is administered subcutaneously compared with the agent administered by other means (e.g. intravenously). By providing a slow absorption from the injection site, renal clearance and degradation can be controlled, thereby achieving the desired PK profile.

Advantageously, the compositions provide for prolonged pharmacokinetic exposure due to sustained release of the active agent. In particular aspects, the maximal exposure level may be achieved at about 10 hours, about 24 hours, about 48 hours or about 72 hours after administration; typically the maximum exposure level is achieved between about 10 hours and about 48 hours after administration. After the maximal exposure level is achieved the compositions may achieve a sustained rate of release whereby a substantial percentage of the maximal level is obtained for a period of time. For example, the sustained rate may about 50%, about 60%, about 70%, about 80%, about 90% or about 100% of the maximal exposure level. Exemplary periods of time for maintaining the sustained rate are about 3 days, about 4 days, about 5 days, about 6 days, about 1 week, about 2 weeks, about 4 weeks, about 6 weeks, or about 8 weeks, after the maximal exposure level is achieved. Subsequently, the sustained rate may lower to a reduced exposure rate. Such reduced exposure rates may be about 5%, about 10%, about 20%, about 30%, about 40%, about 50% or about 60% of the maximal exposure level. For example, in one embodiment (PE0256) a maximal exposure level of 1000 ng/mL is obtained within about 1-2 days. After this period, a sustained rate of about 70-100% of the maximal exposure level is maintained until about days 10-12 whereupon a reduced exposure rate from about 60% decreasing down to about 10% is obtained for the remainder of the study.

In various instances, the plasma concentration of the active agent does not change by more than a factor of about 20, or a factor of about 10, or a factor of about 5, or a factor of about 3 over the course of a plurality of administrations, such as at least 2, at least about 5, or at least about 10 administrations of the formulation. In some instances, the plasma concentration of the active agent does not change by more than a factor of about 20, or a factor of about 10, or a factor of about 5, or a factor of about 3 between each administration. In some instances, there is some accumulation until steady state is reached (*e.g*. after about 3 to about 4 administrations). The administrations are substantially evenly spaced, such as, for example, about daily, or about once per week, or from one to about five times per month, or about once every two months, or about once every three months. In other instances, the dose may be steadily increased over several administrations, so steady state is reached after 5 or more administrations.

The pharmaceutical compositions disclosed herein may be administered in smaller doses and/or less frequently than unfused or unconjugated counterparts. While one of skill in the art can determine the desirable dose in each case, a suitable dose of the therapeutic agent for achievement of therapeutic benefit, may, for example, be in a range of about 1 microgram (µg) to about 100 milligrams (mg) per kilogram body weight of the recipient per dose, preferably in a range of about 10 µg to about 50 mg per kilogram body weight per dose and most preferably in a range of about 10 µg to about 50 mg per kilogram body weight per dose. In some instances, the pharmaceutical composition is administered at a low dose. In some instances, the pharmaceutical composition is administered at a dose between 1 mg per kilogram per body weight per dose to about 9 mg per kilogram per body weight per dose. In some instances, the pharmaceutical composition is administered at about 1 mg per kilogram body weight per dose, about 3 mg per kilogram body weight per dose, and/or about 9 mg per kilogram body weight per dose. The desired dose may be presented as one dose or two or more sub-doses administered at appropriate intervals throughout the day. These sub-doses can be administered in unit dosage forms, for example, containing from about 10 µg to about 1000 mg, preferably from about 50 µg to about 500 mg, and most preferably from about 50 µg to about 250 mg of active ingredient per unit dosage form. Alternatively, if the condition of the recipient so requires, the doses may be administered as a continuous infusion.

In certain instances, the subject is a human, but in other instances may be a non-human mammal, such as a domesticated pet (*e.g.,* dog or cat), or livestock or farm animal (*e.g.,* horse, cow, sheep, or pig).

It should be understood that singular forms such as "a," "an," and "the" are used throughout this application for convenience, however, except where context or an explicit statement indicates otherwise, the singular forms are intended to include the plural. All numerical ranges should be understood to include each and every numerical point within the numerical range, and should be interpreted as reciting each and every numerical point individually. The endpoints of all ranges directed to the same component or property are inclusive, and intended to be independently combinable.

The term "about" when used in connection with a referenced numeric indication means the referenced numeric indication plus or minus up to 10% of that referenced numeric indication. For example, the language "about 50" covers the range of 45 to 55.

As used herein, the word "include," and its variants, is intended to be non-limiting, such that recitation of items in a list is not to the exclusion of other like items that may also be useful in the materials, compositions, devices, and methods of this technology. Similarly, the terms "can" and "may" and their variants are intended to be non-limiting, such that recitation that an embodiment can or may comprise certain elements or features does not exclude other embodiments of the present technology that do not contain those elements or features. Although the open-ended term "comprising," as a synonym of terms such as including, containing, or having, is used herein to describe and claim the disclosure, the present technology, or embodiments thereof, may alternatively be described using more limiting terms such as "consisting of' or "consisting essentially of' the recited ingredients.

As used herein, "half-life" (which generally refers to *in vivo* half-life or circulatory half-life) is the period of time that is required for a 50% diminution of bioactivity of the active agent to occur. In some instances, this term includes both prolonged exposure and a long half-life *(e.g.* both a slow uptake from the injection site and retardation of clearance compared to the unconjugated peptide).

Unless defined otherwise, all technical and scientific terms herein have the same meaning as commonly understood by one of ordinary skill in the art to which this disclosure belongs.

This disclosure is further illustrated by the following non-limiting examples.

### EXAMPLES

### Comparative Example 1 - Preparation of CNP ELP constructs

DNA encoding a 37 amino acid version of the CNP sequence was synthesized, digested with restriction enzymes *Bgl*I/*Eco*RI*,* and then sub-cloned into plasmid pPE0003 to provide plasmid pPE0493, placing the CNP sequence on the C-terminus of the ELP1-120 sequence.

DNA encoding a 37 amino acid version of the CNP sequence was synthesized, digested with restriction enzymes *Xba*I and *BsrGI* and then sub-cloned into plasmid pPE0003 cut with *Xba*I and *Acc65*I to provide plasmid pPE0531, placing the CNP sequence on the N-terminus of the ELP1-120 sequence.

DNA encoding a 37 amino acid version of the CNP sequence was synthesized to include a glycine and serine repeat linker (Gly-Gly-Ser-Gly-Gly-Ser). This was digested with restriction enzymes *Xba*I and *BsrGI* and then sub-cloned into plasmid pPE0003 cut with *Xba*I and *Acc65I* to provide plasmid pPE0552, placing the CNP sequence on the N-terminus of the ELP1-120 sequence with linker positioned between the two.

DNA encoding a 22 amino acid version of the CNP sequence was synthesized, digested with restriction enzymes *Bgl*I and *Eco*RI*,* and then sub-cloned into plasmid pPE0003 to provide plasmid pPE0514, placing the CNP sequence on the C-terminus of the ELP1-120 sequence.

DNA encoding a 22 amino acid version of the CNP sequence was synthesized, digested with restriction enzymes *Xba*I and *BsrGI* and then sub-cloned into plasmid pPE0003 cut with *Xba*I and *Acc65*I to provide plasmid pPE0550, placing the CNP sequence on the N-terminus of the ELP1-120 sequence.

DNA encoding a 22 amino acid version of the CNP sequence was synthesized which includes a glycine and serine repeat linker (Gly-Gly-Ser-Gly-Gly-Ser). This was digested with restriction enzymes *Xba*I and *BsrGI* and then sub-cloned into plasmid pPE0003 cut with *Xba*I and *Acc651* to provide plasmid pPE0565, placing the CNP sequence on the N-terminus of the ELP1-120 sequence with linker positioned between the two.

### Comparative Example 2 - Potency determination of CNP constructs

Potency of purified CNP constructs described in **Example 1** was demonstrated utilizing B-type natriuretic receptor (NPRB) expressing primary uterine fibroblast cells and a cGMP Fluorescent Assay Kit (CatchPoint, Molecular Devices, Sunnyvale, CA). When the receptor becomes activated, it causes generation of cGMP within the cells. These cells are lysed and the amount of cGMP is detected via a competitive immunoassay for cGMP. The cGMP in each sample competes with a horse radish peroxidase (HRP)-labeled cGMP conjugate for binding sites on the anti-cGMP antibodies. In the absence of cGMP, most of the conjugate is bound to the antibody. Increasing concentration of cGMP competitively decreases the amount of bound conjugate, decreasing measured HRP activity.

The day prior to the assay, primary uterine fibroblast cells were plated on a 96-well tissue culture plate and incubated overnight at 37°C, 5% CO₂. The following day, serial dilutions of CNP constructs were prepared in Dulbecco's phosphate-buffered saline (DPBS). The cells were rinsed with Krebs Ringer Bicarbonate Buffer (KRB) and then incubated at room temperature with 2mM 3-isobutyl-1-methylxanthine (IBMX) in KRB for 10 min to prevent the degradation of cGMP. The serial dilutions of samples were then added to the plate in duplicate and the plate was incubated at 37°C, 5% CO₂ for 40 min. Cell lysis buffer was then added to each well to lyse the cells and release the cGMP. These samples were then transferred to the cGMP assay plate. Both anti-cGMP antibody and HRP-cGMP were added to the assay plate and incubated at room temperature for 2 hr. The plate was then washed with cGMP wash buffer. Following the wash, stoplight red substrate was added to each well and the plate was incubated for 1 hr at room temperature. The plate was then read on a fluorescence plate reader with 530 nm excitation, 590 nm emission, and 570 nm cutoff.

The constructs in which the CNP was positioned at the N-terminus of the fusion protein were more potent than those in which the CNP was positioned at the C-terminus. The most potent construct was PE0552, which was approximately 30-fold less potent than the CNP peptide. See **Figure 1A-B****.**

The PE0552 construct was injected subcutaneously three times per week in normal mice (strain FVB/nj) for up to five weeks. The mice were 3-5 weeks of age at the start of the experiment. The effect on linear growth of the mice was determined by measuring tail length, nose to anal length and nose to tail length after each week of dosing in comparison to a control group injected with normal saline. **Figure 2A-C** shows that both dose levels of PE0552 resulted in a faster rate of linear growth.

### Comparative Example 3-Preparation of protease-cleavable CNP ELP constructs

Five different genes were synthesized each containing a 37 amino acid version of the CNP sequence and one of five different protease cleavage sites **(Table 4).**

| **Protease** | **Cleavage site** | **Construct** | **SEQ ID NOs:** |
|---|---|---|---|
| Factor Xa | Ile-Glu-Gly-Arg/ | PE9206 | 121 & 122 |
| Thrombin | Leu-Val-Pro-Arg / Gly-Ser | PE9216 | 123 & 124 |
| Cathepsin K site 1 | Arg-Lys-Pro-Arg / Gly | PE9306 | 125 & 126 |
| Cathepsin K site 2 | Arg-Lys-Leu-Arg / Gly | PE9316 | 127 & 128 |
| Matrix metalloprotease consensus | Pro-Leu-Gly / Leu-Trp-Ala-Gly | PE9326 | 129 & 130 |

Each gene sequence was digested with restriction enzymes *Xba*I/*Bsr*GI*,* and then sub-cloned into plasmid pPE0003 digested with *Xba*I/*Acc65*i to provide plasmids pPE9206, pPE9216, pPE9306, pPE9316, and pPE9326. These fusions place the CNP sequence on the N-terminus of the ELP1-120 sequence with the different protease-sensitive site between the two to enable cleavage to release the CNP sequence *in vivo.*

Potency of purified CNP constructs described in **Example 3** was demonstrated utilizing B-type natriuretic receptor (NPRB) expressing primary uterine fibroblast cell as described in **Example 2.** Pretreatment of the protease-sensitive CNP ELP constructs increased potency compared to untreated construct due to liberation of the CNP moiety.

The above constructs were injected daily in normal mice (strain FVB/nj) for three weeks. The mice were 3 weeks of age at the start of the experiment. The effect on linear growth of the mice was determined by measuring tail length, nose to anal length and nose to tail length after each week of dosing in comparison to a control group injected with normal saline. **Figure 3A-C** shows that only PE9306 resulted in a faster rate of linear growth. There was no significant effect on body weight.

Additional exemplary constructs were made comprising CNP53 at the N-terminus linked to ELP1-120 via a cathepsin K cleavage site linker (PE9446; SEQ ID NOs: 111 and 112), CNP22 at the N-terminus linked to ELP1-120 via a cathepsin K cleavage site linker (PE9456; SEQ ID NOs: 113 and 114), CNP37 at the C-terminus linked to ELP1-120 via a cathepsin K cleavage site linker (PE9486; SEQ ID NOs: 115 and 116), CNP53 at the C-terminus linked to ELP1-120 via a cathepsin K cleavage site linker (PE9496; SEQ ID NOs: 117 and 118), and CNP22 at the C-terminus linked to ELP1-120 via a cathepsin K cleavage site linker (PE9506; SEQ ID NOs: 119 and 120).

### Example 4 - Preparation of GLP-2 ELP construct

DNA encoding the GLP-2 sequence was synthesized to incorporate the A2G mutation for increased DPP-IV resistance. This was digested with restriction enzymes *Xba*I and *BsrGI* and then sub-cloned into plasmid pPE0003 cut with *Xba*I and *Acc65I* to provide plasmid pPE0503. The fusion places the GLP-2 sequence on the N-terminus of the ELP1-120 sequence..

Purified PE0503 protein was analysed in a cell-based cAMP potency assay and shown to have an EC₅₀ of 1.75 nM, compared to 0.24 nM for GLP-2 peptide.

### Example 5 - Evaluation of GLP-2 ELP construct in vivo

Male Sprague Dawley rats (200 - 220 g, n = 12/group) were injected subcutaneously with PE0503 or GLP-2 (A2G) peptide over an eleven-day dosing period as shown in **Table 5.**

**Table 5**

| Compound | Dosing frequency | Dosing days | Dose (mg/kg/dose) | Dose (nmol/kg/dose) |
|---|---|---|---|---|
| Saline | Daily | 1, 2, 3, 4, 5, 6, 7, 8, 9, 10, 11 | NA | NA |
| PE0503 | Daily | 1, 2, 3, 4, 5, 6, 7, 8, 9, 10, 11 | 1.3 | 25 |
| PE0503 | Every other day | 1, 3, 5, 7, 9, 11 | 5.1 | 100 |
| PE0503 | Every fourth day | 1,5,9 | 20.5 | 400 |
| GLP-1 (A2G) | Twice a day | 1, 2, 3, 4, 5, 6, 7, 8, 9, 10, 11 | 0.1 | 25 |

The animals were sacrificed on day 12 and a mid-line incision was made, the small intestines were removed, stretched to their maximum length and measured. The fecal material was then flushed from the lumen and the small intestine weight was recorded. **Figure 4** shows that treatment with PE0503 resulted in a highly significant increase in small intestine weight compared to placebo, even when dosed every fourth day (Q4D). Histological analysis of sections of the small intestine indicated a clear increase in villus height compared to vehicle (saline), as expected for a GLP-2 receptor agonist. Since the half-life is significantly longer in humans, the data verify that PE0503 is suitable for dosing once per week or less in humans.

### Comparative Example 6 - Preparation of FGF21 ELP constructs

The FGF21 gene sequence was synthesized, digested with restriction enzymes *Bgl*I/*Eco*RI*,* and then sub-cloned into plasmid pPE0003 to provide plasmid pPE9183 (SEQ ID NOs: 131 and 132), placing the FGF21 sequence on the C-terminus of the ELP1-120 sequence.

The FGF21 gene sequence was synthesized, digested with restriction enzymes *Bgl*I/*Eco*RI*,* and then sub-cloned into plasmid pPB1023 to provide plasmid pPE9193 (SEQ ID NOs: 133 and 134), placing the FGF21 sequence on the C-terminus of the ELP1-120 sequence. This creates a dual agonist with GLP-1 on one end of the ELP polymer and FGF21 on the other.

### REFERENCES

This application makes reference to the following publications and applications in their entireties: US 2001/0034050 A1; US 2009/0220455; US 8,334,257; US 2013/0310538; US 2013/0172274; US 2011/0236384; US 6,582,926; US 7,429,458; US 7,364,859; US 8,178,495; US 2013/0079277; US 2013/0085099; US 2013/0143802; US 2014/0024600; US 2011/0178017; US 7,709,227; US 2011/0123487; US 8,729,018; US 2014/0171370; US 2013/0150291; WO/2014/113434; US 2014/0213516, PCT/US2015/061955; and US Provisional Application Nos. 62/113,943, 62/145,770, and 62/150,679.

### REFERENCES

Amarante-Mendes and Griffith. Therapeutic applications of TRAIL receptor agonists in cancer and beyond. Pharm. & Thera. 155: 117-131 (2015).
Anker et al. Ularitide for the treatment of acute decompensated heart failure: from preclinical to clinical studies. European Heart J. 36: 715-723 (2015).
Barnes et al. Sustained Cardiovasuclar Actions of APJ Agonism During Renin-Angiotensin System Activation and in Patients with Heart Failure. Circ Heart Fail. 6:482-491 (2013).
Brame et al. Design, Characterization, and First-In-Human Study of the Vascular Actions of a Novel Biased Apelin Receptor Agonist. Hypertension 65:834-840 (2015).
Bukulmez et al. Protective Effects of C-Type natruiretic Peptide on Linear Growth and Articular Cartilage Integrity in a Mouse Model of Inlfammtory Arthritis. Arthritis & Rheum. 66(1):78-89 (2014).
Calabria et al. GLP-1 Receptor Antagonist Exendin-(9-39) Elevates Fasting Blood Glucose Levels in Congenital Hyperinsulinism Owing to Inactivating Mutations in the ATP-Sensitive K+ Channel. Diabetes 61:2585-2591 (2012).
Chou et al. Substrate Profiling of Cysteine Proteases Using a Combinatorial Peptide Library Identifies Functionally Unique Specificities. J. Biol. Chem. 281, 12824-12832 (2006)
Chua et al. Small cyclic agonists of iron regulatory hormone hepcidin. Bioorg. & Med. Chem. Ltr. 25:4961-4969 (2015).
Dalzell et al. The Emerging Potential of the Apelin-APJ System in Heart Failure. J. Card. Fail. 21:489-498 (2015).
De Leon et al. Exendin-(-39) Corrects Fasting Hypoglycemia in SUR-1-/- Mice by Lowering cAMP in Pancreatic β-cells and Inhibiting Insulin Secretion. J.Biol. Chem. 283:25786-25793 (2008).
Folino et al. Effects of apelin on the cardiovascular system. Heart Fail Rev. 20:505-518 (2015).
Gaich et al. The Effects of LY2405319, an FGF21 Analog, in Obese Human Subjects with Type 2 Diabetes. Cell Metabolism 18, 333-340 (2013).
Gimeno and Moller FGF21-based pharmacotherapy - potential utility for metabolic disorders. Trends Endocrinol. Metab. 25, 303-311 (2014).
Hecht et al. Rationale-Based Engineering of a Potent Long-Acting FGF21 Analog for the Treatment of Type 2 Diabetes. PLOS One, 7(11): e49345 (2012).
Jia et al. Cardiovascular effects of a PEGylated apelin. Peptides 38:181-188 (2012).
Kharitonenkov et al. FGF-21 as a novel metabolic regulator. J. Clin. Invest. 115; 1627-1635 (2005).
Kharitonenkov et al. The metabolic state of diabetic monkeys is regulated by fibroblast growth factor-21. Endocrinol. 48:774-781 (2007).
Kharitonenkov et al. Rational Design of a Fibroblast Growth Factor 21-Based Clinical Candidate, LY2405319. PLOS One, 8, e58575 (2013).
Lemke et al. Getting TRAIL back on track for cancer therapy. Cell Death & Diff. 21:1350-1364 (2014).
Long and Kharitonenkov Hormone-like fibroblast growth factors and metabolic regulation. Biochim. Biophys. Acta 1812:791-795 (2011).
Lord and De Leon. Monogenic hyperinsulinemic hypoglycemia: current insights into the pathogenesis and management. Int. J. Ped. Endocrin. 2013:3 (2013).
Lorget et al. Evaluation of the Therapeutic potential of a CNP analog in a Fgfr3 mouse model recapitulating achondroplasia. Am. J. Hum. Genet. 91:1108-1114 (2012).
Mitrovic et al. Effects of the renal natriuretic peptide urodilatin (ularitide) in patients with decompensated chronic heart failure: A double-blind, placebo-controlled, ascending-dose trial. Am. Heart J. 150:1239.e1-1239.e8 (2005).
Ogawa et al. BetaKlotho is required for metabolic activity of fibroblast growth factor 21. Proc. Natl. Acad. Sci. USA 104:7432-7437 (2007).
Oh et al. Systemic PEGylated TRAIL treatment ameliorates liver cirrhosis in rats by eliminating activated hepatic stellate cells. Hepatology. Dec 28. doi: 10.1002/hep.28432 (2015).
Peake et al. C-type natriuretic peptide signalling drives homeostatic effects in human chondrocytes. Biochem. & Biophys. Res. Comm. 465:784-789 (2015).
Preza et al. Minihepcidins are rationally designed small peptides that mimic hepcidin activity in mice and may be useful for the treatment of iron overload. J. Clin. Invest. 121:4880-4888 (2011).
Rochette et al. The iron-regulatory hormone hepcidin: A possible therapeutic target? Pharm. & Ther. 146:35-52 (2015).
Ruchala and Nemeth. The pathophysiology and pharmacology of hepcidin. Trends in Pharm. Sci. 35:155-161. (2014).
Salehi et al. Blockade of glucagon-like Peptide 1 receptor corrects postprandial hypoglycemia after gastric bypass. Gastroenterology 146:669-680 (2014).
Suzuki et al. betaKlotho is required for fibroblast growth factor (FGF) 21 signaling through FGF receptor (FGFR) 1c and FGFR3c. Mol. Endocrinol. 22:1006-1014 (2008).
Wendt et al. Neutral Endopeptidase-Resistant C-Type Natriuretic Peptide Variant Represents a New Therapeutic Approach for Treatment of Fibroblast Growth Factor Receptor 3-Related Dwarfism. J. Pharm. & Exp. Therap. 353:132-149 (2015).
Yang et al. Apelin, Elabela/Toddler, and biased agonists as novel therapeutic agents in the cardiovascular system. Trends Pharm. Sci. 36:560-567 (2015).
Yasoda et al. Systemic Administration of C-Type natriuretic Peptide as a Novel Therapeutic Strategy for Skeletal Dysplasias. Endocrinology. 150(7):3138-3144 (2009).
Yorifuji, Tohru. Congenital hyperinsulinism: current status and future perspectives. Ann Pediatr Endocrinol Metab.19:57-68 (2014).

## Claims

1. A sustained release pharmaceutical formulation comprising:
a therapeutic agent for systemic administration, the therapeutic agent comprising a protein active agent and at least 60 elastin-like peptide (ELP) structural units of SEQ ID NO: 3, and
wherein the protein active agent is a Glucagon-like peptide (GLP)-2 receptor agonist comprising the amino acid sequence of SEQ ID NO: 70 .

2. The pharmaceutical formulation of claim 1, wherein each X is selected from V, G, and A.

3. The pharmaceutical formulation of claim 1 or claim 2, wherein the formulation provides slow absorption from an injection site upon administration.

4. The pharmaceutical formulation of claim 1 or claim 2, wherein the formulation provides a flat PK profile upon administration, as compared to the PK profile for the active agent in the absence of the amino acid sequence forming a reversible matrix,
optionally wherein the PK profile has a low peak to trough (Cₘₐₓ to Cₘᵢₙ) and delayed or late Tₘₐₓ.

5. The pharmaceutical formulation of any one of claims 1 to 4, wherein a reversible matrix formed at body temperature reverses as protein concentration decreases.

6. The pharmaceutical formulation of claim 2, wherein the ratio of V:G:A is 5:3:2.

7. The pharmaceutical formulation of any one of claims 1 to 6, wherein the subject is human or a non-human mammal.

8. The pharmaceutical formulation of any one of the preceding claims, wherein the therapeutic agent is a recombinant fusion protein between the protein active agent and ELP.

9. The pharmaceutical formulation of any one of the preceding claims, wherein the protein active agent has a circulatory half-life in the range of from about 30 seconds to about 10 hours, or about 30 seconds to about 1 hour.

10. The pharmaceutical formulation of any one of claims 1 to 7 and 9, wherein the therapeutic agent is a chemical conjugate between the protein active agent and ELP.

11. The pharmaceutical formulation of any one of claims 1 to 10, wherein the therapeutic agent is present in the range of 0.5 mg/mL to 200 mg/mL, optionally 30 mg/mL to 150 mg/mL, 50 mg/mL to 125 mg/mL, or 75 mg/mL to 110 mg/mL, optionally wherein the therapeutic agent is present in the amount of 100 mg/mL.

12. The pharmaceutical composition of any one of claims 1 to 11, wherein the therapeutic agent does not form a phase-transitioned matrix at storage conditions.

13. The pharmaceutical composition of claim 12, wherein the storage conditions are less than 40°C, or less than 37°C, less than 30°C, less than 27°C, less than 25°C, less than 0°C, less than -15°C, or less than -60°C,
optionally wherein the formulation is stable for more than 1 month at the storage conditions, optionally wherein the formulation is stable for more than about 1 month at a temperature selected from the group consisting of:
a. 25°C;
b. 2°C;
c. 8°C;
d. -15°C; and
e. -80°C.

14. The pharmaceutical formulation of any of claims 1-13, wherein the formulation comprises two or more of calcium chloride, magnesium chloride, potassium chloride, potassium phosphate monobasic, sodium chloride, polysorbate 20, polysorbate 80, sodium phosphate, sodium phosphate monobasic, histidine, and sodium phosphate dibasic,
optionally wherein the formulation comprises sodium phosphate, sodium chloride and polysorbate 20,
optionally wherein the formulation comprises 10 mM sodium phosphate, 110 mM sodium chloride, and 0.1% polysorbate 20, or
wherein the formulation comprises sodium chloride and histidine.

15. The pharmaceutical formulation of any one of claims 1 to 14, wherein the formulation is packaged in the form of pre-dosed pens or syringes for administration about once per week, about twice per week, or from one to eight times per month.

16. The formulation of any one of claims 1 to 15 for use as a medicament,
optionally wherein the therapeutic agent is administered to a subject about 1 to about 8 times per month, optionally wherein the formulation is administered about weekly,
optionally wherein the formulation is administered subcutaneously or intramuscularly,
optionally wherein the site of administration is not a pathological site.

17. The pharmaceutical formulation of claim 1, wherein the therapeutic agent is a polypeptide comprising the amino acid sequence of SEQ ID NO: 71.

18. The pharmaceutical formulation of any one of claims 8-9 and 11-15 or the formulation for use according to claim 16, wherein the ELP is fused to the C terminus or the N terminus of the protein active agent.

## Patentansprüche

1. Pharmazeutische Formulierung zur verzögerten Freisetzung, umfassend:
einen therapeutischen Wirkstoff zur systemischen Verabreichung, wobei der therapeutische Wirkstoff einen Proteinwirkstoff und mindestens 60 Elastin-ähnliche Peptid (ELP)-Struktureinheiten von SEQ ID NR: 3 umfasst, und worin der Proteinwirkstoff ein Glucagon-ähnlicher Peptid (GLP)-2-Rezeptoragonist ist, der die Aminosäuresequenz SEQ ID NR: 70 umfasst.

2. Pharmazeutische Formulierung nach Anspruch 1, worin X jeweils ausgewählt ist aus V, G und A.

3. Pharmazeutische Formulierung nach Anspruch 1 oder Anspruch 2, worin die Formulierung eine langsame Absorption von einer Injektionsstelle nach der Verabreichung bereitstellt.

4. Pharmazeutische Formulierung nach Anspruch 1 oder Anspruch 2, worin die Formulierung ein flaches PK-Profil nach der Verabreichung bereitstellt, verglichen mit dem PK-Profil für den Wirkstoff, in Abwesenheit der Aminosäuresequenz, die eine reversible Matrix bildet, worin wahlweise das PK-Profil einen geringen Abstand vom Höchst- zum Tiefstwert (Cₘₐₓ zu Cₘᵢₙ) und eine verzögerte oder verspätete Tₘₐₓ aufweist.

5. Pharmazeutische Formulierung nach einem der Ansprüche 1 bis 4, worin eine reversible Matrix, die bei Körpertemperatur gebildet wird, sich mit abnehmender Proteinkonzentration zurückbildet.

6. Pharmazeutische Formulierung nach Anspruch 2, worin das Verhältnis V:G:A 5:3:2 beträgt.

7. Pharmazeutische Formulierung nach einem der Ansprüche 1 bis 6, worin das Subjekt ein Mensch oder ein nicht-humanes Säugetier ist.

8. Pharmazeutische Formulierung nach einem der vorhergehenden Ansprüche, worin der therapeutische Wirkstoff ein rekombinantes Fusionsprotein zwischen dem Proteinwirkstoff und ELP ist.

9. Pharmazeutische Formulierung nach einem der vorhergehenden Ansprüche, worin der Proteinwirkstoff eine Halbwertzeit im Kreislauf im Bereich von etwa 30 Sekunden bis etwa 10 Stunden oder etwa 30 Sekunden bis etwa 1 Stunde hat.

10. Pharmazeutische Formulierung nach einem der Ansprüche 1 bis 7 und 9, worin der therapeutische Wirkstoff ein chemisches Konjugat zwischen dem Proteinwirkstoff und ELP ist.

11. Pharmazeutische Formulierung nach einem der Ansprüche 1 bis 10, worin der therapeutische Wirkstoff im Bereich von 0,5 mg/ml bis 200 mg/ml, wahlweise von 30 mg/ml bis 150 mg/ml, 50 mg/ml bis 125 mg/ml oder 75 mg/ml bis 110 mg/ml vorhanden ist, worin wahlweise der therapeutische Wirkstoff in der Menge von 100 mg/ml vorhanden ist.

12. Pharmazeutische Zusammensetzung nach einem der Ansprüche 1 bis 11, worin der therapeutische Wirkstoff keine Phasen-überschreitende Matrix bei Lagerbedingungen bildet.

13. Pharmazeutische Zusammensetzung nach Anspruch 12, worin die Lagerbedingungen weniger als 40°C oder weniger als 37°C, weniger als 30°C, weniger als 27°C, weniger als 25°C, weniger als 0°C, weniger als -15°C oder weniger als -60°C betragen, worin wahlweise die Formulierung mehr als 1 Monat lang bei den Lagerbedingungen stabil ist, worin wahlweise die Formulierung mehr als 1 Monat lang bei einer Temperatur stabil ist, die ausgewählt ist aus der Gruppe, bestehend aus:
a. 25°C
b. 2°C
c. 8°C
d. -15°C; und
e. -80°C.

14. Pharmazeutische Formulierung nach einem der Ansprüche 1-13, worin die Formulierung zwei oder mehr von Calciumchlorid, Magnesiumchlorid, Kaliumchlorid, monobasischem Kaliumphosphat, Natriumchlorid, Polysorbat 20, Polysorbat 80, Natriumphosphat, monobasischem Natriumphosphat, Histidin und dibasischem Natriumphosphat, umfasst,
worin wahlweise die Formulierung Natriumphosphat, Natriumchlorid und Polysorbat 20 umfasst,
worin wahlweise die Formulierung 10 mM Natriumphosphat, 110 mM Natriumchlorid und 0,1% Polysorbat 20 umfasst, oder worin die Formulierung Natriumchlorid und Histidin umfasst.

15. Pharmazeutische Formulierung nach einem der Ansprüche 1 bis 14, worin die Formulierung in der Form von vordosierten Pens oder Spritzen zur Verabreichung etwa einmal pro Woche, etwa zweimal pro Woche oder von einmal bis achtmal pro Monat abgepackt ist.

16. Formulierung nach einem der Ansprüche 1 bis 15 zur Anwendung als Medikament,
worin wahlweise der therapeutische Wirkstoff an ein Subjekt etwa 1 bis etwa 8 mal pro Monat verabreicht wird, worin wahlweise die Formulierung etwa wöchentlich verabreicht wird, worin wahlweise die Formulierung subkutan oder intramuskulär verabreicht wird, worin wahlweise die Verabreichungsstelle keine pathologische Stelle ist.

17. Pharmazeutische Formulierung nach Anspruch 1, worin der therapeutische Wirkstoff ein Polypeptid ist, das die Aminosäuresequenz SEQ ID NR: 71 umfasst.

18. Pharmazeutische Formulierung nach einem der Ansprüche 8-9 und 11-15 oder Formulierung zur Anwendung nach Anspruch 16, worin das ELP mit dem C-Terminus oder dem N-Terminus des Proteinwirkstoffs fusioniert ist.

## Revendications

1. Formulation pharmaceutique à libération prolongée, comprenant :
un agent thérapeutique pour administration systémique, l'agent thérapeutique comprenant un agent protéique actif et au moins 60 unités structurelles de peptide de type élastine (ELP, elastin-like peptide) de SEQ ID N° 3, et
dans laquelle l'agent protéique actif est un agoniste du récepteur du peptide de type glucagon (GLP)-2 comprenant la séquence d'acides aminés de SEQ ID N° 70.

2. Formulation pharmaceutique selon la revendication 1, dans laquelle chaque X est choisi parmi V, G et A.

3. Formulation pharmaceutique selon la revendication 1 ou la revendication 2, dans laquelle la formulation assure une absorption lente à partir d'un site d'injection lors de l'administration.

4. Formulation pharmaceutique selon la revendication 1 ou la revendication 2, dans laquelle la formulation assure un profil PK plat lors de l'administration, comparé au profil PK pour l'agent actif en l'absence de la séquence d'acides aminés formant une matrice réversible,
facultativement dans laquelle le profil PK a un pic à creux (Cₘₐₓ à Cₘᵢₙ) faible et un Tₘₐₓ différé ou tardif.

5. Formulation pharmaceutique selon l'une quelconque des revendications 1 à 4, dans laquelle une matrice réversible formée à la température du corps s'inverse à mesure que la concentration en protéine diminue.

6. Formulation pharmaceutique selon la revendication 2, dans laquelle le ratio V:G:A est égal à 5:3:2.

7. Formulation pharmaceutique selon l'une quelconque des revendications 1 à 6, dans laquelle le sujet est un être humain ou un mammifère non humain.

8. Formulation pharmaceutique selon l'une quelconque des revendications précédentes, dans laquelle l'agent thérapeutique est une protéine de fusion recombinante entre l'agent protéique actif et l'ELP.

9. Formulation pharmaceutique selon l'une quelconque des revendications précédentes, dans laquelle l'agent protéique actif a une demi-vie circulatoire dans la plage allant d'environ 30 secondes à environ 10 heures ou d'environ 30 secondes à environ 1 heure.

10. Formulation pharmaceutique selon l'une quelconque des revendications 1 à 7 et 9, dans laquelle l'agent thérapeutique est un conjugué chimique entre l'agent protéique actif et l'ELP.

11. Formulation pharmaceutique selon l'une quelconque des revendications 1 à 10, dans laquelle l'agent thérapeutique est présent dans la plage de 0,5 mg/ml à 200 mg/ml, facultativement de 30 mg/ml à 150 mg/ml, de 50 mg/ml à 125 mg/ml ou de 75 mg/ml à 110 mg/ml, facultativement dans laquelle l'agent thérapeutique est présent en une quantité de 100 mg/ml.

12. Composition pharmaceutique selon l'une quelconque des revendications 1 à 11, dans laquelle l'agent thérapeutique ne forme pas de matrice ayant subi une transition de phase dans les conditions de stockage.

13. Composition pharmaceutique selon la revendication 12, dans laquelle les conditions de stockage sont inférieures à 40 °C, ou inférieures à 37 °C, inférieures à 30 °C, inférieures à 27 °C, inférieures à 25 °C, inférieures à 0 °C, inférieures à -15 °C ou inférieures à -60 °C,
facultativement dans laquelle la formulation est stable pendant plus de 1 mois dans les conditions de stockage, facultativement dans laquelle la formulation est stable pendant plus d'environ 1 mois à une température choisie dans le groupe constitué par :
a) 25 °C ;
b) 2 °C ;
c) 8 °C ;
d) -15 °C ; et
e) -80 °C.

14. Formulation pharmaceutique selon l'une quelconque des revendications 1 à 13, dans laquelle la formulation comprend deux ou plusieurs parmi le chlorure de calcium, le chlorure de magnésium, le chlorure de potassium, le phosphate de potassium monobasique, le chlorure de sodium, le polysorbate 20, le polysorbate 80, le phosphate de sodium, le phosphate de sodium monobasique, l'histidine et le phosphate de sodium dibasique,
facultativement dans laquelle la formulation comprend du phosphate de sodium, du chlorure de sodium et du polysorbate 20,
facultativement dans laquelle la formulation comprend 10 mM de phosphate de sodium, 110 mM de chlorure de sodium et 0,1 % de polysorbate 20, ou
dans laquelle la formulation comprend du chlorure de sodium et de l'histidine.

15. Formulation pharmaceutique selon l'une quelconque des revendications 1 à 14, dans laquelle la formulation est conditionnée sous la forme de stylos ou seringues prédosés pour l'administration environ une fois par semaine, environ deux fois par semaine, ou d'une à huit fois par mois.

16. Formulation selon l'une quelconque des revendications 1 à 15 pour une utilisation comme médicament,
facultativement dans laquelle l'agent thérapeutique est administré à un sujet environ 1 à environ 8 fois par mois, facultativement dans laquelle la formulation est administrée environ une fois par semaine,
facultativement dans laquelle la formulation est administrée par voie sous-cutanée ou intramusculaire,
facultativement dans laquelle le site d'administration n'est pas un site pathologique.

17. Formulation pharmaceutique selon la revendication 1, dans laquelle l'agent thérapeutique est un polypeptide comprenant la séquence d'acides aminés de SEQ ID N° 71.

18. Formulation pharmaceutique selon l'une quelconque des revendications 8 à 9 et 11 à 15 ou formulation pour une utilisation selon la revendication 16, dans laquelle l'ELP est fusionné à la terminaison C ou à la terminaison N de l'agent protéique actif.


### SEQUENCE LISTING

<110> PhaseBio Pharmaceuticals, Inc. Jowett, James Ballance, David James
<120> ELP FUSION PROTEINS FOR CONTROLLED AND SUSTAINED RELEASE
<130> PHAS-033/02WO 309646-2248
<140> PCT/US2017/030403
   <141> 2017-05-01
<150> US 62/442,057
   <151> 2017-01-04
<150> US 62/332,803
   <151> 2016-05-06
<160> 134
<170> PatentIn version 3.5
<210> 1
   <211> 4
   <212> PRT
   <213> Artificial Sequence
<220>
   <223> ELP component sequence
<400> 1
   Val Pro Gly Gly
   1
<210> 2
   <211> 4
   <212> PRT
   <213> Artificial Sequence
<220>
   <223> ELP component sequence
<400> 2
   Ile Pro Gly Gly
   1
<210> 3
   <211> 5
   <212> PRT
   <213> Artificial Sequence
<220>
   <223> ELP component sequence
<220>
   <221> misc_feature
   <222> (4)..(4)
   <223> Xaa can be any naturally occurring or non-natural amino acid
<400> 3
   Val Pro Gly Xaa Gly
   1               5
<210> 4
   <211> 5
   <212> PRT
   <213> Artificial Sequence
<220>
   <223> ELP component sequence
<400> 4
   Ala Val Gly Val Pro
   1               5
<210> 5
   <211> 5
   <212> PRT
   <213> Artificial Sequence
<220>
   <223> ELP component sequence
<220>
   <221> misc_feature
   <222> (4)..(4)
   <223> Xaa can be any naturally occurring or non-natural amino acid
<400> 5
   Ile Pro Gly Xaa Gly
   1               5
<210> 6
   <211> 5
   <212> PRT
   <213> Artificial Sequence
<220>
   <223> ELP component sequence
<400> 6
   Ile Pro Gly Val Gly
   1               5
<210> 7
   <211> 5
   <212> PRT
   <213> Artificial Sequence
<220>
   <223> ELP component sequence
<220>
   <221> misc_feature
   <222> (4)..(4)
   <223> Xaa can be any naturally occurring or non-natural amino acid
<400> 7
   Leu Pro Gly Xaa Gly
   1               5
<210> 8
   <211> 5
   <212> PRT
   <213> Artificial Sequence
<220>
   <223> ELP component sequence
<400> 8
   Leu Pro Gly Val Gly
   1               5
<210> 9
   <211> 6
   <212> PRT
   <213> Artificial Sequence
<220>
   <223> ELP component sequence
<400> 9
   Val Ala Pro Gly Val Gly
   1               5
<210> 10
   <211> 8
   <212> PRT
   <213> Artificial Sequence
<220>
   <223> ELP component sequence
<400> 10
   Gly Val Gly Val Pro Gly Val Gly
   1               5
<210> 11
   <211> 9
   <212> PRT
   <213> Artificial Sequence
<220>
   <223> ELP component sequence
<400> 11
   Val Pro Gly Phe Gly Val Gly Ala Gly
   1 5
<210> 12
   <211> 9
   <212> PRT
   <213> Artificial Sequence
<220>
   <223> ELP component sequence
<400> 12
   Val Pro Gly Val Gly Val Pro Gly Gly
   1 5
<210> 13
   <211> 5
   <212> PRT
   <213> Artificial Sequence
<220>
   <223> ELP component sequence
<220>
   <221> misc_feature
   <222> (1)..(1)
   <223> Xaa can be any naturally occurring or non-natural amino acid
<400> 13
   Xaa Pro Gly Val Gly
   1 5
<210> 14
   <211> 600
   <212> PRT
   <213> Artificial Sequence
<220>
   <223> ELP1-120
<400> 14
   Val Pro Gly Val Gly Val Pro Gly Val Gly Val Pro Gly Gly Gly Val
   1 5 10 15
   Pro Gly Ala Gly Val Pro Gly Val Gly Val Pro Gly Val Gly Val Pro
   20 25 30
   Gly Val Gly Val Pro Gly Gly Gly Val Pro Gly Ala Gly Val Pro Gly
   35 40 45
   Gly Gly Val Pro Gly Val Gly Val Pro Gly Val Gly Val Pro Gly Gly
       50                  55                  60
   Gly Val Pro Gly Ala Gly Val Pro Gly Val Gly Val Pro Gly Val Gly
   65                  70                  75                  80
   Val Pro Gly Val Gly Val Pro Gly Gly Gly Val Pro Gly Ala Gly Val
                   85                  90                  95
   Pro Gly Gly Gly Val Pro Gly Val Gly Val Pro Gly Val Gly Val Pro
               100                 105                 110
   Gly Gly Gly Val Pro Gly Ala Gly Val Pro Gly Val Gly Val Pro Gly
           115                 120                 125
   Val Gly Val Pro Gly Val Gly Val Pro Gly Gly Gly Val Pro Gly Ala
       130                 135                 140
   Gly Val Pro Gly Gly Gly Val Pro Gly Val Gly Val Pro Gly Val Gly
   145                 150                 155                 160
   Val Pro Gly Gly Gly Val Pro Gly Ala Gly Val Pro Gly Val Gly Val
                   165                 170                 175
   Pro Gly Val Gly Val Pro Gly Val Gly Val Pro Gly Gly Gly Val Pro
               180                 185                 190
   Gly Ala Gly Val Pro Gly Gly Gly Val Pro Gly Val Gly Val Pro Gly
           195                 200                 205
   Val Gly Val Pro Gly Gly Gly Val Pro Gly Ala Gly Val Pro Gly Val
       210                 215                 220
   Gly Val Pro Gly Val Gly Val Pro Gly Val Gly Val Pro Gly Gly Gly
   225                 230                 235                 240
   Val Pro Gly Ala Gly Val Pro Gly Gly Gly Val Pro Gly Val Gly Val
                   245                 250                 255
   Pro Gly Val Gly Val Pro Gly Gly Gly Val Pro Gly Ala Gly Val Pro
               260                 265                 270
   Gly Val Gly Val Pro Gly Val Gly Val Pro Gly Val Gly Val Pro Gly
           275                 280                 285
   Gly Gly Val Pro Gly Ala Gly Val Pro Gly Gly Gly Val Pro Gly Val
       290                 295                 300
   Gly Val Pro Gly Val Gly Val Pro Gly Gly Gly Val Pro Gly Ala Gly
   305                 310                 315                 320
   Val Pro Gly Val Gly Val Pro Gly Val Gly Val Pro Gly Val Gly Val
                   325                 330                 335
   Pro Gly Gly Gly Val Pro Gly Ala Gly Val Pro Gly Gly Gly Val Pro
               340                 345                 350
   Gly Val Gly Val Pro Gly Val Gly Val Pro Gly Gly Gly Val Pro Gly
           355                 360                 365
   Ala Gly Val Pro Gly Val Gly Val Pro Gly Val Gly Val Pro Gly Val
       370                 375                 380
   Gly Val Pro Gly Gly Gly Val Pro Gly Ala Gly Val Pro Gly Gly Gly
   385                 390                 395                 400
   Val Pro Gly Val Gly Val Pro Gly Val Gly Val Pro Gly Gly Gly Val
                   405                 410                 415
   Pro Gly Ala Gly Val Pro Gly Val Gly Val Pro Gly Val Gly Val Pro
               420                 425                 430
   Gly Val Gly Val Pro Gly Gly Gly Val Pro Gly Ala Gly Val Pro Gly
           435                 440                 445
   Gly Gly Val Pro Gly Val Gly Val Pro Gly Val Gly Val Pro Gly Gly
       450                 455                 460
   Gly Val Pro Gly Ala Gly Val Pro Gly Val Gly Val Pro Gly Val Gly
   465                 470                 475                 480
   Val Pro Gly Val Gly Val Pro Gly Gly Gly Val Pro Gly Ala Gly Val
                   485                 490                 495
   Pro Gly Gly Gly Val Pro Gly Val Gly Val Pro Gly Val Gly Val Pro
               500                 505                 510
   Gly Gly Gly Val Pro Gly Ala Gly Val Pro Gly Val Gly Val Pro Gly
           515                 520                 525
   Val Gly Val Pro Gly Val Gly Val Pro Gly Gly Gly Val Pro Gly Ala
       530                 535                 540
   Gly Val Pro Gly Gly Gly Val Pro Gly Val Gly Val Pro Gly Val Gly
   545                 550                 555                 560
   Val Pro Gly Gly Gly Val Pro Gly Ala Gly Val Pro Gly Val Gly Val
   565 570 575
   Pro Gly Val Gly Val Pro Gly Val Gly Val Pro Gly Gly Gly Val Pro
   580 585 590
   Gly Ala Gly Val Pro Gly Gly Gly
   595 600
<210> 15
   <211> 155
   <212> PRT
   <213> Artificial Sequence
<220>
   <223> ELP1-30
<400> 15
   Val Pro Gly Val Gly Val Pro Gly Val Gly Val Pro Gly Gly Gly Val
   1 5 10 15
   Pro Gly Ala Gly Val Pro Gly Val Gly Val Pro Gly Val Gly Val Pro
   20 25 30
   Gly Val Gly Val Pro Gly Gly Gly Val Pro Gly Ala Gly Val Pro Gly
   35 40 45
   Gly Gly Val Pro Gly Val Gly Val Pro Gly Val Gly Val Pro Gly Gly
   50 55 60
   Gly Val Pro Gly Ala Gly Val Pro Gly Val Gly Val Pro Gly Val Gly
   65 70 75 80
   Val Pro Gly Val Gly Val Pro Gly Gly Gly Val Pro Gly Ala Gly Val
   85 90 95
   Pro Gly Gly Gly Val Pro Gly Val Gly Val Pro Gly Val Gly Val Pro
   100 105 110
   Gly Gly Gly Val Pro Gly Ala Gly Val Pro Gly Val Gly Val Pro Gly
   115 120 125
   Val Gly Val Pro Gly Val Gly Val Pro Gly Gly Gly Val Pro Gly Ala
   130 135 140
   Gly Val Pro Gly Gly Gly Val Pro Gly Trp Pro
   145 150 155
<210> 16
   <211> 730
   <212> PRT
   <213> Artificial Sequence
<220>
   <223> ELPbetaV2-144
<400> 16
   Val Pro Gly Val Gly Val Pro Gly Val Gly Val Pro Gly Ala Gly Val
   1               5                   10                  15
   Pro Gly Val Gly Val Pro Gly Ala Gly Val Pro Gly Val Gly Val Pro
               20                  25                  30
   Gly Val Gly Val Pro Gly Ala Gly Val Pro Gly Val Gly Val Pro Gly
           35                  40                  45
   Ala Gly Val Pro Gly Val Gly Val Pro Gly Ala Gly Val Pro Gly Val
       50                  55                  60
   Gly Val Pro Gly Ala Gly Val Pro Gly Val Gly Val Pro Gly Val Gly
   65                  70                  75                  80
   Val Pro Gly Ala Gly Val Pro Gly Val Gly Val Pro Gly Ala Gly Val
                   85                  90                  95
   Pro Gly Val Gly Val Pro Gly Ala Gly Val Pro Gly Val Gly Val Pro
               100                 105                 110
   Gly Ala Gly Val Pro Gly Val Gly Val Pro Gly Val Gly Val Pro Gly
           115                 120                 125
   Ala Gly Val Pro Gly Val Gly Val Pro Gly Ala Gly Val Pro Gly Val
       130                 135                 140
   Gly Val Pro Gly Ala Gly Val Pro Gly Val Gly Val Pro Gly Ala Gly
   145                 150                 155                 160
   Val Pro Gly Val Gly Val Pro Gly Val Gly Val Pro Gly Ala Gly Val
                   165                 170                 175
   Pro Gly Val Gly Val Pro Gly Ala Gly Val Pro Gly Val Gly Val Pro
               180                 185                 190
   Gly Ala Gly Val Pro Gly Val Gly Val Pro Gly Ala Gly Val Pro Gly
           195                 200                 205
   Val Gly Val Pro Gly Val Gly Val Pro Gly Ala Gly Val Pro Gly Val
   210 215 220
   Gly Val Pro Gly Ala Gly Val Pro Gly Val Gly Val Pro Gly Ala Gly
   225 230 235 240
   Val Pro Gly Val Gly Val Pro Gly Ala Gly Val Pro Gly Val Gly Val
   245 250 255
   Pro Gly Val Gly Val Pro Gly Ala Gly Val Pro Gly Val Gly Val Pro
   260 265 270
   Gly Ala Gly Val Pro Gly Val Gly Val Pro Gly Ala Gly Val Pro Gly
   275 280 285
   Val Gly Val Pro Gly Ala Gly Val Pro Gly Val Gly Val Pro Gly Val
   290 295 300
   Gly Val Pro Gly Ala Gly Val Pro Gly Val Gly Val Pro Gly Ala Gly
   305 310 315 320
   Val Pro Gly Val Gly Val Pro Gly Ala Gly Val Pro Gly Val Gly Val
   325 330 335
   Pro Gly Ala Gly Val Pro Gly Val Gly Val Pro Gly Val Gly Val Pro
   340 345 350
   Gly Ala Gly Val Pro Gly Val Gly Val Pro Gly Ala Gly Val Pro Gly
   355 360 365
   Val Gly Val Pro Gly Ala Gly Val Pro Gly Val Gly Val Pro Gly Ala
   370 375 380
   Gly Val Pro Gly Val Gly Val Pro Gly Val Gly Val Pro Gly Ala Gly
   385 390 395 400
   Val Pro Gly Val Gly Val Pro Gly Ala Gly Val Pro Gly Val Gly Val
   405 410 415
   Pro Gly Ala Gly Val Pro Gly Val Gly Val Pro Gly Ala Gly Val Pro
   420 425 430
   Gly Val Gly Val Pro Gly Val Gly Val Pro Gly Ala Gly Val Pro Gly
   435 440 445
   Val Gly Val Pro Gly Ala Gly Val Pro Gly Val Gly Val Pro Gly Ala
   450 455 460
   Gly Val Pro Gly Val Gly Val Pro Gly Ala Gly Val Pro Gly Val Gly
   465 470 475 480
   Val Pro Gly Val Gly Val Pro Gly Ala Gly Val Pro Gly Val Gly Val
   485 490 495
   Pro Gly Ala Gly Val Pro Gly Val Gly Val Pro Gly Ala Gly Val Pro
   500 505 510
   Gly Val Gly Val Pro Gly Ala Gly Val Pro Gly Val Gly Val Pro Gly
   515 520 525
   Val Gly Val Pro Gly Ala Gly Val Pro Gly Val Gly Val Pro Gly Ala
   530 535 540
   Gly Val Pro Gly Val Gly Val Pro Gly Ala Gly Val Pro Gly Val Gly
   545 550 555 560
   Val Pro Gly Ala Gly Val Pro Gly Val Gly Val Pro Gly Val Gly Val
   565 570 575
   Pro Gly Ala Gly Val Pro Gly Val Gly Val Pro Gly Ala Gly Val Pro
   580 585 590
   Gly Val Gly Val Pro Gly Ala Gly Val Pro Gly Val Gly Val Pro Gly
   595 600 605
   Ala Gly Val Pro Gly Val Gly Val Pro Gly Val Gly Val Pro Gly Ala
   610 615 620
   Gly Val Pro Gly Val Gly Val Pro Gly Ala Gly Val Pro Gly Val Gly
   625 630 635 640
   Val Pro Gly Ala Gly Val Pro Gly Val Gly Val Pro Gly Ala Gly Val
   645 650 655
   Pro Gly Val Gly Val Pro Gly Val Gly Val Pro Gly Ala Gly Val Pro
   660 665 670
   Gly Val Gly Val Pro Gly Ala Gly Val Pro Gly Val Gly Val Pro Gly
   675 680 685
   Ala Gly Val Pro Gly Val Gly Val Pro Gly Ala Gly Val Pro Gly Val
   690 695 700
   Gly Val Pro Gly Val Gly Val Pro Gly Ala Gly Val Pro Gly Val Gly
   705 710 715 720
   Val Pro Gly Ala Gly Val Pro Gly Val Gly
   725 730
<210> 17
   <211> 39
   <212> PRT
   <213> Artificial Sequence
<220>
   <223> Human recombinant Exendin-4
<400> 17
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 18
   <211> 31
   <212> PRT
   <213> Unknown
<220>
   <223> Exendin-4 (1-31)
<400> 18
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro
   20 25 30
<210> 19
   <211> 30
   <212> PRT
   <213> Unknown
<220>
   <223> Exendin-4 (1-30)
<400> 19
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly
               20                  25                  30
<210> 20
   <211> 32
   <212> PRT
   <213> Unknown
<220>
   <223> Exendin-4 (1-32)
<400> 20
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
<210> 21
   <211> 33
   <212> PRT
   <213> Unknown
<220>
   <223> Exendin-4 (1-33)
<400> 21
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser
<210> 22
   <211> 34
   <212> PRT
   <213> Unknown
<220>
   <223> Exendin-4 (1-34)
<400> 22
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly
<210> 23
   <211> 35
   <212> PRT
   <213> Unknown
<220>
   <223> Exendin-4 (1-35)
<400> 23
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala
           35
<210> 24
   <211> 36
   <212> PRT
   <213> Unknown
<220>
   <223> Exendin-4 (1-36)
<400> 24
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro
           35
<210> 25
   <211> 37
   <212> PRT
   <213> Unknown
<220>
   <223> Exendin-4 (1-37)
<400> 25
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro
           35
<210> 26
   <211> 38
   <212> PRT
   <213> Unknown
<220>
   <223> Exendin-4 (1-38)
<400> 26
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro
           35
<210> 27
   <211> 31
   <212> PRT
   <213> Artificial Sequence
<220>
   <223> Human recombinant Exendin-4 (9-39)
<400> 27
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser
               20                  25                  30
<210> 28
   <211> 23
   <212> PRT
   <213> Artificial Sequence
<220>
   <223> Human recombinant Exendin-4 (9-31)
<400> 28
   Asp Leu Ser Lys Gln Met Glu Glu Glu Ala Val Arg Leu Phe Ile Glu
   1               5                   10                  15
   Trp Leu Lys Asn Gly Gly Pro
               20
<210> 29
   <211> 46
   <212> PRT
   <213> Unknown
<220>
   <223> Exendin-4-ELP sequence with N-terminal Tev cleavage site
<400> 29
   Met Glu Asn Leu Tyr Phe Gln His Gly Glu Gly Thr Phe Thr Ser Asp
   1               5                   10                  15
   Leu Ser Lys Gln Met Glu Glu Glu Ala Val Arg Leu Phe Ile Glu Trp
               20                  25                  30
   Leu Lys Asn Gly Gly Pro Ser Ser Gly Ala Pro Pro Pro Ser
           35                  40                  45
<210> 30
   <211> 58
   <212> PRT
   <213> Unknown
<220>
   <223> Exendin-4-ELP sequence with DsbA leader sequence
<400> 30
   Met Lys Lys Ile Trp Leu Ala Leu Ala Gly Leu Val Leu Ala Phe Ser
   1               5                   10                  15
   Ala Ser Ala His Gly Glu Gly Thr Phe Thr Ser Asp Leu Ser Lys Gln
               20                  25                  30
   Met Glu Glu Glu Ala Val Arg Leu Phe Ile Glu Trp Leu Lys Asn Gly
           35                  40                  45
   Gly Pro Ser Ser Gly Ala Pro Pro Pro Ser
       50                  55
<210> 31
   <211> 31
   <212> PRT
   <213> Unknown
<220>
   <223> GLP-1 (A8G 7-37)
<400> 31
   His Gly Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Gly Arg Gly
               20                  25                  30
<210> 32
   <211> 30
   <212> PRT
   <213> Unknown
<220>
   <223> GLP-1(7-36)
<400> 32
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Gly Arg
               20                  25                  30
<210> 33
   <211> 30
   <212> PRT
   <213> Unknown
<220>
   <223> GLP-1(7-36) with G at position 2
<400> 33
   His Gly Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Gly Arg
               20                  25                  30
<210> 34
   <211> 38
   <212> PRT
   <213> Unknown
<220>
   <223> GLP-1(A8G 1-37)
<400> 34
   Met Glu Asn Leu Tyr Phe Gln His Gly Glu Gly Thr Phe Thr Ser Asp
   1               5                   10                  15
   Val Ser Ser Tyr Leu Glu Gly Gln Ala Ala Lys Glu Phe Ile Ala Trp
               20                  25                  30
   Leu Val Lys Gly Arg Gly
           35
<210> 35
   <211> 44
   <212> PRT
   <213> Unknown
<220>
   <223> GLP-1 receptor agonist
<400> 35
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Ser Lys Lys Lys Lys Lys Lys
           35                  40
<210> 36
   <211> 31
   <212> PRT
   <213> Unknown
<220>
   <223> GLP-1 receptor agonist
<400> 36
   His Val Glu Gly Thr Phe Thr Ser Asp Val Ser Ser Tyr Leu Glu Glu
   1               5                   10                  15
   Gln Ala Ala Lys Glu Phe Ile Ala Trp Leu Ile Lys Gly Arg Gly
               20                  25                  30
<210> 37
   <211> 48
   <212> PRT
   <213> Unknown
<220>
   <223> GLP-1 A8G
<400> 37
   Met Glu Asn Leu Tyr Phe Gln His Gly Glu Gly Thr Phe Thr Ser Asp
   1               5                   10                  15
   Val Ser Ser Tyr Leu Glu Gly Gln Ala Ala Lys Glu Phe Ile Ala Trp
               20                  25                  30
   Leu Val Lys Gly Arg Gly Leu Glu Gly Met Gly Gly Pro Gly Val Gly
           35                  40                  45
<210> 38
   <211> 22
   <212> PRT
   <213> Unknown
<220>
   <223> Exendin-4 (9-30)
<400> 38
   Asp Leu Ser Lys Gln Met Glu Glu Glu Ala Val Arg Leu Phe Ile Glu
   1               5                   10                  15
   Trp Leu Lys Asn Gly Gly
               20
<210> 39
   <211> 36
   <212> PRT
   <213> Artificial Sequence
<220>
   <223> Human recombinant apelin-36
<400> 39
   Leu Val Gln Pro Arg Gly Ser Arg Asn Gly Pro Gly Pro Trp Gln Gly
   1               5                   10                  15
   Gly Arg Arg Lys Phe Arg Arg Gln Arg Pro Arg Leu Ser His Lys Gly
               20                  25                  30
   Pro Met Pro Phe
           35
<210> 40
   <211> 642
   <212> PRT
   <213> Artificial Sequence
<220>
   <223> pPE0518-ELP1-120 apelin-36
<400> 40
   Met Val Pro Gly Val Gly Val Pro Gly Val Gly Val Pro Gly Gly Gly
   1               5                   10                  15
   Val Pro Gly Ala Gly Val Pro Gly Val Gly Val Pro Gly Val Gly Val
               20                  25                  30
   Pro Gly Val Gly Val Pro Gly Gly Gly Val Pro Gly Ala Gly Val Pro
           35                  40                  45
   Gly Gly Gly Val Pro Gly Val Gly Val Pro Gly Val Gly Val Pro Gly
       50                  55                  60
   Gly Gly Val Pro Gly Ala Gly Val Pro Gly Val Gly Val Pro Gly Val
   65                  70                  75                  80
   Gly Val Pro Gly Val Gly Val Pro Gly Gly Gly Val Pro Gly Ala Gly
                   85                  90                  95
   Val Pro Gly Gly Gly Val Pro Gly Val Gly Val Pro Gly Val Gly Val
               100                 105                 110
   Pro Gly Gly Gly Val Pro Gly Ala Gly Val Pro Gly Val Gly Val Pro
           115                 120                 125
   Gly Val Gly Val Pro Gly Val Gly Val Pro Gly Gly Gly Val Pro Gly
       130                 135                 140
   Ala Gly Val Pro Gly Gly Gly Val Pro Gly Val Gly Val Pro Gly Val
   145                 150                 155                 160
   Gly Val Pro Gly Gly Gly Val Pro Gly Ala Gly Val Pro Gly Val Gly
                   165                 170                 175
   Val Pro Gly Val Gly Val Pro Gly Val Gly Val Pro Gly Gly Gly Val
               180                 185                 190
   Pro Gly Ala Gly Val Pro Gly Gly Gly Val Pro Gly Val Gly Val Pro
           195                 200                 205
   Gly Val Gly Val Pro Gly Gly Gly Val Pro Gly Ala Gly Val Pro Gly
       210                 215                 220
   Val Gly Val Pro Gly Val Gly Val Pro Gly Val Gly Val Pro Gly Gly
   225                 230                 235                 240
   Gly Val Pro Gly Ala Gly Val Pro Gly Gly Gly Val Pro Gly Val Gly
                   245                 250                 255
   Val Pro Gly Val Gly Val Pro Gly Gly Gly Val Pro Gly Ala Gly Val
               260                 265                 270
   Pro Gly Val Gly Val Pro Gly Val Gly Val Pro Gly Val Gly Val Pro
           275                 280                 285
   Gly Gly Gly Val Pro Gly Ala Gly Val Pro Gly Gly Gly Val Pro Gly
       290                 295                 300
   Val Gly Val Pro Gly Val Gly Val Pro Gly Gly Gly Val Pro Gly Ala
   305                 310                 315                 320
   Gly Val Pro Gly Val Gly Val Pro Gly Val Gly Val Pro Gly Val Gly
   325 330 335
   Val Pro Gly Gly Gly Val Pro Gly Ala Gly Val Pro Gly Gly Gly Val
   340 345 350
   Pro Gly Val Gly Val Pro Gly Val Gly Val Pro Gly Gly Gly Val Pro
   355 360 365
   Gly Ala Gly Val Pro Gly Val Gly Val Pro Gly Val Gly Val Pro Gly
   370 375 380
   Val Gly Val Pro Gly Gly Gly Val Pro Gly Ala Gly Val Pro Gly Gly
   385 390 395 400
   Gly Val Pro Gly Val Gly Val Pro Gly Val Gly Val Pro Gly Gly Gly
   405 410 415
   Val Pro Gly Ala Gly Val Pro Gly Val Gly Val Pro Gly Val Gly Val
   420 425 430
   Pro Gly Val Gly Val Pro Gly Gly Gly Val Pro Gly Ala Gly Val Pro
   435 440 445
   Gly Gly Gly Val Pro Gly Val Gly Val Pro Gly Val Gly Val Pro Gly
   450 455 460
   Gly Gly Val Pro Gly Ala Gly Val Pro Gly Val Gly Val Pro Gly Val
   465 470 475 480
   Gly Val Pro Gly Val Gly Val Pro Gly Gly Gly Val Pro Gly Ala Gly
   485 490 495
   Val Pro Gly Gly Gly Val Pro Gly Val Gly Val Pro Gly Val Gly Val
   500 505 510
   Pro Gly Gly Gly Val Pro Gly Ala Gly Val Pro Gly Val Gly Val Pro
   515 520 525
   Gly Val Gly Val Pro Gly Val Gly Val Pro Gly Gly Gly Val Pro Gly
   530 535 540
   Ala Gly Val Pro Gly Gly Gly Val Pro Gly Val Gly Val Pro Gly Val
   545 550 555 560
   Gly Val Pro Gly Gly Gly Val Pro Gly Ala Gly Val Pro Gly Val Gly
   565 570 575
   Val Pro Gly Val Gly Val Pro Gly Val Gly Val Pro Gly Gly Gly Val
               580                 585                 590
   Pro Gly Ala Gly Val Pro Gly Gly Gly Val Pro Gly Val Gly Leu Val
           595                 600                 605
   Gln Pro Arg Gly Ser Arg Asn Gly Pro Gly Pro Trp Gln Gly Gly Arg
       610                 615                 620
   Arg Lys Phe Arg Arg Gln Arg Pro Arg Leu Ser His Lys Gly Pro Met
   625                 630                 635                 640
   Pro Phe
<210> 41
   <211> 17
   <212> PRT
   <213> Artificial Sequence
<220>
   <223> Human recombinant Apelin-17
<400> 41
   Lys Phe Arg Arg Gln Arg Pro Arg Leu Ser His Lys Gly Pro Met Pro
   1               5                   10                  15
   Phe
<210> 42
   <211> 12
   <212> PRT
   <213> Artificial Sequence
<220>
   <223> Human recombinant Pyr-apelin-13
<220>
   <221> MISC_FEATURE
   <222> (1)..()
   <223> May be modified with a Pyroglutamate moiety
<400> 42
   Arg Pro Arg Leu Ser His Lys Gly Pro Met Pro Phe
   1               5                   10
<210> 43
   <211> 13
   <212> PRT
   <213> Artificial Sequence
<220>
   <223> Human recombinant Apelin-13
<400> 43
   Gln Arg Pro Arg Leu Ser His Lys Gly Pro Met Pro Phe
   1               5                   10
<210> 44
   <211> 12
   <212> PRT
   <213> Artificial Sequence
<220>
   <223> Human recombinant Apelin-12
<400> 44
   Arg Pro Arg Leu Ser His Lys Gly Pro Met Pro Phe
   1               5                   10
<210> 45
   <211> 322
   <212> PRT
   <213> Artificial Sequence
<220>
   <223> Human recombinant arginase
<400> 45
<210> 46
   <211> 927
   <212> PRT
   <213> Artificial Sequence
<220>
   <223> pPE0140-arginase ELP1-120
<400> 46
   Met Ser Ala Lys Ser Arg Thr Ile Gly Ile Ile Gly Ala Pro Phe Ser
   1 5 10 15
   Lys Gly Gln Pro Arg Gly Gly Val Glu Glu Gly Pro Thr Val Leu Arg
   20 25 30
   Lys Ala Gly Leu Leu Glu Lys Leu Lys Glu Gln Glu Cys Asp Val Lys
   35 40 45
   Asp Tyr Gly Asp Leu Pro Phe Ala Asp Ile Pro Asn Asp Ser Pro Phe
   50 55 60
   Gln Ile Val Lys Asn Pro Arg Ser Val Gly Lys Ala Ser Glu Gln Leu
   65 70 75 80
   Ala Gly Lys Val Ala Glu Val Lys Lys Asn Gly Arg Ile Ser Leu Val
   85 90 95
   Leu Gly Gly Asp His Ser Leu Ala Ile Gly Ser Ile Ser Gly His Ala
   100 105 110
   Arg Val His Pro Asp Leu Gly Val Ile Trp Val Asp Ala His Thr Asp
   115 120 125
   Ile Asn Thr Pro Leu Thr Thr Thr Ser Gly Asn Leu His Gly Gln Pro
   130 135 140
   Val Ser Phe Leu Leu Lys Glu Leu Lys Gly Lys Ile Pro Asp Val Pro
   145 150 155 160
   Gly Phe Ser Trp Val Thr Pro Cys Ile Ser Ala Lys Asp Ile Val Tyr
   165 170 175
   Ile Gly Leu Arg Asp Val Asp Pro Gly Glu His Tyr Ile Leu Lys Thr
   180 185 190
   Leu Gly Ile Lys Tyr Phe Ser Met Thr Glu Val Asp Arg Leu Gly Ile
   195 200 205
   Gly Lys Val Met Glu Glu Thr Leu Ser Tyr Leu Leu Gly Arg Lys Lys
   210 215 220
   Arg Pro Ile His Leu Ser Phe Asp Val Asp Gly Leu Asp Pro Ser Phe
   225 230 235 240
   Thr Pro Ala Thr Gly Thr Pro Val Val Gly Gly Leu Thr Tyr Arg Glu
   245 250 255
   Gly Leu Tyr Ile Thr Glu Glu Ile Tyr Lys Thr Gly Leu Leu Ser Gly
   260 265 270
   Leu Asp Ile Met Glu Val Asn Pro Ser Leu Gly Lys Thr Pro Glu Glu
   275 280 285
   Val Thr Arg Thr Val Asn Thr Ala Val Ala Ile Thr Leu Ala Cys Phe
   290 295 300
   Gly Leu Ala Arg Glu Gly Asn His Lys Pro Ile Asp Tyr Leu Asn Pro
   305 310 315 320
   Pro Lys Val Pro Gly Val Gly Val Pro Gly Val Gly Val Pro Gly Gly
   325 330 335
   Gly Val Pro Gly Ala Gly Val Pro Gly Val Gly Val Pro Gly Val Gly
   340 345 350
   Val Pro Gly Val Gly Val Pro Gly Gly Gly Val Pro Gly Ala Gly Val
   355 360 365
   Pro Gly Gly Gly Val Pro Gly Val Gly Val Pro Gly Val Gly Val Pro
   370 375 380
   Gly Gly Gly Val Pro Gly Ala Gly Val Pro Gly Val Gly Val Pro Gly
   385 390 395 400
   Val Gly Val Pro Gly Val Gly Val Pro Gly Gly Gly Val Pro Gly Ala
   405 410 415
   Gly Val Pro Gly Gly Gly Val Pro Gly Val Gly Val Pro Gly Val Gly
   420 425 430
   Val Pro Gly Gly Gly Val Pro Gly Ala Gly Val Pro Gly Val Gly Val
   435 440 445
   Pro Gly Val Gly Val Pro Gly Val Gly Val Pro Gly Gly Gly Val Pro
   450 455 460
   Gly Ala Gly Val Pro Gly Gly Gly Val Pro Gly Val Gly Val Pro Gly
   465 470 475 480
   Val Gly Val Pro Gly Gly Gly Val Pro Gly Ala Gly Val Pro Gly Val
   485 490 495
   Gly Val Pro Gly Val Gly Val Pro Gly Val Gly Val Pro Gly Gly Gly
               500                 505                 510
   Val Pro Gly Ala Gly Val Pro Gly Gly Gly Val Pro Gly Val Gly Val
           515                 520                 525
   Pro Gly Val Gly Val Pro Gly Gly Gly Val Pro Gly Ala Gly Val Pro
       530                 535                 540
   Gly Val Gly Val Pro Gly Val Gly Val Pro Gly Val Gly Val Pro Gly
   545                 550                 555                 560
   Gly Gly Val Pro Gly Ala Gly Val Pro Gly Gly Gly Val Pro Gly Val
                   565                 570                 575
   Gly Val Pro Gly Val Gly Val Pro Gly Gly Gly Val Pro Gly Ala Gly
               580                 585                 590
   Val Pro Gly Val Gly Val Pro Gly Val Gly Val Pro Gly Val Gly Val
           595                 600                 605
   Pro Gly Gly Gly Val Pro Gly Ala Gly Val Pro Gly Gly Gly Val Pro
       610                 615                 620
   Gly Val Gly Val Pro Gly Val Gly Val Pro Gly Gly Gly Val Pro Gly
   625                 630                 635                 640
   Ala Gly Val Pro Gly Val Gly Val Pro Gly Val Gly Val Pro Gly Val
                   645                 650                 655
   Gly Val Pro Gly Gly Gly Val Pro Gly Ala Gly Val Pro Gly Gly Gly
               660                 665                 670
   Val Pro Gly Val Gly Val Pro Gly Val Gly Val Pro Gly Gly Gly Val
           675                 680                 685
   Pro Gly Ala Gly Val Pro Gly Val Gly Val Pro Gly Val Gly Val Pro
       690                 695                 700
   Gly Val Gly Val Pro Gly Gly Gly Val Pro Gly Ala Gly Val Pro Gly
   705                 710                 715                 720
   Gly Gly Val Pro Gly Val Gly Val Pro Gly Val Gly Val Pro Gly Gly
                   725                 730                 735
   Gly Val Pro Gly Ala Gly Val Pro Gly Val Gly Val Pro Gly Val Gly
               740                 745                 750
   Val Pro Gly Val Gly Val Pro Gly Gly Gly Val Pro Gly Ala Gly Val
           755                 760                 765
   Pro Gly Gly Gly Val Pro Gly Val Gly Val Pro Gly Val Gly Val Pro
       770                 775                 780
   Gly Gly Gly Val Pro Gly Ala Gly Val Pro Gly Val Gly Val Pro Gly
   785                 790                 795                 800
   Val Gly Val Pro Gly Val Gly Val Pro Gly Gly Gly Val Pro Gly Ala
                   805                 810                 815
   Gly Val Pro Gly Gly Gly Val Pro Gly Val Gly Val Pro Gly Val Gly
               820                 825                 830
   Val Pro Gly Gly Gly Val Pro Gly Ala Gly Val Pro Gly Val Gly Val
           835                 840                 845
   Pro Gly Val Gly Val Pro Gly Val Gly Val Pro Gly Gly Gly Val Pro
       850                 855                 860
   Gly Ala Gly Val Pro Gly Gly Gly Val Pro Gly Val Gly Val Pro Gly
   865                 870                 875                 880
   Val Gly Val Pro Gly Gly Gly Val Pro Gly Ala Gly Val Pro Gly Val
                   885                 890                 895
   Gly Val Pro Gly Val Gly Val Pro Gly Val Gly Val Pro Gly Gly Gly
               900                 905                 910
   Val Pro Gly Ala Gly Val Pro Gly Gly Gly Val Pro Gly Trp Pro
           915                 920                 925
<210> 47
   <211> 22
   <212> PRT
   <213> Artificial Sequence
<220>
   <223> Human recombinant CNP22
<400> 47
   Gly Leu Ser Lys Gly Cys Phe Gly Leu Lys Leu Asp Arg Ile Gly Ser
   1               5                   10                  15
   Met Ser Gly Leu Gly Cys
               20
<210> 48
   <211> 628
   <212> PRT
   <213> Artificial Sequence
<220>
   <223> ELP1-120 CNP22 fusion
<400> 48
   Met Val Pro Gly Val Gly Val Pro Gly Val Gly Val Pro Gly Gly Gly
   1               5                   10                  15
   Val Pro Gly Ala Gly Val Pro Gly Val Gly Val Pro Gly Val Gly Val
               20                  25                  30
   Pro Gly Val Gly Val Pro Gly Gly Gly Val Pro Gly Ala Gly Val Pro
           35                  40                  45
   Gly Gly Gly Val Pro Gly Val Gly Val Pro Gly Val Gly Val Pro Gly
       50                  55                  60
   Gly Gly Val Pro Gly Ala Gly Val Pro Gly Val Gly Val Pro Gly Val
   65                  70                  75                  80
   Gly Val Pro Gly Val Gly Val Pro Gly Gly Gly Val Pro Gly Ala Gly
                   85                  90                  95
   Val Pro Gly Gly Gly Val Pro Gly Val Gly Val Pro Gly Val Gly Val
               100                 105                 110
   Pro Gly Gly Gly Val Pro Gly Ala Gly Val Pro Gly Val Gly Val Pro
           115                 120                 125
   Gly Val Gly Val Pro Gly Val Gly Val Pro Gly Gly Gly Val Pro Gly
       130                 135                 140
   Ala Gly Val Pro Gly Gly Gly Val Pro Gly Val Gly Val Pro Gly Val
   145                 150                 155                 160
   Gly Val Pro Gly Gly Gly Val Pro Gly Ala Gly Val Pro Gly Val Gly
                   165                 170                 175
   Val Pro Gly Val Gly Val Pro Gly Val Gly Val Pro Gly Gly Gly Val
               180                 185                 190
   Pro Gly Ala Gly Val Pro Gly Gly Gly Val Pro Gly Val Gly Val Pro
           195                 200                 205
   Gly Val Gly Val Pro Gly Gly Gly Val Pro Gly Ala Gly Val Pro Gly
       210                 215                 220
   Val Gly Val Pro Gly Val Gly Val Pro Gly Val Gly Val Pro Gly Gly
   225                 230                 235                 240
   Gly Val Pro Gly Ala Gly Val Pro Gly Gly Gly Val Pro Gly Val Gly
                   245                 250                 255
   Val Pro Gly Val Gly Val Pro Gly Gly Gly Val Pro Gly Ala Gly Val
               260                 265                 270
   Pro Gly Val Gly Val Pro Gly Val Gly Val Pro Gly Val Gly Val Pro
           275                 280                 285
   Gly Gly Gly Val Pro Gly Ala Gly Val Pro Gly Gly Gly Val Pro Gly
       290                 295                 300
   Val Gly Val Pro Gly Val Gly Val Pro Gly Gly Gly Val Pro Gly Ala
   305                 310                 315                 320
   Gly Val Pro Gly Val Gly Val Pro Gly Val Gly Val Pro Gly Val Gly
                   325                 330                 335
   Val Pro Gly Gly Gly Val Pro Gly Ala Gly Val Pro Gly Gly Gly Val
               340                 345                 350
   Pro Gly Val Gly Val Pro Gly Val Gly Val Pro Gly Gly Gly Val Pro
           355                 360                 365
   Gly Ala Gly Val Pro Gly Val Gly Val Pro Gly Val Gly Val Pro Gly
       370                 375                 380
   Val Gly Val Pro Gly Gly Gly Val Pro Gly Ala Gly Val Pro Gly Gly
   385                 390                 395                 400
   Gly Val Pro Gly Val Gly Val Pro Gly Val Gly Val Pro Gly Gly Gly
                   405                 410                 415
   Val Pro Gly Ala Gly Val Pro Gly Val Gly Val Pro Gly Val Gly Val
               420                 425                 430
   Pro Gly Val Gly Val Pro Gly Gly Gly Val Pro Gly Ala Gly Val Pro
           435                 440                 445
   Gly Gly Gly Val Pro Gly Val Gly Val Pro Gly Val Gly Val Pro Gly
       450                 455                 460
   Gly Gly Val Pro Gly Ala Gly Val Pro Gly Val Gly Val Pro Gly Val
   465                 470                 475                 480
   Gly Val Pro Gly Val Gly Val Pro Gly Gly Gly Val Pro Gly Ala Gly
                   485                 490                 495
   Val Pro Gly Gly Gly Val Pro Gly Val Gly Val Pro Gly Val Gly Val
               500                 505                 510
   Pro Gly Gly Gly Val Pro Gly Ala Gly Val Pro Gly Val Gly Val Pro
           515                 520                 525
   Gly Val Gly Val Pro Gly Val Gly Val Pro Gly Gly Gly Val Pro Gly
       530                 535                 540
   Ala Gly Val Pro Gly Gly Gly Val Pro Gly Val Gly Val Pro Gly Val
   545                 550                 555                 560
   Gly Val Pro Gly Gly Gly Val Pro Gly Ala Gly Val Pro Gly Val Gly
                   565                 570                 575
   Val Pro Gly Val Gly Val Pro Gly Val Gly Val Pro Gly Gly Gly Val
               580                 585                 590
   Pro Gly Ala Gly Val Pro Gly Gly Gly Val Pro Gly Trp Gly Gly Leu
           595                 600                 605
   Ser Lys Gly Cys Phe Gly Leu Lys Leu Asp Arg Ile Gly Ser Met Ser
       610                 615                 620
   Gly Leu Gly Cys
   625
<210> 49
   <211> 37
   <212> PRT
   <213> Artificial Sequence
<220>
   <223> Human recombinant CNP37
<400> 49
   Gln Glu His Pro Asn Ala Arg Lys Tyr Lys Gly Ala Asn Lys Lys Gly
   1               5                   10                  15
   Leu Ser Lys Gly Cys Phe Gly Leu Lys Leu Asp Arg Ile Gly Ser Met
               20                  25                  30
   Ser Gly Leu Gly Cys
   35
<210> 50
   <211> 643
   <212> PRT
   <213> Artificial Sequence
<220>
   <223> pPE0493-ELP1-120 CNP37
<400> 50
   Met Val Pro Gly Val Gly Val Pro Gly Val Gly Val Pro Gly Gly Gly
   1               5                   10                  15
   Val Pro Gly Ala Gly Val Pro Gly Val Gly Val Pro Gly Val Gly Val
               20                  25                  30
   Pro Gly Val Gly Val Pro Gly Gly Gly Val Pro Gly Ala Gly Val Pro
           35                  40                  45
   Gly Gly Gly Val Pro Gly Val Gly Val Pro Gly Val Gly Val Pro Gly
       50                  55                  60
   Gly Gly Val Pro Gly Ala Gly Val Pro Gly Val Gly Val Pro Gly Val
   65                  70                  75                  80
   Gly Val Pro Gly Val Gly Val Pro Gly Gly Gly Val Pro Gly Ala Gly
                   85                  90                  95
   Val Pro Gly Gly Gly Val Pro Gly Val Gly Val Pro Gly Val Gly Val
               100                 105                 110
   Pro Gly Gly Gly Val Pro Gly Ala Gly Val Pro Gly Val Gly Val Pro
           115                 120                 125
   Gly Val Gly Val Pro Gly Val Gly Val Pro Gly Gly Gly Val Pro Gly
       130                 135                 140
   Ala Gly Val Pro Gly Gly Gly Val Pro Gly Val Gly Val Pro Gly Val
   145                 150                 155                 160
   Gly Val Pro Gly Gly Gly Val Pro Gly Ala Gly Val Pro Gly Val Gly
                   165                 170                 175
   Val Pro Gly Val Gly Val Pro Gly Val Gly Val Pro Gly Gly Gly Val
               180                 185                 190
   Pro Gly Ala Gly Val Pro Gly Gly Gly Val Pro Gly Val Gly Val Pro
           195                 200                 205
   Gly Val Gly Val Pro Gly Gly Gly Val Pro Gly Ala Gly Val Pro Gly
       210                 215                 220
   Val Gly Val Pro Gly Val Gly Val Pro Gly Val Gly Val Pro Gly Gly
   225                 230                 235                 240
   Gly Val Pro Gly Ala Gly Val Pro Gly Gly Gly Val Pro Gly Val Gly
                   245                 250                 255
   Val Pro Gly Val Gly Val Pro Gly Gly Gly Val Pro Gly Ala Gly Val
               260                 265                 270
   Pro Gly Val Gly Val Pro Gly Val Gly Val Pro Gly Val Gly Val Pro
           275                 280                 285
   Gly Gly Gly Val Pro Gly Ala Gly Val Pro Gly Gly Gly Val Pro Gly
       290                 295                 300
   Val Gly Val Pro Gly Val Gly Val Pro Gly Gly Gly Val Pro Gly Ala
   305                 310                 315                 320
   Gly Val Pro Gly Val Gly Val Pro Gly Val Gly Val Pro Gly Val Gly
                   325                 330                 335
   Val Pro Gly Gly Gly Val Pro Gly Ala Gly Val Pro Gly Gly Gly Val
               340                 345                 350
   Pro Gly Val Gly Val Pro Gly Val Gly Val Pro Gly Gly Gly Val Pro
           355                 360                 365
   Gly Ala Gly Val Pro Gly Val Gly Val Pro Gly Val Gly Val Pro Gly
       370                 375                 380
   Val Gly Val Pro Gly Gly Gly Val Pro Gly Ala Gly Val Pro Gly Gly
   385                 390                 395                 400
   Gly Val Pro Gly Val Gly Val Pro Gly Val Gly Val Pro Gly Gly Gly
                   405                 410                 415
   Val Pro Gly Ala Gly Val Pro Gly Val Gly Val Pro Gly Val Gly Val
               420                 425                 430
   Pro Gly Val Gly Val Pro Gly Gly Gly Val Pro Gly Ala Gly Val Pro
           435                 440                 445
   Gly Gly Gly Val Pro Gly Val Gly Val Pro Gly Val Gly Val Pro Gly
   450 455 460
   Gly Gly Val Pro Gly Ala Gly Val Pro Gly Val Gly Val Pro Gly Val
   465 470 475 480
   Gly Val Pro Gly Val Gly Val Pro Gly Gly Gly Val Pro Gly Ala Gly
   485 490 495
   Val Pro Gly Gly Gly Val Pro Gly Val Gly Val Pro Gly Val Gly Val
   500 505 510
   Pro Gly Gly Gly Val Pro Gly Ala Gly Val Pro Gly Val Gly Val Pro
   515 520 525
   Gly Val Gly Val Pro Gly Val Gly Val Pro Gly Gly Gly Val Pro Gly
   530 535 540
   Ala Gly Val Pro Gly Gly Gly Val Pro Gly Val Gly Val Pro Gly Val
   545 550 555 560
   Gly Val Pro Gly Gly Gly Val Pro Gly Ala Gly Val Pro Gly Val Gly
   565 570 575
   Val Pro Gly Val Gly Val Pro Gly Val Gly Val Pro Gly Gly Gly Val
   580 585 590
   Pro Gly Ala Gly Val Pro Gly Gly Gly Val Pro Gly Trp Gly Gln Glu
   595 600 605
   His Pro Asn Ala Arg Lys Tyr Lys Gly Ala Asn Lys Lys Gly Leu Ser
   610 615 620
   Lys Gly Cys Phe Gly Leu Lys Leu Asp Arg Ile Gly Ser Met Ser Gly
   625 630 635 640
   Leu Gly Cys
<210> 51
   <211> 53
   <212> PRT
   <213> Artificial Sequence
<220>
   <223> Human recombinant CNP-53
<400> 51
   Asp Leu Arg Val Asp Thr Lys Ser Arg Ala Ala Trp Ala Arg Leu Leu
   1 5 10 15
   Gln Glu His Pro Asn Ala Arg Lys Tyr Lys Gly Ala Asn Lys Lys Gly
   20 25 30
   Leu Ser Lys Gly Cys Phe Gly Leu Lys Leu Asp Arg Ile Gly Ser Met
   35 40 45
   Ser Gly Leu Gly Cys
   50
<210> 52
   <211> 39
   <212> PRT
   <213> Artificial Sequence
<220>
   <223> Human recombinant CNP-39
<400> 52
   Pro Gly Gln Glu His Pro Asn Ala Arg Lys Tyr Lys Gly Ala Asn Lys
   1 5 10 15
   Lys Gly Leu Ser Lys Gly Cys Phe Gly Leu Lys Leu Asp Arg Ile Gly
   20 25 30
   Ser Met Ser Gly Leu Gly Cys
   35
<210> 53
   <211> 31
   <212> PRT
   <213> Artificial Sequence
<220>
   <223> Human recombinant GLP-1
<400> 53
   His Ala Glu Gly Thr Phe Thr Ser Asp Val Ser Ser Tyr Leu Glu Gly
   1 5 10 15
   Gln Ala Ala Lys Glu Phe Ile Ala Trp Leu Val Lys Gly Arg Gly
   20 25 30
<210> 54
   <211> 22
   <212> PRT
   <213> Artificial Sequence
<220>
   <223> Human recombinant GLP-1 (9-31)
<400> 54
   Asp Val Ser Ser Tyr Leu Glu Gly Gln Ala Ala Lys Glu Phe Ile Ala
   1               5                   10                  15
   Trp Leu Val Lys Gly Arg
               20
<210> 55
   <211> 633
   <212> PRT
   <213> Artificial Sequence
<220>
   <223> CNP22 GGS2X ELP1-120 fusion
<400> 55
   Gly Leu Ser Lys Gly Cys Phe Gly Leu Lys Leu Asp Arg Ile Gly Ser
   1               5                   10                  15
   Met Ser Gly Leu Gly Cys Gly Gly Ser Gly Gly Ser Val Pro Gly Val
               20                  25                  30
   Gly Val Pro Gly Val Gly Val Pro Gly Gly Gly Val Pro Gly Ala Gly
           35                  40                  45
   Val Pro Gly Val Gly Val Pro Gly Val Gly Val Pro Gly Val Gly Val
       50                  55                  60
   Pro Gly Gly Gly Val Pro Gly Ala Gly Val Pro Gly Gly Gly Val Pro
   65                  70                  75                  80
   Gly Val Gly Val Pro Gly Val Gly Val Pro Gly Gly Gly Val Pro Gly
                   85                  90                  95
   Ala Gly Val Pro Gly Val Gly Val Pro Gly Val Gly Val Pro Gly Val
               100                 105                 110
   Gly Val Pro Gly Gly Gly Val Pro Gly Ala Gly Val Pro Gly Gly Gly
           115                 120                 125
   Val Pro Gly Val Gly Val Pro Gly Val Gly Val Pro Gly Gly Gly Val
       130                 135                 140
   Pro Gly Ala Gly Val Pro Gly Val Gly Val Pro Gly Val Gly Val Pro
   145                 150                 155                 160
   Gly Val Gly Val Pro Gly Gly Gly Val Pro Gly Ala Gly Val Pro Gly
                   165                 170                 175
   Gly Gly Val Pro Gly Val Gly Val Pro Gly Val Gly Val Pro Gly Gly
               180                 185                 190
   Gly Val Pro Gly Ala Gly Val Pro Gly Val Gly Val Pro Gly Val Gly
           195                 200                 205
   Val Pro Gly Val Gly Val Pro Gly Gly Gly Val Pro Gly Ala Gly Val
       210                 215                 220
   Pro Gly Gly Gly Val Pro Gly Val Gly Val Pro Gly Val Gly Val Pro
   225                 230                 235                 240
   Gly Gly Gly Val Pro Gly Ala Gly Val Pro Gly Val Gly Val Pro Gly
                   245                 250                 255
   Val Gly Val Pro Gly Val Gly Val Pro Gly Gly Gly Val Pro Gly Ala
               260                 265                 270
   Gly Val Pro Gly Gly Gly Val Pro Gly Val Gly Val Pro Gly Val Gly
           275                 280                 285
   Val Pro Gly Gly Gly Val Pro Gly Ala Gly Val Pro Gly Val Gly Val
       290                 295                 300
   Pro Gly Val Gly Val Pro Gly Val Gly Val Pro Gly Gly Gly Val Pro
   305                 310                 315                 320
   Gly Ala Gly Val Pro Gly Gly Gly Val Pro Gly Val Gly Val Pro Gly
                   325                 330                 335
   Val Gly Val Pro Gly Gly Gly Val Pro Gly Ala Gly Val Pro Gly Val
               340                 345                 350
   Gly Val Pro Gly Val Gly Val Pro Gly Val Gly Val Pro Gly Gly Gly
           355                 360                 365
   Val Pro Gly Ala Gly Val Pro Gly Gly Gly Val Pro Gly Val Gly Val
       370                 375                 380
   Pro Gly Val Gly Val Pro Gly Gly Gly Val Pro Gly Ala Gly Val Pro
   385                 390                 395                 400
   Gly Val Gly Val Pro Gly Val Gly Val Pro Gly Val Gly Val Pro Gly
                   405                 410                 415
   Gly Gly Val Pro Gly Ala Gly Val Pro Gly Gly Gly Val Pro Gly Val
               420                 425                 430
   Gly Val Pro Gly Val Gly Val Pro Gly Gly Gly Val Pro Gly Ala Gly
           435                 440                 445
   Val Pro Gly Val Gly Val Pro Gly Val Gly Val Pro Gly Val Gly Val
       450                 455                 460
   Pro Gly Gly Gly Val Pro Gly Ala Gly Val Pro Gly Gly Gly Val Pro
   465                 470                 475                 480
   Gly Val Gly Val Pro Gly Val Gly Val Pro Gly Gly Gly Val Pro Gly
                   485                 490                 495
   Ala Gly Val Pro Gly Val Gly Val Pro Gly Val Gly Val Pro Gly Val
               500                 505                 510
   Gly Val Pro Gly Gly Gly Val Pro Gly Ala Gly Val Pro Gly Gly Gly
           515                 520                 525
   Val Pro Gly Val Gly Val Pro Gly Val Gly Val Pro Gly Gly Gly Val
       530                 535                 540
   Pro Gly Ala Gly Val Pro Gly Val Gly Val Pro Gly Val Gly Val Pro
   545                 550                 555                 560
   Gly Val Gly Val Pro Gly Gly Gly Val Pro Gly Ala Gly Val Pro Gly
                   565                 570                 575
   Gly Gly Val Pro Gly Val Gly Val Pro Gly Val Gly Val Pro Gly Gly
               580                 585                 590
   Gly Val Pro Gly Ala Gly Val Pro Gly Val Gly Val Pro Gly Val Gly
           595                 600                 605
   Val Pro Gly Val Gly Val Pro Gly Gly Gly Val Pro Gly Ala Gly Val
       610                 615                 620
   Pro Gly Gly Gly Val Pro Gly Trp Pro
   625                 630
<210> 56
   <211> 732
   <212> PRT
   <213> Artificial Sequence
<220>
   <223> ELP1-120 pro-CNP
<400> 56
   Met Val Pro Gly Val Gly Val Pro Gly Val Gly Val Pro Gly Gly Gly
   1               5                   10                  15
   Val Pro Gly Ala Gly Val Pro Gly Val Gly Val Pro Gly Val Gly Val
               20                  25                  30
   Pro Gly Val Gly Val Pro Gly Gly Gly Val Pro Gly Ala Gly Val Pro
           35                  40                  45
   Gly Gly Gly Val Pro Gly Val Gly Val Pro Gly Val Gly Val Pro Gly
       50                  55                  60
   Gly Gly Val Pro Gly Ala Gly Val Pro Gly Val Gly Val Pro Gly Val
   65                  70                  75                  80
   Gly Val Pro Gly Val Gly Val Pro Gly Gly Gly Val Pro Gly Ala Gly
                   85                  90                  95
   Val Pro Gly Gly Gly Val Pro Gly Val Gly Val Pro Gly Val Gly Val
               100                 105                 110
   Pro Gly Gly Gly Val Pro Gly Ala Gly Val Pro Gly Val Gly Val Pro
           115                 120                 125
   Gly Val Gly Val Pro Gly Val Gly Val Pro Gly Gly Gly Val Pro Gly
       130                 135                 140
   Ala Gly Val Pro Gly Gly Gly Val Pro Gly Val Gly Val Pro Gly Val
   145                 150                 155                 160
   Gly Val Pro Gly Gly Gly Val Pro Gly Ala Gly Val Pro Gly Val Gly
                   165                 170                 175
   Val Pro Gly Val Gly Val Pro Gly Val Gly Val Pro Gly Gly Gly Val
               180                 185                 190
   Pro Gly Ala Gly Val Pro Gly Gly Gly Val Pro Gly Val Gly Val Pro
           195                 200                 205
   Gly Val Gly Val Pro Gly Gly Gly Val Pro Gly Ala Gly Val Pro Gly
       210                 215                 220
   Val Gly Val Pro Gly Val Gly Val Pro Gly Val Gly Val Pro Gly Gly
   225                 230                 235                 240
   Gly Val Pro Gly Ala Gly Val Pro Gly Gly Gly Val Pro Gly Val Gly
                   245                 250                 255
   Val Pro Gly Val Gly Val Pro Gly Gly Gly Val Pro Gly Ala Gly Val
   260 265 270
   Pro Gly Val Gly Val Pro Gly Val Gly Val Pro Gly Val Gly Val Pro
   275 280 285
   Gly Gly Gly Val Pro Gly Ala Gly Val Pro Gly Gly Gly Val Pro Gly
   290 295 300
   Val Gly Val Pro Gly Val Gly Val Pro Gly Gly Gly Val Pro Gly Ala
   305 310 315 320
   Gly Val Pro Gly Val Gly Val Pro Gly Val Gly Val Pro Gly Val Gly
   325 330 335
   Val Pro Gly Gly Gly Val Pro Gly Ala Gly Val Pro Gly Gly Gly Val
   340 345 350
   Pro Gly Val Gly Val Pro Gly Val Gly Val Pro Gly Gly Gly Val Pro
   355 360 365
   Gly Ala Gly Val Pro Gly Val Gly Val Pro Gly Val Gly Val Pro Gly
   370 375 380
   Val Gly Val Pro Gly Gly Gly Val Pro Gly Ala Gly Val Pro Gly Gly
   385 390 395 400
   Gly Val Pro Gly Val Gly Val Pro Gly Val Gly Val Pro Gly Gly Gly
   405 410 415
   Val Pro Gly Ala Gly Val Pro Gly Val Gly Val Pro Gly Val Gly Val
   420 425 430
   Pro Gly Val Gly Val Pro Gly Gly Gly Val Pro Gly Ala Gly Val Pro
   435 440 445
   Gly Gly Gly Val Pro Gly Val Gly Val Pro Gly Val Gly Val Pro Gly
   450 455 460
   Gly Gly Val Pro Gly Ala Gly Val Pro Gly Val Gly Val Pro Gly Val
   465 470 475 480
   Gly Val Pro Gly Val Gly Val Pro Gly Gly Gly Val Pro Gly Ala Gly
   485 490 495
   Val Pro Gly Gly Gly Val Pro Gly Val Gly Val Pro Gly Val Gly Val
   500 505 510
   Pro Gly Gly Gly Val Pro Gly Ala Gly Val Pro Gly Val Gly Val Pro
   515 520 525
   Gly Val Gly Val Pro Gly Val Gly Val Pro Gly Gly Gly Val Pro Gly
   530 535 540
   Ala Gly Val Pro Gly Gly Gly Val Pro Gly Val Gly Val Pro Gly Val
   545 550 555 560
   Gly Val Pro Gly Gly Gly Val Pro Gly Ala Gly Val Pro Gly Val Gly
   565 570 575
   Val Pro Gly Val Gly Val Pro Gly Val Gly Val Pro Gly Gly Gly Val
   580 585 590
   Pro Gly Ala Gly Val Pro Gly Gly Gly Val Pro Gly Val Gly Met His
   595 600 605
   Leu Ser Gln Leu Leu Ala Cys Ala Leu Leu Leu Thr Leu Leu Ser Leu
   610 615 620
   Arg Pro Ser Glu Ala Lys Pro Gly Ala Pro Pro Lys Val Pro Arg Thr
   625 630 635 640
   Pro Pro Ala Glu Glu Leu Ala Glu Pro Gln Ala Ala Gly Gly Gly Gln
   645 650 655
   Lys Lys Gly Asp Lys Ala Pro Gly Gly Gly Gly Ala Asn Leu Lys Gly
   660 665 670
   Asp Arg Ser Arg Leu Leu Arg Asp Leu Arg Val Asp Thr Lys Ser Arg
   675 680 685
   Ala Ala Trp Ala Arg Leu Leu Gln Glu His Pro Asn Ala Arg Lys Tyr
   690 695 700
   Lys Gly Ala Asn Lys Lys Gly Leu Ser Lys Gly Cys Phe Gly Leu Lys
   705 710 715 720
   Leu Asp Arg Ile Gly Ser Met Ser Gly Leu Gly Cys
   725 730
<210> 57
   <211> 643
   <212> PRT
   <213> Artificial Sequence
<220>
   <223> CNP37 ELP1-120 fusion
<400> 57
   Gln Glu His Pro Asn Ala Arg Lys Tyr Lys Gly Ala Asn Lys Lys Gly
   1               5                   10                  15
   Leu Ser Lys Gly Cys Phe Gly Leu Lys Leu Asp Arg Ile Gly Ser Met
               20                  25                  30
   Ser Gly Leu Gly Cys Gly Val Pro Gly Val Gly Val Pro Gly Val Gly
           35                  40                  45
   Val Pro Gly Gly Gly Val Pro Gly Ala Gly Val Pro Gly Val Gly Val
       50                  55                  60
   Pro Gly Val Gly Val Pro Gly Val Gly Val Pro Gly Gly Gly Val Pro
   65                  70                  75                  80
   Gly Ala Gly Val Pro Gly Gly Gly Val Pro Gly Val Gly Val Pro Gly
                   85                  90                  95
   Val Gly Val Pro Gly Gly Gly Val Pro Gly Ala Gly Val Pro Gly Val
               100                 105                 110
   Gly Val Pro Gly Val Gly Val Pro Gly Val Gly Val Pro Gly Gly Gly
           115                 120                 125
   Val Pro Gly Ala Gly Val Pro Gly Gly Gly Val Pro Gly Val Gly Val
       130                 135                 140
   Pro Gly Val Gly Val Pro Gly Gly Gly Val Pro Gly Ala Gly Val Pro
   145                 150                 155                 160
   Gly Val Gly Val Pro Gly Val Gly Val Pro Gly Val Gly Val Pro Gly
                   165                 170                 175
   Gly Gly Val Pro Gly Ala Gly Val Pro Gly Gly Gly Val Pro Gly Val
               180                 185                 190
   Gly Val Pro Gly Val Gly Val Pro Gly Gly Gly Val Pro Gly Ala Gly
           195                 200                 205
   Val Pro Gly Val Gly Val Pro Gly Val Gly Val Pro Gly Val Gly Val
       210                 215                 220
   Pro Gly Gly Gly Val Pro Gly Ala Gly Val Pro Gly Gly Gly Val Pro
   225                 230                 235                 240
   Gly Val Gly Val Pro Gly Val Gly Val Pro Gly Gly Gly Val Pro Gly
                   245                 250                 255
   Ala Gly Val Pro Gly Val Gly Val Pro Gly Val Gly Val Pro Gly Val
               260                 265                 270
   Gly Val Pro Gly Gly Gly Val Pro Gly Ala Gly Val Pro Gly Gly Gly
           275                 280                 285
   Val Pro Gly Val Gly Val Pro Gly Val Gly Val Pro Gly Gly Gly Val
       290                 295                 300
   Pro Gly Ala Gly Val Pro Gly Val Gly Val Pro Gly Val Gly Val Pro
   305                 310                 315                 320
   Gly Val Gly Val Pro Gly Gly Gly Val Pro Gly Ala Gly Val Pro Gly
                   325                 330                 335
   Gly Gly Val Pro Gly Val Gly Val Pro Gly Val Gly Val Pro Gly Gly
               340                 345                 350
   Gly Val Pro Gly Ala Gly Val Pro Gly Val Gly Val Pro Gly Val Gly
           355                 360                 365
   Val Pro Gly Val Gly Val Pro Gly Gly Gly Val Pro Gly Ala Gly Val
       370                 375                 380
   Pro Gly Gly Gly Val Pro Gly Val Gly Val Pro Gly Val Gly Val Pro
   385                 390                 395                 400
   Gly Gly Gly Val Pro Gly Ala Gly Val Pro Gly Val Gly Val Pro Gly
                   405                 410                 415
   Val Gly Val Pro Gly Val Gly Val Pro Gly Gly Gly Val Pro Gly Ala
               420                 425                 430
   Gly Val Pro Gly Gly Gly Val Pro Gly Val Gly Val Pro Gly Val Gly
           435                 440                 445
   Val Pro Gly Gly Gly Val Pro Gly Ala Gly Val Pro Gly Val Gly Val
       450                 455                 460
   Pro Gly Val Gly Val Pro Gly Val Gly Val Pro Gly Gly Gly Val Pro
   465                 470                 475                 480
   Gly Ala Gly Val Pro Gly Gly Gly Val Pro Gly Val Gly Val Pro Gly
                   485                 490                 495
   Val Gly Val Pro Gly Gly Gly Val Pro Gly Ala Gly Val Pro Gly Val
               500                 505                 510
   Gly Val Pro Gly Val Gly Val Pro Gly Val Gly Val Pro Gly Gly Gly
           515                 520                 525
   Val Pro Gly Ala Gly Val Pro Gly Gly Gly Val Pro Gly Val Gly Val
       530                 535                 540
   Pro Gly Val Gly Val Pro Gly Gly Gly Val Pro Gly Ala Gly Val Pro
   545                 550                 555                 560
   Gly Val Gly Val Pro Gly Val Gly Val Pro Gly Val Gly Val Pro Gly
                   565                 570                 575
   Gly Gly Val Pro Gly Ala Gly Val Pro Gly Gly Gly Val Pro Gly Val
               580                 585                 590
   Gly Val Pro Gly Val Gly Val Pro Gly Gly Gly Val Pro Gly Ala Gly
           595                 600                 605
   Val Pro Gly Val Gly Val Pro Gly Val Gly Val Pro Gly Val Gly Val
       610                 615                 620
   Pro Gly Gly Gly Val Pro Gly Ala Gly Val Pro Gly Gly Gly Val Pro
   625                 630                 635                 640
   Gly Trp Pro
<210> 58
   <211> 628
   <212> PRT
   <213> Artificial Sequence
<220>
   <223> pPE0497-Exendin-4 (9-31)-ELP1-120
<400> 58
   Asp Leu Ser Lys Gln Met Glu Glu Glu Ala Val Arg Leu Phe Ile Glu
   1               5                   10                  15
   Trp Leu Lys Asn Gly Gly Pro Val Pro Gly Val Gly Val Pro Gly Val
               20                  25                  30
   Gly Val Pro Gly Gly Gly Val Pro Gly Ala Gly Val Pro Gly Val Gly
           35                  40                  45
   Val Pro Gly Val Gly Val Pro Gly Val Gly Val Pro Gly Gly Gly Val
       50                  55                  60
   Pro Gly Ala Gly Val Pro Gly Gly Gly Val Pro Gly Val Gly Val Pro
   65                  70                  75                  80
   Gly Val Gly Val Pro Gly Gly Gly Val Pro Gly Ala Gly Val Pro Gly
                   85                  90                  95
   Val Gly Val Pro Gly Val Gly Val Pro Gly Val Gly Val Pro Gly Gly
               100                 105                 110
   Gly Val Pro Gly Ala Gly Val Pro Gly Gly Gly Val Pro Gly Val Gly
           115                 120                 125
   Val Pro Gly Val Gly Val Pro Gly Gly Gly Val Pro Gly Ala Gly Val
       130                 135                 140
   Pro Gly Val Gly Val Pro Gly Val Gly Val Pro Gly Val Gly Val Pro
   145                 150                 155                 160
   Gly Gly Gly Val Pro Gly Ala Gly Val Pro Gly Gly Gly Val Pro Gly
                   165                 170                 175
   Val Gly Val Pro Gly Val Gly Val Pro Gly Gly Gly Val Pro Gly Ala
               180                 185                 190
   Gly Val Pro Gly Val Gly Val Pro Gly Val Gly Val Pro Gly Val Gly
           195                 200                 205
   Val Pro Gly Gly Gly Val Pro Gly Ala Gly Val Pro Gly Gly Gly Val
       210                 215                 220
   Pro Gly Val Gly Val Pro Gly Val Gly Val Pro Gly Gly Gly Val Pro
   225                 230                 235                 240
   Gly Ala Gly Val Pro Gly Val Gly Val Pro Gly Val Gly Val Pro Gly
                   245                 250                 255
   Val Gly Val Pro Gly Gly Gly Val Pro Gly Ala Gly Val Pro Gly Gly
               260                 265                 270
   Gly Val Pro Gly Val Gly Val Pro Gly Val Gly Val Pro Gly Gly Gly
           275                 280                 285
   Val Pro Gly Ala Gly Val Pro Gly Val Gly Val Pro Gly Val Gly Val
   290 295 300
   Pro Gly Val Gly Val Pro Gly Gly Gly Val Pro Gly Ala Gly Val Pro
   305 310 315 320
   Gly Gly Gly Val Pro Gly Val Gly Val Pro Gly Val Gly Val Pro Gly
   325 330 335
   Gly Gly Val Pro Gly Ala Gly Val Pro Gly Val Gly Val Pro Gly Val
   340 345 350
   Gly Val Pro Gly Val Gly Val Pro Gly Gly Gly Val Pro Gly Ala Gly
   355 360 365
   Val Pro Gly Gly Gly Val Pro Gly Val Gly Val Pro Gly Val Gly Val
   370 375 380
   Pro Gly Gly Gly Val Pro Gly Ala Gly Val Pro Gly Val Gly Val Pro
   385 390 395 400
   Gly Val Gly Val Pro Gly Val Gly Val Pro Gly Gly Gly Val Pro Gly
   405 410 415
   Ala Gly Val Pro Gly Gly Gly Val Pro Gly Val Gly Val Pro Gly Val
   420 425 430
   Gly Val Pro Gly Gly Gly Val Pro Gly Ala Gly Val Pro Gly Val Gly
   435 440 445
   Val Pro Gly Val Gly Val Pro Gly Val Gly Val Pro Gly Gly Gly Val
   450 455 460
   Pro Gly Ala Gly Val Pro Gly Gly Gly Val Pro Gly Val Gly Val Pro
   465 470 475 480
   Gly Val Gly Val Pro Gly Gly Gly Val Pro Gly Ala Gly Val Pro Gly
   485 490 495
   Val Gly Val Pro Gly Val Gly Val Pro Gly Val Gly Val Pro Gly Gly
   500 505 510
   Gly Val Pro Gly Ala Gly Val Pro Gly Gly Gly Val Pro Gly Val Gly
   515 520 525
   Val Pro Gly Val Gly Val Pro Gly Gly Gly Val Pro Gly Ala Gly Val
   530 535 540
   Pro Gly Val Gly Val Pro Gly Val Gly Val Pro Gly Val Gly Val Pro
   545                 550                 555                 560
   Gly Gly Gly Val Pro Gly Ala Gly Val Pro Gly Gly Gly Val Pro Gly
                   565                 570                 575
   Val Gly Val Pro Gly Val Gly Val Pro Gly Gly Gly Val Pro Gly Ala
               580                 585                 590
   Gly Val Pro Gly Val Gly Val Pro Gly Val Gly Val Pro Gly Val Gly
           595                 600                 605
   Val Pro Gly Gly Gly Val Pro Gly Ala Gly Val Pro Gly Gly Gly Val
       610                 615                 620
   Pro Gly Trp Pro
   625
<210> 59
   <211> 636
   <212> PRT
   <213> Artificial Sequence
<220>
   <223> pPE0496-Exendin-4 (9-39) ELP1-120
<400> 59
   Asp Leu Ser Lys Gln Met Glu Glu Glu Ala Val Arg Leu Phe Ile Glu
   1               5                   10                  15
   Trp Leu Lys Asn Gly Gly Pro Ser Ser Gly Ala Pro Pro Pro Ser Val
               20                  25                  30
   Pro Gly Val Gly Val Pro Gly Val Gly Val Pro Gly Gly Gly Val Pro
           35                  40                  45
   Gly Ala Gly Val Pro Gly Val Gly Val Pro Gly Val Gly Val Pro Gly
       50                  55                  60
   Val Gly Val Pro Gly Gly Gly Val Pro Gly Ala Gly Val Pro Gly Gly
   65                  70                  75                  80
   Gly Val Pro Gly Val Gly Val Pro Gly Val Gly Val Pro Gly Gly Gly
                   85                  90                  95
   Val Pro Gly Ala Gly Val Pro Gly Val Gly Val Pro Gly Val Gly Val
               100                 105                 110
   Pro Gly Val Gly Val Pro Gly Gly Gly Val Pro Gly Ala Gly Val Pro
           115                 120                 125
   Gly Gly Gly Val Pro Gly Val Gly Val Pro Gly Val Gly Val Pro Gly
       130                 135                 140
   Gly Gly Val Pro Gly Ala Gly Val Pro Gly Val Gly Val Pro Gly Val
   145                 150                 155                 160
   Gly Val Pro Gly Val Gly Val Pro Gly Gly Gly Val Pro Gly Ala Gly
                   165                 170                 175
   Val Pro Gly Gly Gly Val Pro Gly Val Gly Val Pro Gly Val Gly Val
               180                 185                 190
   Pro Gly Gly Gly Val Pro Gly Ala Gly Val Pro Gly Val Gly Val Pro
           195                 200                 205
   Gly Val Gly Val Pro Gly Val Gly Val Pro Gly Gly Gly Val Pro Gly
       210                 215                 220
   Ala Gly Val Pro Gly Gly Gly Val Pro Gly Val Gly Val Pro Gly Val
   225                 230                 235                 240
   Gly Val Pro Gly Gly Gly Val Pro Gly Ala Gly Val Pro Gly Val Gly
                   245                 250                 255
   Val Pro Gly Val Gly Val Pro Gly Val Gly Val Pro Gly Gly Gly Val
               260                 265                 270
   Pro Gly Ala Gly Val Pro Gly Gly Gly Val Pro Gly Val Gly Val Pro
           275                 280                 285
   Gly Val Gly Val Pro Gly Gly Gly Val Pro Gly Ala Gly Val Pro Gly
       290                 295                 300
   Val Gly Val Pro Gly Val Gly Val Pro Gly Val Gly Val Pro Gly Gly
   305                 310                 315                 320
   Gly Val Pro Gly Ala Gly Val Pro Gly Gly Gly Val Pro Gly Val Gly
                   325                 330                 335
   Val Pro Gly Val Gly Val Pro Gly Gly Gly Val Pro Gly Ala Gly Val
               340                 345                 350
   Pro Gly Val Gly Val Pro Gly Val Gly Val Pro Gly Val Gly Val Pro
           355                 360                 365
   Gly Gly Gly Val Pro Gly Ala Gly Val Pro Gly Gly Gly Val Pro Gly
       370                 375                 380
   Val Gly Val Pro Gly Val Gly Val Pro Gly Gly Gly Val Pro Gly Ala
   385                 390                 395                 400
   Gly Val Pro Gly Val Gly Val Pro Gly Val Gly Val Pro Gly Val Gly
                   405                 410                 415
   Val Pro Gly Gly Gly Val Pro Gly Ala Gly Val Pro Gly Gly Gly Val
               420                 425                 430
   Pro Gly Val Gly Val Pro Gly Val Gly Val Pro Gly Gly Gly Val Pro
           435                 440                 445
   Gly Ala Gly Val Pro Gly Val Gly Val Pro Gly Val Gly Val Pro Gly
       450                 455                 460
   Val Gly Val Pro Gly Gly Gly Val Pro Gly Ala Gly Val Pro Gly Gly
   465                 470                 475                 480
   Gly Val Pro Gly Val Gly Val Pro Gly Val Gly Val Pro Gly Gly Gly
                   485                 490                 495
   Val Pro Gly Ala Gly Val Pro Gly Val Gly Val Pro Gly Val Gly Val
               500                 505                 510
   Pro Gly Val Gly Val Pro Gly Gly Gly Val Pro Gly Ala Gly Val Pro
           515                 520                 525
   Gly Gly Gly Val Pro Gly Val Gly Val Pro Gly Val Gly Val Pro Gly
       530                 535                 540
   Gly Gly Val Pro Gly Ala Gly Val Pro Gly Val Gly Val Pro Gly Val
   545                 550                 555                 560
   Gly Val Pro Gly Val Gly Val Pro Gly Gly Gly Val Pro Gly Ala Gly
                   565                 570                 575
   Val Pro Gly Gly Gly Val Pro Gly Val Gly Val Pro Gly Val Gly Val
               580                 585                 590
   Pro Gly Gly Gly Val Pro Gly Ala Gly Val Pro Gly Val Gly Val Pro
           595                 600                 605
   Gly Val Gly Val Pro Gly Val Gly Val Pro Gly Gly Gly Val Pro Gly
       610                 615                 620
   Ala Gly Val Pro Gly Gly Gly Val Pro Gly Trp Pro
   625                 630                 635
<210> 60
   <211> 22
   <212> PRT
   <213> Artificial Sequence
<220>
   <223> Human recombinant Exendin-4 (9-30)
<400> 60
   Asp Leu Ser Lys Gln Met Glu Glu Glu Ala Val Arg Leu Phe Ile Glu
   1               5                   10                  15
   Trp Leu Lys Asn Gly Gly
               20
<210> 61
   <211> 24
   <212> PRT
   <213> Artificial Sequence
<220>
   <223> Human recombinant M-Exendin-4 (9-31)
<400> 61
   Met Asp Leu Ser Lys Gln Met Glu Glu Glu Ala Val Arg Leu Phe Ile
   1               5                   10                  15
   Glu Trp Leu Lys Asn Gly Gly Pro
               20
<210> 62
   <211> 32
   <212> PRT
   <213> Artificial Sequence
<220>
   <223> Human recombinant M-Exendin-4 (9-39)
<400> 62
   Met Asp Leu Ser Lys Gln Met Glu Glu Glu Ala Val Arg Leu Phe Ile
   1               5                   10                  15
   Glu Trp Leu Lys Asn Gly Gly Pro Ser Ser Gly Ala Pro Pro Pro Ser
               20                  25                  30
<210> 63
   <211> 23
   <212> PRT
   <213> Artificial Sequence
<220>
   <223> Human recombinant M-Exendin-4 (9-30)
<400> 63
   Met Asp Leu Ser Lys Gln Met Glu Glu Glu Ala Val Arg Leu Phe Ile
   1               5                   10                  15
   Glu Trp Leu Lys Asn Gly Gly
               20
<210> 64
   <211> 21
   <212> PRT
   <213> Artificial Sequence
<220>
   <223> Human recombinant GLP-1 (9-29)
<400> 64
   Asp Val Ser Ser Tyr Leu Glu Gly Gln Ala Ala Lys Glu Phe Ile Ala
   1               5                   10                  15
   Trp Leu Val Lys Gly
               20
<210> 65
   <211> 31
   <212> PRT
   <213> Artificial Sequence
<220>
   <223> Human recombinant GLP-1-exendin-4
<400> 65
   Asp Val Ser Ser Tyr Leu Glu Gly Gln Ala Ala Lys Glu Phe Ile Ala
   1               5                   10                  15
   Trp Leu Val Lys Gly Arg Pro Ser Ser Gly Ala Pro Pro Pro Ser
               20                  25                  30
<210> 66
   <211> 22
   <212> PRT
   <213> Artificial Sequence
<220>
   <223> Human recombinant Jant-4 (9-30)
<400> 66
   Asp Val Ser Ser Tyr Leu Glu Glu Gln Ala Val Arg Glu Phe Ile Ala
   1               5                   10                  15
   Trp Leu Val Lys Gly Arg
   20
<210> 67
   <211> 31
   <212> PRT
   <213> Artificial Sequence
<220>
   <223> Human recombinant Jant-4 (9-39)
<400> 67
   Asp Val Ser Ser Tyr Leu Glu Glu Gln Ala Val Arg Glu Phe Ile Ala
   1 5 10 15
   Trp Leu Val Lys Gly Arg Pro Ser Ser Gly Ala Pro Pro Pro Ser
   20 25 30
<210> 68
   <211> 33
   <212> PRT
   <213> Artificial Sequence
<220>
   <223> Human recombinant GLP-2
<400> 68
   His Ala Asp Gly Ser Phe Ser Asp Glu Met Asn Thr Ile Leu Asp Asn
   1 5 10 15
   Leu Ala Ala Arg Asp Phe Ile Asn Trp Leu Ile Gln Thr Lys Ile Thr
   20 25 30
   Asp
<210> 69
   <211> 638
   <212> PRT
   <213> Artificial Sequence
<220>
   <223> pPE0527-GLP-2-ELP1-120
<400> 69
   His Ala Asp Gly Ser Phe Ser Asp Glu Met Asn Thr Ile Leu Asp Asn
   1 5 10 15
   Leu Ala Ala Arg Asp Phe Ile Asn Trp Leu Ile Gln Thr Lys Ile Thr
   20 25 30
   Asp Val Pro Gly Val Gly Val Pro Gly Val Gly Val Pro Gly Gly Gly
           35                  40                  45
   Val Pro Gly Ala Gly Val Pro Gly Val Gly Val Pro Gly Val Gly Val
       50                  55                  60
   Pro Gly Val Gly Val Pro Gly Gly Gly Val Pro Gly Ala Gly Val Pro
   65                  70                  75                  80
   Gly Gly Gly Val Pro Gly Val Gly Val Pro Gly Val Gly Val Pro Gly
                   85                  90                  95
   Gly Gly Val Pro Gly Ala Gly Val Pro Gly Val Gly Val Pro Gly Val
               100                 105                 110
   Gly Val Pro Gly Val Gly Val Pro Gly Gly Gly Val Pro Gly Ala Gly
           115                 120                 125
   Val Pro Gly Gly Gly Val Pro Gly Val Gly Val Pro Gly Val Gly Val
       130                 135                 140
   Pro Gly Gly Gly Val Pro Gly Ala Gly Val Pro Gly Val Gly Val Pro
   145                 150                 155                 160
   Gly Val Gly Val Pro Gly Val Gly Val Pro Gly Gly Gly Val Pro Gly
                   165                 170                 175
   Ala Gly Val Pro Gly Gly Gly Val Pro Gly Val Gly Val Pro Gly Val
               180                 185                 190
   Gly Val Pro Gly Gly Gly Val Pro Gly Ala Gly Val Pro Gly Val Gly
           195                 200                 205
   Val Pro Gly Val Gly Val Pro Gly Val Gly Val Pro Gly Gly Gly Val
       210                 215                 220
   Pro Gly Ala Gly Val Pro Gly Gly Gly Val Pro Gly Val Gly Val Pro
   225                 230                 235                 240
   Gly Val Gly Val Pro Gly Gly Gly Val Pro Gly Ala Gly Val Pro Gly
                   245                 250                 255
   Val Gly Val Pro Gly Val Gly Val Pro Gly Val Gly Val Pro Gly Gly
               260                 265                 270
   Gly Val Pro Gly Ala Gly Val Pro Gly Gly Gly Val Pro Gly Val Gly
           275                 280                 285
   Val Pro Gly Val Gly Val Pro Gly Gly Gly Val Pro Gly Ala Gly Val
   290 295 300
   Pro Gly Val Gly Val Pro Gly Val Gly Val Pro Gly Val Gly Val Pro
   305 310 315 320
   Gly Gly Gly Val Pro Gly Ala Gly Val Pro Gly Gly Gly Val Pro Gly
   325 330 335
   Val Gly Val Pro Gly Val Gly Val Pro Gly Gly Gly Val Pro Gly Ala
   340 345 350
   Gly Val Pro Gly Val Gly Val Pro Gly Val Gly Val Pro Gly Val Gly
   355 360 365
   Val Pro Gly Gly Gly Val Pro Gly Ala Gly Val Pro Gly Gly Gly Val
   370 375 380
   Pro Gly Val Gly Val Pro Gly Val Gly Val Pro Gly Gly Gly Val Pro
   385 390 395 400
   Gly Ala Gly Val Pro Gly Val Gly Val Pro Gly Val Gly Val Pro Gly
   405 410 415
   Val Gly Val Pro Gly Gly Gly Val Pro Gly Ala Gly Val Pro Gly Gly
   420 425 430
   Gly Val Pro Gly Val Gly Val Pro Gly Val Gly Val Pro Gly Gly Gly
   435 440 445
   Val Pro Gly Ala Gly Val Pro Gly Val Gly Val Pro Gly Val Gly Val
   450 455 460
   Pro Gly Val Gly Val Pro Gly Gly Gly Val Pro Gly Ala Gly Val Pro
   465 470 475 480
   Gly Gly Gly Val Pro Gly Val Gly Val Pro Gly Val Gly Val Pro Gly
   485 490 495
   Gly Gly Val Pro Gly Ala Gly Val Pro Gly Val Gly Val Pro Gly Val
   500 505 510
   Gly Val Pro Gly Val Gly Val Pro Gly Gly Gly Val Pro Gly Ala Gly
   515 520 525
   Val Pro Gly Gly Gly Val Pro Gly Val Gly Val Pro Gly Val Gly Val
   530 535 540
   Pro Gly Gly Gly Val Pro Gly Ala Gly Val Pro Gly Val Gly Val Pro
   545 550 555 560
   Gly Val Gly Val Pro Gly Val Gly Val Pro Gly Gly Gly Val Pro Gly
   565 570 575
   Ala Gly Val Pro Gly Gly Gly Val Pro Gly Val Gly Val Pro Gly Val
   580 585 590
   Gly Val Pro Gly Gly Gly Val Pro Gly Ala Gly Val Pro Gly Val Gly
   595 600 605
   Val Pro Gly Val Gly Val Pro Gly Val Gly Val Pro Gly Gly Gly Val
   610 615 620
   Pro Gly Ala Gly Val Pro Gly Gly Gly Val Pro Gly Trp Pro
   625 630 635
<210> 70
   <211> 33
   <212> PRT
   <213> Artificial Sequence
<220>
   <223> Human recombinant GLP-2(A2G)
<400> 70
   His Gly Asp Gly Ser Phe Ser Asp Glu Met Asn Thr Ile Leu Asp Asn
   1 5 10 15
   Leu Ala Ala Arg Asp Phe Ile Asn Trp Leu Ile Gln Thr Lys Ile Thr
   20 25 30
   Asp
<210> 71
   <211> 638
   <212> PRT
   <213> Artificial Sequence
<220>
   <223> pPE0503-GLP-2(A2G)-ELP1-120
<400> 71
   His Gly Asp Gly Ser Phe Ser Asp Glu Met Asn Thr Ile Leu Asp Asn
   1 5 10 15
   Leu Ala Ala Arg Asp Phe Ile Asn Trp Leu Ile Gln Thr Lys Ile Thr
               20                  25                  30
   Asp Val Pro Gly Val Gly Val Pro Gly Val Gly Val Pro Gly Gly Gly
           35                  40                  45
   Val Pro Gly Ala Gly Val Pro Gly Val Gly Val Pro Gly Val Gly Val
       50                  55                  60
   Pro Gly Val Gly Val Pro Gly Gly Gly Val Pro Gly Ala Gly Val Pro
   65                  70                  75                  80
   Gly Gly Gly Val Pro Gly Val Gly Val Pro Gly Val Gly Val Pro Gly
                   85                  90                  95
   Gly Gly Val Pro Gly Ala Gly Val Pro Gly Val Gly Val Pro Gly Val
               100                 105                 110
   Gly Val Pro Gly Val Gly Val Pro Gly Gly Gly Val Pro Gly Ala Gly
           115                 120                 125
   Val Pro Gly Gly Gly Val Pro Gly Val Gly Val Pro Gly Val Gly Val
       130                 135                 140
   Pro Gly Gly Gly Val Pro Gly Ala Gly Val Pro Gly Val Gly Val Pro
   145                 150                 155                 160
   Gly Val Gly Val Pro Gly Val Gly Val Pro Gly Gly Gly Val Pro Gly
                   165                 170                 175
   Ala Gly Val Pro Gly Gly Gly Val Pro Gly Val Gly Val Pro Gly Val
               180                 185                 190
   Gly Val Pro Gly Gly Gly Val Pro Gly Ala Gly Val Pro Gly Val Gly
           195                 200                 205
   Val Pro Gly Val Gly Val Pro Gly Val Gly Val Pro Gly Gly Gly Val
       210                 215                 220
   Pro Gly Ala Gly Val Pro Gly Gly Gly Val Pro Gly Val Gly Val Pro
   225                 230                 235                 240
   Gly Val Gly Val Pro Gly Gly Gly Val Pro Gly Ala Gly Val Pro Gly
                   245                 250                 255
   Val Gly Val Pro Gly Val Gly Val Pro Gly Val Gly Val Pro Gly Gly
               260                 265                 270
   Gly Val Pro Gly Ala Gly Val Pro Gly Gly Gly Val Pro Gly Val Gly
           275                 280                 285
   Val Pro Gly Val Gly Val Pro Gly Gly Gly Val Pro Gly Ala Gly Val
       290                 295                 300
   Pro Gly Val Gly Val Pro Gly Val Gly Val Pro Gly Val Gly Val Pro
   305                 310                 315                 320
   Gly Gly Gly Val Pro Gly Ala Gly Val Pro Gly Gly Gly Val Pro Gly
                   325                 330                 335
   Val Gly Val Pro Gly Val Gly Val Pro Gly Gly Gly Val Pro Gly Ala
               340                 345                 350
   Gly Val Pro Gly Val Gly Val Pro Gly Val Gly Val Pro Gly Val Gly
           355                 360                 365
   Val Pro Gly Gly Gly Val Pro Gly Ala Gly Val Pro Gly Gly Gly Val
       370                 375                 380
   Pro Gly Val Gly Val Pro Gly Val Gly Val Pro Gly Gly Gly Val Pro
   385                 390                 395                 400
   Gly Ala Gly Val Pro Gly Val Gly Val Pro Gly Val Gly Val Pro Gly
                   405                 410                 415
   Val Gly Val Pro Gly Gly Gly Val Pro Gly Ala Gly Val Pro Gly Gly
               420                 425                 430
   Gly Val Pro Gly Val Gly Val Pro Gly Val Gly Val Pro Gly Gly Gly
           435                 440                 445
   Val Pro Gly Ala Gly Val Pro Gly Val Gly Val Pro Gly Val Gly Val
       450                 455                 460
   Pro Gly Val Gly Val Pro Gly Gly Gly Val Pro Gly Ala Gly Val Pro
   465                 470                 475                 480
   Gly Gly Gly Val Pro Gly Val Gly Val Pro Gly Val Gly Val Pro Gly
                   485                 490                 495
   Gly Gly Val Pro Gly Ala Gly Val Pro Gly Val Gly Val Pro Gly Val
               500                 505                 510
   Gly Val Pro Gly Val Gly Val Pro Gly Gly Gly Val Pro Gly Ala Gly
           515                 520                 525
   Val Pro Gly Gly Gly Val Pro Gly Val Gly Val Pro Gly Val Gly Val
       530                 535                 540
   Pro Gly Gly Gly Val Pro Gly Ala Gly Val Pro Gly Val Gly Val Pro
   545                 550                 555                 560
   Gly Val Gly Val Pro Gly Val Gly Val Pro Gly Gly Gly Val Pro Gly
                   565                 570                 575
   Ala Gly Val Pro Gly Gly Gly Val Pro Gly Val Gly Val Pro Gly Val
               580                 585                 590
   Gly Val Pro Gly Gly Gly Val Pro Gly Ala Gly Val Pro Gly Val Gly
           595                 600                 605
   Val Pro Gly Val Gly Val Pro Gly Val Gly Val Pro Gly Gly Gly Val
       610                 615                 620
   Pro Gly Ala Gly Val Pro Gly Gly Gly Val Pro Gly Trp Pro
   625                 630                 635
<210> 72
   <211> 39
   <212> PRT
   <213> Artificial Sequence
<220>
   <223> Human recombinant GLP-2(A2G)-(GGS)2x linker
<400> 72
   His Gly Asp Gly Ser Phe Ser Asp Glu Met Asn Thr Ile Leu Asp Asn
   1               5                   10                  15
   Leu Ala Ala Arg Asp Phe Ile Asn Trp Leu Ile Gln Thr Lys Ile Thr
               20                  25                  30
   Asp Gly Gly Ser Gly Gly Ser
           35
<210> 73
   <211> 644
   <212> PRT
   <213> Artificial Sequence
<220>
   <223> pPE0526 GLP-2(A2G)-(GGS)2x linker ELP1-120
<400> 73
   His Gly Asp Gly Ser Phe Ser Asp Glu Met Asn Thr Ile Leu Asp Asn
   1               5                   10                  15
   Leu Ala Ala Arg Asp Phe Ile Asn Trp Leu Ile Gln Thr Lys Ile Thr
               20                  25                  30
   Asp Gly Gly Ser Gly Gly Ser Val Pro Gly Val Gly Val Pro Gly Val
           35                  40                  45
   Gly Val Pro Gly Gly Gly Val Pro Gly Ala Gly Val Pro Gly Val Gly
       50                  55                  60
   Val Pro Gly Val Gly Val Pro Gly Val Gly Val Pro Gly Gly Gly Val
   65                  70                  75                  80
   Pro Gly Ala Gly Val Pro Gly Gly Gly Val Pro Gly Val Gly Val Pro
                   85                  90                  95
   Gly Val Gly Val Pro Gly Gly Gly Val Pro Gly Ala Gly Val Pro Gly
               100                 105                 110
   Val Gly Val Pro Gly Val Gly Val Pro Gly Val Gly Val Pro Gly Gly
           115                 120                 125
   Gly Val Pro Gly Ala Gly Val Pro Gly Gly Gly Val Pro Gly Val Gly
       130                 135                 140
   Val Pro Gly Val Gly Val Pro Gly Gly Gly Val Pro Gly Ala Gly Val
   145                 150                 155                 160
   Pro Gly Val Gly Val Pro Gly Val Gly Val Pro Gly Val Gly Val Pro
                   165                 170                 175
   Gly Gly Gly Val Pro Gly Ala Gly Val Pro Gly Gly Gly Val Pro Gly
               180                 185                 190
   Val Gly Val Pro Gly Val Gly Val Pro Gly Gly Gly Val Pro Gly Ala
           195                 200                 205
   Gly Val Pro Gly Val Gly Val Pro Gly Val Gly Val Pro Gly Val Gly
       210                 215                 220
   Val Pro Gly Gly Gly Val Pro Gly Ala Gly Val Pro Gly Gly Gly Val
   225                 230                 235                 240
   Pro Gly Val Gly Val Pro Gly Val Gly Val Pro Gly Gly Gly Val Pro
                   245                 250                 255
   Gly Ala Gly Val Pro Gly Val Gly Val Pro Gly Val Gly Val Pro Gly
               260                 265                 270
   Val Gly Val Pro Gly Gly Gly Val Pro Gly Ala Gly Val Pro Gly Gly
           275                 280                 285
   Gly Val Pro Gly Val Gly Val Pro Gly Val Gly Val Pro Gly Gly Gly
       290                 295                 300
   Val Pro Gly Ala Gly Val Pro Gly Val Gly Val Pro Gly Val Gly Val
   305                 310                 315                 320
   Pro Gly Val Gly Val Pro Gly Gly Gly Val Pro Gly Ala Gly Val Pro
                   325                 330                 335
   Gly Gly Gly Val Pro Gly Val Gly Val Pro Gly Val Gly Val Pro Gly
               340                 345                 350
   Gly Gly Val Pro Gly Ala Gly Val Pro Gly Val Gly Val Pro Gly Val
           355                 360                 365
   Gly Val Pro Gly Val Gly Val Pro Gly Gly Gly Val Pro Gly Ala Gly
       370                 375                 380
   Val Pro Gly Gly Gly Val Pro Gly Val Gly Val Pro Gly Val Gly Val
   385                 390                 395                 400
   Pro Gly Gly Gly Val Pro Gly Ala Gly Val Pro Gly Val Gly Val Pro
                   405                 410                 415
   Gly Val Gly Val Pro Gly Val Gly Val Pro Gly Gly Gly Val Pro Gly
               420                 425                 430
   Ala Gly Val Pro Gly Gly Gly Val Pro Gly Val Gly Val Pro Gly Val
           435                 440                 445
   Gly Val Pro Gly Gly Gly Val Pro Gly Ala Gly Val Pro Gly Val Gly
       450                 455                 460
   Val Pro Gly Val Gly Val Pro Gly Val Gly Val Pro Gly Gly Gly Val
   465                 470                 475                 480
   Pro Gly Ala Gly Val Pro Gly Gly Gly Val Pro Gly Val Gly Val Pro
                   485                 490                 495
   Gly Val Gly Val Pro Gly Gly Gly Val Pro Gly Ala Gly Val Pro Gly
               500                 505                 510
   Val Gly Val Pro Gly Val Gly Val Pro Gly Val Gly Val Pro Gly Gly
   515 520 525
   Gly Val Pro Gly Ala Gly Val Pro Gly Gly Gly Val Pro Gly Val Gly
   530 535 540
   Val Pro Gly Val Gly Val Pro Gly Gly Gly Val Pro Gly Ala Gly Val
   545 550 555 560
   Pro Gly Val Gly Val Pro Gly Val Gly Val Pro Gly Val Gly Val Pro
   565 570 575
   Gly Gly Gly Val Pro Gly Ala Gly Val Pro Gly Gly Gly Val Pro Gly
   580 585 590
   Val Gly Val Pro Gly Val Gly Val Pro Gly Gly Gly Val Pro Gly Ala
   595 600 605
   Gly Val Pro Gly Val Gly Val Pro Gly Val Gly Val Pro Gly Val Gly
   610 615 620
   Val Pro Gly Gly Gly Val Pro Gly Ala Gly Val Pro Gly Gly Gly Val
   625 630 635 640
   Pro Gly Trp Pro
<210> 74
   <211> 33
   <212> PRT
   <213> Artificial Sequence
<220>
   <223> Human recombinant GLP-2-consensus
<220>
   <221> MISC_FEATURE
   <222> (2)..(2)
   <223> Xaa may be Ala, Gly, Leu, Ile or Val
<400> 74
   His Xaa Asp Gly Ser Phe Ser Asp Glu Met Asn Thr Ile Leu Asp Asn
   1 5 10 15
   Leu Ala Ala Arg Asp Phe Ile Asn Trp Leu Ile Gln Thr Lys Ile Thr
   20 25 30
   Asp
<210> 75
   <211> 25
   <212> PRT
   <213> Artificial Sequence
<220>
   <223> Human recombinant hepcidin
<400> 75
   Asp Thr His Phe Pro Ile Cys Ile Phe Cys Cys Gly Cys Cys His Arg
   1               5                   10                  15
   Ser Lys Cys Gly Met Cys Cys Lys Thr
               20                  25
<210> 76
   <211> 631
   <212> PRT
   <213> Artificial Sequence
<220>
   <223> pPE0479 ELP1-120 hepcidin
<400> 76
   Met Val Pro Gly Val Gly Val Pro Gly Val Gly Val Pro Gly Gly Gly
   1               5                   10                  15
   Val Pro Gly Ala Gly Val Pro Gly Val Gly Val Pro Gly Val Gly Val
               20                  25                  30
   Pro Gly Val Gly Val Pro Gly Gly Gly Val Pro Gly Ala Gly Val Pro
           35                  40                  45
   Gly Gly Gly Val Pro Gly Val Gly Val Pro Gly Val Gly Val Pro Gly
       50                  55                  60
   Gly Gly Val Pro Gly Ala Gly Val Pro Gly Val Gly Val Pro Gly Val
   65                  70                  75                  80
   Gly Val Pro Gly Val Gly Val Pro Gly Gly Gly Val Pro Gly Ala Gly
                   85                  90                  95
   Val Pro Gly Gly Gly Val Pro Gly Val Gly Val Pro Gly Val Gly Val
               100                 105                 110
   Pro Gly Gly Gly Val Pro Gly Ala Gly Val Pro Gly Val Gly Val Pro
           115                 120                 125
   Gly Val Gly Val Pro Gly Val Gly Val Pro Gly Gly Gly Val Pro Gly
       130                 135                 140
   Ala Gly Val Pro Gly Gly Gly Val Pro Gly Val Gly Val Pro Gly Val
   145                 150                 155                 160
   Gly Val Pro Gly Gly Gly Val Pro Gly Ala Gly Val Pro Gly Val Gly
                   165                 170                 175
   Val Pro Gly Val Gly Val Pro Gly Val Gly Val Pro Gly Gly Gly Val
               180                 185                 190
   Pro Gly Ala Gly Val Pro Gly Gly Gly Val Pro Gly Val Gly Val Pro
           195                 200                 205
   Gly Val Gly Val Pro Gly Gly Gly Val Pro Gly Ala Gly Val Pro Gly
       210                 215                 220
   Val Gly Val Pro Gly Val Gly Val Pro Gly Val Gly Val Pro Gly Gly
   225                 230                 235                 240
   Gly Val Pro Gly Ala Gly Val Pro Gly Gly Gly Val Pro Gly Val Gly
                   245                 250                 255
   Val Pro Gly Val Gly Val Pro Gly Gly Gly Val Pro Gly Ala Gly Val
               260                 265                 270
   Pro Gly Val Gly Val Pro Gly Val Gly Val Pro Gly Val Gly Val Pro
           275                 280                 285
   Gly Gly Gly Val Pro Gly Ala Gly Val Pro Gly Gly Gly Val Pro Gly
       290                 295                 300
   Val Gly Val Pro Gly Val Gly Val Pro Gly Gly Gly Val Pro Gly Ala
   305                 310                 315                 320
   Gly Val Pro Gly Val Gly Val Pro Gly Val Gly Val Pro Gly Val Gly
                   325                 330                 335
   Val Pro Gly Gly Gly Val Pro Gly Ala Gly Val Pro Gly Gly Gly Val
               340                 345                 350
   Pro Gly Val Gly Val Pro Gly Val Gly Val Pro Gly Gly Gly Val Pro
           355                 360                 365
   Gly Ala Gly Val Pro Gly Val Gly Val Pro Gly Val Gly Val Pro Gly
       370                 375                 380
   Val Gly Val Pro Gly Gly Gly Val Pro Gly Ala Gly Val Pro Gly Gly
   385                 390                 395                 400
   Gly Val Pro Gly Val Gly Val Pro Gly Val Gly Val Pro Gly Gly Gly
                   405                 410                 415
   Val Pro Gly Ala Gly Val Pro Gly Val Gly Val Pro Gly Val Gly Val
               420                 425                 430
   Pro Gly Val Gly Val Pro Gly Gly Gly Val Pro Gly Ala Gly Val Pro
           435                 440                 445
   Gly Gly Gly Val Pro Gly Val Gly Val Pro Gly Val Gly Val Pro Gly
       450                 455                 460
   Gly Gly Val Pro Gly Ala Gly Val Pro Gly Val Gly Val Pro Gly Val
   465                 470                 475                 480
   Gly Val Pro Gly Val Gly Val Pro Gly Gly Gly Val Pro Gly Ala Gly
                   485                 490                 495
   Val Pro Gly Gly Gly Val Pro Gly Val Gly Val Pro Gly Val Gly Val
               500                 505                 510
   Pro Gly Gly Gly Val Pro Gly Ala Gly Val Pro Gly Val Gly Val Pro
           515                 520                 525
   Gly Val Gly Val Pro Gly Val Gly Val Pro Gly Gly Gly Val Pro Gly
       530                 535                 540
   Ala Gly Val Pro Gly Gly Gly Val Pro Gly Val Gly Val Pro Gly Val
   545                 550                 555                 560
   Gly Val Pro Gly Gly Gly Val Pro Gly Ala Gly Val Pro Gly Val Gly
                   565                 570                 575
   Val Pro Gly Val Gly Val Pro Gly Val Gly Val Pro Gly Gly Gly Val
               580                 585                 590
   Pro Gly Ala Gly Val Pro Gly Gly Gly Val Pro Trp Val Gly Asp Thr
           595                 600                 605
   His Phe Pro Ile Cys Ile Phe Cys Cys Gly Cys Cys His Arg Ser Lys
       610                 615                 620
   Cys Gly Met Cys Cys Lys Thr
   625                 630
<210> 77
   <211> 630
   <212> PRT
   <213> Artificial Sequence
<220>
   <223> pPE0487 hepcidin ELP1-120
<400> 77
   Asp Thr His Phe Pro Ile Cys Ile Phe Cys Cys Gly Cys Cys His Arg
   1               5                   10                  15
   Ser Lys Cys Gly Met Cys Cys Lys Thr Val Pro Gly Val Gly Val Pro
               20                  25                  30
   Gly Val Gly Val Pro Gly Gly Gly Val Pro Gly Ala Gly Val Pro Gly
           35                  40                  45
   Val Gly Val Pro Gly Val Gly Val Pro Gly Val Gly Val Pro Gly Gly
       50                  55                  60
   Gly Val Pro Gly Ala Gly Val Pro Gly Gly Gly Val Pro Gly Val Gly
   65                  70                  75                  80
   Val Pro Gly Val Gly Val Pro Gly Gly Gly Val Pro Gly Ala Gly Val
                   85                  90                  95
   Pro Gly Val Gly Val Pro Gly Val Gly Val Pro Gly Val Gly Val Pro
               100                 105                 110
   Gly Gly Gly Val Pro Gly Ala Gly Val Pro Gly Gly Gly Val Pro Gly
           115                 120                 125
   Val Gly Val Pro Gly Val Gly Val Pro Gly Gly Gly Val Pro Gly Ala
       130                 135                 140
   Gly Val Pro Gly Val Gly Val Pro Gly Val Gly Val Pro Gly Val Gly
   145                 150                 155                 160
   Val Pro Gly Gly Gly Val Pro Gly Ala Gly Val Pro Gly Gly Gly Val
                   165                 170                 175
   Pro Gly Val Gly Val Pro Gly Val Gly Val Pro Gly Gly Gly Val Pro
               180                 185                 190
   Gly Ala Gly Val Pro Gly Val Gly Val Pro Gly Val Gly Val Pro Gly
           195                 200                 205
   Val Gly Val Pro Gly Gly Gly Val Pro Gly Ala Gly Val Pro Gly Gly
   210 215 220
   Gly Val Pro Gly Val Gly Val Pro Gly Val Gly Val Pro Gly Gly Gly
   225 230 235 240
   Val Pro Gly Ala Gly Val Pro Gly Val Gly Val Pro Gly Val Gly Val
   245 250 255
   Pro Gly Val Gly Val Pro Gly Gly Gly Val Pro Gly Ala Gly Val Pro
   260 265 270
   Gly Gly Gly Val Pro Gly Val Gly Val Pro Gly Val Gly Val Pro Gly
   275 280 285
   Gly Gly Val Pro Gly Ala Gly Val Pro Gly Val Gly Val Pro Gly Val
   290 295 300
   Gly Val Pro Gly Val Gly Val Pro Gly Gly Gly Val Pro Gly Ala Gly
   305 310 315 320
   Val Pro Gly Gly Gly Val Pro Gly Val Gly Val Pro Gly Val Gly Val
   325 330 335
   Pro Gly Gly Gly Val Pro Gly Ala Gly Val Pro Gly Val Gly Val Pro
   340 345 350
   Gly Val Gly Val Pro Gly Val Gly Val Pro Gly Gly Gly Val Pro Gly
   355 360 365
   Ala Gly Val Pro Gly Gly Gly Val Pro Gly Val Gly Val Pro Gly Val
   370 375 380
   Gly Val Pro Gly Gly Gly Val Pro Gly Ala Gly Val Pro Gly Val Gly
   385 390 395 400
   Val Pro Gly Val Gly Val Pro Gly Val Gly Val Pro Gly Gly Gly Val
   405 410 415
   Pro Gly Ala Gly Val Pro Gly Gly Gly Val Pro Gly Val Gly Val Pro
   420 425 430
   Gly Val Gly Val Pro Gly Gly Gly Val Pro Gly Ala Gly Val Pro Gly
   435 440 445
   Val Gly Val Pro Gly Val Gly Val Pro Gly Val Gly Val Pro Gly Gly
   450 455 460
   Gly Val Pro Gly Ala Gly Val Pro Gly Gly Gly Val Pro Gly Val Gly
   465 470 475 480
   Val Pro Gly Val Gly Val Pro Gly Gly Gly Val Pro Gly Ala Gly Val
   485 490 495
   Pro Gly Val Gly Val Pro Gly Val Gly Val Pro Gly Val Gly Val Pro
   500 505 510
   Gly Gly Gly Val Pro Gly Ala Gly Val Pro Gly Gly Gly Val Pro Gly
   515 520 525
   Val Gly Val Pro Gly Val Gly Val Pro Gly Gly Gly Val Pro Gly Ala
   530 535 540
   Gly Val Pro Gly Val Gly Val Pro Gly Val Gly Val Pro Gly Val Gly
   545 550 555 560
   Val Pro Gly Gly Gly Val Pro Gly Ala Gly Val Pro Gly Gly Gly Val
   565 570 575
   Pro Gly Val Gly Val Pro Gly Val Gly Val Pro Gly Gly Gly Val Pro
   580 585 590
   Gly Ala Gly Val Pro Gly Val Gly Val Pro Gly Val Gly Val Pro Gly
   595 600 605
   Val Gly Val Pro Gly Gly Gly Val Pro Gly Ala Gly Val Pro Gly Gly
   610 615 620
   Gly Val Pro Gly Trp Pro
   625 630
<210> 78
   <211> 70
   <212> PRT
   <213> Artificial Sequence
<220>
   <223> Human recombinant IGF-1
<400> 78
   Gly Pro Glu Thr Leu Cys Gly Ala Glu Leu Val Asp Ala Leu Gln Phe
   1 5 10 15
   Val Cys Gly Asp Arg Gly Phe Tyr Phe Asn Lys Pro Thr Gly Tyr Gly
   20 25 30
   Ser Ser Ser Arg Arg Ala Pro Gln Thr Gly Ile Val Asp Glu Cys Cys
           35                  40                  45
   Phe Arg Ser Cys Asp Leu Arg Arg Leu Glu Met Tyr Cys Ala Pro Leu
       50                  55                  60
   Lys Pro Ala Lys Ser Ala
   65                  70
<210> 79
   <211> 675
   <212> PRT
   <213> Artificial Sequence
<220>
   <223> pPE0410 IGF-1 ELP1-120
<400> 79
   Gly Pro Glu Thr Leu Cys Gly Ala Glu Leu Val Asp Ala Leu Gln Phe
   1               5                   10                  15
   Val Cys Gly Asp Arg Gly Phe Tyr Phe Asn Lys Pro Thr Gly Tyr Gly
               20                  25                  30
   Ser Ser Ser Arg Arg Ala Pro Gln Thr Gly Ile Val Asp Glu Cys Cys
           35                  40                  45
   Phe Arg Ser Cys Asp Leu Arg Arg Leu Glu Met Tyr Cys Ala Pro Leu
       50                  55                  60
   Lys Pro Ala Lys Ser Ala Val Pro Gly Val Gly Val Pro Gly Val Gly
   65                  70                  75                  80
   Val Pro Gly Gly Gly Val Pro Gly Ala Gly Val Pro Gly Val Gly Val
                   85                  90                  95
   Pro Gly Val Gly Val Pro Gly Val Gly Val Pro Gly Gly Gly Val Pro
               100                 105                 110
   Gly Ala Gly Val Pro Gly Gly Gly Val Pro Gly Val Gly Val Pro Gly
           115                 120                 125
   Val Gly Val Pro Gly Gly Gly Val Pro Gly Ala Gly Val Pro Gly Val
       130                 135                 140
   Gly Val Pro Gly Val Gly Val Pro Gly Val Gly Val Pro Gly Gly Gly
   145                 150                 155                 160
   Val Pro Gly Ala Gly Val Pro Gly Gly Gly Val Pro Gly Val Gly Val
   165 170 175
   Pro Gly Val Gly Val Pro Gly Gly Gly Val Pro Gly Ala Gly Val Pro
   180 185 190
   Gly Val Gly Val Pro Gly Val Gly Val Pro Gly Val Gly Val Pro Gly
   195 200 205
   Gly Gly Val Pro Gly Ala Gly Val Pro Gly Gly Gly Val Pro Gly Val
   210 215 220
   Gly Val Pro Gly Val Gly Val Pro Gly Gly Gly Val Pro Gly Ala Gly
   225 230 235 240
   Val Pro Gly Val Gly Val Pro Gly Val Gly Val Pro Gly Val Gly Val
   245 250 255
   Pro Gly Gly Gly Val Pro Gly Ala Gly Val Pro Gly Gly Gly Val Pro
   260 265 270
   Gly Val Gly Val Pro Gly Val Gly Val Pro Gly Gly Gly Val Pro Gly
   275 280 285
   Ala Gly Val Pro Gly Val Gly Val Pro Gly Val Gly Val Pro Gly Val
   290 295 300
   Gly Val Pro Gly Gly Gly Val Pro Gly Ala Gly Val Pro Gly Gly Gly
   305 310 315 320
   Val Pro Gly Val Gly Val Pro Gly Val Gly Val Pro Gly Gly Gly Val
   325 330 335
   Pro Gly Ala Gly Val Pro Gly Val Gly Val Pro Gly Val Gly Val Pro
   340 345 350
   Gly Val Gly Val Pro Gly Gly Gly Val Pro Gly Ala Gly Val Pro Gly
   355 360 365
   Gly Gly Val Pro Gly Val Gly Val Pro Gly Val Gly Val Pro Gly Gly
   370 375 380
   Gly Val Pro Gly Ala Gly Val Pro Gly Val Gly Val Pro Gly Val Gly
   385 390 395 400
   Val Pro Gly Val Gly Val Pro Gly Gly Gly Val Pro Gly Ala Gly Val
   405 410 415
   Pro Gly Gly Gly Val Pro Gly Val Gly Val Pro Gly Val Gly Val Pro
               420                 425                 430
   Gly Gly Gly Val Pro Gly Ala Gly Val Pro Gly Val Gly Val Pro Gly
           435                 440                 445
   Val Gly Val Pro Gly Val Gly Val Pro Gly Gly Gly Val Pro Gly Ala
       450                 455                 460
   Gly Val Pro Gly Gly Gly Val Pro Gly Val Gly Val Pro Gly Val Gly
   465                 470                 475                 480
   Val Pro Gly Gly Gly Val Pro Gly Ala Gly Val Pro Gly Val Gly Val
                   485                 490                 495
   Pro Gly Val Gly Val Pro Gly Val Gly Val Pro Gly Gly Gly Val Pro
               500                 505                 510
   Gly Ala Gly Val Pro Gly Gly Gly Val Pro Gly Val Gly Val Pro Gly
           515                 520                 525
   Val Gly Val Pro Gly Gly Gly Val Pro Gly Ala Gly Val Pro Gly Val
       530                 535                 540
   Gly Val Pro Gly Val Gly Val Pro Gly Val Gly Val Pro Gly Gly Gly
   545                 550                 555                 560
   Val Pro Gly Ala Gly Val Pro Gly Gly Gly Val Pro Gly Val Gly Val
                   565                 570                 575
   Pro Gly Val Gly Val Pro Gly Gly Gly Val Pro Gly Ala Gly Val Pro
               580                 585                 590
   Gly Val Gly Val Pro Gly Val Gly Val Pro Gly Val Gly Val Pro Gly
           595                 600                 605
   Gly Gly Val Pro Gly Ala Gly Val Pro Gly Gly Gly Val Pro Gly Val
       610                 615                 620
   Gly Val Pro Gly Val Gly Val Pro Gly Gly Gly Val Pro Gly Ala Gly
   625                 630                 635                 640
   Val Pro Gly Val Gly Val Pro Gly Val Gly Val Pro Gly Val Gly Val
                   645                 650                 655
   Pro Gly Gly Gly Val Pro Gly Ala Gly Val Pro Gly Gly Gly Val Pro
               660                 665                 670
   Gly Trp Pro
   675
<210> 80
   <211> 800
   <212> PRT
   <213> Artificial Sequence
<220>
   <223> pPE0413 IGF-1 ELPbetav2 144mer
<400> 80
   Gly Pro Glu Thr Leu Cys Gly Ala Glu Leu Val Asp Ala Leu Gln Phe
   1 5 10 15
   Val Cys Gly Asp Arg Gly Phe Tyr Phe Asn Lys Pro Thr Gly Tyr Gly
   20 25 30
   Ser Ser Ser Arg Arg Ala Pro Gln Thr Gly Ile Val Asp Glu Cys Cys
   35 40 45
   Phe Arg Ser Cys Asp Leu Arg Arg Leu Glu Met Tyr Cys Ala Pro Leu
   50 55 60
   Lys Pro Ala Lys Ser Ala Val Pro Gly Val Gly Val Pro Gly Val Gly
   65 70 75 80
   Val Pro Gly Ala Gly Val Pro Gly Val Gly Val Pro Gly Ala Gly Val
   85 90 95
   Pro Gly Val Gly Val Pro Gly Val Gly Val Pro Gly Ala Gly Val Pro
   100 105 110
   Gly Val Gly Val Pro Gly Ala Gly Val Pro Gly Val Gly Val Pro Gly
   115 120 125
   Ala Gly Val Pro Gly Val Gly Val Pro Gly Ala Gly Val Pro Gly Val
   130 135 140
   Gly Val Pro Gly Val Gly Val Pro Gly Ala Gly Val Pro Gly Val Gly
   145 150 155 160
   Val Pro Gly Ala Gly Val Pro Gly Val Gly Val Pro Gly Ala Gly Val
   165 170 175
   Pro Gly Val Gly Val Pro Gly Ala Gly Val Pro Gly Val Gly Val Pro
   180 185 190
   Gly Val Gly Val Pro Gly Ala Gly Val Pro Gly Val Gly Val Pro Gly
           195                 200                 205
   Ala Gly Val Pro Gly Val Gly Val Pro Gly Ala Gly Val Pro Gly Val
       210                 215                 220
   Gly Val Pro Gly Ala Gly Val Pro Gly Val Gly Val Pro Gly Val Gly
   225                 230                 235                 240
   Val Pro Gly Ala Gly Val Pro Gly Val Gly Val Pro Gly Ala Gly Val
                   245                 250                 255
   Pro Gly Val Gly Val Pro Gly Ala Gly Val Pro Gly Val Gly Val Pro
               260                 265                 270
   Gly Ala Gly Val Pro Gly Val Gly Val Pro Gly Val Gly Val Pro Gly
           275                 280                 285
   Ala Gly Val Pro Gly Val Gly Val Pro Gly Ala Gly Val Pro Gly Val
       290                 295                 300
   Gly Val Pro Gly Ala Gly Val Pro Gly Val Gly Val Pro Gly Ala Gly
   305                 310                 315                 320
   Val Pro Gly Val Gly Val Pro Gly Val Gly Val Pro Gly Ala Gly Val
                   325                 330                 335
   Pro Gly Val Gly Val Pro Gly Ala Gly Val Pro Gly Val Gly Val Pro
               340                 345                 350
   Gly Ala Gly Val Pro Gly Val Gly Val Pro Gly Ala Gly Val Pro Gly
           355                 360                 365
   Val Gly Val Pro Gly Val Gly Val Pro Gly Ala Gly Val Pro Gly Val
       370                 375                 380
   Gly Val Pro Gly Ala Gly Val Pro Gly Val Gly Val Pro Gly Ala Gly
   385                 390                 395                 400
   Val Pro Gly Val Gly Val Pro Gly Ala Gly Val Pro Gly Val Gly Val
                   405                 410                 415
   Pro Gly Val Gly Val Pro Gly Ala Gly Val Pro Gly Val Gly Val Pro
               420                 425                 430
   Gly Ala Gly Val Pro Gly Val Gly Val Pro Gly Ala Gly Val Pro Gly
   435 440 445
   Val Gly Val Pro Gly Ala Gly Val Pro Gly Val Gly Val Pro Gly Val
   450 455 460
   Gly Val Pro Gly Ala Gly Val Pro Gly Val Gly Val Pro Gly Ala Gly
   465 470 475 480
   Val Pro Gly Val Gly Val Pro Gly Ala Gly Val Pro Gly Val Gly Val
   485 490 495
   Pro Gly Ala Gly Val Pro Gly Val Gly Val Pro Gly Val Gly Val Pro
   500 505 510
   Gly Ala Gly Val Pro Gly Val Gly Val Pro Gly Ala Gly Val Pro Gly
   515 520 525
   Val Gly Val Pro Gly Ala Gly Val Pro Gly Val Gly Val Pro Gly Ala
   530 535 540
   Gly Val Pro Gly Val Gly Val Pro Gly Val Gly Val Pro Gly Ala Gly
   545 550 555 560
   Val Pro Gly Val Gly Val Pro Gly Ala Gly Val Pro Gly Val Gly Val
   565 570 575
   Pro Gly Ala Gly Val Pro Gly Val Gly Val Pro Gly Ala Gly Val Pro
   580 585 590
   Gly Val Gly Val Pro Gly Val Gly Val Pro Gly Ala Gly Val Pro Gly
   595 600 605
   Val Gly Val Pro Gly Ala Gly Val Pro Gly Val Gly Val Pro Gly Ala
   610 615 620
   Gly Val Pro Gly Val Gly Val Pro Gly Ala Gly Val Pro Gly Val Gly
   625 630 635 640
   Val Pro Gly Val Gly Val Pro Gly Ala Gly Val Pro Gly Val Gly Val
   645 650 655
   Pro Gly Ala Gly Val Pro Gly Val Gly Val Pro Gly Ala Gly Val Pro
   660 665 670
   Gly Val Gly Val Pro Gly Ala Gly Val Pro Gly Val Gly Val Pro Gly
   675 680 685
   Val Gly Val Pro Gly Ala Gly Val Pro Gly Val Gly Val Pro Gly Ala
       690                 695                 700
   Gly Val Pro Gly Val Gly Val Pro Gly Ala Gly Val Pro Gly Val Gly
   705                 710                 715                 720
   Val Pro Gly Ala Gly Val Pro Gly Val Gly Val Pro Gly Val Gly Val
                   725                 730                 735
   Pro Gly Ala Gly Val Pro Gly Val Gly Val Pro Gly Ala Gly Val Pro
               740                 745                 750
   Gly Val Gly Val Pro Gly Ala Gly Val Pro Gly Val Gly Val Pro Gly
           755                 760                 765
   Ala Gly Val Pro Gly Val Gly Val Pro Gly Val Gly Val Pro Gly Ala
       770                 775                 780
   Gly Val Pro Gly Val Gly Val Pro Gly Ala Gly Val Pro Gly Trp Pro
   785                 790                 795                 800
<210> 81
   <211> 1935
   <212> DNA
   <213> Artificial Sequence
<220>
   <223> ELP1-120 CNP37 fusion
<400> 81
   atggtaccgg gcgtgggtgt tccgggcgtg ggtgttccgg gtggcggtgt gccgggcgca 60
   ggtgttcctg gtgtaggtgt gccgggtgtt ggtgtgccgg gtgttggtgt accaggtggc 120
   ggtgttccgg gtgcaggcgt tccgggtggc ggtgtgccgg gcgtgggtgt tccgggcgtg 180
   ggtgttccgg gtggcggtgt gccgggcgca ggtgttcctg gtgtaggtgt gccgggtgtt 240
   ggtgtgccgg gtgttggtgt accaggtggc ggtgttccgg gtgcaggcgt tccgggtggc 300
   ggtgtgccgg gcgtgggtgt tccgggcgtg ggtgttccgg gtggcggtgt gccgggcgca 360
   ggtgttcctg gtgtaggtgt gccgggtgtt ggtgtgccgg gtgttggtgt accaggtggc 420
   ggtgttccgg gtgcaggcgt tccgggtggc ggtgtgccgg gcgtgggtgt tccgggcgtg 480
   ggtgttccgg gtggcggtgt gccgggcgca ggtgttcctg gtgtaggtgt gccgggtgtt 540
   ggtgtgccgg gtgttggtgt accaggtggc ggtgttccgg gtgcaggcgt tccgggtggc 600
   ggtgtgccgg gcgtgggtgt tccgggcgtg ggtgttccgg gtggcggtgt gccgggcgca 660
   ggtgttcctg gtgtaggtgt gccgggtgtt ggtgtgccgg gtgttggtgt accaggtggc 720
   ggtgttccgg gtgcaggcgt tccgggtggc ggtgtgccgg gcgtgggtgt tccgggcgtg 780
   ggtgttccgg gtggcggtgt gccgggcgca ggtgttcctg gtgtaggtgt gccgggtgtt 840
   ggtgtgccgg gtgttggtgt accaggtggc ggtgttccgg gtgcaggcgt tccgggtggc 900
   ggtgtgccgg gcgtgggtgt tccgggcgtg ggtgttccgg gtggcggtgt gccgggcgca 960
   ggtgttcctg gtgtaggtgt gccgggtgtt ggtgtgccgg gtgttggtgt accaggtggc 1020
   ggtgttccgg gtgcaggcgt tccgggtggc ggtgtgccgg gcgtgggtgt tccgggcgtg 1080
   ggtgttccgg gtggcggtgt gccgggcgca ggtgttcctg gtgtaggtgt gccgggtgtt 1140
   ggtgtgccgg gtgttggtgt accaggtggc ggtgttccgg gtgcaggcgt tccgggtggc 1200
   ggtgtgccgg gcgtgggtgt tccgggcgtg ggtgttccgg gtggcggtgt gccgggcgca 1260
   ggtgttcctg gtgtaggtgt gccgggtgtt ggtgtgccgg gtgttggtgt accaggtggc 1320
   ggtgttccgg gtgcaggcgt tccgggtggc ggtgtgccgg gcgtgggtgt tccgggcgtg 1380
   ggtgttccgg gtggcggtgt gccgggcgca ggtgttcctg gtgtaggtgt gccgggtgtt 1440
   ggtgtgccgg gtgttggtgt accaggtggc ggtgttccgg gtgcaggcgt tccgggtggc 1500
   ggtgtgccgg gcgtgggtgt tccgggcgtg ggtgttccgg gtggcggtgt gccgggcgca 1560
   ggtgttcctg gtgtaggtgt gccgggtgtt ggtgtgccgg gtgttggtgt accaggtggc 1620
   ggtgttccgg gtgcaggcgt tccgggtggc ggtgtgccgg gcgtgggtgt tccgggcgtg 1680
   ggtgttccgg gtggcggtgt gccgggcgca ggtgttcctg gtgtaggtgt gccgggtgtt 1740
   ggtgtgccgg gtgttggtgt accaggtggc ggtgttccgg gtgcaggcgt tccgggtggc 1800
   ggtgtgccgg gcgtggggca ggaacatccg aacgcgcgta aatataaagg tgcgaacaaa 1860
   aaaggcctga gcaaaggctg ctttggcctg aaactggatc gcattggcag catgagcggc 1920
   ctgggctgct gataa 1935
<210> 82
   <211> 1935
   <212> DNA
   <213> Artificial Sequence
<220>
   <223> CNP37 ELP1-120 fusion
<400> 82
   caggaacatc cgaacgcgcg taaatataaa ggtgcgaaca aaaaaggcct gagcaaaggc       60
   tgctttggcc tgaaactgga tcgcattggc agcatgagcg gcctgggctg cggtgtaccg      120
   ggcgtgggtg ttccgggcgt gggtgttccg ggtggcggtg tgccgggcgc aggtgttcct      180
   ggtgtaggtg tgccgggtgt tggtgtgccg ggtgttggtg taccaggtgg cggtgttccg      240
   ggtgcaggcg ttccgggtgg cggtgtgccg ggcgtgggtg ttccgggcgt gggtgttccg      300
   ggtggcggtg tgccgggcgc aggtgttcct ggtgtaggtg tgccgggtgt tggtgtgccg      360
   ggtgttggtg taccaggtgg cggtgttccg ggtgcaggcg ttccgggtgg cggtgtgccg      420
   ggcgtgggtg ttccgggcgt gggtgttccg ggtggcggtg tgccgggcgc aggtgttcct     480
   ggtgtaggtg tgccgggtgt tggtgtgccg ggtgttggtg taccaggtgg cggtgttccg     540
   ggtgcaggcg ttccgggtgg cggtgtgccg ggcgtgggtg ttccgggcgt gggtgttccg     600
   ggtggcggtg tgccgggcgc aggtgttcct ggtgtaggtg tgccgggtgt tggtgtgccg     660
   ggtgttggtg taccaggtgg cggtgttccg ggtgcaggcg ttccgggtgg cggtgtgccg     720
   ggcgtgggtg ttccgggcgt gggtgttccg ggtggcggtg tgccgggcgc aggtgttcct     780
   ggtgtaggtg tgccgggtgt tggtgtgccg ggtgttggtg taccaggtgg cggtgttccg     840
   ggtgcaggcg ttccgggtgg cggtgtgccg ggcgtgggtg ttccgggcgt gggtgttccg     900
   ggtggcggtg tgccgggcgc aggtgttcct ggtgtaggtg tgccgggtgt tggtgtgccg     960
   ggtgttggtg taccaggtgg cggtgttccg ggtgcaggcg ttccgggtgg cggtgtgccg    1020
   ggcgtgggtg ttccgggcgt gggtgttccg ggtggcggtg tgccgggcgc aggtgttcct    1080
   ggtgtaggtg tgccgggtgt tggtgtgccg ggtgttggtg taccaggtgg cggtgttccg    1140
   ggtgcaggcg ttccgggtgg cggtgtgccg ggcgtgggtg ttccgggcgt gggtgttccg    1200
   ggtggcggtg tgccgggcgc aggtgttcct ggtgtaggtg tgccgggtgt tggtgtgccg    1260
   ggtgttggtg taccaggtgg cggtgttccg ggtgcaggcg ttccgggtgg cggtgtgccg    1320
   ggcgtgggtg ttccgggcgt gggtgttccg ggtggcggtg tgccgggcgc aggtgttcct    1380
   ggtgtaggtg tgccgggtgt tggtgtgccg ggtgttggtg taccaggtgg cggtgttccg    1440
   ggtgcaggcg ttccgggtgg cggtgtgccg ggcgtgggtg ttccgggcgt gggtgttccg    1500
   ggtggcggtg tgccgggcgc aggtgttcct ggtgtaggtg tgccgggtgt tggtgtgccg    1560
   ggtgttggtg taccaggtgg cggtgttccg ggtgcaggcg ttccgggtgg cggtgtgccg    1620
   ggcgtgggtg ttccgggcgt gggtgttccg ggtggcggtg tgccgggcgc aggtgttcct    1680
   ggtgtaggtg tgccgggtgt tggtgtgccg ggtgttggtg taccaggtgg cggtgttccg    1740
   ggtgcaggcg ttccgggtgg cggtgtgccg ggcgtgggtg ttccgggcgt gggtgttccg    1800
   ggtggcggtg tgccgggcgc aggtgttcct ggtgtaggtg tgccgggtgt tggtgtgccg    1860
   ggtgttggtg taccaggtgg cggtgttccg ggtgcaggcg ttccgggtgg cggtgtgccg    1920
   ggctggccgt gataa  1935
<210> 83
   <211> 648
   <212> PRT
   <213> Artificial Sequence
<220>
   <223> CNP37 GGS2x ELP1-120 fusion
<400> 83
   Gln Glu His Pro Asn Ala Arg Lys Tyr Lys Gly Ala Asn Lys Lys Gly
   1               5                   10                  15
   Leu Ser Lys Gly Cys Phe Gly Leu Lys Leu Asp Arg Ile Gly Ser Met
               20                  25                  30
   Ser Gly Leu Gly Cys Gly Gly Ser Gly Gly Ser Val Pro Gly Val Gly
           35                  40                  45
   Val Pro Gly Val Gly Val Pro Gly Gly Gly Val Pro Gly Ala Gly Val
       50                  55                  60
   Pro Gly Val Gly Val Pro Gly Val Gly Val Pro Gly Val Gly Val Pro
   65                  70                  75                  80
   Gly Gly Gly Val Pro Gly Ala Gly Val Pro Gly Gly Gly Val Pro Gly
                   85                  90                  95
   Val Gly Val Pro Gly Val Gly Val Pro Gly Gly Gly Val Pro Gly Ala
               100                 105                 110
   Gly Val Pro Gly Val Gly Val Pro Gly Val Gly Val Pro Gly Val Gly
           115                 120                 125
   Val Pro Gly Gly Gly Val Pro Gly Ala Gly Val Pro Gly Gly Gly Val
       130                 135                 140
   Pro Gly Val Gly Val Pro Gly Val Gly Val Pro Gly Gly Gly Val Pro
   145                 150                 155                 160
   Gly Ala Gly Val Pro Gly Val Gly Val Pro Gly Val Gly Val Pro Gly
                   165                 170                 175
   Val Gly Val Pro Gly Gly Gly Val Pro Gly Ala Gly Val Pro Gly Gly
               180                 185                 190
   Gly Val Pro Gly Val Gly Val Pro Gly Val Gly Val Pro Gly Gly Gly
           195                 200                 205
   Val Pro Gly Ala Gly Val Pro Gly Val Gly Val Pro Gly Val Gly Val
       210                 215                 220
   Pro Gly Val Gly Val Pro Gly Gly Gly Val Pro Gly Ala Gly Val Pro
   225                 230                 235                 240
   Gly Gly Gly Val Pro Gly Val Gly Val Pro Gly Val Gly Val Pro Gly
                   245                 250                 255
   Gly Gly Val Pro Gly Ala Gly Val Pro Gly Val Gly Val Pro Gly Val
               260                 265                 270
   Gly Val Pro Gly Val Gly Val Pro Gly Gly Gly Val Pro Gly Ala Gly
           275                 280                 285
   Val Pro Gly Gly Gly Val Pro Gly Val Gly Val Pro Gly Val Gly Val
       290                 295                 300
   Pro Gly Gly Gly Val Pro Gly Ala Gly Val Pro Gly Val Gly Val Pro
   305                 310                 315                 320
   Gly Val Gly Val Pro Gly Val Gly Val Pro Gly Gly Gly Val Pro Gly
                   325                 330                 335
   Ala Gly Val Pro Gly Gly Gly Val Pro Gly Val Gly Val Pro Gly Val
               340                 345                 350
   Gly Val Pro Gly Gly Gly Val Pro Gly Ala Gly Val Pro Gly Val Gly
           355                 360                 365
   Val Pro Gly Val Gly Val Pro Gly Val Gly Val Pro Gly Gly Gly Val
       370                 375                 380
   Pro Gly Ala Gly Val Pro Gly Gly Gly Val Pro Gly Val Gly Val Pro
   385                 390                 395                 400
   Gly Val Gly Val Pro Gly Gly Gly Val Pro Gly Ala Gly Val Pro Gly
                   405                 410                 415
   Val Gly Val Pro Gly Val Gly Val Pro Gly Val Gly Val Pro Gly Gly
               420                 425                 430
   Gly Val Pro Gly Ala Gly Val Pro Gly Gly Gly Val Pro Gly Val Gly
           435                 440                 445
   Val Pro Gly Val Gly Val Pro Gly Gly Gly Val Pro Gly Ala Gly Val
       450                 455                 460
   Pro Gly Val Gly Val Pro Gly Val Gly Val Pro Gly Val Gly Val Pro
   465                 470                 475                 480
   Gly Gly Gly Val Pro Gly Ala Gly Val Pro Gly Gly Gly Val Pro Gly
                   485                 490                 495
   Val Gly Val Pro Gly Val Gly Val Pro Gly Gly Gly Val Pro Gly Ala
   500 505 510
   Gly Val Pro Gly Val Gly Val Pro Gly Val Gly Val Pro Gly Val Gly
   515 520 525
   Val Pro Gly Gly Gly Val Pro Gly Ala Gly Val Pro Gly Gly Gly Val
   530 535 540
   Pro Gly Val Gly Val Pro Gly Val Gly Val Pro Gly Gly Gly Val Pro
   545 550 555 560
   Gly Ala Gly Val Pro Gly Val Gly Val Pro Gly Val Gly Val Pro Gly
   565 570 575
   Val Gly Val Pro Gly Gly Gly Val Pro Gly Ala Gly Val Pro Gly Gly
   580 585 590
   Gly Val Pro Gly Val Gly Val Pro Gly Val Gly Val Pro Gly Gly Gly
   595 600 605
   Val Pro Gly Ala Gly Val Pro Gly Val Gly Val Pro Gly Val Gly Val
   610 615 620
   Pro Gly Val Gly Val Pro Gly Gly Gly Val Pro Gly Ala Gly Val Pro
   625 630 635 640
   Gly Gly Gly Val Pro Gly Trp Pro
   645
<210> 84
   <211> 32
   <212> PRT
   <213> Artificial Sequence
<220>
   <223> Human recombinant urodilatin
<400> 84
   Thr Ala Pro Arg Ser Leu Arg Arg Ser Ser Cys Phe Gly Gly Arg Met
   1 5 10 15
   Asp Arg Ile Gly Ala Gln Ser Gly Leu Gly Cys Asn Ser Phe Arg Tyr
   20 25 30
<210> 85
   <211> 637
   <212> PRT
   <213> Artificial Sequence
<220>
   <223> pPE0483 urodilatin ELP1-120
<400> 85
   Thr Ala Pro Arg Ser Leu Arg Arg Ser Ser Cys Phe Gly Gly Arg Met
   1               5                   10                  15
   Asp Arg Ile Gly Ala Gln Ser Gly Leu Gly Cys Asn Ser Phe Arg Tyr
               20                  25                  30
   Val Pro Gly Val Gly Val Pro Gly Val Gly Val Pro Gly Gly Gly Val
           35                  40                  45
   Pro Gly Ala Gly Val Pro Gly Val Gly Val Pro Gly Val Gly Val Pro
       50                  55                  60
   Gly Val Gly Val Pro Gly Gly Gly Val Pro Gly Ala Gly Val Pro Gly
   65                  70                  75                  80
   Gly Gly Val Pro Gly Val Gly Val Pro Gly Val Gly Val Pro Gly Gly
                   85                  90                  95
   Gly Val Pro Gly Ala Gly Val Pro Gly Val Gly Val Pro Gly Val Gly
               100                 105                 110
   Val Pro Gly Val Gly Val Pro Gly Gly Gly Val Pro Gly Ala Gly Val
           115                 120                 125
   Pro Gly Gly Gly Val Pro Gly Val Gly Val Pro Gly Val Gly Val Pro
       130                 135                 140
   Gly Gly Gly Val Pro Gly Ala Gly Val Pro Gly Val Gly Val Pro Gly
   145                 150                 155                 160
   Val Gly Val Pro Gly Val Gly Val Pro Gly Gly Gly Val Pro Gly Ala
                   165                 170                 175
   Gly Val Pro Gly Gly Gly Val Pro Gly Val Gly Val Pro Gly Val Gly
               180                 185                 190
   Val Pro Gly Gly Gly Val Pro Gly Ala Gly Val Pro Gly Val Gly Val
           195                 200                 205
   Pro Gly Val Gly Val Pro Gly Val Gly Val Pro Gly Gly Gly Val Pro
       210                 215                 220
   Gly Ala Gly Val Pro Gly Gly Gly Val Pro Gly Val Gly Val Pro Gly
   225                 230                 235                 240
   Val Gly Val Pro Gly Gly Gly Val Pro Gly Ala Gly Val Pro Gly Val
   245 250 255
   Gly Val Pro Gly Val Gly Val Pro Gly Val Gly Val Pro Gly Gly Gly
   260 265 270
   Val Pro Gly Ala Gly Val Pro Gly Gly Gly Val Pro Gly Val Gly Val
   275 280 285
   Pro Gly Val Gly Val Pro Gly Gly Gly Val Pro Gly Ala Gly Val Pro
   290 295 300
   Gly Val Gly Val Pro Gly Val Gly Val Pro Gly Val Gly Val Pro Gly
   305 310 315 320
   Gly Gly Val Pro Gly Ala Gly Val Pro Gly Gly Gly Val Pro Gly Val
   325 330 335
   Gly Val Pro Gly Val Gly Val Pro Gly Gly Gly Val Pro Gly Ala Gly
   340 345 350
   Val Pro Gly Val Gly Val Pro Gly Val Gly Val Pro Gly Val Gly Val
   355 360 365
   Pro Gly Gly Gly Val Pro Gly Ala Gly Val Pro Gly Gly Gly Val Pro
   370 375 380
   Gly Val Gly Val Pro Gly Val Gly Val Pro Gly Gly Gly Val Pro Gly
   385 390 395 400
   Ala Gly Val Pro Gly Val Gly Val Pro Gly Val Gly Val Pro Gly Val
   405 410 415
   Gly Val Pro Gly Gly Gly Val Pro Gly Ala Gly Val Pro Gly Gly Gly
   420 425 430
   Val Pro Gly Val Gly Val Pro Gly Val Gly Val Pro Gly Gly Gly Val
   435 440 445
   Pro Gly Ala Gly Val Pro Gly Val Gly Val Pro Gly Val Gly Val Pro
   450 455 460
   Gly Val Gly Val Pro Gly Gly Gly Val Pro Gly Ala Gly Val Pro Gly
   465 470 475 480
   Gly Gly Val Pro Gly Val Gly Val Pro Gly Val Gly Val Pro Gly Gly
   485 490 495
   Gly Val Pro Gly Ala Gly Val Pro Gly Val Gly Val Pro Gly Val Gly
   500 505 510
   Val Pro Gly Val Gly Val Pro Gly Gly Gly Val Pro Gly Ala Gly Val
   515 520 525
   Pro Gly Gly Gly Val Pro Gly Val Gly Val Pro Gly Val Gly Val Pro
   530 535 540
   Gly Gly Gly Val Pro Gly Ala Gly Val Pro Gly Val Gly Val Pro Gly
   545 550 555 560
   Val Gly Val Pro Gly Val Gly Val Pro Gly Gly Gly Val Pro Gly Ala
   565 570 575
   Gly Val Pro Gly Gly Gly Val Pro Gly Val Gly Val Pro Gly Val Gly
   580 585 590
   Val Pro Gly Gly Gly Val Pro Gly Ala Gly Val Pro Gly Val Gly Val
   595 600 605
   Pro Gly Val Gly Val Pro Gly Val Gly Val Pro Gly Gly Gly Val Pro
   610 615 620
   Gly Ala Gly Val Pro Gly Gly Gly Val Pro Gly Trp Pro
   625 630 635
<210> 86
   <211> 638
   <212> PRT
   <213> Artificial Sequence
<220>
   <223> pPE0484 ELP1-120 urodilatin
<400> 86
   Met Val Pro Gly Val Gly Val Pro Gly Val Gly Val Pro Gly Gly Gly
   1 5 10 15
   Val Pro Gly Ala Gly Val Pro Gly Val Gly Val Pro Gly Val Gly Val
   20 25 30
   Pro Gly Val Gly Val Pro Gly Gly Gly Val Pro Gly Ala Gly Val Pro
   35 40 45
   Gly Gly Gly Val Pro Gly Val Gly Val Pro Gly Val Gly Val Pro Gly
       50                  55                  60
   Gly Gly Val Pro Gly Ala Gly Val Pro Gly Val Gly Val Pro Gly Val
   65                  70                  75                  80
   Gly Val Pro Gly Val Gly Val Pro Gly Gly Gly Val Pro Gly Ala Gly
                   85                  90                  95
   Val Pro Gly Gly Gly Val Pro Gly Val Gly Val Pro Gly Val Gly Val
               100                 105                 110
   Pro Gly Gly Gly Val Pro Gly Ala Gly Val Pro Gly Val Gly Val Pro
           115                 120                 125
   Gly Val Gly Val Pro Gly Val Gly Val Pro Gly Gly Gly Val Pro Gly
       130                 135                 140
   Ala Gly Val Pro Gly Gly Gly Val Pro Gly Val Gly Val Pro Gly Val
   145                 150                 155                 160
   Gly Val Pro Gly Gly Gly Val Pro Gly Ala Gly Val Pro Gly Val Gly
                   165                 170                 175
   Val Pro Gly Val Gly Val Pro Gly Val Gly Val Pro Gly Gly Gly Val
               180                 185                 190
   Pro Gly Ala Gly Val Pro Gly Gly Gly Val Pro Gly Val Gly Val Pro
           195                 200                 205
   Gly Val Gly Val Pro Gly Gly Gly Val Pro Gly Ala Gly Val Pro Gly
       210                 215                 220
   Val Gly Val Pro Gly Val Gly Val Pro Gly Val Gly Val Pro Gly Gly
   225                 230                 235                 240
   Gly Val Pro Gly Ala Gly Val Pro Gly Gly Gly Val Pro Gly Val Gly
                   245                 250                 255
   Val Pro Gly Val Gly Val Pro Gly Gly Gly Val Pro Gly Ala Gly Val
               260                 265                 270
   Pro Gly Val Gly Val Pro Gly Val Gly Val Pro Gly Val Gly Val Pro
           275                 280                 285
   Gly Gly Gly Val Pro Gly Ala Gly Val Pro Gly Gly Gly Val Pro Gly
       290                 295                 300
   Val Gly Val Pro Gly Val Gly Val Pro Gly Gly Gly Val Pro Gly Ala
   305                 310                 315                 320
   Gly Val Pro Gly Val Gly Val Pro Gly Val Gly Val Pro Gly Val Gly
                   325                 330                 335
   Val Pro Gly Gly Gly Val Pro Gly Ala Gly Val Pro Gly Gly Gly Val
               340                 345                 350
   Pro Gly Val Gly Val Pro Gly Val Gly Val Pro Gly Gly Gly Val Pro
           355                 360                 365
   Gly Ala Gly Val Pro Gly Val Gly Val Pro Gly Val Gly Val Pro Gly
       370                 375                 380
   Val Gly Val Pro Gly Gly Gly Val Pro Gly Ala Gly Val Pro Gly Gly
   385                 390                 395                 400
   Gly Val Pro Gly Val Gly Val Pro Gly Val Gly Val Pro Gly Gly Gly
                   405                 410                 415
   Val Pro Gly Ala Gly Val Pro Gly Val Gly Val Pro Gly Val Gly Val
               420                 425                 430
   Pro Gly Val Gly Val Pro Gly Gly Gly Val Pro Gly Ala Gly Val Pro
           435                 440                 445
   Gly Gly Gly Val Pro Gly Val Gly Val Pro Gly Val Gly Val Pro Gly
       450                 455                 460
   Gly Gly Val Pro Gly Ala Gly Val Pro Gly Val Gly Val Pro Gly Val
   465                 470                 475                 480
   Gly Val Pro Gly Val Gly Val Pro Gly Gly Gly Val Pro Gly Ala Gly
                   485                 490                 495
   Val Pro Gly Gly Gly Val Pro Gly Val Gly Val Pro Gly Val Gly Val
               500                 505                 510
   Pro Gly Gly Gly Val Pro Gly Ala Gly Val Pro Gly Val Gly Val Pro
           515                 520                 525
   Gly Val Gly Val Pro Gly Val Gly Val Pro Gly Gly Gly Val Pro Gly
       530                 535                 540
   Ala Gly Val Pro Gly Gly Gly Val Pro Gly Val Gly Val Pro Gly Val
   545                 550                 555                 560
   Gly Val Pro Gly Gly Gly Val Pro Gly Ala Gly Val Pro Gly Val Gly
   565 570 575
   Val Pro Gly Val Gly Val Pro Gly Val Gly Val Pro Gly Gly Gly Val
   580 585 590
   Pro Gly Ala Gly Val Pro Gly Gly Gly Val Pro Gly Trp Gly Thr Ala
   595 600 605
   Pro Arg Ser Leu Arg Arg Ser Ser Cys Phe Gly Gly Arg Met Asp Arg
   610 615 620
   Ile Gly Ala Gln Ser Gly Leu Gly Cys Asn Ser Phe Arg Tyr
   625 630 635
<210> 87
   <211> 3
   <212> PRT
   <213> Artificial Sequence
<220>
   <223> GGS tripeptide
<400> 87
   Gly Gly Ser
   1
<210> 88
   <211> 9
   <212> PRT
   <213> Artificial Sequence
<220>
   <223> Hepcidin (1-9)
<400> 88
   Asp Thr His Phe Pro Ile Cys Ile Phe
   1 5
<210> 89
   <211> 43
   <212> PRT
   <213> Artificial Sequence
<220>
   <223> Thymosin beta 4
<400> 89
   Ser Asp Lys Pro Asp Met Ala Glu Ile Glu Lys Phe Asp Lys Ser Lys
   1 5 10 15
   Leu Lys Lys Thr Glu Thr Gln Glu Lys Asn Pro Leu Pro Ser Lys Glu
   20 25 30
   Thr Ile Glu Gln Glu Lys Gln Ala Gly Glu Ser
   35 40
<210> 90
   <211> 648
   <212> PRT
   <213> Artificial Sequence
<220>
   <223> pPE0320 thymosin beta 4 ELP1-120
<400> 90
   Ser Asp Lys Pro Asp Met Ala Glu Ile Glu Lys Phe Asp Lys Ser Lys
   1 5 10 15
   Leu Lys Lys Thr Glu Thr Gln Glu Lys Asn Pro Leu Pro Ser Lys Glu
   20 25 30
   Thr Ile Glu Gln Glu Lys Gln Ala Gly Glu Ser Val Pro Gly Val Gly
   35 40 45
   Val Pro Gly Val Gly Val Pro Gly Gly Gly Val Pro Gly Ala Gly Val
   50 55 60
   Pro Gly Val Gly Val Pro Gly Val Gly Val Pro Gly Val Gly Val Pro
   65 70 75 80
   Gly Gly Gly Val Pro Gly Ala Gly Val Pro Gly Gly Gly Val Pro Gly
   85 90 95
   Val Gly Val Pro Gly Val Gly Val Pro Gly Gly Gly Val Pro Gly Ala
   100 105 110
   Gly Val Pro Gly Val Gly Val Pro Gly Val Gly Val Pro Gly Val Gly
   115 120 125
   Val Pro Gly Gly Gly Val Pro Gly Ala Gly Val Pro Gly Gly Gly Val
   130 135 140
   Pro Gly Val Gly Val Pro Gly Val Gly Val Pro Gly Gly Gly Val Pro
   145 150 155 160
   Gly Ala Gly Val Pro Gly Val Gly Val Pro Gly Val Gly Val Pro Gly
   165 170 175
   Val Gly Val Pro Gly Gly Gly Val Pro Gly Ala Gly Val Pro Gly Gly
               180                 185                 190
   Gly Val Pro Gly Val Gly Val Pro Gly Val Gly Val Pro Gly Gly Gly
           195                 200                 205
   Val Pro Gly Ala Gly Val Pro Gly Val Gly Val Pro Gly Val Gly Val
       210                 215                 220
   Pro Gly Val Gly Val Pro Gly Gly Gly Val Pro Gly Ala Gly Val Pro
   225                 230                 235                 240
   Gly Gly Gly Val Pro Gly Val Gly Val Pro Gly Val Gly Val Pro Gly
                   245                 250                 255
   Gly Gly Val Pro Gly Ala Gly Val Pro Gly Val Gly Val Pro Gly Val
               260                 265                 270
   Gly Val Pro Gly Val Gly Val Pro Gly Gly Gly Val Pro Gly Ala Gly
           275                 280                 285
   Val Pro Gly Gly Gly Val Pro Gly Val Gly Val Pro Gly Val Gly Val
       290                 295                 300
   Pro Gly Gly Gly Val Pro Gly Ala Gly Val Pro Gly Val Gly Val Pro
   305                 310                 315                 320
   Gly Val Gly Val Pro Gly Val Gly Val Pro Gly Gly Gly Val Pro Gly
                   325                 330                 335
   Ala Gly Val Pro Gly Gly Gly Val Pro Gly Val Gly Val Pro Gly Val
               340                 345                 350
   Gly Val Pro Gly Gly Gly Val Pro Gly Ala Gly Val Pro Gly Val Gly
           355                 360                 365
   Val Pro Gly Val Gly Val Pro Gly Val Gly Val Pro Gly Gly Gly Val
       370                 375                 380
   Pro Gly Ala Gly Val Pro Gly Gly Gly Val Pro Gly Val Gly Val Pro
   385                 390                 395                 400
   Gly Val Gly Val Pro Gly Gly Gly Val Pro Gly Ala Gly Val Pro Gly
                   405                 410                 415
   Val Gly Val Pro Gly Val Gly Val Pro Gly Val Gly Val Pro Gly Gly
               420                 425                 430
   Gly Val Pro Gly Ala Gly Val Pro Gly Gly Gly Val Pro Gly Val Gly
           435                 440                 445
   Val Pro Gly Val Gly Val Pro Gly Gly Gly Val Pro Gly Ala Gly Val
       450                 455                 460
   Pro Gly Val Gly Val Pro Gly Val Gly Val Pro Gly Val Gly Val Pro
   465                 470                 475                 480
   Gly Gly Gly Val Pro Gly Ala Gly Val Pro Gly Gly Gly Val Pro Gly
                   485                 490                 495
   Val Gly Val Pro Gly Val Gly Val Pro Gly Gly Gly Val Pro Gly Ala
               500                 505                 510
   Gly Val Pro Gly Val Gly Val Pro Gly Val Gly Val Pro Gly Val Gly
           515                 520                 525
   Val Pro Gly Gly Gly Val Pro Gly Ala Gly Val Pro Gly Gly Gly Val
       530                 535                 540
   Pro Gly Val Gly Val Pro Gly Val Gly Val Pro Gly Gly Gly Val Pro
   545                 550                 555                 560
   Gly Ala Gly Val Pro Gly Val Gly Val Pro Gly Val Gly Val Pro Gly
                   565                 570                 575
   Val Gly Val Pro Gly Gly Gly Val Pro Gly Ala Gly Val Pro Gly Gly
               580                 585                 590
   Gly Val Pro Gly Val Gly Val Pro Gly Val Gly Val Pro Gly Gly Gly
           595                 600                 605
   Val Pro Gly Ala Gly Val Pro Gly Val Gly Val Pro Gly Val Gly Val
       610                 615                 620
   Pro Gly Val Gly Val Pro Gly Gly Gly Val Pro Gly Ala Gly Val Pro
   625                 630                 635                 640
   Gly Gly Gly Val Pro Gly Trp Pro
                   645
<210> 91
   <211> 168
   <212> PRT
   <213> Artificial Sequence
<220>
   <223> TRAIL
<400> 91
   Val Arg Glu Arg Gly Pro Gln Arg Val Ala Ala His Ile Thr Gly Thr
   1               5                   10                  15
   Arg Gly Arg Ser Asn Thr Leu Ser Ser Pro Asn Ser Lys Asn Glu Lys
               20                  25                  30
   Ala Leu Gly Arg Lys Ile Asn Ser Trp Glu Ser Ser Arg Ser Gly His
           35                  40                  45
   Ser Phe Leu Ser Asn Leu His Leu Arg Asn Gly Glu Leu Val Ile His
       50                  55                  60
   Glu Lys Gly Phe Tyr Tyr Ile Tyr Ser Gln Thr Tyr Phe Arg Phe Gln
   65                  70                  75                  80
   Glu Glu Ile Lys Glu Asn Thr Lys Asn Asp Lys Gln Met Val Gln Tyr
                   85                  90                  95
   Ile Tyr Lys Tyr Thr Ser Tyr Pro Asp Pro Ile Leu Leu Met Lys Ser
               100                 105                 110
   Ala Arg Asn Ser Cys Trp Ser Lys Asp Ala Glu Tyr Gly Leu Tyr Ser
           115                 120                 125
   Ile Tyr Gln Gly Gly Ile Phe Glu Leu Lys Glu Asn Asp Arg Ile Phe
       130                 135                 140
   Val Ser Val Thr Asn Glu His Leu Ile Asp Met Asp His Glu Ala Ser
   145                 150                 155                 160
   Phe Phe Gly Ala Phe Leu Val Gly
                   165
<210> 92
   <211> 777
   <212> PRT
   <213> Artificial Sequence
<220>
   <223> pPE0141 ELP1-120 TRAIL
<400> 92
   Met Val Pro Gly Val Gly Val Pro Gly Val Gly Val Pro Gly Gly Gly
   1               5                   10                  15
   Val Pro Gly Ala Gly Val Pro Gly Val Gly Val Pro Gly Val Gly Val
               20                  25                  30
   Pro Gly Val Gly Val Pro Gly Gly Gly Val Pro Gly Ala Gly Val Pro
           35                  40                  45
   Gly Gly Gly Val Pro Gly Val Gly Val Pro Gly Val Gly Val Pro Gly
       50                  55                  60
   Gly Gly Val Pro Gly Ala Gly Val Pro Gly Val Gly Val Pro Gly Val
   65                  70                  75                  80
   Gly Val Pro Gly Val Gly Val Pro Gly Gly Gly Val Pro Gly Ala Gly
                   85                  90                  95
   Val Pro Gly Gly Gly Val Pro Gly Val Gly Val Pro Gly Val Gly Val
               100                 105                 110
   Pro Gly Gly Gly Val Pro Gly Ala Gly Val Pro Gly Val Gly Val Pro
           115                 120                 125
   Gly Val Gly Val Pro Gly Val Gly Val Pro Gly Gly Gly Val Pro Gly
       130                 135                 140
   Ala Gly Val Pro Gly Gly Gly Val Pro Gly Val Gly Val Pro Gly Val
   145                 150                 155                 160
   Gly Val Pro Gly Gly Gly Val Pro Gly Ala Gly Val Pro Gly Val Gly
                   165                 170                 175
   Val Pro Gly Val Gly Val Pro Gly Val Gly Val Pro Gly Gly Gly Val
               180                 185                 190
   Pro Gly Ala Gly Val Pro Gly Gly Gly Val Pro Gly Val Gly Val Pro
           195                 200                 205
   Gly Val Gly Val Pro Gly Gly Gly Val Pro Gly Ala Gly Val Pro Gly
       210                 215                 220
   Val Gly Val Pro Gly Val Gly Val Pro Gly Val Gly Val Pro Gly Gly
   225                 230                 235                 240
   Gly Val Pro Gly Ala Gly Val Pro Gly Gly Gly Val Pro Gly Val Gly
                   245                 250                 255
   Val Pro Gly Val Gly Val Pro Gly Gly Gly Val Pro Gly Ala Gly Val
               260                 265                 270
   Pro Gly Val Gly Val Pro Gly Val Gly Val Pro Gly Val Gly Val Pro
   275 280 285
   Gly Gly Gly Val Pro Gly Ala Gly Val Pro Gly Gly Gly Val Pro Gly
   290 295 300
   Val Gly Val Pro Gly Val Gly Val Pro Gly Gly Gly Val Pro Gly Ala
   305 310 315 320
   Gly Val Pro Gly Val Gly Val Pro Gly Val Gly Val Pro Gly Val Gly
   325 330 335
   Val Pro Gly Gly Gly Val Pro Gly Ala Gly Val Pro Gly Gly Gly Val
   340 345 350
   Pro Gly Val Gly Val Pro Gly Val Gly Val Pro Gly Gly Gly Val Pro
   355 360 365
   Gly Ala Gly Val Pro Gly Val Gly Val Pro Gly Val Gly Val Pro Gly
   370 375 380
   Val Gly Val Pro Gly Gly Gly Val Pro Gly Ala Gly Val Pro Gly Gly
   385 390 395 400
   Gly Val Pro Gly Val Gly Val Pro Gly Val Gly Val Pro Gly Gly Gly
   405 410 415
   Val Pro Gly Ala Gly Val Pro Gly Val Gly Val Pro Gly Val Gly Val
   420 425 430
   Pro Gly Val Gly Val Pro Gly Gly Gly Val Pro Gly Ala Gly Val Pro
   435 440 445
   Gly Gly Gly Val Pro Gly Val Gly Val Pro Gly Val Gly Val Pro Gly
   450 455 460
   Gly Gly Val Pro Gly Ala Gly Val Pro Gly Val Gly Val Pro Gly Val
   465 470 475 480
   Gly Val Pro Gly Val Gly Val Pro Gly Gly Gly Val Pro Gly Ala Gly
   485 490 495
   Val Pro Gly Gly Gly Val Pro Gly Val Gly Val Pro Gly Val Gly Val
   500 505 510
   Pro Gly Gly Gly Val Pro Gly Ala Gly Val Pro Gly Val Gly Val Pro
   515 520 525
   Gly Val Gly Val Pro Gly Val Gly Val Pro Gly Gly Gly Val Pro Gly
   530 535 540
   Ala Gly Val Pro Gly Gly Gly Val Pro Gly Val Gly Val Pro Gly Val
   545 550 555 560
   Gly Val Pro Gly Gly Gly Val Pro Gly Ala Gly Val Pro Gly Val Gly
   565 570 575
   Val Pro Gly Val Gly Val Pro Gly Val Gly Val Pro Gly Gly Gly Val
   580 585 590
   Pro Gly Ala Gly Val Pro Gly Gly Gly Val Pro Gly Val Pro Gly Gly
   595 600 605
   Gly Val Arg Glu Arg Gly Pro Gln Arg Val Ala Ala His Ile Thr Gly
   610 615 620
   Thr Arg Gly Arg Ser Asn Thr Leu Ser Ser Pro Asn Ser Lys Asn Glu
   625 630 635 640
   Lys Ala Leu Gly Arg Lys Ile Asn Ser Trp Glu Ser Ser Arg Ser Gly
   645 650 655
   His Ser Phe Leu Ser Asn Leu His Leu Arg Asn Gly Glu Leu Val Ile
   660 665 670
   His Glu Lys Gly Phe Tyr Tyr Ile Tyr Ser Gln Thr Tyr Phe Arg Phe
   675 680 685
   Gln Glu Glu Ile Lys Glu Asn Thr Lys Asn Asp Lys Gln Met Val Gln
   690 695 700
   Tyr Ile Tyr Lys Tyr Thr Ser Tyr Pro Asp Pro Ile Leu Leu Met Lys
   705 710 715 720
   Ser Ala Arg Asn Ser Cys Trp Ser Lys Asp Ala Glu Tyr Gly Leu Tyr
   725 730 735
   Ser Ile Tyr Gln Gly Gly Ile Phe Glu Leu Lys Glu Asn Asp Arg Ile
   740 745 750
   Phe Val Ser Val Thr Asn Glu His Leu Ile Asp Met Asp His Glu Ala
   755 760 765
   Ser Phe Phe Gly Ala Phe Leu Val Gly
   770 775
<210> 93
   <211> 5
   <212> PRT
   <213> Artificial Sequence
<220>
   <223> GGGGS linker
<400> 93
   Gly Gly Gly Gly Ser
   1 5
<210> 94
   <211> 5
   <212> PRT
   <213> Artificial Sequence
<220>
   <223> PAPAP linker
<400> 94
   Pro Ala Pro Ala Pro
   1 5
<210> 95
   <211> 5
   <212> PRT
   <213> Artificial Sequence
<220>
   <223> EAAAK linker
<400> 95
   Glu Ala Ala Ala Lys
   1 5
<210> 96
   <211> 1950
   <212> DNA
   <213> Artificial Sequence
<220>
   <223> CNP37 GGS2x ELP1-120 fusion
<400> 96
   caggaacatc cgaacgcgcg taaatataaa ggtgcgaaca aaaaaggcct gagcaaaggc        60
   tgctttggcc tgaaactgga tcgcattggc agcatgagcg gcctgggctg cggtggctct       120
   ggcggttctg taccgggcgt gggtgttccg ggcgtgggtg ttccgggtgg cggtgtgccg       180
   ggcgcaggtg ttcctggtgt aggtgtgccg ggtgttggtg tgccgggtgt tggtgtacca       240
   ggtggcggtg ttccgggtgc aggcgttccg ggtggcggtg tgccgggcgt gggtgttccg       300
   ggcgtgggtg ttccgggtgg cggtgtgccg ggcgcaggtg ttcctggtgt aggtgtgccg       360
   ggtgttggtg tgccgggtgt tggtgtacca ggtggcggtg ttccgggtgc aggcgttccg      420
   ggtggcggtg tgccgggcgt gggtgttccg ggcgtgggtg ttccgggtgg cggtgtgccg      480
   ggcgcaggtg ttcctggtgt aggtgtgccg ggtgttggtg tgccgggtgt tggtgtacca      540
   ggtggcggtg ttccgggtgc aggcgttccg ggtggcggtg tgccgggcgt gggtgttccg      600
   ggcgtgggtg ttccgggtgg cggtgtgccg ggcgcaggtg ttcctggtgt aggtgtgccg      660
   ggtgttggtg tgccgggtgt tggtgtacca ggtggcggtg ttccgggtgc aggcgttccg      720
   ggtggcggtg tgccgggcgt gggtgttccg ggcgtgggtg ttccgggtgg cggtgtgccg      780
   ggcgcaggtg ttcctggtgt aggtgtgccg ggtgttggtg tgccgggtgt tggtgtacca      840
   ggtggcggtg ttccgggtgc aggcgttccg ggtggcggtg tgccgggcgt gggtgttccg      900
   ggcgtgggtg ttccgggtgg cggtgtgccg ggcgcaggtg ttcctggtgt aggtgtgccg      960
   ggtgttggtg tgccgggtgt tggtgtacca ggtggcggtg ttccgggtgc aggcgttccg     1020
   ggtggcggtg tgccgggcgt gggtgttccg ggcgtgggtg ttccgggtgg cggtgtgccg     1080
   ggcgcaggtg ttcctggtgt aggtgtgccg ggtgttggtg tgccgggtgt tggtgtacca     1140
   ggtggcggtg ttccgggtgc aggcgttccg ggtggcggtg tgccgggcgt gggtgttccg     1200
   ggcgtgggtg ttccgggtgg cggtgtgccg ggcgcaggtg ttcctggtgt aggtgtgccg     1260
   ggtgttggtg tgccgggtgt tggtgtacca ggtggcggtg ttccgggtgc aggcgttccg     1320
   ggtggcggtg tgccgggcgt gggtgttccg ggcgtgggtg ttccgggtgg cggtgtgccg     1380
   ggcgcaggtg ttcctggtgt aggtgtgccg ggtgttggtg tgccgggtgt tggtgtacca     1440
   ggtggcggtg ttccgggtgc aggcgttccg ggtggcggtg tgccgggcgt gggtgttccg     1500
   ggcgtgggtg ttccgggtgg cggtgtgccg ggcgcaggtg ttcctggtgt aggtgtgccg     1560
   ggtgttggtg tgccgggtgt tggtgtacca ggtggcggtg ttccgggtgc aggcgttccg     1620
   ggtggcggtg tgccgggcgt gggtgttccg ggcgtgggtg ttccgggtgg cggtgtgccg     1680
   ggcgcaggtg ttcctggtgt aggtgtgccg ggtgttggtg tgccgggtgt tggtgtacca     1740
   ggtggcggtg ttccgggtgc aggcgttccg ggtggcggtg tgccgggcgt gggtgttccg     1800
   ggcgtgggtg ttccgggtgg cggtgtgccg ggcgcaggtg ttcctggtgt aggtgtgccg     1860
   ggtgttggtg tgccgggtgt tggtgtacca ggtggcggtg ttccgggtgc aggcgttccg     1920
   ggtggcggtg tgccgggctg gccgtgataa                                      1950
<210> 97
   <211> 1890
   <212> DNA
   <213> Artificial Sequence
<220>
   <223> ELP1-120 CNP22 fusion
<400> 97
   atggtaccgg gcgtgggtgt tccgggcgtg ggtgttccgg gtggcggtgt gccgggcgca      60
   ggtgttcctg gtgtaggtgt gccgggtgtt ggtgtgccgg gtgttggtgt accaggtggc     120
   ggtgttccgg gtgcaggcgt tccgggtggc ggtgtgccgg gcgtgggtgt tccgggcgtg     180
   ggtgttccgg gtggcggtgt gccgggcgca ggtgttcctg gtgtaggtgt gccgggtgtt     240
   ggtgtgccgg gtgttggtgt accaggtggc ggtgttccgg gtgcaggcgt tccgggtggc     300
   ggtgtgccgg gcgtgggtgt tccgggcgtg ggtgttccgg gtggcggtgt gccgggcgca     360
   ggtgttcctg gtgtaggtgt gccgggtgtt ggtgtgccgg gtgttggtgt accaggtggc     420
   ggtgttccgg gtgcaggcgt tccgggtggc ggtgtgccgg gcgtgggtgt tccgggcgtg     480
   ggtgttccgg gtggcggtgt gccgggcgca ggtgttcctg gtgtaggtgt gccgggtgtt     540
   ggtgtgccgg gtgttggtgt accaggtggc ggtgttccgg gtgcaggcgt tccgggtggc     600
   ggtgtgccgg gcgtgggtgt tccgggcgtg ggtgttccgg gtggcggtgt gccgggcgca     660
   ggtgttcctg gtgtaggtgt gccgggtgtt ggtgtgccgg gtgttggtgt accaggtggc     720
   ggtgttccgg gtgcaggcgt tccgggtggc ggtgtgccgg gcgtgggtgt tccgggcgtg     780
   ggtgttccgg gtggcggtgt gccgggcgca ggtgttcctg gtgtaggtgt gccgggtgtt     840
   ggtgtgccgg gtgttggtgt accaggtggc ggtgttccgg gtgcaggcgt tccgggtggc     900
   ggtgtgccgg gcgtgggtgt tccgggcgtg ggtgttccgg gtggcggtgt gccgggcgca     960
   ggtgttcctg gtgtaggtgt gccgggtgtt ggtgtgccgg gtgttggtgt accaggtggc    1020
   ggtgttccgg gtgcaggcgt tccgggtggc ggtgtgccgg gcgtgggtgt tccgggcgtg    1080
   ggtgttccgg gtggcggtgt gccgggcgca ggtgttcctg gtgtaggtgt gccgggtgtt    1140
   ggtgtgccgg gtgttggtgt accaggtggc ggtgttccgg gtgcaggcgt tccgggtggc    1200
   ggtgtgccgg gcgtgggtgt tccgggcgtg ggtgttccgg gtggcggtgt gccgggcgca    1260
   ggtgttcctg gtgtaggtgt gccgggtgtt ggtgtgccgg gtgttggtgt accaggtggc    1320
   ggtgttccgg gtgcaggcgt tccgggtggc ggtgtgccgg gcgtgggtgt tccgggcgtg    1380
   ggtgttccgg gtggcggtgt gccgggcgca ggtgttcctg gtgtaggtgt gccgggtgtt    1440
   ggtgtgccgg gtgttggtgt accaggtggc ggtgttccgg gtgcaggcgt tccgggtggc    1500
   ggtgtgccgg gcgtgggtgt tccgggcgtg ggtgttccgg gtggcggtgt gccgggcgca    1560
   ggtgttcctg gtgtaggtgt gccgggtgtt ggtgtgccgg gtgttggtgt accaggtggc    1620
   ggtgttccgg gtgcaggcgt tccgggtggc ggtgtgccgg gcgtgggtgt tccgggcgtg    1680
   ggtgttccgg gtggcggtgt gccgggcgca ggtgttcctg gtgtaggtgt gccgggtgtt    1740
   ggtgtgccgg gtgttggtgt accaggtggc ggtgttccgg gtgcaggcgt tccgggtggc    1800
   ggtgtgccgg gctggccggg cctgagcaaa ggctgctttg gcctgaaact ggatcgcatt    1860
   ggcagcatga gcggcctggg ctgctgataa 1890
<210> 98
   <211> 628
   <212> PRT
   <213> Artificial Sequence
<220>
   <223> CNP22 ELP1-120 fusion
<400> 98
   Gly Leu Ser Lys Gly Cys Phe Gly Leu Lys Leu Asp Arg Ile Gly Ser
   1               5                   10                  15
   Met Ser Gly Leu Gly Cys Gly Val Pro Gly Val Gly Val Pro Gly Val
               20                  25                  30
   Gly Val Pro Gly Gly Gly Val Pro Gly Ala Gly Val Pro Gly Val Gly
           35                  40                  45
   Val Pro Gly Val Gly Val Pro Gly Val Gly Val Pro Gly Gly Gly Val
       50                  55                  60
   Pro Gly Ala Gly Val Pro Gly Gly Gly Val Pro Gly Val Gly Val Pro
   65                  70                  75                  80
   Gly Val Gly Val Pro Gly Gly Gly Val Pro Gly Ala Gly Val Pro Gly
                   85                  90                  95
   Val Gly Val Pro Gly Val Gly Val Pro Gly Val Gly Val Pro Gly Gly
               100                 105                 110
   Gly Val Pro Gly Ala Gly Val Pro Gly Gly Gly Val Pro Gly Val Gly
           115                 120                 125
   Val Pro Gly Val Gly Val Pro Gly Gly Gly Val Pro Gly Ala Gly Val
       130                 135                 140
   Pro Gly Val Gly Val Pro Gly Val Gly Val Pro Gly Val Gly Val Pro
   145                 150                 155                 160
   Gly Gly Gly Val Pro Gly Ala Gly Val Pro Gly Gly Gly Val Pro Gly
                   165                 170                 175
   Val Gly Val Pro Gly Val Gly Val Pro Gly Gly Gly Val Pro Gly Ala
               180                 185                 190
   Gly Val Pro Gly Val Gly Val Pro Gly Val Gly Val Pro Gly Val Gly
           195                 200                 205
   Val Pro Gly Gly Gly Val Pro Gly Ala Gly Val Pro Gly Gly Gly Val
       210                 215                 220
   Pro Gly Val Gly Val Pro Gly Val Gly Val Pro Gly Gly Gly Val Pro
   225                 230                 235                 240
   Gly Ala Gly Val Pro Gly Val Gly Val Pro Gly Val Gly Val Pro Gly
                   245                 250                 255
   Val Gly Val Pro Gly Gly Gly Val Pro Gly Ala Gly Val Pro Gly Gly
               260                 265                 270
   Gly Val Pro Gly Val Gly Val Pro Gly Val Gly Val Pro Gly Gly Gly
           275                 280                 285
   Val Pro Gly Ala Gly Val Pro Gly Val Gly Val Pro Gly Val Gly Val
       290                 295                 300
   Pro Gly Val Gly Val Pro Gly Gly Gly Val Pro Gly Ala Gly Val Pro
   305                 310                 315                 320
   Gly Gly Gly Val Pro Gly Val Gly Val Pro Gly Val Gly Val Pro Gly
                   325                 330                 335
   Gly Gly Val Pro Gly Ala Gly Val Pro Gly Val Gly Val Pro Gly Val
               340                 345                 350
   Gly Val Pro Gly Val Gly Val Pro Gly Gly Gly Val Pro Gly Ala Gly
           355                 360                 365
   Val Pro Gly Gly Gly Val Pro Gly Val Gly Val Pro Gly Val Gly Val
       370                 375                 380
   Pro Gly Gly Gly Val Pro Gly Ala Gly Val Pro Gly Val Gly Val Pro
   385                 390                 395                 400
   Gly Val Gly Val Pro Gly Val Gly Val Pro Gly Gly Gly Val Pro Gly
                   405                 410                 415
   Ala Gly Val Pro Gly Gly Gly Val Pro Gly Val Gly Val Pro Gly Val
               420                 425                 430
   Gly Val Pro Gly Gly Gly Val Pro Gly Ala Gly Val Pro Gly Val Gly
           435                 440                 445
   Val Pro Gly Val Gly Val Pro Gly Val Gly Val Pro Gly Gly Gly Val
       450                 455                 460
   Pro Gly Ala Gly Val Pro Gly Gly Gly Val Pro Gly Val Gly Val Pro
   465                 470                 475                 480
   Gly Val Gly Val Pro Gly Gly Gly Val Pro Gly Ala Gly Val Pro Gly
                   485                 490                 495
   Val Gly Val Pro Gly Val Gly Val Pro Gly Val Gly Val Pro Gly Gly
               500                 505                 510
   Gly Val Pro Gly Ala Gly Val Pro Gly Gly Gly Val Pro Gly Val Gly
           515                 520                 525
   Val Pro Gly Val Gly Val Pro Gly Gly Gly Val Pro Gly Ala Gly Val
       530                 535                 540
   Pro Gly Val Gly Val Pro Gly Val Gly Val Pro Gly Val Gly Val Pro
   545                 550                 555                 560
   Gly Gly Gly Val Pro Gly Ala Gly Val Pro Gly Gly Gly Val Pro Gly
                   565                 570                 575
   Val Gly Val Pro Gly Val Gly Val Pro Gly Gly Gly Val Pro Gly Ala
               580                 585                 590
   Gly Val Pro Gly Val Gly Val Pro Gly Val Gly Val Pro Gly Val Gly
           595                 600                 605
   Val Pro Gly Gly Gly Val Pro Gly Ala Gly Val Pro Gly Gly Gly Val
       610                 615                 620
   Pro Gly Trp Pro
   625
<210> 99
   <211> 1890
   <212> DNA
   <213> Artificial Sequence
<220>
   <223> CNP ELP1-120 fusion
<400> 99
   ggcctgagca aaggctgctt tggcctgaaa ctggatcgca ttggcagcat gagcggcctg      60
   ggctgcggtg taccgggcgt gggtgttccg ggcgtgggtg ttccgggtgg cggtgtgccg     120
   ggcgcaggtg ttcctggtgt aggtgtgccg ggtgttggtg tgccgggtgt tggtgtacca     180
   ggtggcggtg ttccgggtgc aggcgttccg ggtggcggtg tgccgggcgt gggtgttccg     240
   ggcgtgggtg ttccgggtgg cggtgtgccg ggcgcaggtg ttcctggtgt aggtgtgccg 300
   ggtgttggtg tgccgggtgt tggtgtacca ggtggcggtg ttccgggtgc aggcgttccg 360
   ggtggcggtg tgccgggcgt gggtgttccg ggcgtgggtg ttccgggtgg cggtgtgccg 420
   ggcgcaggtg ttcctggtgt aggtgtgccg ggtgttggtg tgccgggtgt tggtgtacca 480
   ggtggcggtg ttccgggtgc aggcgttccg ggtggcggtg tgccgggcgt gggtgttccg 540
   ggcgtgggtg ttccgggtgg cggtgtgccg ggcgcaggtg ttcctggtgt aggtgtgccg 600
   ggtgttggtg tgccgggtgt tggtgtacca ggtggcggtg ttccgggtgc aggcgttccg 660
   ggtggcggtg tgccgggcgt gggtgttccg ggcgtgggtg ttccgggtgg cggtgtgccg 720
   ggcgcaggtg ttcctggtgt aggtgtgccg ggtgttggtg tgccgggtgt tggtgtacca 780
   ggtggcggtg ttccgggtgc aggcgttccg ggtggcggtg tgccgggcgt gggtgttccg 840
   ggcgtgggtg ttccgggtgg cggtgtgccg ggcgcaggtg ttcctggtgt aggtgtgccg 900
   ggtgttggtg tgccgggtgt tggtgtacca ggtggcggtg ttccgggtgc aggcgttccg 960
   ggtggcggtg tgccgggcgt gggtgttccg ggcgtgggtg ttccgggtgg cggtgtgccg 1020
   ggcgcaggtg ttcctggtgt aggtgtgccg ggtgttggtg tgccgggtgt tggtgtacca 1080
   ggtggcggtg ttccgggtgc aggcgttccg ggtggcggtg tgccgggcgt gggtgttccg 1140
   ggcgtgggtg ttccgggtgg cggtgtgccg ggcgcaggtg ttcctggtgt aggtgtgccg 1200
   ggtgttggtg tgccgggtgt tggtgtacca ggtggcggtg ttccgggtgc aggcgttccg 1260
   ggtggcggtg tgccgggcgt gggtgttccg ggcgtgggtg ttccgggtgg cggtgtgccg 1320
   ggcgcaggtg ttcctggtgt aggtgtgccg ggtgttggtg tgccgggtgt tggtgtacca 1380
   ggtggcggtg ttccgggtgc aggcgttccg ggtggcggtg tgccgggcgt gggtgttccg 1440
   ggcgtgggtg ttccgggtgg cggtgtgccg ggcgcaggtg ttcctggtgt aggtgtgccg 1500
   ggtgttggtg tgccgggtgt tggtgtacca ggtggcggtg ttccgggtgc aggcgttccg 1560
   ggtggcggtg tgccgggcgt gggtgttccg ggcgtgggtg ttccgggtgg cggtgtgccg 1620
   ggcgcaggtg ttcctggtgt aggtgtgccg ggtgttggtg tgccgggtgt tggtgtacca 1680
   ggtggcggtg ttccgggtgc aggcgttccg ggtggcggtg tgccgggcgt gggtgttccg 1740
   ggcgtgggtg ttccgggtgg cggtgtgccg ggcgcaggtg ttcctggtgt aggtgtgccg 1800
   ggtgttggtg tgccgggtgt tggtgtacca ggtggcggtg ttccgggtgc aggcgttccg 1860
   ggtggcggtg tgccgggctg gccgtgataa 1890
<210> 100
   <211> 1905
   <212> DNA
   <213> Artificial Sequence
<220>
   <223> CNP22 GGS2X ELP1-120 fusion
<400> 100
   ggcctgagca aaggctgctt tggcctgaaa ctggatcgca ttggcagcat gagcggcctg      60
   ggctgcggtg gctctggcgg ttctgtaccg ggcgtgggtg ttccgggcgt gggtgttccg     120
   ggtggcggtg tgccgggcgc aggtgttcct ggtgtaggtg tgccgggtgt tggtgtgccg     180
   ggtgttggtg taccaggtgg cggtgttccg ggtgcaggcg ttccgggtgg cggtgtgccg     240
   ggcgtgggtg ttccgggcgt gggtgttccg ggtggcggtg tgccgggcgc aggtgttcct     300
   ggtgtaggtg tgccgggtgt tggtgtgccg ggtgttggtg taccaggtgg cggtgttccg     360
   ggtgcaggcg ttccgggtgg cggtgtgccg ggcgtgggtg ttccgggcgt gggtgttccg     420
   ggtggcggtg tgccgggcgc aggtgttcct ggtgtaggtg tgccgggtgt tggtgtgccg     480
   ggtgttggtg taccaggtgg cggtgttccg ggtgcaggcg ttccgggtgg cggtgtgccg     540
   ggcgtgggtg ttccgggcgt gggtgttccg ggtggcggtg tgccgggcgc aggtgttcct     600
   ggtgtaggtg tgccgggtgt tggtgtgccg ggtgttggtg taccaggtgg cggtgttccg     660
   ggtgcaggcg ttccgggtgg cggtgtgccg ggcgtgggtg ttccgggcgt gggtgttccg     720
   ggtggcggtg tgccgggcgc aggtgttcct ggtgtaggtg tgccgggtgt tggtgtgccg     780
   ggtgttggtg taccaggtgg cggtgttccg ggtgcaggcg ttccgggtgg cggtgtgccg     840
   ggcgtgggtg ttccgggcgt gggtgttccg ggtggcggtg tgccgggcgc aggtgttcct     900
   ggtgtaggtg tgccgggtgt tggtgtgccg ggtgttggtg taccaggtgg cggtgttccg     960
   ggtgcaggcg ttccgggtgg cggtgtgccg ggcgtgggtg ttccgggcgt gggtgttccg    1020
   ggtggcggtg tgccgggcgc aggtgttcct ggtgtaggtg tgccgggtgt tggtgtgccg    1080
   ggtgttggtg taccaggtgg cggtgttccg ggtgcaggcg ttccgggtgg cggtgtgccg    1140
   ggcgtgggtg ttccgggcgt gggtgttccg ggtggcggtg tgccgggcgc aggtgttcct    1200
   ggtgtaggtg tgccgggtgt tggtgtgccg ggtgttggtg taccaggtgg cggtgttccg    1260
   ggtgcaggcg ttccgggtgg cggtgtgccg ggcgtgggtg ttccgggcgt gggtgttccg    1320
   ggtggcggtg tgccgggcgc aggtgttcct ggtgtaggtg tgccgggtgt tggtgtgccg    1380
   ggtgttggtg taccaggtgg cggtgttccg ggtgcaggcg ttccgggtgg cggtgtgccg    1440
   ggcgtgggtg ttccgggcgt gggtgttccg ggtggcggtg tgccgggcgc aggtgttcct    1500
   ggtgtaggtg tgccgggtgt tggtgtgccg ggtgttggtg taccaggtgg cggtgttccg    1560
   ggtgcaggcg ttccgggtgg cggtgtgccg ggcgtgggtg ttccgggcgt gggtgttccg    1620
   ggtggcggtg tgccgggcgc aggtgttcct ggtgtaggtg tgccgggtgt tggtgtgccg    1680
   ggtgttggtg taccaggtgg cggtgttccg ggtgcaggcg ttccgggtgg cggtgtgccg    1740
   ggcgtgggtg ttccgggcgt gggtgttccg ggtggcggtg tgccgggcgc aggtgttcct    1800
   ggtgtaggtg tgccgggtgt tggtgtgccg ggtgttggtg taccaggtgg cggtgttccg 1860
   ggtgcaggcg ttccgggtgg cggtgtgccg ggctggccgt gataa 1905
<210> 101
   <211> 1920
   <212> DNA
   <213> Artificial Sequence
<220>
   <223> GLP-2 ELP1-120 fusion
<400> 101
   cacggcgatg gcagcttttc tgatgaaatg aataccattc tggacaacct ggcagcacgc      60
   gactttatta actggctgat tcagacgaaa attactgatg taccgggcgt gggtgttccg     120
   ggcgtgggtg ttccgggtgg cggtgtgccg ggcgcaggtg ttcctggtgt aggtgtgccg     180
   ggtgttggtg tgccgggtgt tggtgtacca ggtggcggtg ttccgggtgc aggcgttccg     240
   ggtggcggtg tgccgggcgt gggtgttccg ggcgtgggtg ttccgggtgg cggtgtgccg     300
   ggcgcaggtg ttcctggtgt aggtgtgccg ggtgttggtg tgccgggtgt tggtgtacca     360
   ggtggcggtg ttccgggtgc aggcgttccg ggtggcggtg tgccgggcgt gggtgttccg     420
   ggcgtgggtg ttccgggtgg cggtgtgccg ggcgcaggtg ttcctggtgt aggtgtgccg     480
   ggtgttggtg tgccgggtgt tggtgtacca ggtggcggtg ttccgggtgc aggcgttccg     540
   ggtggcggtg tgccgggcgt gggtgttccg ggcgtgggtg ttccgggtgg cggtgtgccg     600
   ggcgcaggtg ttcctggtgt aggtgtgccg ggtgttggtg tgccgggtgt tggtgtacca     660
   ggtggcggtg ttccgggtgc aggcgttccg ggtggcggtg tgccgggcgt gggtgttccg     720
   ggcgtgggtg ttccgggtgg cggtgtgccg ggcgcaggtg ttcctggtgt aggtgtgccg     780
   ggtgttggtg tgccgggtgt tggtgtacca ggtggcggtg ttccgggtgc aggcgttccg     840
   ggtggcggtg tgccgggcgt gggtgttccg ggcgtgggtg ttccgggtgg cggtgtgccg     900
   ggcgcaggtg ttcctggtgt aggtgtgccg ggtgttggtg tgccgggtgt tggtgtacca     960
   ggtggcggtg ttccgggtgc aggcgttccg ggtggcggtg tgccgggcgt gggtgttccg    1020
   ggcgtgggtg ttccgggtgg cggtgtgccg ggcgcaggtg ttcctggtgt aggtgtgccg    1080
   ggtgttggtg tgccgggtgt tggtgtacca ggtggcggtg ttccgggtgc aggcgttccg    1140
   ggtggcggtg tgccgggcgt gggtgttccg ggcgtgggtg ttccgggtgg cggtgtgccg    1200
   ggcgcaggtg ttcctggtgt aggtgtgccg ggtgttggtg tgccgggtgt tggtgtacca    1260
   ggtggcggtg ttccgggtgc aggcgttccg ggtggcggtg tgccgggcgt gggtgttccg    1320
   ggcgtgggtg ttccgggtgg cggtgtgccg ggcgcaggtg ttcctggtgt aggtgtgccg    1380
   ggtgttggtg tgccgggtgt tggtgtacca ggtggcggtg ttccgggtgc aggcgttccg    1440
   ggtggcggtg tgccgggcgt gggtgttccg ggcgtgggtg ttccgggtgg cggtgtgccg    1500
   ggcgcaggtg ttcctggtgt aggtgtgccg ggtgttggtg tgccgggtgt tggtgtacca     1560
   ggtggcggtg ttccgggtgc aggcgttccg ggtggcggtg tgccgggcgt gggtgttccg     1620
   ggcgtgggtg ttccgggtgg cggtgtgccg ggcgcaggtg ttcctggtgt aggtgtgccg     1680
   ggtgttggtg tgccgggtgt tggtgtacca ggtggcggtg ttccgggtgc aggcgttccg     1740
   ggtggcggtg tgccgggcgt gggtgttccg ggcgtgggtg ttccgggtgg cggtgtgccg     1800
   ggcgcaggtg ttcctggtgt aggtgtgccg ggtgttggtg tgccgggtgt tggtgtacca     1860
   ggtggcggtg ttccgggtgc aggcgttccg ggtggcggtg tgccgggctg gccgtgataa     1920
<210> 102
   <211> 6
   <212> PRT
   <213> Artificial Sequence
<220>
   <223> GGS 2X
<400> 102
   Gly Gly Ser Gly Gly Ser
   1               5
<210> 103
   <211> 9
   <212> PRT
   <213> Artificial Sequence
<220>
   <223> GGS 3X
<400> 103
   Gly Gly Ser Gly Gly Ser Gly Gly Ser
   1               5
<210> 104
   <211> 10
   <212> PRT
   <213> Artificial Sequence
<220>
   <223> GGGGS 2X
<400> 104
   Gly Gly Gly Gly Ser Gly Gly Gly Gly Ser
   1               5                   10
<210> 105
   <211> 15
   <212> PRT
   <213> Artificial Sequence
<220>
   <223> GGGGS 3X
<400> 105
   Gly Gly Gly Gly Ser Gly Gly Gly Gly Ser Gly Gly Gly Gly Ser
   1               5                   10                  15
<210> 106
   <211> 10
   <212> PRT
   <213> Artificial Sequence
<220>
   <223> PAPAP 2X
<400> 106
   Pro Ala Pro Ala Pro Pro Ala Pro Ala Pro
   1               5                   10
<210> 107
   <211> 15
   <212> PRT
   <213> Artificial Sequence
<220>
   <223> PAPAP 3X
<400> 107
   Pro Ala Pro Ala Pro Pro Ala Pro Ala Pro Pro Ala Pro Ala Pro
   1               5                   10                  15
<210> 108
   <211> 10
   <212> PRT
   <213> Artificial Sequence
<220>
   <223> EAAAK 2X
<400> 108
   Glu Ala Ala Ala Lys Glu Ala Ala Ala Lys
   1               5                   10
<210> 109
   <211> 15
   <212> PRT
   <213> Artificial Sequence
<220>
   <223> EAAAK 3X
<400> 109
   Glu Ala Ala Ala Lys Glu Ala Ala Ala Lys Glu Ala Ala Ala Lys
   1 5 10 15
<210> 110
   <211> 209
   <212> PRT
   <213> Artificial Sequence
<220>
   <223> FGF21
<400> 110
   Met Asp Ser Asp Glu Thr Gly Phe Glu His Ser Gly Leu Trp Val Ser
   1               5                   10                  15
   Val Leu Ala Gly Leu Leu Leu Gly Ala Cys Gln Ala His Pro Ile Pro
               20                  25                  30
   Asp Ser Ser Pro Leu Leu Gln Phe Gly Gly Gln Val Arg Gln Arg Tyr
           35                  40                  45
   Leu Tyr Thr Asp Asp Ala Gln Gln Thr Glu Ala His Leu Glu Ile Arg
       50                  55                  60
   Glu Asp Gly Thr Val Gly Gly Ala Ala Asp Gln Ser Pro Glu Ser Leu
   65                  70                  75                  80
   Leu Gln Leu Lys Ala Leu Lys Pro Gly Val Ile Gln Ile Leu Gly Val
                   85                  90                  95
   Lys Thr Ser Arg Phe Leu Cys Gln Arg Pro Asp Gly Ala Leu Tyr Gly
               100                 105                 110
   Ser Leu His Phe Asp Pro Glu Ala Cys Ser Phe Arg Glu Leu Leu Leu
           115                 120                 125
   Glu Asp Gly Tyr Asn Val Tyr Gln Ser Glu Ala His Gly Leu Pro Leu
       130                 135                 140
   His Leu Pro Gly Asn Lys Ser Pro His Arg Asp Pro Ala Pro Arg Gly
   145                 150                 155                 160
   Pro Ala Arg Phe Leu Pro Leu Pro Gly Leu Pro Pro Ala Leu Pro Glu
                   165                 170                 175
   Pro Pro Gly Ile Leu Ala Pro Gln Pro Pro Asp Val Gly Ser Ser Asp
               180                 185                 190
   Pro Leu Ser Met Val Gly Pro Ser Gln Gly Arg Ser Pro Ser Tyr Ala
           195                 200                 205
   Ser
<210> 111
   <211> 663
   <212> PRT
   <213> Artificial Sequence
<220>
   <223> CNP53 CatK ELP1-120 fusion PE9446
<400> 111
   Asp Leu Arg Val Asp Thr Lys Ser Arg Ala Ala Trp Ala Arg Leu Leu
   1               5                   10                  15
   Gln Glu His Pro Asn Ala Arg Lys Tyr Lys Gly Ala Asn Lys Lys Gly
               20                  25                  30
   Leu Ser Lys Gly Cys Phe Gly Leu Lys Leu Asp Arg Ile Gly Ser Met
           35                  40                  45
   Ser Gly Leu Gly Cys Arg Lys Pro Arg Gly Val Pro Gly Val Gly Val
       50                  55                  60
   Pro Gly Val Gly Val Pro Gly Gly Gly Val Pro Gly Ala Gly Val Pro
   65                  70                  75                  80
   Gly Val Gly Val Pro Gly Val Gly Val Pro Gly Val Gly Val Pro Gly
                   85                  90                  95
   Gly Gly Val Pro Gly Ala Gly Val Pro Gly Gly Gly Val Pro Gly Val
               100                 105                 110
   Gly Val Pro Gly Val Gly Val Pro Gly Gly Gly Val Pro Gly Ala Gly
           115                 120                 125
   Val Pro Gly Val Gly Val Pro Gly Val Gly Val Pro Gly Val Gly Val
       130                 135                 140
   Pro Gly Gly Gly Val Pro Gly Ala Gly Val Pro Gly Gly Gly Val Pro
   145                 150                 155                 160
   Gly Val Gly Val Pro Gly Val Gly Val Pro Gly Gly Gly Val Pro Gly
                   165                 170                 175
   Ala Gly Val Pro Gly Val Gly Val Pro Gly Val Gly Val Pro Gly Val
               180                 185                 190
   Gly Val Pro Gly Gly Gly Val Pro Gly Ala Gly Val Pro Gly Gly Gly
           195                 200                 205
   Val Pro Gly Val Gly Val Pro Gly Val Gly Val Pro Gly Gly Gly Val
       210                 215                 220
   Pro Gly Ala Gly Val Pro Gly Val Gly Val Pro Gly Val Gly Val Pro
   225                 230                 235                 240
   Gly Val Gly Val Pro Gly Gly Gly Val Pro Gly Ala Gly Val Pro Gly
                   245                 250                 255
   Gly Gly Val Pro Gly Val Gly Val Pro Gly Val Gly Val Pro Gly Gly
               260                 265                 270
   Gly Val Pro Gly Ala Gly Val Pro Gly Val Gly Val Pro Gly Val Gly
           275                 280                 285
   Val Pro Gly Val Gly Val Pro Gly Gly Gly Val Pro Gly Ala Gly Val
       290                 295                 300
   Pro Gly Gly Gly Val Pro Gly Val Gly Val Pro Gly Val Gly Val Pro
   305                 310                 315                 320
   Gly Gly Gly Val Pro Gly Ala Gly Val Pro Gly Val Gly Val Pro Gly
                   325                 330                 335
   Val Gly Val Pro Gly Val Gly Val Pro Gly Gly Gly Val Pro Gly Ala
               340                 345                 350
   Gly Val Pro Gly Gly Gly Val Pro Gly Val Gly Val Pro Gly Val Gly
           355                 360                 365
   Val Pro Gly Gly Gly Val Pro Gly Ala Gly Val Pro Gly Val Gly Val
       370                 375                 380
   Pro Gly Val Gly Val Pro Gly Val Gly Val Pro Gly Gly Gly Val Pro
   385                 390                 395                 400
   Gly Ala Gly Val Pro Gly Gly Gly Val Pro Gly Val Gly Val Pro Gly
                   405                 410                 415
   Val Gly Val Pro Gly Gly Gly Val Pro Gly Ala Gly Val Pro Gly Val
               420                 425                 430
   Gly Val Pro Gly Val Gly Val Pro Gly Val Gly Val Pro Gly Gly Gly
           435                 440                 445
   Val Pro Gly Ala Gly Val Pro Gly Gly Gly Val Pro Gly Val Gly Val
       450                 455                 460
   Pro Gly Val Gly Val Pro Gly Gly Gly Val Pro Gly Ala Gly Val Pro
   465                 470                 475                 480
   Gly Val Gly Val Pro Gly Val Gly Val Pro Gly Val Gly Val Pro Gly
                   485                 490                 495
   Gly Gly Val Pro Gly Ala Gly Val Pro Gly Gly Gly Val Pro Gly Val
               500                 505                 510
   Gly Val Pro Gly Val Gly Val Pro Gly Gly Gly Val Pro Gly Ala Gly
           515                 520                 525
   Val Pro Gly Val Gly Val Pro Gly Val Gly Val Pro Gly Val Gly Val
       530                 535                 540
   Pro Gly Gly Gly Val Pro Gly Ala Gly Val Pro Gly Gly Gly Val Pro
   545                 550                 555                 560
   Gly Val Gly Val Pro Gly Val Gly Val Pro Gly Gly Gly Val Pro Gly
                   565                 570                 575
   Ala Gly Val Pro Gly Val Gly Val Pro Gly Val Gly Val Pro Gly Val
               580                 585                 590
   Gly Val Pro Gly Gly Gly Val Pro Gly Ala Gly Val Pro Gly Gly Gly
           595                 600                 605
   Val Pro Gly Val Gly Val Pro Gly Val Gly Val Pro Gly Gly Gly Val
       610                 615                 620
   Pro Gly Ala Gly Val Pro Gly Val Gly Val Pro Gly Val Gly Val Pro
   625                 630                 635                 640
   Gly Val Gly Val Pro Gly Gly Gly Val Pro Gly Ala Gly Val Pro Gly
                   645                 650                 655
   Gly Gly Val Pro Gly Trp Pro
               660
<210> 112
   <211> 1989
   <212> DNA
   <213> Artificial Sequence
<220>
   <223> CNP53 CatK ELP1-120 fusion PE9446
<400> 112
   gatctgcgcg tggataccaa aagccgcgcg gcgtgggcgc gcctgctgca ggaacatccg 60
   aacgcgcgca aatataaagg cgcgaacaaa aaaggcctga gcaaaggctg ctttggcctg       120
   aaactggatc gcattggcag catgagcggc ctgggctgcc gcaaaccgcg cggtgtaccg       180
   ggcgtgggtg ttccgggcgt gggtgttccg ggtggcggtg tgccgggcgc aggtgttcct       240
   ggtgtaggtg tgccgggtgt tggtgtgccg ggtgttggtg taccaggtgg cggtgttccg       300
   ggtgcaggcg ttccgggtgg cggtgtgccg ggcgtgggtg ttccgggcgt gggtgttccg       360
   ggtggcggtg tgccgggcgc aggtgttcct ggtgtaggtg tgccgggtgt tggtgtgccg       420
   ggtgttggtg taccaggtgg cggtgttccg ggtgcaggcg ttccgggtgg cggtgtgccg       480
   ggcgtgggtg ttccgggcgt gggtgttccg ggtggcggtg tgccgggcgc aggtgttcct       540
   ggtgtaggtg tgccgggtgt tggtgtgccg ggtgttggtg taccaggtgg cggtgttccg       600
   ggtgcaggcg ttccgggtgg cggtgtgccg ggcgtgggtg ttccgggcgt gggtgttccg       660
   ggtggcggtg tgccgggcgc aggtgttcct ggtgtaggtg tgccgggtgt tggtgtgccg       720
   ggtgttggtg taccaggtgg cggtgttccg ggtgcaggcg ttccgggtgg cggtgtgccg       780
   ggcgtgggtg ttccgggcgt gggtgttccg ggtggcggtg tgccgggcgc aggtgttcct       840
   ggtgtaggtg tgccgggtgt tggtgtgccg ggtgttggtg taccaggtgg cggtgttccg       900
   ggtgcaggcg ttccgggtgg cggtgtgccg ggcgtgggtg ttccgggcgt gggtgttccg       960
   ggtggcggtg tgccgggcgc aggtgttcct ggtgtaggtg tgccgggtgt tggtgtgccg      1020
   ggtgttggtg taccaggtgg cggtgttccg ggtgcaggcg ttccgggtgg cggtgtgccg      1080
   ggcgtgggtg ttccgggcgt gggtgttccg ggtggcggtg tgccgggcgc aggtgttcct      1140
   ggtgtaggtg tgccgggtgt tggtgtgccg ggtgttggtg taccaggtgg cggtgttccg      1200
   ggtgcaggcg ttccgggtgg cggtgtgccg ggcgtgggtg ttccgggcgt gggtgttccg      1260
   ggtggcggtg tgccgggcgc aggtgttcct ggtgtaggtg tgccgggtgt tggtgtgccg      1320
   ggtgttggtg taccaggtgg cggtgttccg ggtgcaggcg ttccgggtgg cggtgtgccg      1380
   ggcgtgggtg ttccgggcgt gggtgttccg ggtggcggtg tgccgggcgc aggtgttcct      1440
   ggtgtaggtg tgccgggtgt tggtgtgccg ggtgttggtg taccaggtgg cggtgttccg      1500
   ggtgcaggcg ttccgggtgg cggtgtgccg ggcgtgggtg ttccgggcgt gggtgttccg      1560
   ggtggcggtg tgccgggcgc aggtgttcct ggtgtaggtg tgccgggtgt tggtgtgccg      1620
   ggtgttggtg taccaggtgg cggtgttccg ggtgcaggcg ttccgggtgg cggtgtgccg      1680
   ggcgtgggtg ttccgggcgt gggtgttccg ggtggcggtg tgccgggcgc aggtgttcct      1740
   ggtgtaggtg tgccgggtgt tggtgtgccg ggtgttggtg taccaggtgg cggtgttccg      1800
   ggtgcaggcg ttccgggtgg cggtgtgccg ggcgtgggtg ttccgggcgt gggtgttccg      1860
   ggtggcggtg tgccgggcgc aggtgttcct ggtgtaggtg tgccgggtgt tggtgtgccg      1920
   ggtgttggtg taccaggtgg cggtgttccg ggtgcaggcg ttccgggtgg cggtgtgccg 1980
   ggctggccg 1989
<210> 113
   <211> 632
   <212> PRT
   <213> Artificial Sequence
<220>
   <223> CNP22 CatK ELP1-120 fusion PE9456
<400> 113
   Gly Leu Ser Lys Gly Cys Phe Gly Leu Lys Leu Asp Arg Ile Gly Ser
   1               5                   10                  15
   Met Ser Gly Leu Gly Cys Arg Lys Pro Arg Gly Val Pro Gly Val Gly
               20                  25                  30
   Val Pro Gly Val Gly Val Pro Gly Gly Gly Val Pro Gly Ala Gly Val
           35                  40                  45
   Pro Gly Val Gly Val Pro Gly Val Gly Val Pro Gly Val Gly Val Pro
       50                  55                  60
   Gly Gly Gly Val Pro Gly Ala Gly Val Pro Gly Gly Gly Val Pro Gly
   65                  70                  75                  80
   Val Gly Val Pro Gly Val Gly Val Pro Gly Gly Gly Val Pro Gly Ala
                   85                  90                  95
   Gly Val Pro Gly Val Gly Val Pro Gly Val Gly Val Pro Gly Val Gly
               100                 105                 110
   Val Pro Gly Gly Gly Val Pro Gly Ala Gly Val Pro Gly Gly Gly Val
           115                 120                 125
   Pro Gly Val Gly Val Pro Gly Val Gly Val Pro Gly Gly Gly Val Pro
       130                 135                 140
   Gly Ala Gly Val Pro Gly Val Gly Val Pro Gly Val Gly Val Pro Gly
   145                 150                 155                 160
   Val Gly Val Pro Gly Gly Gly Val Pro Gly Ala Gly Val Pro Gly Gly
                   165                 170                 175
   Gly Val Pro Gly Val Gly Val Pro Gly Val Gly Val Pro Gly Gly Gly
               180                 185                 190
   Val Pro Gly Ala Gly Val Pro Gly Val Gly Val Pro Gly Val Gly Val
           195                 200                 205
   Pro Gly Val Gly Val Pro Gly Gly Gly Val Pro Gly Ala Gly Val Pro
       210                 215                 220
   Gly Gly Gly Val Pro Gly Val Gly Val Pro Gly Val Gly Val Pro Gly
   225                 230                 235                 240
   Gly Gly Val Pro Gly Ala Gly Val Pro Gly Val Gly Val Pro Gly Val
                   245                 250                 255
   Gly Val Pro Gly Val Gly Val Pro Gly Gly Gly Val Pro Gly Ala Gly
               260                 265                 270
   Val Pro Gly Gly Gly Val Pro Gly Val Gly Val Pro Gly Val Gly Val
           275                 280                 285
   Pro Gly Gly Gly Val Pro Gly Ala Gly Val Pro Gly Val Gly Val Pro
       290                 295                 300
   Gly Val Gly Val Pro Gly Val Gly Val Pro Gly Gly Gly Val Pro Gly
   305                 310                 315                 320
   Ala Gly Val Pro Gly Gly Gly Val Pro Gly Val Gly Val Pro Gly Val
                   325                 330                 335
   Gly Val Pro Gly Gly Gly Val Pro Gly Ala Gly Val Pro Gly Val Gly
               340                 345                 350
   Val Pro Gly Val Gly Val Pro Gly Val Gly Val Pro Gly Gly Gly Val
           355                 360                 365
   Pro Gly Ala Gly Val Pro Gly Gly Gly Val Pro Gly Val Gly Val Pro
       370                 375                 380
   Gly Val Gly Val Pro Gly Gly Gly Val Pro Gly Ala Gly Val Pro Gly
   385                 390                 395                 400
   Val Gly Val Pro Gly Val Gly Val Pro Gly Val Gly Val Pro Gly Gly
                   405                 410                 415
   Gly Val Pro Gly Ala Gly Val Pro Gly Gly Gly Val Pro Gly Val Gly
               420                 425                 430
   Val Pro Gly Val Gly Val Pro Gly Gly Gly Val Pro Gly Ala Gly Val
           435                 440                 445
   Pro Gly Val Gly Val Pro Gly Val Gly Val Pro Gly Val Gly Val Pro
       450                 455                 460
   Gly Gly Gly Val Pro Gly Ala Gly Val Pro Gly Gly Gly Val Pro Gly
   465                 470                 475                 480
   Val Gly Val Pro Gly Val Gly Val Pro Gly Gly Gly Val Pro Gly Ala
                   485                 490                 495
   Gly Val Pro Gly Val Gly Val Pro Gly Val Gly Val Pro Gly Val Gly
               500                 505                 510
   Val Pro Gly Gly Gly Val Pro Gly Ala Gly Val Pro Gly Gly Gly Val
           515                 520                 525
   Pro Gly Val Gly Val Pro Gly Val Gly Val Pro Gly Gly Gly Val Pro
       530                 535                 540
   Gly Ala Gly Val Pro Gly Val Gly Val Pro Gly Val Gly Val Pro Gly
   545                 550                 555                 560
   Val Gly Val Pro Gly Gly Gly Val Pro Gly Ala Gly Val Pro Gly Gly
                   565                 570                 575
   Gly Val Pro Gly Val Gly Val Pro Gly Val Gly Val Pro Gly Gly Gly
               580                 585                 590
   Val Pro Gly Ala Gly Val Pro Gly Val Gly Val Pro Gly Val Gly Val
           595                 600                 605
   Pro Gly Val Gly Val Pro Gly Gly Gly Val Pro Gly Ala Gly Val Pro
       610                 615                 620
   Gly Gly Gly Val Pro Gly Trp Pro
   625                 630
<210> 114
   <211> 1896
   <212> DNA
   <213> Artificial Sequence
<220>
   <223> CNP22 CatK ELP1-120 fusion PE9456
<400> 114
   ggcctgagca aaggctgctt tggcctgaaa ctggatcgca ttggcagcat gagcggcctg 60
   ggctgccgca aaccgcgcgg tgtaccgggc gtgggtgttc cgggcgtggg tgttccgggt 120
   ggcggtgtgc cgggcgcagg tgttcctggt gtaggtgtgc cgggtgttgg tgtgccgggt 180
   gttggtgtac caggtggcgg tgttccgggt gcaggcgttc cgggtggcgg tgtgccgggc 240
   gtgggtgttc cgggcgtggg tgttccgggt ggcggtgtgc cgggcgcagg tgttcctggt 300
   gtaggtgtgc cgggtgttgg tgtgccgggt gttggtgtac caggtggcgg tgttccgggt 360
   gcaggcgttc cgggtggcgg tgtgccgggc gtgggtgttc cgggcgtggg tgttccgggt 420
   ggcggtgtgc cgggcgcagg tgttcctggt gtaggtgtgc cgggtgttgg tgtgccgggt 480
   gttggtgtac caggtggcgg tgttccgggt gcaggcgttc cgggtggcgg tgtgccgggc 540
   gtgggtgttc cgggcgtggg tgttccgggt ggcggtgtgc cgggcgcagg tgttcctggt 600
   gtaggtgtgc cgggtgttgg tgtgccgggt gttggtgtac caggtggcgg tgttccgggt 660
   gcaggcgttc cgggtggcgg tgtgccgggc gtgggtgttc cgggcgtggg tgttccgggt 720
   ggcggtgtgc cgggcgcagg tgttcctggt gtaggtgtgc cgggtgttgg tgtgccgggt 780
   gttggtgtac caggtggcgg tgttccgggt gcaggcgttc cgggtggcgg tgtgccgggc 840
   gtgggtgttc cgggcgtggg tgttccgggt ggcggtgtgc cgggcgcagg tgttcctggt 900
   gtaggtgtgc cgggtgttgg tgtgccgggt gttggtgtac caggtggcgg tgttccgggt 960
   gcaggcgttc cgggtggcgg tgtgccgggc gtgggtgttc cgggcgtggg tgttccgggt 1020
   ggcggtgtgc cgggcgcagg tgttcctggt gtaggtgtgc cgggtgttgg tgtgccgggt 1080
   gttggtgtac caggtggcgg tgttccgggt gcaggcgttc cgggtggcgg tgtgccgggc 1140
   gtgggtgttc cgggcgtggg tgttccgggt ggcggtgtgc cgggcgcagg tgttcctggt 1200
   gtaggtgtgc cgggtgttgg tgtgccgggt gttggtgtac caggtggcgg tgttccgggt 1260
   gcaggcgttc cgggtggcgg tgtgccgggc gtgggtgttc cgggcgtggg tgttccgggt 1320
   ggcggtgtgc cgggcgcagg tgttcctggt gtaggtgtgc cgggtgttgg tgtgccgggt 1380
   gttggtgtac caggtggcgg tgttccgggt gcaggcgttc cgggtggcgg tgtgccgggc 1440
   gtgggtgttc cgggcgtggg tgttccgggt ggcggtgtgc cgggcgcagg tgttcctggt 1500
   gtaggtgtgc cgggtgttgg tgtgccgggt gttggtgtac caggtggcgg tgttccgggt 1560
   gcaggcgttc cgggtggcgg tgtgccgggc gtgggtgttc cgggcgtggg tgttccgggt 1620
   ggcggtgtgc cgggcgcagg tgttcctggt gtaggtgtgc cgggtgttgg tgtgccgggt 1680
   gttggtgtac caggtggcgg tgttccgggt gcaggcgttc cgggtggcgg tgtgccgggc 1740
   gtgggtgttc cgggcgtggg tgttccgggt ggcggtgtgc cgggcgcagg tgttcctggt 1800
   gtaggtgtgc cgggtgttgg tgtgccgggt gttggtgtac caggtggcgg tgttccgggt 1860
   gcaggcgttc cgggtggcgg tgtgccgggc tggccg 1896
<210> 115
   <211> 648
   <212> PRT
   <213> Artificial Sequence
<220>
   <223> ELP1-120 CatK CNP37 fusion PE9486
<400> 115
   Met Val Pro Gly Val Gly Val Pro Gly Val Gly Val Pro Gly Gly Gly
   1               5                   10                  15
   Val Pro Gly Ala Gly Val Pro Gly Val Gly Val Pro Gly Val Gly Val
               20                  25                  30
   Pro Gly Val Gly Val Pro Gly Gly Gly Val Pro Gly Ala Gly Val Pro
           35                  40                  45
   Gly Gly Gly Val Pro Gly Val Gly Val Pro Gly Val Gly Val Pro Gly
       50                  55                  60
   Gly Gly Val Pro Gly Ala Gly Val Pro Gly Val Gly Val Pro Gly Val
   65                  70                  75                  80
   Gly Val Pro Gly Val Gly Val Pro Gly Gly Gly Val Pro Gly Ala Gly
                   85                  90                  95
   Val Pro Gly Gly Gly Val Pro Gly Val Gly Val Pro Gly Val Gly Val
               100                 105                 110
   Pro Gly Gly Gly Val Pro Gly Ala Gly Val Pro Gly Val Gly Val Pro
           115                 120                 125
   Gly Val Gly Val Pro Gly Val Gly Val Pro Gly Gly Gly Val Pro Gly
       130                 135                 140
   Ala Gly Val Pro Gly Gly Gly Val Pro Gly Val Gly Val Pro Gly Val
   145                 150                 155                 160
   Gly Val Pro Gly Gly Gly Val Pro Gly Ala Gly Val Pro Gly Val Gly
                   165                 170                 175
   Val Pro Gly Val Gly Val Pro Gly Val Gly Val Pro Gly Gly Gly Val
               180                 185                 190
   Pro Gly Ala Gly Val Pro Gly Gly Gly Val Pro Gly Val Gly Val Pro
           195                 200                 205
   Gly Val Gly Val Pro Gly Gly Gly Val Pro Gly Ala Gly Val Pro Gly
       210                 215                 220
   Val Gly Val Pro Gly Val Gly Val Pro Gly Val Gly Val Pro Gly Gly
   225 230 235 240
   Gly Val Pro Gly Ala Gly Val Pro Gly Gly Gly Val Pro Gly Val Gly
   245 250 255
   Val Pro Gly Val Gly Val Pro Gly Gly Gly Val Pro Gly Ala Gly Val
   260 265 270
   Pro Gly Val Gly Val Pro Gly Val Gly Val Pro Gly Val Gly Val Pro
   275 280 285
   Gly Gly Gly Val Pro Gly Ala Gly Val Pro Gly Gly Gly Val Pro Gly
   290 295 300
   Val Gly Val Pro Gly Val Gly Val Pro Gly Gly Gly Val Pro Gly Ala
   305 310 315 320
   Gly Val Pro Gly Val Gly Val Pro Gly Val Gly Val Pro Gly Val Gly
   325 330 335
   Val Pro Gly Gly Gly Val Pro Gly Ala Gly Val Pro Gly Gly Gly Val
   340 345 350
   Pro Gly Val Gly Val Pro Gly Val Gly Val Pro Gly Gly Gly Val Pro
   355 360 365
   Gly Ala Gly Val Pro Gly Val Gly Val Pro Gly Val Gly Val Pro Gly
   370 375 380
   Val Gly Val Pro Gly Gly Gly Val Pro Gly Ala Gly Val Pro Gly Gly
   385 390 395 400
   Gly Val Pro Gly Val Gly Val Pro Gly Val Gly Val Pro Gly Gly Gly
   405 410 415
   Val Pro Gly Ala Gly Val Pro Gly Val Gly Val Pro Gly Val Gly Val
   420 425 430
   Pro Gly Val Gly Val Pro Gly Gly Gly Val Pro Gly Ala Gly Val Pro
   435 440 445
   Gly Gly Gly Val Pro Gly Val Gly Val Pro Gly Val Gly Val Pro Gly
   450 455 460
   Gly Gly Val Pro Gly Ala Gly Val Pro Gly Val Gly Val Pro Gly Val
   465 470 475 480
   Gly Val Pro Gly Val Gly Val Pro Gly Gly Gly Val Pro Gly Ala Gly
   485 490 495
   Val Pro Gly Gly Gly Val Pro Gly Val Gly Val Pro Gly Val Gly Val
   500 505 510
   Pro Gly Gly Gly Val Pro Gly Ala Gly Val Pro Gly Val Gly Val Pro
   515 520 525
   Gly Val Gly Val Pro Gly Val Gly Val Pro Gly Gly Gly Val Pro Gly
   530 535 540
   Ala Gly Val Pro Gly Gly Gly Val Pro Gly Val Gly Val Pro Gly Val
   545 550 555 560
   Gly Val Pro Gly Gly Gly Val Pro Gly Ala Gly Val Pro Gly Val Gly
   565 570 575
   Val Pro Gly Val Gly Val Pro Gly Val Gly Val Pro Gly Gly Gly Val
   580 585 590
   Pro Gly Ala Gly Val Pro Gly Gly Gly Val Pro Gly Trp Pro Arg Lys
   595 600 605
   Pro Arg Gly Gln Glu His Pro Asn Ala Arg Lys Tyr Lys Gly Ala Asn
   610 615 620
   Lys Lys Gly Leu Ser Lys Gly Cys Phe Gly Leu Lys Leu Asp Arg Ile
   625 630 635 640
   Gly Ser Met Ser Gly Leu Gly Cys
   645
<210> 116
   <211> 1945
   <212> DNA
   <213> Artificial Sequence
<220>
   <223> ELP1-120 CatK CNP37 fusion PE9486
<400> 116
   atggtaccgg gcgtgggtgt tccgggcgtg ggtgttccgg gtggcggtgt gccgggcgca        60
   ggtgttcctg gtgtaggtgt gccgggtgtt ggtgtgccgg gtgttggtgt accaggtggc       120
   ggtgttccgg gtgcaggcgt tccgggtggc ggtgtgccgg gcgtgggtgt tccgggcgtg       180
   ggtgttccgg gtggcggtgt gccgggcgca ggtgttcctg gtgtaggtgt gccgggtgtt       240
   ggtgtgccgg gtgttggtgt accaggtggc ggtgttccgg gtgcaggcgt tccgggtggc       300
   ggtgtgccgg gcgtgggtgt tccgggcgtg ggtgttccgg gtggcggtgt gccgggcgca 360
   ggtgttcctg gtgtaggtgt gccgggtgtt ggtgtgccgg gtgttggtgt accaggtggc 420
   ggtgttccgg gtgcaggcgt tccgggtggc ggtgtgccgg gcgtgggtgt tccgggcgtg 480
   ggtgttccgg gtggcggtgt gccgggcgca ggtgttcctg gtgtaggtgt gccgggtgtt 540
   ggtgtgccgg gtgttggtgt accaggtggc ggtgttccgg gtgcaggcgt tccgggtggc 600
   ggtgtgccgg gcgtgggtgt tccgggcgtg ggtgttccgg gtggcggtgt gccgggcgca 660
   ggtgttcctg gtgtaggtgt gccgggtgtt ggtgtgccgg gtgttggtgt accaggtggc 720
   ggtgttccgg gtgcaggcgt tccgggtggc ggtgtgccgg gcgtgggtgt tccgggcgtg 780
   ggtgttccgg gtggcggtgt gccgggcgca ggtgttcctg gtgtaggtgt gccgggtgtt 840
   ggtgtgccgg gtgttggtgt accaggtggc ggtgttccgg gtgcaggcgt tccgggtggc 900
   ggtgtgccgg gcgtgggtgt tccgggcgtg ggtgttccgg gtggcggtgt gccgggcgca 960
   ggtgttcctg gtgtaggtgt gccgggtgtt ggtgtgccgg gtgttggtgt accaggtggc 1020
   ggtgttccgg gtgcaggcgt tccgggtggc ggtgtgccgg gcgtgggtgt tccgggcgtg 1080
   ggtgttccgg gtggcggtgt gccgggcgca ggtgttcctg gtgtaggtgt gccgggtgtt 1140
   ggtgtgccgg gtgttggtgt accaggtggc ggtgttccgg gtgcaggcgt tccgggtggc 1200
   ggtgtgccgg gcgtgggtgt tccgggcgtg ggtgttccgg gtggcggtgt gccgggcgca 1260
   ggtgttcctg gtgtaggtgt gccgggtgtt ggtgtgccgg gtgttggtgt accaggtggc 1320
   ggtgttccgg gtgcaggcgt tccgggtggc ggtgtgccgg gcgtgggtgt tccgggcgtg 1380
   ggtgttccgg gtggcggtgt gccgggcgca ggtgttcctg gtgtaggtgt gccgggtgtt 1440
   ggtgtgccgg gtgttggtgt accaggtggc ggtgttccgg gtgcaggcgt tccgggtggc 1500
   ggtgtgccgg gcgtgggtgt tccgggcgtg ggtgttccgg gtggcggtgt gccgggcgca 1560
   ggtgttcctg gtgtaggtgt gccgggtgtt ggtgtgccgg gtgttggtgt accaggtggc 1620
   ggtgttccgg gtgcaggcgt tccgggtggc ggtgtgccgg gcgtgggtgt tccgggcgtg 1680
   ggtgttccgg gtggcggtgt gccgggcgca ggtgttcctg gtgtaggtgt gccgggtgtt 1740
   ggtgtgccgg gtgttggtgt accaggtggc ggtgttccgg gtgcaggcgt tccgggtggc 1800
   ggtgtgccgg gctggccgcc gcaaaccgcg cggtcaggaa catccgaacg cgcgtaaata 1860
   taaaggtgcg aacaaaaaag gcctgagcaa aggctgcttt ggcctgaaac tggatcgcat 1920
   tggcagcatg agcggcctgg gctgc 1945
<210> 117
   <211> 664
   <212> PRT
   <213> Artificial Sequence
<220>
   <223> ELP1-120 CatK CNP53 fusion PE9496
<400> 117
   Met Val Pro Gly Val Gly Val Pro Gly Val Gly Val Pro Gly Gly Gly
   1               5                   10                  15
   Val Pro Gly Ala Gly Val Pro Gly Val Gly Val Pro Gly Val Gly Val
               20                  25                  30
   Pro Gly Val Gly Val Pro Gly Gly Gly Val Pro Gly Ala Gly Val Pro
           35                  40                  45
   Gly Gly Gly Val Pro Gly Val Gly Val Pro Gly Val Gly Val Pro Gly
       50                  55                  60
   Gly Gly Val Pro Gly Ala Gly Val Pro Gly Val Gly Val Pro Gly Val
   65                  70                  75                  80
   Gly Val Pro Gly Val Gly Val Pro Gly Gly Gly Val Pro Gly Ala Gly
                   85                  90                  95
   Val Pro Gly Gly Gly Val Pro Gly Val Gly Val Pro Gly Val Gly Val
               100                 105                 110
   Pro Gly Gly Gly Val Pro Gly Ala Gly Val Pro Gly Val Gly Val Pro
           115                 120                 125
   Gly Val Gly Val Pro Gly Val Gly Val Pro Gly Gly Gly Val Pro Gly
       130                 135                 140
   Ala Gly Val Pro Gly Gly Gly Val Pro Gly Val Gly Val Pro Gly Val
   145                 150                 155                 160
   Gly Val Pro Gly Gly Gly Val Pro Gly Ala Gly Val Pro Gly Val Gly
                   165                 170                 175
   Val Pro Gly Val Gly Val Pro Gly Val Gly Val Pro Gly Gly Gly Val
               180                 185                 190
   Pro Gly Ala Gly Val Pro Gly Gly Gly Val Pro Gly Val Gly Val Pro
           195                 200                 205
   Gly Val Gly Val Pro Gly Gly Gly Val Pro Gly Ala Gly Val Pro Gly
       210                 215                 220
   Val Gly Val Pro Gly Val Gly Val Pro Gly Val Gly Val Pro Gly Gly
   225                 230                 235                 240
   Gly Val Pro Gly Ala Gly Val Pro Gly Gly Gly Val Pro Gly Val Gly
                   245                 250                 255
   Val Pro Gly Val Gly Val Pro Gly Gly Gly Val Pro Gly Ala Gly Val
               260                 265                 270
   Pro Gly Val Gly Val Pro Gly Val Gly Val Pro Gly Val Gly Val Pro
           275                 280                 285
   Gly Gly Gly Val Pro Gly Ala Gly Val Pro Gly Gly Gly Val Pro Gly
       290                 295                 300
   Val Gly Val Pro Gly Val Gly Val Pro Gly Gly Gly Val Pro Gly Ala
   305                 310                 315                 320
   Gly Val Pro Gly Val Gly Val Pro Gly Val Gly Val Pro Gly Val Gly
                   325                 330                 335
   Val Pro Gly Gly Gly Val Pro Gly Ala Gly Val Pro Gly Gly Gly Val
               340                 345                 350
   Pro Gly Val Gly Val Pro Gly Val Gly Val Pro Gly Gly Gly Val Pro
           355                 360                 365
   Gly Ala Gly Val Pro Gly Val Gly Val Pro Gly Val Gly Val Pro Gly
       370                 375                 380
   Val Gly Val Pro Gly Gly Gly Val Pro Gly Ala Gly Val Pro Gly Gly
   385                 390                 395                 400
   Gly Val Pro Gly Val Gly Val Pro Gly Val Gly Val Pro Gly Gly Gly
                   405                 410                 415
   Val Pro Gly Ala Gly Val Pro Gly Val Gly Val Pro Gly Val Gly Val
               420                 425                 430
   Pro Gly Val Gly Val Pro Gly Gly Gly Val Pro Gly Ala Gly Val Pro
           435                 440                 445
   Gly Gly Gly Val Pro Gly Val Gly Val Pro Gly Val Gly Val Pro Gly
       450                 455                 460
   Gly Gly Val Pro Gly Ala Gly Val Pro Gly Val Gly Val Pro Gly Val
   465                 470                 475                 480
   Gly Val Pro Gly Val Gly Val Pro Gly Gly Gly Val Pro Gly Ala Gly
   485 490 495
   Val Pro Gly Gly Gly Val Pro Gly Val Gly Val Pro Gly Val Gly Val
   500 505 510
   Pro Gly Gly Gly Val Pro Gly Ala Gly Val Pro Gly Val Gly Val Pro
   515 520 525
   Gly Val Gly Val Pro Gly Val Gly Val Pro Gly Gly Gly Val Pro Gly
   530 535 540
   Ala Gly Val Pro Gly Gly Gly Val Pro Gly Val Gly Val Pro Gly Val
   545 550 555 560
   Gly Val Pro Gly Gly Gly Val Pro Gly Ala Gly Val Pro Gly Val Gly
   565 570 575
   Val Pro Gly Val Gly Val Pro Gly Val Gly Val Pro Gly Gly Gly Val
   580 585 590
   Pro Gly Ala Gly Val Pro Gly Gly Gly Val Pro Gly Trp Pro Arg Lys
   595 600 605
   Pro Arg Gly Asp Leu Arg Val Asp Thr Lys Ser Arg Ala Ala Trp Ala
   610 615 620
   Arg Leu Leu Gln Glu His Pro Asn Ala Arg Lys Tyr Lys Gly Ala Asn
   625 630 635 640
   Lys Lys Gly Leu Ser Lys Gly Cys Phe Gly Leu Lys Leu Asp Arg Ile
   645 650 655
   Gly Ser Met Ser Gly Leu Gly Cys
   660
<210> 118
   <211> 1993
   <212> DNA
   <213> Artificial Sequence
<220>
   <223> ELP1-120 CatK CNP53 fusion PE9496
<400> 118
   atggtaccgg gcgtgggtgt tccgggcgtg ggtgttccgg gtggcggtgt gccgggcgca        60
   ggtgttcctg gtgtaggtgt gccgggtgtt ggtgtgccgg gtgttggtgt accaggtggc       120
   ggtgttccgg gtgcaggcgt tccgggtggc ggtgtgccgg gcgtgggtgt tccgggcgtg       180
   ggtgttccgg gtggcggtgt gccgggcgca ggtgttcctg gtgtaggtgt gccgggtgtt       240
   ggtgtgccgg gtgttggtgt accaggtggc ggtgttccgg gtgcaggcgt tccgggtggc 300
   ggtgtgccgg gcgtgggtgt tccgggcgtg ggtgttccgg gtggcggtgt gccgggcgca 360
   ggtgttcctg gtgtaggtgt gccgggtgtt ggtgtgccgg gtgttggtgt accaggtggc 420
   ggtgttccgg gtgcaggcgt tccgggtggc ggtgtgccgg gcgtgggtgt tccgggcgtg 480
   ggtgttccgg gtggcggtgt gccgggcgca ggtgttcctg gtgtaggtgt gccgggtgtt 540
   ggtgtgccgg gtgttggtgt accaggtggc ggtgttccgg gtgcaggcgt tccgggtggc 600
   ggtgtgccgg gcgtgggtgt tccgggcgtg ggtgttccgg gtggcggtgt gccgggcgca 660
   ggtgttcctg gtgtaggtgt gccgggtgtt ggtgtgccgg gtgttggtgt accaggtggc 720
   ggtgttccgg gtgcaggcgt tccgggtggc ggtgtgccgg gcgtgggtgt tccgggcgtg 780
   ggtgttccgg gtggcggtgt gccgggcgca ggtgttcctg gtgtaggtgt gccgggtgtt 840
   ggtgtgccgg gtgttggtgt accaggtggc ggtgttccgg gtgcaggcgt tccgggtggc 900
   ggtgtgccgg gcgtgggtgt tccgggcgtg ggtgttccgg gtggcggtgt gccgggcgca 960
   ggtgttcctg gtgtaggtgt gccgggtgtt ggtgtgccgg gtgttggtgt accaggtggc 1020
   ggtgttccgg gtgcaggcgt tccgggtggc ggtgtgccgg gcgtgggtgt tccgggcgtg 1080
   ggtgttccgg gtggcggtgt gccgggcgca ggtgttcctg gtgtaggtgt gccgggtgtt 1140
   ggtgtgccgg gtgttggtgt accaggtggc ggtgttccgg gtgcaggcgt tccgggtggc 1200
   ggtgtgccgg gcgtgggtgt tccgggcgtg ggtgttccgg gtggcggtgt gccgggcgca 1260
   ggtgttcctg gtgtaggtgt gccgggtgtt ggtgtgccgg gtgttggtgt accaggtggc 1320
   ggtgttccgg gtgcaggcgt tccgggtggc ggtgtgccgg gcgtgggtgt tccgggcgtg 1380
   ggtgttccgg gtggcggtgt gccgggcgca ggtgttcctg gtgtaggtgt gccgggtgtt 1440
   ggtgtgccgg gtgttggtgt accaggtggc ggtgttccgg gtgcaggcgt tccgggtggc 1500
   ggtgtgccgg gcgtgggtgt tccgggcgtg ggtgttccgg gtggcggtgt gccgggcgca 1560
   ggtgttcctg gtgtaggtgt gccgggtgtt ggtgtgccgg gtgttggtgt accaggtggc 1620
   ggtgttccgg gtgcaggcgt tccgggtggc ggtgtgccgg gcgtgggtgt tccgggcgtg 1680
   ggtgttccgg gtggcggtgt gccgggcgca ggtgttcctg gtgtaggtgt gccgggtgtt 1740
   ggtgtgccgg gtgttggtgt accaggtggc ggtgttccgg gtgcaggcgt tccgggtggc 1800
   ggtgtgccgg gctggccgcc gcaaaccgcg cggtgatctg cgcgtggata ccaaaagccg 1860
   cgcggcgtgg gcgcgcctgc tgcaggaaca tccgaacgcg cgcaaatata aaggcgcgaa 1920
   caaaaaaggc ctgagcaaag gctgctttgg cctgaaactg gatcgcattg gcagcatgag 1980
   cggcctgggc tgc 1993
<210> 119
   <211> 633
   <212> PRT
   <213> Artificial Sequence
<220>
   <223> ELP1-120 CatK CNP22 fusion PE9506
<400> 119
   Met Val Pro Gly Val Gly Val Pro Gly Val Gly Val Pro Gly Gly Gly
   1               5                   10                  15
   Val Pro Gly Ala Gly Val Pro Gly Val Gly Val Pro Gly Val Gly Val
               20                  25                  30
   Pro Gly Val Gly Val Pro Gly Gly Gly Val Pro Gly Ala Gly Val Pro
           35                  40                  45
   Gly Gly Gly Val Pro Gly Val Gly Val Pro Gly Val Gly Val Pro Gly
       50                  55                  60
   Gly Gly Val Pro Gly Ala Gly Val Pro Gly Val Gly Val Pro Gly Val
   65                  70                  75                  80
   Gly Val Pro Gly Val Gly Val Pro Gly Gly Gly Val Pro Gly Ala Gly
                   85                  90                  95
   Val Pro Gly Gly Gly Val Pro Gly Val Gly Val Pro Gly Val Gly Val
               100                 105                 110
   Pro Gly Gly Gly Val Pro Gly Ala Gly Val Pro Gly Val Gly Val Pro
           115                 120                 125
   Gly Val Gly Val Pro Gly Val Gly Val Pro Gly Gly Gly Val Pro Gly
       130                 135                 140
   Ala Gly Val Pro Gly Gly Gly Val Pro Gly Val Gly Val Pro Gly Val
   145                 150                 155                 160
   Gly Val Pro Gly Gly Gly Val Pro Gly Ala Gly Val Pro Gly Val Gly
                   165                 170                 175
   Val Pro Gly Val Gly Val Pro Gly Val Gly Val Pro Gly Gly Gly Val
               180                 185                 190
   Pro Gly Ala Gly Val Pro Gly Gly Gly Val Pro Gly Val Gly Val Pro
           195                 200                 205
   Gly Val Gly Val Pro Gly Gly Gly Val Pro Gly Ala Gly Val Pro Gly
       210                 215                 220
   Val Gly Val Pro Gly Val Gly Val Pro Gly Val Gly Val Pro Gly Gly
   225 230 235 240
   Gly Val Pro Gly Ala Gly Val Pro Gly Gly Gly Val Pro Gly Val Gly
   245 250 255
   Val Pro Gly Val Gly Val Pro Gly Gly Gly Val Pro Gly Ala Gly Val
   260 265 270
   Pro Gly Val Gly Val Pro Gly Val Gly Val Pro Gly Val Gly Val Pro
   275 280 285
   Gly Gly Gly Val Pro Gly Ala Gly Val Pro Gly Gly Gly Val Pro Gly
   290 295 300
   Val Gly Val Pro Gly Val Gly Val Pro Gly Gly Gly Val Pro Gly Ala
   305 310 315 320
   Gly Val Pro Gly Val Gly Val Pro Gly Val Gly Val Pro Gly Val Gly
   325 330 335
   Val Pro Gly Gly Gly Val Pro Gly Ala Gly Val Pro Gly Gly Gly Val
   340 345 350
   Pro Gly Val Gly Val Pro Gly Val Gly Val Pro Gly Gly Gly Val Pro
   355 360 365
   Gly Ala Gly Val Pro Gly Val Gly Val Pro Gly Val Gly Val Pro Gly
   370 375 380
   Val Gly Val Pro Gly Gly Gly Val Pro Gly Ala Gly Val Pro Gly Gly
   385 390 395 400
   Gly Val Pro Gly Val Gly Val Pro Gly Val Gly Val Pro Gly Gly Gly
   405 410 415
   Val Pro Gly Ala Gly Val Pro Gly Val Gly Val Pro Gly Val Gly Val
   420 425 430
   Pro Gly Val Gly Val Pro Gly Gly Gly Val Pro Gly Ala Gly Val Pro
   435 440 445
   Gly Gly Gly Val Pro Gly Val Gly Val Pro Gly Val Gly Val Pro Gly
   450 455 460
   Gly Gly Val Pro Gly Ala Gly Val Pro Gly Val Gly Val Pro Gly Val
   465 470 475 480
   Gly Val Pro Gly Val Gly Val Pro Gly Gly Gly Val Pro Gly Ala Gly
   485 490 495
   Val Pro Gly Gly Gly Val Pro Gly Val Gly Val Pro Gly Val Gly Val
   500 505 510
   Pro Gly Gly Gly Val Pro Gly Ala Gly Val Pro Gly Val Gly Val Pro
   515 520 525
   Gly Val Gly Val Pro Gly Val Gly Val Pro Gly Gly Gly Val Pro Gly
   530 535 540
   Ala Gly Val Pro Gly Gly Gly Val Pro Gly Val Gly Val Pro Gly Val
   545 550 555 560
   Gly Val Pro Gly Gly Gly Val Pro Gly Ala Gly Val Pro Gly Val Gly
   565 570 575
   Val Pro Gly Val Gly Val Pro Gly Val Gly Val Pro Gly Gly Gly Val
   580 585 590
   Pro Gly Ala Gly Val Pro Gly Gly Gly Val Pro Gly Trp Pro Arg Lys
   595 600 605
   Pro Arg Gly Gly Leu Ser Lys Gly Cys Phe Gly Leu Lys Leu Asp Arg
   610 615 620
   Ile Gly Ser Met Ser Gly Leu Gly Cys
   625 630
<210> 120
   <211> 1900
   <212> DNA
   <213> Artificial Sequence
<220>
   <223> ELP1-120 CatK CNP22 fusion PE9506
<400> 120
   atggtaccgg gcgtgggtgt tccgggcgtg ggtgttccgg gtggcggtgt gccgggcgca      60
   ggtgttcctg gtgtaggtgt gccgggtgtt ggtgtgccgg gtgttggtgt accaggtggc     120
   ggtgttccgg gtgcaggcgt tccgggtggc ggtgtgccgg gcgtgggtgt tccgggcgtg     180
   ggtgttccgg gtggcggtgt gccgggcgca ggtgttcctg gtgtaggtgt gccgggtgtt     240
   ggtgtgccgg gtgttggtgt accaggtggc ggtgttccgg gtgcaggcgt tccgggtggc     300
   ggtgtgccgg gcgtgggtgt tccgggcgtg ggtgttccgg gtggcggtgt gccgggcgca     360
   ggtgttcctg gtgtaggtgt gccgggtgtt ggtgtgccgg gtgttggtgt accaggtggc 420
   ggtgttccgg gtgcaggcgt tccgggtggc ggtgtgccgg gcgtgggtgt tccgggcgtg 480
   ggtgttccgg gtggcggtgt gccgggcgca ggtgttcctg gtgtaggtgt gccgggtgtt 540
   ggtgtgccgg gtgttggtgt accaggtggc ggtgttccgg gtgcaggcgt tccgggtggc 600
   ggtgtgccgg gcgtgggtgt tccgggcgtg ggtgttccgg gtggcggtgt gccgggcgca 660
   ggtgttcctg gtgtaggtgt gccgggtgtt ggtgtgccgg gtgttggtgt accaggtggc 720
   ggtgttccgg gtgcaggcgt tccgggtggc ggtgtgccgg gcgtgggtgt tccgggcgtg 780
   ggtgttccgg gtggcggtgt gccgggcgca ggtgttcctg gtgtaggtgt gccgggtgtt 840
   ggtgtgccgg gtgttggtgt accaggtggc ggtgttccgg gtgcaggcgt tccgggtggc 900
   ggtgtgccgg gcgtgggtgt tccgggcgtg ggtgttccgg gtggcggtgt gccgggcgca 960
   ggtgttcctg gtgtaggtgt gccgggtgtt ggtgtgccgg gtgttggtgt accaggtggc 1020
   ggtgttccgg gtgcaggcgt tccgggtggc ggtgtgccgg gcgtgggtgt tccgggcgtg 1080
   ggtgttccgg gtggcggtgt gccgggcgca ggtgttcctg gtgtaggtgt gccgggtgtt 1140
   ggtgtgccgg gtgttggtgt accaggtggc ggtgttccgg gtgcaggcgt tccgggtggc 1200
   ggtgtgccgg gcgtgggtgt tccgggcgtg ggtgttccgg gtggcggtgt gccgggcgca 1260
   ggtgttcctg gtgtaggtgt gccgggtgtt ggtgtgccgg gtgttggtgt accaggtggc 1320
   ggtgttccgg gtgcaggcgt tccgggtggc ggtgtgccgg gcgtgggtgt tccgggcgtg 1380
   ggtgttccgg gtggcggtgt gccgggcgca ggtgttcctg gtgtaggtgt gccgggtgtt 1440
   ggtgtgccgg gtgttggtgt accaggtggc ggtgttccgg gtgcaggcgt tccgggtggc 1500
   ggtgtgccgg gcgtgggtgt tccgggcgtg ggtgttccgg gtggcggtgt gccgggcgca 1560
   ggtgttcctg gtgtaggtgt gccgggtgtt ggtgtgccgg gtgttggtgt accaggtggc 1620
   ggtgttccgg gtgcaggcgt tccgggtggc ggtgtgccgg gcgtgggtgt tccgggcgtg 1680
   ggtgttccgg gtggcggtgt gccgggcgca ggtgttcctg gtgtaggtgt gccgggtgtt 1740
   ggtgtgccgg gtgttggtgt accaggtggc ggtgttccgg gtgcaggcgt tccgggtggc 1800
   ggtgtgccgg gctggccgcc gcaaaccgcg cggtggcctg agcaaaggct gctttggcct 1860
   gaaactggat cgcattggca gcatgagcgg cctgggctgc 1900
<210> 121
   <211> 647
   <212> PRT
   <213> Artificial Sequence
<220>
   <223> CNP37 Fxa ELP1-120 fusion PE9206
<400> 121
   Gln Glu His Pro Asn Ala Arg Lys Tyr Lys Gly Ala Asn Lys Lys Gly
   1               5                   10                  15
   Leu Ser Lys Gly Cys Phe Gly Leu Lys Leu Asp Arg Ile Gly Ser Met
               20                  25                  30
   Ser Gly Leu Gly Cys Ile Glu Gly Arg Gly Val Pro Gly Val Gly Val
           35                  40                  45
   Pro Gly Val Gly Val Pro Gly Gly Gly Val Pro Gly Ala Gly Val Pro
       50                  55                  60
   Gly Val Gly Val Pro Gly Val Gly Val Pro Gly Val Gly Val Pro Gly
   65                  70                  75                  80
   Gly Gly Val Pro Gly Ala Gly Val Pro Gly Gly Gly Val Pro Gly Val
                   85                  90                  95
   Gly Val Pro Gly Val Gly Val Pro Gly Gly Gly Val Pro Gly Ala Gly
               100                 105                 110
   Val Pro Gly Val Gly Val Pro Gly Val Gly Val Pro Gly Val Gly Val
           115                 120                 125
   Pro Gly Gly Gly Val Pro Gly Ala Gly Val Pro Gly Gly Gly Val Pro
       130                 135                 140
   Gly Val Gly Val Pro Gly Val Gly Val Pro Gly Gly Gly Val Pro Gly
   145                 150                 155                 160
   Ala Gly Val Pro Gly Val Gly Val Pro Gly Val Gly Val Pro Gly Val
                   165                 170                 175
   Gly Val Pro Gly Gly Gly Val Pro Gly Ala Gly Val Pro Gly Gly Gly
               180                 185                 190
   Val Pro Gly Val Gly Val Pro Gly Val Gly Val Pro Gly Gly Gly Val
           195                 200                 205
   Pro Gly Ala Gly Val Pro Gly Val Gly Val Pro Gly Val Gly Val Pro
       210                 215                 220
   Gly Val Gly Val Pro Gly Gly Gly Val Pro Gly Ala Gly Val Pro Gly
   225                 230                 235                 240
   Gly Gly Val Pro Gly Val Gly Val Pro Gly Val Gly Val Pro Gly Gly
                   245                 250                 255
   Gly Val Pro Gly Ala Gly Val Pro Gly Val Gly Val Pro Gly Val Gly
   260 265 270
   Val Pro Gly Val Gly Val Pro Gly Gly Gly Val Pro Gly Ala Gly Val
   275 280 285
   Pro Gly Gly Gly Val Pro Gly Val Gly Val Pro Gly Val Gly Val Pro
   290 295 300
   Gly Gly Gly Val Pro Gly Ala Gly Val Pro Gly Val Gly Val Pro Gly
   305 310 315 320
   Val Gly Val Pro Gly Val Gly Val Pro Gly Gly Gly Val Pro Gly Ala
   325 330 335
   Gly Val Pro Gly Gly Gly Val Pro Gly Val Gly Val Pro Gly Val Gly
   340 345 350
   Val Pro Gly Gly Gly Val Pro Gly Ala Gly Val Pro Gly Val Gly Val
   355 360 365
   Pro Gly Val Gly Val Pro Gly Val Gly Val Pro Gly Gly Gly Val Pro
   370 375 380
   Gly Ala Gly Val Pro Gly Gly Gly Val Pro Gly Val Gly Val Pro Gly
   385 390 395 400
   Val Gly Val Pro Gly Gly Gly Val Pro Gly Ala Gly Val Pro Gly Val
   405 410 415
   Gly Val Pro Gly Val Gly Val Pro Gly Val Gly Val Pro Gly Gly Gly
   420 425 430
   Val Pro Gly Ala Gly Val Pro Gly Gly Gly Val Pro Gly Val Gly Val
   435 440 445
   Pro Gly Val Gly Val Pro Gly Gly Gly Val Pro Gly Ala Gly Val Pro
   450 455 460
   Gly Val Gly Val Pro Gly Val Gly Val Pro Gly Val Gly Val Pro Gly
   465 470 475 480
   Gly Gly Val Pro Gly Ala Gly Val Pro Gly Gly Gly Val Pro Gly Val
   485 490 495
   Gly Val Pro Gly Val Gly Val Pro Gly Gly Gly Val Pro Gly Ala Gly
                   500                 505                 510
   Val Pro Gly Val Gly Val Pro Gly Val Gly Val Pro Gly Val Gly Val
           515                 520                 525
   Pro Gly Gly Gly Val Pro Gly Ala Gly Val Pro Gly Gly Gly Val Pro
       530                 535                 540
   Gly Val Gly Val Pro Gly Val Gly Val Pro Gly Gly Gly Val Pro Gly
   545                 550                 555                 560
   Ala Gly Val Pro Gly Val Gly Val Pro Gly Val Gly Val Pro Gly Val
                   565                 570                 575
   Gly Val Pro Gly Gly Gly Val Pro Gly Ala Gly Val Pro Gly Gly Gly
               580                 585                 590
   Val Pro Gly Val Gly Val Pro Gly Val Gly Val Pro Gly Gly Gly Val
           595                 600                 605
   Pro Gly Ala Gly Val Pro Gly Val Gly Val Pro Gly Val Gly Val Pro
       610                 615                 620
   Gly Val Gly Val Pro Gly Gly Gly Val Pro Gly Ala Gly Val Pro Gly
   625                 630                 635                 640
   Gly Gly Val Pro Gly Trp Pro
                   645
<210> 122
   <211> 1941
   <212> DNA
   <213> Artificial Sequence
<220>
   <223> CNP37 Fxa ELP1-120 fusion PE9206
<400> 122
   caggaacatc cgaacgcgcg taaatataaa ggtgcgaaca aaaaaggcct gagcaaaggc      60
   tgctttggcc tgaaactgga tcgcattggc agcatgagcg gcctgggctg cattgaaggc     120
   cgcggtgtac cgggcgtggg tgttccgggc gtgggtgttc cgggtggcgg tgtgccgggc     180
   gcaggtgttc ctggtgtagg tgtgccgggt gttggtgtgc cgggtgttgg tgtaccaggt     240
   ggcggtgttc cgggtgcagg cgttccgggt ggcggtgtgc cgggcgtggg tgttccgggc     300
   gtgggtgttc cgggtggcgg tgtgccgggc gcaggtgttc ctggtgtagg tgtgccgggt     360
   gttggtgtgc cgggtgttgg tgtaccaggt ggcggtgttc cgggtgcagg cgttccgggt     420
   ggcggtgtgc cgggcgtggg tgttccgggc gtgggtgttc cgggtggcgg tgtgccgggc     480
   gcaggtgttc ctggtgtagg tgtgccgggt gttggtgtgc cgggtgttgg tgtaccaggt 540
   ggcggtgttc cgggtgcagg cgttccgggt ggcggtgtgc cgggcgtggg tgttccgggc 600
   gtgggtgttc cgggtggcgg tgtgccgggc gcaggtgttc ctggtgtagg tgtgccgggt 660
   gttggtgtgc cgggtgttgg tgtaccaggt ggcggtgttc cgggtgcagg cgttccgggt 720
   ggcggtgtgc cgggcgtggg tgttccgggc gtgggtgttc cgggtggcgg tgtgccgggc 780
   gcaggtgttc ctggtgtagg tgtgccgggt gttggtgtgc cgggtgttgg tgtaccaggt 840
   ggcggtgttc cgggtgcagg cgttccgggt ggcggtgtgc cgggcgtggg tgttccgggc 900
   gtgggtgttc cgggtggcgg tgtgccgggc gcaggtgttc ctggtgtagg tgtgccgggt 960
   gttggtgtgc cgggtgttgg tgtaccaggt ggcggtgttc cgggtgcagg cgttccgggt 1020
   ggcggtgtgc cgggcgtggg tgttccgggc gtgggtgttc cgggtggcgg tgtgccgggc 1080
   gcaggtgttc ctggtgtagg tgtgccgggt gttggtgtgc cgggtgttgg tgtaccaggt 1140
   ggcggtgttc cgggtgcagg cgttccgggt ggcggtgtgc cgggcgtggg tgttccgggc 1200
   gtgggtgttc cgggtggcgg tgtgccgggc gcaggtgttc ctggtgtagg tgtgccgggt 1260
   gttggtgtgc cgggtgttgg tgtaccaggt ggcggtgttc cgggtgcagg cgttccgggt 1320
   ggcggtgtgc cgggcgtggg tgttccgggc gtgggtgttc cgggtggcgg tgtgccgggc 1380
   gcaggtgttc ctggtgtagg tgtgccgggt gttggtgtgc cgggtgttgg tgtaccaggt 1440
   ggcggtgttc cgggtgcagg cgttccgggt ggcggtgtgc cgggcgtggg tgttccgggc 1500
   gtgggtgttc cgggtggcgg tgtgccgggc gcaggtgttc ctggtgtagg tgtgccgggt 1560
   gttggtgtgc cgggtgttgg tgtaccaggt ggcggtgttc cgggtgcagg cgttccgggt 1620
   ggcggtgtgc cgggcgtggg tgttccgggc gtgggtgttc cgggtggcgg tgtgccgggc 1680
   gcaggtgttc ctggtgtagg tgtgccgggt gttggtgtgc cgggtgttgg tgtaccaggt 1740
   ggcggtgttc cgggtgcagg cgttccgggt ggcggtgtgc cgggcgtggg tgttccgggc 1800
   gtgggtgttc cgggtggcgg tgtgccgggc gcaggtgttc ctggtgtagg tgtgccgggt 1860
   gttggtgtgc cgggtgttgg tgtaccaggt ggcggtgttc cgggtgcagg cgttccgggt 1920
   ggcggtgtgc cgggctggcc g 1941
<210> 123
   <211> 649
   <212> PRT
   <213> Artificial Sequence
<220>
   <223> CNP37 thrombin ELP1-120 fusion PE9216
<400> 123
   Gln Glu His Pro Asn Ala Arg Lys Tyr Lys Gly Ala Asn Lys Lys Gly
   1               5                   10                  15
   Leu Ser Lys Gly Cys Phe Gly Leu Lys Leu Asp Arg Ile Gly Ser Met
               20                  25                  30
   Ser Gly Leu Gly Cys Leu Val Pro Arg Gly Ser Gly Val Pro Gly Val
           35                  40                  45
   Gly Val Pro Gly Val Gly Val Pro Gly Gly Gly Val Pro Gly Ala Gly
       50                  55                  60
   Val Pro Gly Val Gly Val Pro Gly Val Gly Val Pro Gly Val Gly Val
   65                  70                  75                  80
   Pro Gly Gly Gly Val Pro Gly Ala Gly Val Pro Gly Gly Gly Val Pro
                   85                  90                  95
   Gly Val Gly Val Pro Gly Val Gly Val Pro Gly Gly Gly Val Pro Gly
               100                 105                 110
   Ala Gly Val Pro Gly Val Gly Val Pro Gly Val Gly Val Pro Gly Val
           115                 120                 125
   Gly Val Pro Gly Gly Gly Val Pro Gly Ala Gly Val Pro Gly Gly Gly
       130                 135                 140
   Val Pro Gly Val Gly Val Pro Gly Val Gly Val Pro Gly Gly Gly Val
   145                 150                 155                 160
   Pro Gly Ala Gly Val Pro Gly Val Gly Val Pro Gly Val Gly Val Pro
                   165                 170                 175
   Gly Val Gly Val Pro Gly Gly Gly Val Pro Gly Ala Gly Val Pro Gly
               180                 185                 190
   Gly Gly Val Pro Gly Val Gly Val Pro Gly Val Gly Val Pro Gly Gly
           195                 200                 205
   Gly Val Pro Gly Ala Gly Val Pro Gly Val Gly Val Pro Gly Val Gly
       210                 215                 220
   Val Pro Gly Val Gly Val Pro Gly Gly Gly Val Pro Gly Ala Gly Val
   225                 230                 235                 240
   Pro Gly Gly Gly Val Pro Gly Val Gly Val Pro Gly Val Gly Val Pro
                   245                 250                 255
   Gly Gly Gly Val Pro Gly Ala Gly Val Pro Gly Val Gly Val Pro Gly
               260                 265                 270
   Val Gly Val Pro Gly Val Gly Val Pro Gly Gly Gly Val Pro Gly Ala
           275                 280                 285
   Gly Val Pro Gly Gly Gly Val Pro Gly Val Gly Val Pro Gly Val Gly
       290                 295                 300
   Val Pro Gly Gly Gly Val Pro Gly Ala Gly Val Pro Gly Val Gly Val
   305                 310                 315                 320
   Pro Gly Val Gly Val Pro Gly Val Gly Val Pro Gly Gly Gly Val Pro
                   325                 330                 335
   Gly Ala Gly Val Pro Gly Gly Gly Val Pro Gly Val Gly Val Pro Gly
               340                 345                 350
   Val Gly Val Pro Gly Gly Gly Val Pro Gly Ala Gly Val Pro Gly Val
           355                 360                 365
   Gly Val Pro Gly Val Gly Val Pro Gly Val Gly Val Pro Gly Gly Gly
       370                 375                 380
   Val Pro Gly Ala Gly Val Pro Gly Gly Gly Val Pro Gly Val Gly Val
   385                 390                 395                 400
   Pro Gly Val Gly Val Pro Gly Gly Gly Val Pro Gly Ala Gly Val Pro
                   405                 410                 415
   Gly Val Gly Val Pro Gly Val Gly Val Pro Gly Val Gly Val Pro Gly
               420                 425                 430
   Gly Gly Val Pro Gly Ala Gly Val Pro Gly Gly Gly Val Pro Gly Val
           435                 440                 445
   Gly Val Pro Gly Val Gly Val Pro Gly Gly Gly Val Pro Gly Ala Gly
       450                 455                 460
   Val Pro Gly Val Gly Val Pro Gly Val Gly Val Pro Gly Val Gly Val
   465                 470                 475                 480
   Pro Gly Gly Gly Val Pro Gly Ala Gly Val Pro Gly Gly Gly Val Pro
                   485                 490                 495
   Gly Val Gly Val Pro Gly Val Gly Val Pro Gly Gly Gly Val Pro Gly
               500                 505                 510
   Ala Gly Val Pro Gly Val Gly Val Pro Gly Val Gly Val Pro Gly Val
           515                 520                 525
   Gly Val Pro Gly Gly Gly Val Pro Gly Ala Gly Val Pro Gly Gly Gly
       530                 535                 540
   Val Pro Gly Val Gly Val Pro Gly Val Gly Val Pro Gly Gly Gly Val
   545                 550                 555                 560
   Pro Gly Ala Gly Val Pro Gly Val Gly Val Pro Gly Val Gly Val Pro
                   565                 570                 575
   Gly Val Gly Val Pro Gly Gly Gly Val Pro Gly Ala Gly Val Pro Gly
               580                 585                 590
   Gly Gly Val Pro Gly Val Gly Val Pro Gly Val Gly Val Pro Gly Gly
           595                 600                 605
   Gly Val Pro Gly Ala Gly Val Pro Gly Val Gly Val Pro Gly Val Gly
       610                 615                 620
   Val Pro Gly Val Gly Val Pro Gly Gly Gly Val Pro Gly Ala Gly Val
   625                 630                 635                 640
   Pro Gly Gly Gly Val Pro Gly Trp Pro
                   645
<210> 124
   <211> 1947
   <212> DNA
   <213> Artificial Sequence
<220>
   <223> CNP37 thrombin ELP1-120 fusion PE9216
<400> 124
   caggaacatc cgaacgcgcg taaatataaa ggtgcgaaca aaaaaggcct gagcaaaggc      60
   tgctttggcc tgaaactgga tcgcattggc agcatgagcg gcctgggctg cctggtgccg     120
   cgcggcagcg gtgtaccggg cgtgggtgtt ccgggcgtgg gtgttccggg tggcggtgtg     180
   ccgggcgcag gtgttcctgg tgtaggtgtg ccgggtgttg gtgtgccggg tgttggtgta     240
   ccaggtggcg gtgttccggg tgcaggcgtt ccgggtggcg gtgtgccggg cgtgggtgtt     300
   ccgggcgtgg gtgttccggg tggcggtgtg ccgggcgcag gtgttcctgg tgtaggtgtg     360
   ccgggtgttg gtgtgccggg tgttggtgta ccaggtggcg gtgttccggg tgcaggcgtt     420
   ccgggtggcg gtgtgccggg cgtgggtgtt ccgggcgtgg gtgttccggg tggcggtgtg     480
   ccgggcgcag gtgttcctgg tgtaggtgtg ccgggtgttg gtgtgccggg tgttggtgta     540
   ccaggtggcg gtgttccggg tgcaggcgtt ccgggtggcg gtgtgccggg cgtgggtgtt      600
   ccgggcgtgg gtgttccggg tggcggtgtg ccgggcgcag gtgttcctgg tgtaggtgtg      660
   ccgggtgttg gtgtgccggg tgttggtgta ccaggtggcg gtgttccggg tgcaggcgtt      720
   ccgggtggcg gtgtgccggg cgtgggtgtt ccgggcgtgg gtgttccggg tggcggtgtg      780
   ccgggcgcag gtgttcctgg tgtaggtgtg ccgggtgttg gtgtgccggg tgttggtgta      840
   ccaggtggcg gtgttccggg tgcaggcgtt ccgggtggcg gtgtgccggg cgtgggtgtt      900
   ccgggcgtgg gtgttccggg tggcggtgtg ccgggcgcag gtgttcctgg tgtaggtgtg      960
   ccgggtgttg gtgtgccggg tgttggtgta ccaggtggcg gtgttccggg tgcaggcgtt     1020
   ccgggtggcg gtgtgccggg cgtgggtgtt ccgggcgtgg gtgttccggg tggcggtgtg     1080
   ccgggcgcag gtgttcctgg tgtaggtgtg ccgggtgttg gtgtgccggg tgttggtgta     1140
   ccaggtggcg gtgttccggg tgcaggcgtt ccgggtggcg gtgtgccggg cgtgggtgtt     1200
   ccgggcgtgg gtgttccggg tggcggtgtg ccgggcgcag gtgttcctgg tgtaggtgtg     1260
   ccgggtgttg gtgtgccggg tgttggtgta ccaggtggcg gtgttccggg tgcaggcgtt     1320
   ccgggtggcg gtgtgccggg cgtgggtgtt ccgggcgtgg gtgttccggg tggcggtgtg     1380
   ccgggcgcag gtgttcctgg tgtaggtgtg ccgggtgttg gtgtgccggg tgttggtgta     1440
   ccaggtggcg gtgttccggg tgcaggcgtt ccgggtggcg gtgtgccggg cgtgggtgtt     1500
   ccgggcgtgg gtgttccggg tggcggtgtg ccgggcgcag gtgttcctgg tgtaggtgtg     1560
   ccgggtgttg gtgtgccggg tgttggtgta ccaggtggcg gtgttccggg tgcaggcgtt     1620
   ccgggtggcg gtgtgccggg cgtgggtgtt ccgggcgtgg gtgttccggg tggcggtgtg     1680
   ccgggcgcag gtgttcctgg tgtaggtgtg ccgggtgttg gtgtgccggg tgttggtgta     1740
   ccaggtggcg gtgttccggg tgcaggcgtt ccgggtggcg gtgtgccggg cgtgggtgtt     1800
   ccgggcgtgg gtgttccggg tggcggtgtg ccgggcgcag gtgttcctgg tgtaggtgtg     1860
   ccgggtgttg gtgtgccggg tgttggtgta ccaggtggcg gtgttccggg tgcaggcgtt     1920
   ccgggtggcg gtgtgccggg ctggccg                                         1947
<210> 125
   <211> 647
   <212> PRT
   <213> Artificial Sequence
<220>
   <223> CNP37 CatK ELP1-120 fusion PE9306
<400> 125
   Gln Glu His Pro Asn Ala Arg Lys Tyr Lys Gly Ala Asn Lys Lys Gly
   1               5                   10                  15
   Leu Ser Lys Gly Cys Phe Gly Leu Lys Leu Asp Arg Ile Gly Ser Met
               20                  25                  30
   Ser Gly Leu Gly Cys Arg Lys Pro Arg Gly Val Pro Gly Val Gly Val
           35                  40                  45
   Pro Gly Val Gly Val Pro Gly Gly Gly Val Pro Gly Ala Gly Val Pro
       50                  55                  60
   Gly Val Gly Val Pro Gly Val Gly Val Pro Gly Val Gly Val Pro Gly
   65                  70                  75                  80
   Gly Gly Val Pro Gly Ala Gly Val Pro Gly Gly Gly Val Pro Gly Val
                   85                  90                  95
   Gly Val Pro Gly Val Gly Val Pro Gly Gly Gly Val Pro Gly Ala Gly
               100                 105                 110
   Val Pro Gly Val Gly Val Pro Gly Val Gly Val Pro Gly Val Gly Val
           115                 120                 125
   Pro Gly Gly Gly Val Pro Gly Ala Gly Val Pro Gly Gly Gly Val Pro
       130                 135                 140
   Gly Val Gly Val Pro Gly Val Gly Val Pro Gly Gly Gly Val Pro Gly
   145                 150                 155                 160
   Ala Gly Val Pro Gly Val Gly Val Pro Gly Val Gly Val Pro Gly Val
                   165                 170                 175
   Gly Val Pro Gly Gly Gly Val Pro Gly Ala Gly Val Pro Gly Gly Gly
               180                 185                 190
   Val Pro Gly Val Gly Val Pro Gly Val Gly Val Pro Gly Gly Gly Val
           195                 200                 205
   Pro Gly Ala Gly Val Pro Gly Val Gly Val Pro Gly Val Gly Val Pro
       210                 215                 220
   Gly Val Gly Val Pro Gly Gly Gly Val Pro Gly Ala Gly Val Pro Gly
   225                 230                 235                 240
   Gly Gly Val Pro Gly Val Gly Val Pro Gly Val Gly Val Pro Gly Gly
                   245                 250                 255
   Gly Val Pro Gly Ala Gly Val Pro Gly Val Gly Val Pro Gly Val Gly
               260                 265                 270
   Val Pro Gly Val Gly Val Pro Gly Gly Gly Val Pro Gly Ala Gly Val
   275 280 285
   Pro Gly Gly Gly Val Pro Gly Val Gly Val Pro Gly Val Gly Val Pro
   290 295 300
   Gly Gly Gly Val Pro Gly Ala Gly Val Pro Gly Val Gly Val Pro Gly
   305 310 315 320
   Val Gly Val Pro Gly Val Gly Val Pro Gly Gly Gly Val Pro Gly Ala
   325 330 335
   Gly Val Pro Gly Gly Gly Val Pro Gly Val Gly Val Pro Gly Val Gly
   340 345 350
   Val Pro Gly Gly Gly Val Pro Gly Ala Gly Val Pro Gly Val Gly Val
   355 360 365
   Pro Gly Val Gly Val Pro Gly Val Gly Val Pro Gly Gly Gly Val Pro
   370 375 380
   Gly Ala Gly Val Pro Gly Gly Gly Val Pro Gly Val Gly Val Pro Gly
   385 390 395 400
   Val Gly Val Pro Gly Gly Gly Val Pro Gly Ala Gly Val Pro Gly Val
   405 410 415
   Gly Val Pro Gly Val Gly Val Pro Gly Val Gly Val Pro Gly Gly Gly
   420 425 430
   Val Pro Gly Ala Gly Val Pro Gly Gly Gly Val Pro Gly Val Gly Val
   435 440 445
   Pro Gly Val Gly Val Pro Gly Gly Gly Val Pro Gly Ala Gly Val Pro
   450 455 460
   Gly Val Gly Val Pro Gly Val Gly Val Pro Gly Val Gly Val Pro Gly
   465 470 475 480
   Gly Gly Val Pro Gly Ala Gly Val Pro Gly Gly Gly Val Pro Gly Val
   485 490 495
   Gly Val Pro Gly Val Gly Val Pro Gly Gly Gly Val Pro Gly Ala Gly
   500 505 510
   Val Pro Gly Val Gly Val Pro Gly Val Gly Val Pro Gly Val Gly Val
               515                 520                 525
   Pro Gly Gly Gly Val Pro Gly Ala Gly Val Pro Gly Gly Gly Val Pro
       530                 535                 540
   Gly Val Gly Val Pro Gly Val Gly Val Pro Gly Gly Gly Val Pro Gly
   545                 550                 555                 560
   Ala Gly Val Pro Gly Val Gly Val Pro Gly Val Gly Val Pro Gly Val
                   565                 570                 575
   Gly Val Pro Gly Gly Gly Val Pro Gly Ala Gly Val Pro Gly Gly Gly
               580                 585                 590
   Val Pro Gly Val Gly Val Pro Gly Val Gly Val Pro Gly Gly Gly Val
           595                 600                 605
   Pro Gly Ala Gly Val Pro Gly Val Gly Val Pro Gly Val Gly Val Pro
       610                 615                 620
   Gly Val Gly Val Pro Gly Gly Gly Val Pro Gly Ala Gly Val Pro Gly
   625                 630                 635                 640
   Gly Gly Val Pro Gly Trp Pro
                   645
<210> 126
   <211> 1941
   <212> DNA
   <213> Artificial Sequence
<220>
   <223> CNP37 CatK ELP1-120 fusion PE9306
<400> 126
   caggaacatc cgaacgcgcg taaatataaa ggtgcgaaca aaaaaggcct gagcaaaggc      60
   tgctttggcc tgaaactgga tcgcattggc agcatgagcg gcctgggctg ccgcaaaccg     120
   cgcggtgtac cgggcgtggg tgttccgggc gtgggtgttc cgggtggcgg tgtgccgggc     180
   gcaggtgttc ctggtgtagg tgtgccgggt gttggtgtgc cgggtgttgg tgtaccaggt     240
   ggcggtgttc cgggtgcagg cgttccgggt ggcggtgtgc cgggcgtggg tgttccgggc     300
   gtgggtgttc cgggtggcgg tgtgccgggc gcaggtgttc ctggtgtagg tgtgccgggt     360
   gttggtgtgc cgggtgttgg tgtaccaggt ggcggtgttc cgggtgcagg cgttccgggt     420
   ggcggtgtgc cgggcgtggg tgttccgggc gtgggtgttc cgggtggcgg tgtgccgggc     480
   gcaggtgttc ctggtgtagg tgtgccgggt gttggtgtgc cgggtgttgg tgtaccaggt     540
   ggcggtgttc cgggtgcagg cgttccgggt ggcggtgtgc cgggcgtggg tgttccgggc     600
   gtgggtgttc cgggtggcgg tgtgccgggc gcaggtgttc ctggtgtagg tgtgccgggt      660
   gttggtgtgc cgggtgttgg tgtaccaggt ggcggtgttc cgggtgcagg cgttccgggt      720
   ggcggtgtgc cgggcgtggg tgttccgggc gtgggtgttc cgggtggcgg tgtgccgggc      780
   gcaggtgttc ctggtgtagg tgtgccgggt gttggtgtgc cgggtgttgg tgtaccaggt      840
   ggcggtgttc cgggtgcagg cgttccgggt ggcggtgtgc cgggcgtggg tgttccgggc      900
   gtgggtgttc cgggtggcgg tgtgccgggc gcaggtgttc ctggtgtagg tgtgccgggt      960
   gttggtgtgc cgggtgttgg tgtaccaggt ggcggtgttc cgggtgcagg cgttccgggt     1020
   ggcggtgtgc cgggcgtggg tgttccgggc gtgggtgttc cgggtggcgg tgtgccgggc     1080
   gcaggtgttc ctggtgtagg tgtgccgggt gttggtgtgc cgggtgttgg tgtaccaggt     1140
   ggcggtgttc cgggtgcagg cgttccgggt ggcggtgtgc cgggcgtggg tgttccgggc     1200
   gtgggtgttc cgggtggcgg tgtgccgggc gcaggtgttc ctggtgtagg tgtgccgggt     1260
   gttggtgtgc cgggtgttgg tgtaccaggt ggcggtgttc cgggtgcagg cgttccgggt     1320
   ggcggtgtgc cgggcgtggg tgttccgggc gtgggtgttc cgggtggcgg tgtgccgggc     1380
   gcaggtgttc ctggtgtagg tgtgccgggt gttggtgtgc cgggtgttgg tgtaccaggt     1440
   ggcggtgttc cgggtgcagg cgttccgggt ggcggtgtgc cgggcgtggg tgttccgggc     1500
   gtgggtgttc cgggtggcgg tgtgccgggc gcaggtgttc ctggtgtagg tgtgccgggt     1560
   gttggtgtgc cgggtgttgg tgtaccaggt ggcggtgttc cgggtgcagg cgttccgggt     1620
   ggcggtgtgc cgggcgtggg tgttccgggc gtgggtgttc cgggtggcgg tgtgccgggc     1680
   gcaggtgttc ctggtgtagg tgtgccgggt gttggtgtgc cgggtgttgg tgtaccaggt     1740
   ggcggtgttc cgggtgcagg cgttccgggt ggcggtgtgc cgggcgtggg tgttccgggc     1800
   gtgggtgttc cgggtggcgg tgtgccgggc gcaggtgttc ctggtgtagg tgtgccgggt     1860
   gttggtgtgc cgggtgttgg tgtaccaggt ggcggtgttc cgggtgcagg cgttccgggt     1920
   ggcggtgtgc cgggctggcc g                                               1941
<210> 127
   <211> 647
   <212> PRT
   <213> Artificial Sequence
<220>
   <223> CNP37 CatK ELP1-120 fusion PE9316
<400> 127
   Gln Glu His Pro Asn Ala Arg Lys Tyr Lys Gly Ala Asn Lys Lys Gly
   1               5                   10                  15
   Leu Ser Lys Gly Cys Phe Gly Leu Lys Leu Asp Arg Ile Gly Ser Met
   20 25 30
   Ser Gly Leu Gly Cys Arg Lys Pro Arg Gly Val Pro Gly Val Gly Val
   35 40 45
   Pro Gly Val Gly Val Pro Gly Gly Gly Val Pro Gly Ala Gly Val Pro
   50 55 60
   Gly Val Gly Val Pro Gly Val Gly Val Pro Gly Val Gly Val Pro Gly
   65 70 75 80
   Gly Gly Val Pro Gly Ala Gly Val Pro Gly Gly Gly Val Pro Gly Val
   85 90 95
   Gly Val Pro Gly Val Gly Val Pro Gly Gly Gly Val Pro Gly Ala Gly
   100 105 110
   Val Pro Gly Val Gly Val Pro Gly Val Gly Val Pro Gly Val Gly Val
   115 120 125
   Pro Gly Gly Gly Val Pro Gly Ala Gly Val Pro Gly Gly Gly Val Pro
   130 135 140
   Gly Val Gly Val Pro Gly Val Gly Val Pro Gly Gly Gly Val Pro Gly
   145 150 155 160
   Ala Gly Val Pro Gly Val Gly Val Pro Gly Val Gly Val Pro Gly Val
   165 170 175
   Gly Val Pro Gly Gly Gly Val Pro Gly Ala Gly Val Pro Gly Gly Gly
   180 185 190
   Val Pro Gly Val Gly Val Pro Gly Val Gly Val Pro Gly Gly Gly Val
   195 200 205
   Pro Gly Ala Gly Val Pro Gly Val Gly Val Pro Gly Val Gly Val Pro
   210 215 220
   Gly Val Gly Val Pro Gly Gly Gly Val Pro Gly Ala Gly Val Pro Gly
   225 230 235 240
   Gly Gly Val Pro Gly Val Gly Val Pro Gly Val Gly Val Pro Gly Gly
   245 250 255
   Gly Val Pro Gly Ala Gly Val Pro Gly Val Gly Val Pro Gly Val Gly
   260 265 270
   Val Pro Gly Val Gly Val Pro Gly Gly Gly Val Pro Gly Ala Gly Val
   275 280 285
   Pro Gly Gly Gly Val Pro Gly Val Gly Val Pro Gly Val Gly Val Pro
   290 295 300
   Gly Gly Gly Val Pro Gly Ala Gly Val Pro Gly Val Gly Val Pro Gly
   305 310 315 320
   Val Gly Val Pro Gly Val Gly Val Pro Gly Gly Gly Val Pro Gly Ala
   325 330 335
   Gly Val Pro Gly Gly Gly Val Pro Gly Val Gly Val Pro Gly Val Gly
   340 345 350
   Val Pro Gly Gly Gly Val Pro Gly Ala Gly Val Pro Gly Val Gly Val
   355 360 365
   Pro Gly Val Gly Val Pro Gly Val Gly Val Pro Gly Gly Gly Val Pro
   370 375 380
   Gly Ala Gly Val Pro Gly Gly Gly Val Pro Gly Val Gly Val Pro Gly
   385 390 395 400
   Val Gly Val Pro Gly Gly Gly Val Pro Gly Ala Gly Val Pro Gly Val
   405 410 415
   Gly Val Pro Gly Val Gly Val Pro Gly Val Gly Val Pro Gly Gly Gly
   420 425 430
   Val Pro Gly Ala Gly Val Pro Gly Gly Gly Val Pro Gly Val Gly Val
   435 440 445
   Pro Gly Val Gly Val Pro Gly Gly Gly Val Pro Gly Ala Gly Val Pro
   450 455 460
   Gly Val Gly Val Pro Gly Val Gly Val Pro Gly Val Gly Val Pro Gly
   465 470 475 480
   Gly Gly Val Pro Gly Ala Gly Val Pro Gly Gly Gly Val Pro Gly Val
   485 490 495
   Gly Val Pro Gly Val Gly Val Pro Gly Gly Gly Val Pro Gly Ala Gly
   500 505 510
   Val Pro Gly Val Gly Val Pro Gly Val Gly Val Pro Gly Val Gly Val
   515 520 525
   Pro Gly Gly Gly Val Pro Gly Ala Gly Val Pro Gly Gly Gly Val Pro
       530                 535                 540
   Gly Val Gly Val Pro Gly Val Gly Val Pro Gly Gly Gly Val Pro Gly
   545                 550                 555                 560
   Ala Gly Val Pro Gly Val Gly Val Pro Gly Val Gly Val Pro Gly Val
                   565                 570                 575
   Gly Val Pro Gly Gly Gly Val Pro Gly Ala Gly Val Pro Gly Gly Gly
               580                 585                 590
   Val Pro Gly Val Gly Val Pro Gly Val Gly Val Pro Gly Gly Gly Val
           595                 600                 605
   Pro Gly Ala Gly Val Pro Gly Val Gly Val Pro Gly Val Gly Val Pro
       610                 615                 620
   Gly Val Gly Val Pro Gly Gly Gly Val Pro Gly Ala Gly Val Pro Gly
   625                 630                 635                 640
   Gly Gly Val Pro Gly Trp Pro
                   645
<210> 128
   <211> 1941
   <212> DNA
   <213> Artificial Sequence
<220>
   <223> CNP37 CatK ELP1-120 fusion PE9316
<400> 128
   caggaacatc cgaacgcgcg taaatataaa ggtgcgaaca aaaaaggcct gagcaaaggc        60
   tgctttggcc tgaaactgga tcgcattggc agcatgagcg gcctgggctg ccgcaaaccg       120
   cgcggtgtac cgggcgtggg tgttccgggc gtgggtgttc cgggtggcgg tgtgccgggc       180
   gcaggtgttc ctggtgtagg tgtgccgggt gttggtgtgc cgggtgttgg tgtaccaggt       240
   ggcggtgttc cgggtgcagg cgttccgggt ggcggtgtgc cgggcgtggg tgttccgggc       300
   gtgggtgttc cgggtggcgg tgtgccgggc gcaggtgttc ctggtgtagg tgtgccgggt       360
   gttggtgtgc cgggtgttgg tgtaccaggt ggcggtgttc cgggtgcagg cgttccgggt       420
   ggcggtgtgc cgggcgtggg tgttccgggc gtgggtgttc cgggtggcgg tgtgccgggc       480
   gcaggtgttc ctggtgtagg tgtgccgggt gttggtgtgc cgggtgttgg tgtaccaggt       540
   ggcggtgttc cgggtgcagg cgttccgggt ggcggtgtgc cgggcgtggg tgttccgggc       600
   gtgggtgttc cgggtggcgg tgtgccgggc gcaggtgttc ctggtgtagg tgtgccgggt       660
   gttggtgtgc cgggtgttgg tgtaccaggt ggcggtgttc cgggtgcagg cgttccgggt 720
   ggcggtgtgc cgggcgtggg tgttccgggc gtgggtgttc cgggtggcgg tgtgccgggc 780
   gcaggtgttc ctggtgtagg tgtgccgggt gttggtgtgc cgggtgttgg tgtaccaggt 840
   ggcggtgttc cgggtgcagg cgttccgggt ggcggtgtgc cgggcgtggg tgttccgggc 900
   gtgggtgttc cgggtggcgg tgtgccgggc gcaggtgttc ctggtgtagg tgtgccgggt 960
   gttggtgtgc cgggtgttgg tgtaccaggt ggcggtgttc cgggtgcagg cgttccgggt 1020
   ggcggtgtgc cgggcgtggg tgttccgggc gtgggtgttc cgggtggcgg tgtgccgggc 1080
   gcaggtgttc ctggtgtagg tgtgccgggt gttggtgtgc cgggtgttgg tgtaccaggt 1140
   ggcggtgttc cgggtgcagg cgttccgggt ggcggtgtgc cgggcgtggg tgttccgggc 1200
   gtgggtgttc cgggtggcgg tgtgccgggc gcaggtgttc ctggtgtagg tgtgccgggt 1260
   gttggtgtgc cgggtgttgg tgtaccaggt ggcggtgttc cgggtgcagg cgttccgggt 1320
   ggcggtgtgc cgggcgtggg tgttccgggc gtgggtgttc cgggtggcgg tgtgccgggc 1380
   gcaggtgttc ctggtgtagg tgtgccgggt gttggtgtgc cgggtgttgg tgtaccaggt 1440
   ggcggtgttc cgggtgcagg cgttccgggt ggcggtgtgc cgggcgtggg tgttccgggc 1500
   gtgggtgttc cgggtggcgg tgtgccgggc gcaggtgttc ctggtgtagg tgtgccgggt 1560
   gttggtgtgc cgggtgttgg tgtaccaggt ggcggtgttc cgggtgcagg cgttccgggt 1620
   ggcggtgtgc cgggcgtggg tgttccgggc gtgggtgttc cgggtggcgg tgtgccgggc 1680
   gcaggtgttc ctggtgtagg tgtgccgggt gttggtgtgc cgggtgttgg tgtaccaggt 1740
   ggcggtgttc cgggtgcagg cgttccgggt ggcggtgtgc cgggcgtggg tgttccgggc 1800
   gtgggtgttc cgggtggcgg tgtgccgggc gcaggtgttc ctggtgtagg tgtgccgggt 1860
   gttggtgtgc cgggtgttgg tgtaccaggt ggcggtgttc cgggtgcagg cgttccgggt 1920
   ggcggtgtgc cgggctggcc g 1941
<210> 129
   <211> 649
   <212> PRT
   <213> Artificial Sequence
<220>
   <223> CNP37 MMP ELP1-120 fusion PE09326
<400> 129
   Gln Glu His Pro Asn Ala Arg Lys Tyr Lys Gly Ala Asn Lys Lys Gly
   1 5 10 15
   Leu Ser Lys Gly Cys Phe Gly Leu Lys Leu Asp Arg Ile Gly Ser Met
   20 25 30
   Ser Gly Leu Gly Cys Pro Leu Gly Leu Trp Ala Gly Val Pro Gly Val
           35                  40                  45
   Gly Val Pro Gly Val Gly Val Pro Gly Gly Gly Val Pro Gly Ala Gly
       50                  55                  60
   Val Pro Gly Val Gly Val Pro Gly Val Gly Val Pro Gly Val Gly Val
   65                  70                  75                  80
   Pro Gly Gly Gly Val Pro Gly Ala Gly Val Pro Gly Gly Gly Val Pro
                   85                  90                  95
   Gly Val Gly Val Pro Gly Val Gly Val Pro Gly Gly Gly Val Pro Gly
               100                 105                 110
   Ala Gly Val Pro Gly Val Gly Val Pro Gly Val Gly Val Pro Gly Val
           115                 120                 125
   Gly Val Pro Gly Gly Gly Val Pro Gly Ala Gly Val Pro Gly Gly Gly
       130                 135                 140
   Val Pro Gly Val Gly Val Pro Gly Val Gly Val Pro Gly Gly Gly Val
   145                 150                 155                 160
   Pro Gly Ala Gly Val Pro Gly Val Gly Val Pro Gly Val Gly Val Pro
                   165                 170                 175
   Gly Val Gly Val Pro Gly Gly Gly Val Pro Gly Ala Gly Val Pro Gly
               180                 185                 190
   Gly Gly Val Pro Gly Val Gly Val Pro Gly Val Gly Val Pro Gly Gly
           195                 200                 205
   Gly Val Pro Gly Ala Gly Val Pro Gly Val Gly Val Pro Gly Val Gly
       210                 215                 220
   Val Pro Gly Val Gly Val Pro Gly Gly Gly Val Pro Gly Ala Gly Val
   225                 230                 235                 240
   Pro Gly Gly Gly Val Pro Gly Val Gly Val Pro Gly Val Gly Val Pro
                   245                 250                 255
   Gly Gly Gly Val Pro Gly Ala Gly Val Pro Gly Val Gly Val Pro Gly
               260                 265                 270
   Val Gly Val Pro Gly Val Gly Val Pro Gly Gly Gly Val Pro Gly Ala
           275                 280                 285
   Gly Val Pro Gly Gly Gly Val Pro Gly Val Gly Val Pro Gly Val Gly
       290                 295                 300
   Val Pro Gly Gly Gly Val Pro Gly Ala Gly Val Pro Gly Val Gly Val
   305                 310                 315                 320
   Pro Gly Val Gly Val Pro Gly Val Gly Val Pro Gly Gly Gly Val Pro
                   325                 330                 335
   Gly Ala Gly Val Pro Gly Gly Gly Val Pro Gly Val Gly Val Pro Gly
               340                 345                 350
   Val Gly Val Pro Gly Gly Gly Val Pro Gly Ala Gly Val Pro Gly Val
           355                 360                 365
   Gly Val Pro Gly Val Gly Val Pro Gly Val Gly Val Pro Gly Gly Gly
       370                 375                 380
   Val Pro Gly Ala Gly Val Pro Gly Gly Gly Val Pro Gly Val Gly Val
   385                 390                 395                 400
   Pro Gly Val Gly Val Pro Gly Gly Gly Val Pro Gly Ala Gly Val Pro
                   405                 410                 415
   Gly Val Gly Val Pro Gly Val Gly Val Pro Gly Val Gly Val Pro Gly
               420                 425                 430
   Gly Gly Val Pro Gly Ala Gly Val Pro Gly Gly Gly Val Pro Gly Val
           435                 440                 445
   Gly Val Pro Gly Val Gly Val Pro Gly Gly Gly Val Pro Gly Ala Gly
       450                 455                 460
   Val Pro Gly Val Gly Val Pro Gly Val Gly Val Pro Gly Val Gly Val
   465                 470                 475                 480
   Pro Gly Gly Gly Val Pro Gly Ala Gly Val Pro Gly Gly Gly Val Pro
                   485                 490                 495
   Gly Val Gly Val Pro Gly Val Gly Val Pro Gly Gly Gly Val Pro Gly
               500                 505                 510
   Ala Gly Val Pro Gly Val Gly Val Pro Gly Val Gly Val Pro Gly Val
           515                 520                 525
   Gly Val Pro Gly Gly Gly Val Pro Gly Ala Gly Val Pro Gly Gly Gly
       530                 535                 540
   Val Pro Gly Val Gly Val Pro Gly Val Gly Val Pro Gly Gly Gly Val
   545                 550                 555                 560
   Pro Gly Ala Gly Val Pro Gly Val Gly Val Pro Gly Val Gly Val Pro
                   565                 570                 575
   Gly Val Gly Val Pro Gly Gly Gly Val Pro Gly Ala Gly Val Pro Gly
               580                 585                 590
   Gly Gly Val Pro Gly Val Gly Val Pro Gly Val Gly Val Pro Gly Gly
           595                 600                 605
   Gly Val Pro Gly Ala Gly Val Pro Gly Val Gly Val Pro Gly Val Gly
       610                 615                 620
   Val Pro Gly Val Gly Val Pro Gly Gly Gly Val Pro Gly Ala Gly Val
   625                 630                 635                 640
   Pro Gly Gly Gly Val Pro Gly Trp Pro
                   645
<210> 130
   <211> 1947
   <212> DNA
   <213> Artificial Sequence
<220>
   <223> CNP37 MMP ELP1-120 fusion PE09326
<400> 130
   caggaacatc cgaacgcgcg taaatataaa ggtgcgaaca aaaaaggcct gagcaaaggc 60
   tgctttggcc tgaaactgga tcgcattggc agcatgagcg gcctgggctg cccgctgggc 120
   ctgtgggcgg gtgtaccggg cgtgggtgtt ccgggcgtgg gtgttccggg tggcggtgtg 180
   ccgggcgcag gtgttcctgg tgtaggtgtg ccgggtgttg gtgtgccggg tgttggtgta 240
   ccaggtggcg gtgttccggg tgcaggcgtt ccgggtggcg gtgtgccggg cgtgggtgtt 300
   ccgggcgtgg gtgttccggg tggcggtgtg ccgggcgcag gtgttcctgg tgtaggtgtg 360
   ccgggtgttg gtgtgccggg tgttggtgta ccaggtggcg gtgttccggg tgcaggcgtt 420
   ccgggtggcg gtgtgccggg cgtgggtgtt ccgggcgtgg gtgttccggg tggcggtgtg 480
   ccgggcgcag gtgttcctgg tgtaggtgtg ccgggtgttg gtgtgccggg tgttggtgta 540
   ccaggtggcg gtgttccggg tgcaggcgtt ccgggtggcg gtgtgccggg cgtgggtgtt 600
   ccgggcgtgg gtgttccggg tggcggtgtg ccgggcgcag gtgttcctgg tgtaggtgtg 660
   ccgggtgttg gtgtgccggg tgttggtgta ccaggtggcg gtgttccggg tgcaggcgtt 720
   ccgggtggcg gtgtgccggg cgtgggtgtt ccgggcgtgg gtgttccggg tggcggtgtg 780
   ccgggcgcag gtgttcctgg tgtaggtgtg ccgggtgttg gtgtgccggg tgttggtgta 840
   ccaggtggcg gtgttccggg tgcaggcgtt ccgggtggcg gtgtgccggg cgtgggtgtt 900
   ccgggcgtgg gtgttccggg tggcggtgtg ccgggcgcag gtgttcctgg tgtaggtgtg 960
   ccgggtgttg gtgtgccggg tgttggtgta ccaggtggcg gtgttccggg tgcaggcgtt 1020
   ccgggtggcg gtgtgccggg cgtgggtgtt ccgggcgtgg gtgttccggg tggcggtgtg 1080
   ccgggcgcag gtgttcctgg tgtaggtgtg ccgggtgttg gtgtgccggg tgttggtgta 1140
   ccaggtggcg gtgttccggg tgcaggcgtt ccgggtggcg gtgtgccggg cgtgggtgtt 1200
   ccgggcgtgg gtgttccggg tggcggtgtg ccgggcgcag gtgttcctgg tgtaggtgtg 1260
   ccgggtgttg gtgtgccggg tgttggtgta ccaggtggcg gtgttccggg tgcaggcgtt 1320
   ccgggtggcg gtgtgccggg cgtgggtgtt ccgggcgtgg gtgttccggg tggcggtgtg 1380
   ccgggcgcag gtgttcctgg tgtaggtgtg ccgggtgttg gtgtgccggg tgttggtgta 1440
   ccaggtggcg gtgttccggg tgcaggcgtt ccgggtggcg gtgtgccggg cgtgggtgtt 1500
   ccgggcgtgg gtgttccggg tggcggtgtg ccgggcgcag gtgttcctgg tgtaggtgtg 1560
   ccgggtgttg gtgtgccggg tgttggtgta ccaggtggcg gtgttccggg tgcaggcgtt 1620
   ccgggtggcg gtgtgccggg cgtgggtgtt ccgggcgtgg gtgttccggg tggcggtgtg 1680
   ccgggcgcag gtgttcctgg tgtaggtgtg ccgggtgttg gtgtgccggg tgttggtgta 1740
   ccaggtggcg gtgttccggg tgcaggcgtt ccgggtggcg gtgtgccggg cgtgggtgtt 1800
   ccgggcgtgg gtgttccggg tggcggtgtg ccgggcgcag gtgttcctgg tgtaggtgtg 1860
   ccgggtgttg gtgtgccggg tgttggtgta ccaggtggcg gtgttccggg tgcaggcgtt 1920
   ccgggtggcg gtgtgccggg ctggccg 1947
<210> 131
   <211> 787
   <212> PRT
   <213> Artificial Sequence
<220>
   <223> ELP1-120 FGF21 fusion PE9183
<400> 131
   Met Val Pro Gly Val Gly Val Pro Gly Val Gly Val Pro Gly Gly Gly
   1               5                   10                  15
   Val Pro Gly Ala Gly Val Pro Gly Val Gly Val Pro Gly Val Gly Val
               20                  25                  30
   Pro Gly Val Gly Val Pro Gly Gly Gly Val Pro Gly Ala Gly Val Pro
   35 40 45
   Gly Gly Gly Val Pro Gly Val Gly Val Pro Gly Val Gly Val Pro Gly
   50 55 60
   Gly Gly Val Pro Gly Ala Gly Val Pro Gly Val Gly Val Pro Gly Val
   65 70 75 80
   Gly Val Pro Gly Val Gly Val Pro Gly Gly Gly Val Pro Gly Ala Gly
   85 90 95
   Val Pro Gly Gly Gly Val Pro Gly Val Gly Val Pro Gly Val Gly Val
   100 105 110
   Pro Gly Gly Gly Val Pro Gly Ala Gly Val Pro Gly Val Gly Val Pro
   115 120 125
   Gly Val Gly Val Pro Gly Val Gly Val Pro Gly Gly Gly Val Pro Gly
   130 135 140
   Ala Gly Val Pro Gly Gly Gly Val Pro Gly Val Gly Val Pro Gly Val
   145 150 155 160
   Gly Val Pro Gly Gly Gly Val Pro Gly Ala Gly Val Pro Gly Val Gly
   165 170 175
   Val Pro Gly Val Gly Val Pro Gly Val Gly Val Pro Gly Gly Gly Val
   180 185 190
   Pro Gly Ala Gly Val Pro Gly Gly Gly Val Pro Gly Val Gly Val Pro
   195 200 205
   Gly Val Gly Val Pro Gly Gly Gly Val Pro Gly Ala Gly Val Pro Gly
   210 215 220
   Val Gly Val Pro Gly Val Gly Val Pro Gly Val Gly Val Pro Gly Gly
   225 230 235 240
   Gly Val Pro Gly Ala Gly Val Pro Gly Gly Gly Val Pro Gly Val Gly
   245 250 255
   Val Pro Gly Val Gly Val Pro Gly Gly Gly Val Pro Gly Ala Gly Val
   260 265 270
   Pro Gly Val Gly Val Pro Gly Val Gly Val Pro Gly Val Gly Val Pro
   275 280 285
   Gly Gly Gly Val Pro Gly Ala Gly Val Pro Gly Gly Gly Val Pro Gly
       290                 295                 300
   Val Gly Val Pro Gly Val Gly Val Pro Gly Gly Gly Val Pro Gly Ala
   305                 310                 315                 320
   Gly Val Pro Gly Val Gly Val Pro Gly Val Gly Val Pro Gly Val Gly
                   325                 330                 335
   Val Pro Gly Gly Gly Val Pro Gly Ala Gly Val Pro Gly Gly Gly Val
               340                 345                 350
   Pro Gly Val Gly Val Pro Gly Val Gly Val Pro Gly Gly Gly Val Pro
           355                 360                 365
   Gly Ala Gly Val Pro Gly Val Gly Val Pro Gly Val Gly Val Pro Gly
       370                 375                 380
   Val Gly Val Pro Gly Gly Gly Val Pro Gly Ala Gly Val Pro Gly Gly
   385                 390                 395                 400
   Gly Val Pro Gly Val Gly Val Pro Gly Val Gly Val Pro Gly Gly Gly
                   405                 410                 415
   Val Pro Gly Ala Gly Val Pro Gly Val Gly Val Pro Gly Val Gly Val
               420                 425                 430
   Pro Gly Val Gly Val Pro Gly Gly Gly Val Pro Gly Ala Gly Val Pro
           435                 440                 445
   Gly Gly Gly Val Pro Gly Val Gly Val Pro Gly Val Gly Val Pro Gly
       450                 455                 460
   Gly Gly Val Pro Gly Ala Gly Val Pro Gly Val Gly Val Pro Gly Val
   465                 470                 475                 480
   Gly Val Pro Gly Val Gly Val Pro Gly Gly Gly Val Pro Gly Ala Gly
                   485                 490                 495
   Val Pro Gly Gly Gly Val Pro Gly Val Gly Val Pro Gly Val Gly Val
               500                 505                 510
   Pro Gly Gly Gly Val Pro Gly Ala Gly Val Pro Gly Val Gly Val Pro
           515                 520                 525
   Gly Val Gly Val Pro Gly Val Gly Val Pro Gly Gly Gly Val Pro Gly
       530                 535                 540
   Ala Gly Val Pro Gly Gly Gly Val Pro Gly Val Gly Val Pro Gly Val
   545                 550                 555                 560
   Gly Val Pro Gly Gly Gly Val Pro Gly Ala Gly Val Pro Gly Val Gly
                   565                 570                 575
   Val Pro Gly Val Gly Val Pro Gly Val Gly Val Pro Gly Gly Gly Val
               580                 585                 590
   Pro Gly Ala Gly Val Pro Gly Gly Gly Val Pro Gly Gly Pro His Pro
           595                 600                 605
   Ile Pro Asp Ser Ser Pro Leu Leu Gln Phe Gly Gly Gln Val Arg Gln
       610                 615                 620
   Arg Tyr Leu Tyr Thr Asp Asp Ala Gln Gln Thr Glu Ala His Leu Glu
   625                 630                 635                 640
   Ile Arg Glu Asp Gly Thr Val Gly Gly Ala Ala Asp Gln Ser Pro Glu
                   645                 650                 655
   Ser Leu Leu Gln Leu Lys Ala Leu Lys Pro Gly Val Ile Gln Ile Leu
               660                 665                 670
   Gly Val Lys Thr Ser Arg Phe Leu Cys Gln Arg Pro Asp Gly Ala Leu
           675                 680                 685
   Tyr Gly Ser Leu His Phe Asp Pro Glu Ala Cys Ser Phe Arg Glu Leu
       690                 695                 700
   Leu Leu Glu Asp Gly Tyr Asn Val Tyr Gln Ser Glu Ala His Gly Leu
   705                 710                 715                 720
   Pro Leu His Leu Pro Gly Asn Lys Ser Pro His Arg Asp Pro Ala Pro
                   725                 730                 735
   Arg Gly Pro Ala Arg Phe Leu Pro Leu Pro Gly Leu Pro Pro Ala Pro
               740                 745                 750
   Pro Glu Pro Pro Gly Ile Leu Ala Pro Gln Pro Pro Asp Val Gly Ser
           755                 760                 765
   Ser Asp Pro Leu Ser Met Val Gly Pro Ser Gln Gly Arg Ser Pro Ser
       770                 775                 780
   Tyr Ala Ser
   785
<210> 132
   <211> 2361
   <212> DNA
   <213> Artificial Sequence
<220>
   <223> ELP1-120 FGF21 fusion PE9183
<400> 132
   atggtaccgg gcgtgggtgt tccgggcgtg ggtgttccgg gtggcggtgt gccgggcgca      60
   ggtgttcctg gtgtaggtgt gccgggtgtt ggtgtgccgg gtgttggtgt accaggtggc     120
   ggtgttccgg gtgcaggcgt tccgggtggc ggtgtgccgg gcgtgggtgt tccgggcgtg     180
   ggtgttccgg gtggcggtgt gccgggcgca ggtgttcctg gtgtaggtgt gccgggtgtt     240
   ggtgtgccgg gtgttggtgt accaggtggc ggtgttccgg gtgcaggcgt tccgggtggc     300
   ggtgtgccgg gcgtgggtgt tccgggcgtg ggtgttccgg gtggcggtgt gccgggcgca     360
   ggtgttcctg gtgtaggtgt gccgggtgtt ggtgtgccgg gtgttggtgt accaggtggc     420
   ggtgttccgg gtgcaggcgt tccgggtggc ggtgtgccgg gcgtgggtgt tccgggcgtg     480
   ggtgttccgg gtggcggtgt gccgggcgca ggtgttcctg gtgtaggtgt gccgggtgtt     540
   ggtgtgccgg gtgttggtgt accaggtggc ggtgttccgg gtgcaggcgt tccgggtggc     600
   ggtgtgccgg gcgtgggtgt tccgggcgtg ggtgttccgg gtggcggtgt gccgggcgca     660
   ggtgttcctg gtgtaggtgt gccgggtgtt ggtgtgccgg gtgttggtgt accaggtggc     720
   ggtgttccgg gtgcaggcgt tccgggtggc ggtgtgccgg gcgtgggtgt tccgggcgtg     780
   ggtgttccgg gtggcggtgt gccgggcgca ggtgttcctg gtgtaggtgt gccgggtgtt     840
   ggtgtgccgg gtgttggtgt accaggtggc ggtgttccgg gtgcaggcgt tccgggtggc     900
   ggtgtgccgg gcgtgggtgt tccgggcgtg ggtgttccgg gtggcggtgt gccgggcgca     960
   ggtgttcctg gtgtaggtgt gccgggtgtt ggtgtgccgg gtgttggtgt accaggtggc    1020
   ggtgttccgg gtgcaggcgt tccgggtggc ggtgtgccgg gcgtgggtgt tccgggcgtg    1080
   ggtgttccgg gtggcggtgt gccgggcgca ggtgttcctg gtgtaggtgt gccgggtgtt    1140
   ggtgtgccgg gtgttggtgt accaggtggc ggtgttccgg gtgcaggcgt tccgggtggc    1200
   ggtgtgccgg gcgtgggtgt tccgggcgtg ggtgttccgg gtggcggtgt gccgggcgca    1260
   ggtgttcctg gtgtaggtgt gccgggtgtt ggtgtgccgg gtgttggtgt accaggtggc    1320
   ggtgttccgg gtgcaggcgt tccgggtggc ggtgtgccgg gcgtgggtgt tccgggcgtg    1380
   ggtgttccgg gtggcggtgt gccgggcgca ggtgttcctg gtgtaggtgt gccgggtgtt    1440
   ggtgtgccgg gtgttggtgt accaggtggc ggtgttccgg gtgcaggcgt tccgggtggc    1500
   ggtgtgccgg gcgtgggtgt tccgggcgtg ggtgttccgg gtggcggtgt gccgggcgca    1560
   ggtgttcctg gtgtaggtgt gccgggtgtt ggtgtgccgg gtgttggtgt accaggtggc      1620
   ggtgttccgg gtgcaggcgt tccgggtggc ggtgtgccgg gcgtgggtgt tccgggcgtg      1680
   ggtgttccgg gtggcggtgt gccgggcgca ggtgttcctg gtgtaggtgt gccgggtgtt      1740
   ggtgtgccgg gtgttggtgt accaggtggc ggtgttccgg gtgcaggcgt tccgggtggc      1800
   ggtgtgccgg gcgggccgca cccgatcccg gacagctctc cgctgctgca gtttggcggt      1860
   caggtacgtc agcgttacct gtatactgac gacgcccagc agaccgaagc tcacctggag      1920
   atccgcgaag acggtaccgt aggcggtgct gcagatcaat ctcctgaatc tctgctgcag      1980
   ctgaaggcac tgaaaccggg cgttatccaa atcctgggtg ttaaaacttc tcgtttcctg      2040
   tgccagcgcc cggatggtgc actgtacggt tctctgcatt tcgacccgga ggcctgctct      2100
   ttccgtgaac tgctgctgga agatggttac aatgtatacc aatctgaggc acacggtctg      2160
   ccgctgcacc tgcctggcaa caaatctccg catcgtgacc cggccccgcg cggtccagcc      2220
   cgctttctgc cgctgccggg tctgccgccg gctccaccag aaccgcctgg tatcctggcg      2280
   cctcagccac ctgatgttgg ctcctccgac ccgctgtcta tggtcggtcc gagccaaggc      2340
   cgttccccaa gctacgccag t                                                2361
<210> 133
   <211> 817
   <212> PRT
   <213> Artificial Sequence
<220>
   <223> GLP1 ELP1-120 FGF21 dual fusion PE9193
<400> 133
   His Gly Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Gly Arg Gly Val
               20                  25                  30
   Pro Gly Val Gly Val Pro Gly Val Gly Val Pro Gly Gly Gly Val Pro
           35                  40                  45
   Gly Ala Gly Val Pro Gly Val Gly Val Pro Gly Val Gly Val Pro Gly
       50                  55                  60
   Val Gly Val Pro Gly Gly Gly Val Pro Gly Ala Gly Val Pro Gly Gly
   65                  70                  75                  80
   Gly Val Pro Gly Val Gly Val Pro Gly Val Gly Val Pro Gly Gly Gly
                   85                  90                  95
   Val Pro Gly Ala Gly Val Pro Gly Val Gly Val Pro Gly Val Gly Val
               100                 105                 110
   Pro Gly Val Gly Val Pro Gly Gly Gly Val Pro Gly Ala Gly Val Pro
           115                 120                 125
   Gly Gly Gly Val Pro Gly Val Gly Val Pro Gly Val Gly Val Pro Gly
       130                 135                 140
   Gly Gly Val Pro Gly Ala Gly Val Pro Gly Val Gly Val Pro Gly Val
   145                 150                 155                 160
   Gly Val Pro Gly Val Gly Val Pro Gly Gly Gly Val Pro Gly Ala Gly
                   165                 170                 175
   Val Pro Gly Gly Gly Val Pro Gly Val Gly Val Pro Gly Val Gly Val
               180                 185                 190
   Pro Gly Gly Gly Val Pro Gly Ala Gly Val Pro Gly Val Gly Val Pro
           195                 200                 205
   Gly Val Gly Val Pro Gly Val Gly Val Pro Gly Gly Gly Val Pro Gly
       210                 215                 220
   Ala Gly Val Pro Gly Gly Gly Val Pro Gly Val Gly Val Pro Gly Val
   225                 230                 235                 240
   Gly Val Pro Gly Gly Gly Val Pro Gly Ala Gly Val Pro Gly Val Gly
                   245                 250                 255
   Val Pro Gly Val Gly Val Pro Gly Val Gly Val Pro Gly Gly Gly Val
               260                 265                 270
   Pro Gly Ala Gly Val Pro Gly Gly Gly Val Pro Gly Val Gly Val Pro
           275                 280                 285
   Gly Val Gly Val Pro Gly Gly Gly Val Pro Gly Ala Gly Val Pro Gly
       290                 295                 300
   Val Gly Val Pro Gly Val Gly Val Pro Gly Val Gly Val Pro Gly Gly
   305                 310                 315                 320
   Gly Val Pro Gly Ala Gly Val Pro Gly Gly Gly Val Pro Gly Val Gly
                   325                 330                 335
   Val Pro Gly Val Gly Val Pro Gly Gly Gly Val Pro Gly Ala Gly Val
               340                 345                 350
   Pro Gly Val Gly Val Pro Gly Val Gly Val Pro Gly Val Gly Val Pro
           355                 360                 365
   Gly Gly Gly Val Pro Gly Ala Gly Val Pro Gly Gly Gly Val Pro Gly
       370                 375                 380
   Val Gly Val Pro Gly Val Gly Val Pro Gly Gly Gly Val Pro Gly Ala
   385                 390                 395                 400
   Gly Val Pro Gly Val Gly Val Pro Gly Val Gly Val Pro Gly Val Gly
                   405                 410                 415
   Val Pro Gly Gly Gly Val Pro Gly Ala Gly Val Pro Gly Gly Gly Val
               420                 425                 430
   Pro Gly Val Gly Val Pro Gly Val Gly Val Pro Gly Gly Gly Val Pro
           435                 440                 445
   Gly Ala Gly Val Pro Gly Val Gly Val Pro Gly Val Gly Val Pro Gly
       450                 455                 460
   Val Gly Val Pro Gly Gly Gly Val Pro Gly Ala Gly Val Pro Gly Gly
   465                 470                 475                 480
   Gly Val Pro Gly Val Gly Val Pro Gly Val Gly Val Pro Gly Gly Gly
                   485                 490                 495
   Val Pro Gly Ala Gly Val Pro Gly Val Gly Val Pro Gly Val Gly Val
               500                 505                 510
   Pro Gly Val Gly Val Pro Gly Gly Gly Val Pro Gly Ala Gly Val Pro
           515                 520                 525
   Gly Gly Gly Val Pro Gly Val Gly Val Pro Gly Val Gly Val Pro Gly
       530                 535                 540
   Gly Gly Val Pro Gly Ala Gly Val Pro Gly Val Gly Val Pro Gly Val
   545                 550                 555                 560
   Gly Val Pro Gly Val Gly Val Pro Gly Gly Gly Val Pro Gly Ala Gly
                   565                 570                 575
   Val Pro Gly Gly Gly Val Pro Gly Val Gly Val Pro Gly Val Gly Val
               580                 585                 590
   Pro Gly Gly Gly Val Pro Gly Ala Gly Val Pro Gly Val Gly Val Pro
           595                 600                 605
   Gly Val Gly Val Pro Gly Val Gly Val Pro Gly Gly Gly Val Pro Gly
       610                 615                 620
   Ala Gly Val Pro Gly Gly Gly Val Pro Gly Gly Pro His Pro Ile Pro
   625                 630                 635                 640
   Asp Ser Ser Pro Leu Leu Gln Phe Gly Gly Gln Val Arg Gln Arg Tyr
                   645                 650                 655
   Leu Tyr Thr Asp Asp Ala Gln Gln Thr Glu Ala His Leu Glu Ile Arg
               660                 665                 670
   Glu Asp Gly Thr Val Gly Gly Ala Ala Asp Gln Ser Pro Glu Ser Leu
           675                 680                 685
   Leu Gln Leu Lys Ala Leu Lys Pro Gly Val Ile Gln Ile Leu Gly Val
       690                 695                 700
   Lys Thr Ser Arg Phe Leu Cys Gln Arg Pro Asp Gly Ala Leu Tyr Gly
   705                 710                 715                 720
   Ser Leu His Phe Asp Pro Glu Ala Cys Ser Phe Arg Glu Leu Leu Leu
                   725                 730                 735
   Glu Asp Gly Tyr Asn Val Tyr Gln Ser Glu Ala His Gly Leu Pro Leu
               740                 745                 750
   His Leu Pro Gly Asn Lys Ser Pro His Arg Asp Pro Ala Pro Arg Gly
           755                 760                 765
   Pro Ala Arg Phe Leu Pro Leu Pro Gly Leu Pro Pro Ala Pro Pro Glu
       770                 775                 780
   Pro Pro Gly Ile Leu Ala Pro Gln Pro Pro Asp Val Gly Ser Ser Asp
   785                 790                 795                 800
   Pro Leu Ser Met Val Gly Pro Ser Gln Gly Arg Ser Pro Ser Tyr Ala
                   805                 810                 815
   Ser
<210> 134
   <211> 2451
   <212> DNA
   <213> Artificial Sequence
<220>
   <223> GLP1 ELP1-120 FGF21 dual fusion PE9193
<400> 134
   catggcgaag gtacctttac aagcgatgtt agttcatatc tggagggcca ggcggcaaag      60
   gaatttattg cgtggctggt gaaaggccgc ggcgtgccgg gcgtgggtgt tccgggcgtg     120
   ggtgttccgg gtggcggtgt gccgggcgca ggtgttcctg gtgtaggtgt gccgggtgtt     180
   ggtgtgccgg gtgttggtgt accaggtggc ggtgttccgg gtgcaggcgt tccgggtggc     240
   ggtgtgccgg gcgtgggtgt tccgggcgtg ggtgttccgg gtggcggtgt gccgggcgca     300
   ggtgttcctg gtgtaggtgt gccgggtgtt ggtgtgccgg gtgttggtgt accaggtggc     360
   ggtgttccgg gtgcaggcgt tccgggtggc ggtgtgccgg gcgtgggtgt tccgggcgtg     420
   ggtgttccgg gtggcggtgt gccgggcgca ggtgttcctg gtgtaggtgt gccgggtgtt     480
   ggtgtgccgg gtgttggtgt accaggtggc ggtgttccgg gtgcaggcgt tccgggtggc     540
   ggtgtgccgg gcgtgggtgt tccgggcgtg ggtgttccgg gtggcggtgt gccgggcgca     600
   ggtgttcctg gtgtaggtgt gccgggtgtt ggtgtgccgg gtgttggtgt accaggtggc     660
   ggtgttccgg gtgcaggcgt tccgggtggc ggtgtgccgg gcgtgggtgt tccgggcgtg     720
   ggtgttccgg gtggcggtgt gccgggcgca ggtgttcctg gtgtaggtgt gccgggtgtt     780
   ggtgtgccgg gtgttggtgt accaggtggc ggtgttccgg gtgcaggcgt tccgggtggc     840
   ggtgtgccgg gcgtgggtgt tccgggcgtg ggtgttccgg gtggcggtgt gccgggcgca     900
   ggtgttcctg gtgtaggtgt gccgggtgtt ggtgtgccgg gtgttggtgt accaggtggc     960
   ggtgttccgg gtgcaggcgt tccgggtggc ggtgtgccgg gcgtgggtgt tccgggcgtg    1020
   ggtgttccgg gtggcggtgt gccgggcgca ggtgttcctg gtgtaggtgt gccgggtgtt    1080
   ggtgtgccgg gtgttggtgt accaggtggc ggtgttccgg gtgcaggcgt tccgggtggc    1140
   ggtgtgccgg gcgtgggtgt tccgggcgtg ggtgttccgg gtggcggtgt gccgggcgca    1200
   ggtgttcctg gtgtaggtgt gccgggtgtt ggtgtgccgg gtgttggtgt accaggtggc    1260
   ggtgttccgg gtgcaggcgt tccgggtggc ggtgtgccgg gcgtgggtgt tccgggcgtg    1320
   ggtgttccgg gtggcggtgt gccgggcgca ggtgttcctg gtgtaggtgt gccgggtgtt    1380
   ggtgtgccgg gtgttggtgt accaggtggc ggtgttccgg gtgcaggcgt tccgggtggc    1440
   ggtgtgccgg gcgtgggtgt tccgggcgtg ggtgttccgg gtggcggtgt gccgggcgca    1500
   ggtgttcctg gtgtaggtgt gccgggtgtt ggtgtgccgg gtgttggtgt accaggtggc    1560
   ggtgttccgg gtgcaggcgt tccgggtggc ggtgtgccgg gcgtgggtgt tccgggcgtg    1620
   ggtgttccgg gtggcggtgt gccgggcgca ggtgttcctg gtgtaggtgt gccgggtgtt    1680
   ggtgtgccgg gtgttggtgt accaggtggc ggtgttccgg gtgcaggcgt tccgggtggc    1740
   ggtgtgccgg gcgtgggtgt tccgggcgtg ggtgttccgg gtggcggtgt gccgggcgca    1800
   ggtgttcctg gtgtaggtgt gccgggtgtt ggtgtgccgg gtgttggtgt accaggtggc      1860
   ggtgttccgg gtgcaggcgt tccgggtggc ggtgtgccgg gcgggccgca cccgatcccg      1920
   gacagctctc cgctgctgca gtttggcggt caggtacgtc agcgttacct gtatactgac      1980
   gacgcccagc agaccgaagc tcacctggag atccgcgaag acggtaccgt aggcggtgct      2040
   gcagatcaat ctcctgaatc tctgctgcag ctgaaggcac tgaaaccggg cgttatccaa      2100
   atcctgggtg ttaaaacttc tcgtttcctg tgccagcgcc cggatggtgc actgtacggt      2160
   tctctgcatt tcgacccgga ggcctgctct ttccgtgaac tgctgctgga agatggttac      2220
   aatgtatacc aatctgaggc acacggtctg ccgctgcacc tgcctggcaa caaatctccg      2280
   catcgtgacc cggccccgcg cggtccagcc cgctttctgc cgctgccggg tctgccgccg      2340
   gctccaccag aaccgcctgg tatcctggcg cctcagccac ctgatgttgg ctcctccgac      2400
   ccgctgtcta tggtcggtcc gagccaaggc cgttccccaa gctacgccag t               2451